# EUROPEAN PATENT APPLICATION

(11) **EP 4 851 145 A1**
(43) Date of publication of application: **09.12.2026**
(21) Application number: 25747793.5
(22) Date of filing: 24.01.2025
(51) Int. Cl.: C07D 401/04, A61K 31/496, A61K 31/454, A61K 31/4545, A61P 35/00, A61P 37/06, A61P 29/00, A61P 11/00, A61P 31/00, A61P 3/10, A61P 7/00

(54) **COMPOUNDS WITH NOVEL SUBSTITUTED GLUTARIMIDYL-ISOINDOLINONE SKELETON, AND USES THEREOF**

(30) Priority: 04.02.2024 CN 202410160475
(71) Applicant: Gluetacs Therapeutics (Shanghai) Co., Ltd., Shanghai 201306 (CN)
(72) Inventor: YANG, Xiaobao, Shanghai 201306 (CN); SUN, Renhong, Shanghai 201306 (CN); REN, Chaowei, Shanghai 201306 (CN); ZHAO, Baoyin, Shanghai 201306 (CN); LI, Yan, Shanghai 201306 (CN); ZHOU, Yuedong, Shanghai 201306 (CN); YAO, Shun, Shanghai 201306 (CN); DENG, Meigui, Shanghai 201306 (CN); JIANG, Suping, Shanghai 201306 (CN); REN, Yinbo, Shanghai 201306 (CN)
(74) Representative: J A Kemp LLP
(86) International application number: PCT/CN2025/074559
(87) International publication number: WO 2025/162225

(57) **Abstract**

The present disclosure relates to compounds of Formula (I) and Formula (I'), or a salt, enantiomer, stereoisomer, solvate, or polymorph thereof, and the use thereof. The present disclosure also relates to pharmaceutical compositions comprising, as an active ingredient, a compound of Formula (I) or Formula (II), or a salt, enantiomer, stereoisomer, solvate, or polymorph thereof, and the use thereof. The series of compounds designed and synthesized in the present disclosure can effectively prevent or treat diseases or disorders associated with the cereblon protein.

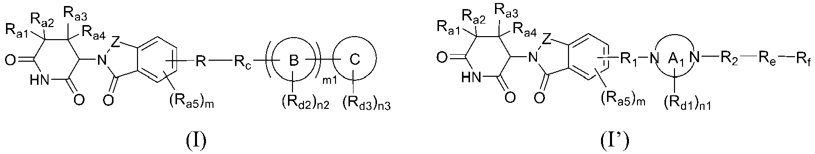

## Description

### Technical Field

The present disclosure relates to compounds of Formula (I) or Formula (I') or salts, enantiomers, stereoisomers, solvates, or polymorphs thereof, and the use thereof, in particular, the use thereof for the prevention or treatment of a disease or disorder associated with cereblon protein.

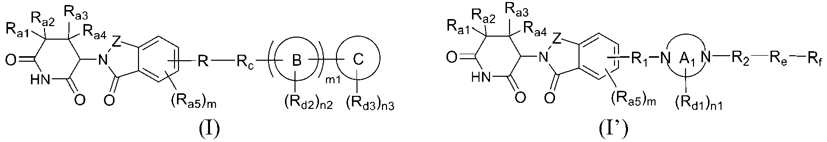

### Background

Phthalimide-based immunomodulatory drugs (IMiDs), such as thalidomide and lenalidomide, have shown significant efficacy in the treatment of hematological malignancies and autoimmune diseases. However, it was not until 2010 that their direct binding target was identified as the E3 ubiquitin ligase cereblon (CRBN). Subsequent studies confirmed that upon binding to CRBN, these drugs act as molecular glues, recruiting substrate proteins (e.g., the transcription factors IKZF1/3) and inducing protein-protein interactions between CRBN and the substrates. This leads to the ubiquitination of the substrate proteins, which are then recognized and degraded by the proteasome, thereby eliciting pharmacological effects such as antitumor and immunomodulatory activities. Molecular glue degraders directly target and degrade specific proteins, offering potential advantages such as targeting "undruggable" proteins. Moreover, molecular glues typically exhibit low molecular weight and favorable drug-like properties, making their development highly challenging. Based on the CRBN E3 ubiquitin ligase and the molecular glue degradation mechanism, a series of compounds have been developed, including the approved pomalidomide and several candidates currently in clinical trials, such as Bristol Myers Squibb's CC-122, CC-220, CC-90009, CC-99282, CC-92480, and BMS-986470; Novartis's DKY709; C4 Therapeutics's CFT7455; and Monte Rosa's MRT-6160. The substrates degraded by these molecular glues have also expanded from the initially identified transcription factors IKZF1/3 to casein kinase 1α (CK1α), zinc finger protein 91 (ZFP91), WIZ, transcription factor IKZF2, translation factor GSPT1, and immune disease target Vav1, among others. Degradation of these protein substrates enables molecular glues to exert immunomodulatory, antiinflammatory, and antitumor activities. Currently, the structural novelty of designed molecular glue candidates remains quite limited, making the diversification of molecular scaffolds and the development of novel molecular glues a key research focus in the field.

Therefore, there is an urgent need for a series of novel CRBN E3 ubiquitin ligase ligand scaffolds, and their application in the development of effective molecular glue degraders for the treatment and/or prevention of diseases or disorders mediated by or associated with the degraded protein.

### Summary of Invention

In view of the above, an object of the present disclosure is to provide protein-degrading compounds having a novel substituted glutarimidyl-isoindolinone skeleton, preparation methods thereof, uses thereof, and methods of using the same.

To achieve the above and other related objects, in one aspect, the present disclosure provides a compound of Formula (I): 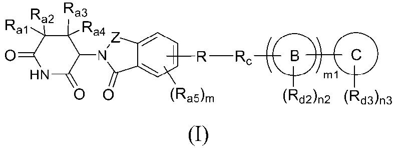 or salts, stereoisomers (including enantiomers and diastereoisomers), isotopically enriched analogs, solvates, or polymorphs thereof,
wherein Z represents C(O), CH₂, or CD₂;
Rₐ₁, Rₐ₂, Rₐ₃, and Rₐ₄ each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkynyl, or C₂₋₆ alkenyl;
m represents an integer of 0, 1, 2, or 3;
R represents: 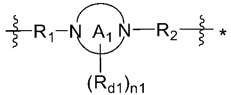
   wherein ring A₁ represents heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
   R₁ represents N(R_{w}), where R_{w} represents hydrogen or C₁₋₃ alkyl, and R₂ represents a bond, CH₂, or C(O); or
   R₁ represents a bond, and R₂ represents N(R_{w}), where R_{w} represents hydrogen or C₁₋₃ alkyl, or R₂ represents N(R_{w})C(O) *, where R_{w} represents hydrogen or C₁₋₃ alkyl, or R₂ represents -N=CH-*; or
R represents: 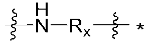 wherein R_{X} represents NH, -N=CH-*, or N(R_{w})C(O) *, where R_{w} represents hydrogen or C₁₋₃ alkyl; or
R represents: 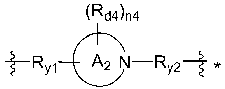
   wherein ring A₂ represents heterocyclylene containing one nitrogen atom, and (R_{d4})ₙ₄ indicates that ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20; and
   wherein R_{y1} represents 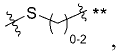 symbol ** indicates the point of attachment to ring A₂; and R₂ represents N(R_{w})C(O) *, where R_{w} represents hydrogen or C₁₋₃ alkyl;
   the symbol * in each of the above formulae indicates the point of attachment to R_{c}; and
R_{c} represents CR_{c1}R_{c2}, where R_{c1} and R_{c2} each independently represent H, deuterium, halogen, optionally substituted linear or branched alkyl, optionally substituted cycloalkyl, optionally substituted heterocyclyl, optionally substituted aryl, or optionally substituted heteroaryl, or R_{c} represents a bond;
ring B represents cycloalkylene, heterocyclylene, arylene, or heteroarylene, ml represents an integer of 0 or 1, and (R_{d2})ₙ₂ indicates that ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n2 represents an integer of 0-20; and
ring C represents heterocyclyl, cycloalkyl, aryl, or heteroaryl, and (R_{d3})ₙ₃ indicates that ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n3 represents an integer of 0-20;
with the proviso that the following compounds are excluded:
   3-(5-((4-benzhydrylpiperazin-1-yl)amino)-1-oxoisoindolin-2-yl)piperidine-2,6-dione;
   3-(5-((4-((4'-chloro-[1,1'-biphenyl]-2-yl)methyl)piperazin-1-yl)amino)-1-oxoisoindolin-2-yl)piperidine-2,6-dione;
   3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione; and
   3-(5-((4-(4'-chloro-5,5-dimethyl-3,4,5,6-tetrahydro-[1,1'-biphenyl]-2-carbonyl)piperazin-1-yl)amino)-1-oxoisoindolin-2-yl)piperidine-2,6-dione.

In another aspect, the present disclosure provides a compound of Formula (I'): 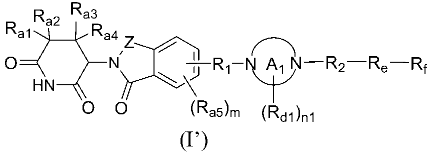 or salts, stereoisomers (including enantiomers and diastereoisomers), isotopically enriched analogs, solvates, or polymorphs thereof,
wherein Z represents C(O), CH₂, or CD₂;
Rₐ₁, Rₐ₂, Rₐ₃, and Rₐ₄ each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, C₂₋₆ alkynyl, or heterocyclyl, wherein said heterocyclyl is optionally substituted with one or more substituents independently selected from the group consisting of halogen, C₁₋₆ alkyl, and tert-butoxycarbonyl;
m represents an integer of 0, 1, 2, or 3;
ring A₁ represents 4- to 20-membered heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, deuterium, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
R₁ represents N(R_{w}), where R_{w} represents hydrogen or C₁₋₃ alkyl optionally substituted with one or more substituents independently selected from the group consisting of optionally substituted aryl and optionally substituted heteroaryl, and R₂ represents a bond; or
R₁ represents a bond, and R₂ represents N(R_{w}), where R_{w} represents hydrogen or C₁₋₃ alkyl; and
Rₑ represents a bond or C(O)O, or Rₑ represents the structure of the following formula: 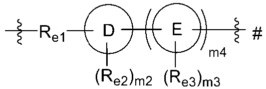
   Rₑ₁ represents C(O), CH₂, or halogenated CH₂;
   ring D represents arylene, and (Rₑ₂)ₘ₂ indicates that the ring D is optionally substituted with m2 Rₑ₂ groups, wherein each Rₑ₂ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m2 represents an integer of 0-20; and
   ring E represents nitrogen-containing heterocyclylene, m4 represents an integer of 0 or 1, and (Rₑ₃)ₘ₃ indicates that the ring E is optionally substituted with m3 Rₑ₃ groups, wherein each Rₑ₃ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m3 represents an integer of 0-20;
   symbol # indicates the point of attachment to R_{f} ; and
R_{f} represents C₁₋₁₀ alkyl, wherein said C₁₋₁₀ alkyl is optionally substituted with one or more substituents independently selected from the group consisting of: halogen, optionally substituted aryl, optionally substituted heteroaryl, optionally substituted heterocyclyl, optionally substituted cycloalkyl, and NR₃R₄, where R₃ and R₄ each independently represent H, C₁₋₃ alkyl, or optionally substituted cycloalkyl;
with the proviso that the following compounds are excluded:
   3-(5-((4-benzhydrylpiperazin-1-yl)amino)-1-oxoisoindolin-2-yl)piperidine-2,6-dione;
   3-(5-((4-((4'-chloro-[1,1'-biphenyl]-2-yl)methyl)piperazin-1-yl)amino)-1-oxoisoindolin-2-yl)piperidine-2,6-dione; and
   3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione.

In a further aspect, the present disclosure provides a pharmaceutical composition comprising:
the compound of Formula (I) or a pharmaceutically acceptable salt thereof, stereoisomer (including enantiomer and diastereoisomer), solvate, isotopically enriched analog, or polymorph thereof, and at least one pharmaceutically acceptable carrier; or
the compound of Formula (I') or a pharmaceutically acceptable salt thereof, stereoisomer (including enantiomer and diastereoisomer), solvate, isotopically enriched analog, or polymorph thereof, and at least one pharmaceutically acceptable carrier.

In a further aspect, the present disclosure further provides a medicine kit or reagent kit comprising:
the compound of Formula (I) or a pharmaceutically acceptable salt, or the pharmaceutical composition comprising the same; or
the compound of Formula (I') or a pharmaceutically acceptable salt, or the pharmaceutical composition comprising the same.

In a further aspect, the present disclosure provides the compound of Formula (I) or Formula (I') or pharmaceutically acceptable salts, enantiomers, diastereoisomers, solvates, or polymorphs thereof, for use as a medicament.

In a further aspect, the present disclosure provides the compound of Formula (I) or Formula (I') or pharmaceutically acceptable salts, stereoisomers (including enantiomers and diastereomers), solvates, isotopically enriched analogs, or polymorphs thereof, or the pharmaceutical composition of the present disclosure for use in the treatment or prevention of a disease or disorder associated with cereblon protein.

In a further aspect, the present disclosure also provides the use of the compound of Formula (I) or Formula (I') or pharmaceutically acceptable salts, stereoisomers (including enantiomers and diastereomers), solvates, isotopically enriched analogs, or polymorphs thereof, or the pharmaceutical composition of the present disclosure, for the manufacture of a medicament for the prevention or treatment of a disease or disorder associated with cereblon protein.

In a further aspect, the present disclosure also provides a method for treating or preventing a disease or disorder associated with cereblon protein in a subject, comprising administering to the subject a therapeutically effective amount of the compound of Formula (I) or Formula (I') or pharmaceutically acceptable salts, stereoisomers (including enantiomers and diastereomers), solvates, isotopically enriched analogs, or polymorphs thereof, or the pharmaceutical of the present disclosure.

In a further aspect, the present disclosure also provides a method for preparing the compound of Formula (I), comprising using a compound of Formula (M1) and a compound of Formula (M2) as starting materials to obtain the compound of Formula (I): 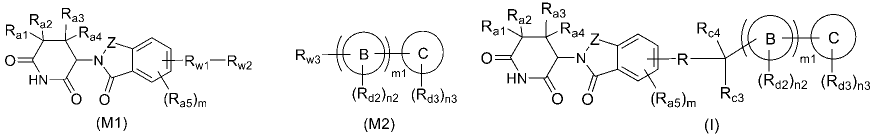
wherein Rₐ₁, Rₐ₂, Rₐ₃, Rₐ₄, (Rₐ₅)ₘ, Z, R, ring B, ring C, (R_{d2})ₙ₂, (R_{d3})ₙ₃, and m1 are as defined in the compounds of Formula (I) of the present disclosure and the respective sub-embodiments thereof; and
R_{w3} represents CHO, COOH, or 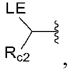 wherein R_{c2} is as defined in the compounds of Formula (I) of the present disclosure and the respective sub-embodiments thereof, and the group LE represents Cl, Br, I, OMs, OTs, or ONs;
wherein (1) -R_{w1}-R_{w2} represents 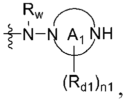 and R represents 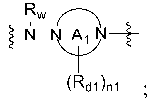 or
(2) -R_{w1}-R_{w2} represents 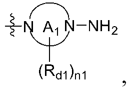 and R represents 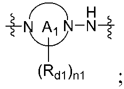 or
(3) -R_{w1}-R_{w2} represents -NH-NH₂, and R represents -NH-NH-; or
(4) -R_{w1}-R_{w2} represents 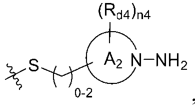 and R represents 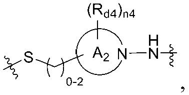
wherein R_{w}, ring A₁, (R_{d1})ₙ₁, ring A₂ and (R_{d4})ₙ₄ are as defined in the compounds of Formula (I) of the present disclosure and the respective sub-embodiments thereof;
wherein when R_{w3} represents CHO, R_{c3} and R_{c4} represents H; or
when R_{w3} represents COOH, R_{c3} and R_{c4} together form a carbonyl group; or
when R_{w3} represents 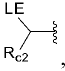 R_{c3} represents H, and R_{c4} represents R_{c2}.

### Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

### I. Compounds

### Compounds of Formula (I)

The present disclosure provides a compound of Formula (I) 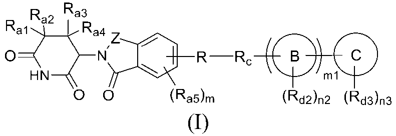
or salts (including pharmaceutically acceptable salts), stereoisomers (including enantiomers and diastereoisomers), solvates, isotopically enriched analogs, or polymorphs thereof,
wherein Z, Rₐ₁, Rₐ₂, Rₐ₃, Rₐ₄, (Rₐ₅)ₘ, R, R_{c}, ring B, (R_{d2})ₙ₂, ring C, (R_{d3})ₙ₃, and ml are as defined in the compounds of Formula (I) above and the various embodiments thereof.

The compounds of Formula (I) of the present disclosure, or salts thereof (including pharmaceutically acceptable salts), stereoisomers (including enantiomers), solvates, isotopically enriched analogs, or polymorphs thereof, exhibit binding affinity to CRBN, facilitate the recruitment of substrate proteins, and can act as molecular glues binding to the CRBN E3 ubiquitin ligase. These compounds of Formula (I), or salts thereof (including pharmaceutically acceptable salts), stereoisomers (including enantiomers), solvates, isotopically enriched analogs, or polymorphs thereof, can also degrade substrate proteins (e.g., IKZF1/2/3/4 proteins, WEE1 protein, CK1α protein, GSPT1 protein, ZFP91 protein, etc.). Therefore, the compounds of Formula (I) of the present disclosure, or salts thereof (including pharmaceutically acceptable salts), stereoisomers (including enantiomers), solvates, isotopically enriched analogs, or polymorphs thereof, can effectively prevent or treat diseases or disorders associated with cereblon protein (e.g., tumors or cancers), or exhibit excellent pharmacokinetic properties, and can be used as therapeutic agents for tumor patients.

In some embodiments of the present disclosure, Rₐ₁, Rₐ₂, Rₐ₃, and Rₐ₄ are identical or different and each independently represents H, deuterium (i.e., D), halogen (e.g., fluorine, chlorine, bromine, or iodine), C₁₋₆ alkyl (e.g., C₁₋₃ alkyl, such as methyl, ethyl, or propyl), halogenated C₁₋₆ alkyl (e.g., halogenated C₁₋₄ alkyl, such as trifluoromethyl), deuterated C₁₋₆ alkyl, C₁₋₆ alkoxy (e.g., C₁₋₄ alkoxy, such as methoxy), deuterated C₁₋₆ alkoxy, or halogenated C₁₋₆ alkoxy (e.g., halogenated C₁₋₄ alkoxy, such as trifluoromethoxy). In some sub-embodiments of the present disclosure, Rₐ₁, Rₐ₂, Rₐ₃, and Rₐ₄ each independently represent H.

In some embodiments of the present disclosure, Z represents C(O), CH₂, or CD₂.

In some embodiments of the present disclosure, Z represents C(O).

In some embodiments of the present disclosure, Z represents CH₂.

In some embodiments of the present disclosure, Z represents CD₂.

In some embodiments of the present disclosure, (Rₐ₅)ₘ indicates that the isoindoline ring in Formula (I) to which it is attached is optionally substituted with m Rₐ₅ groups, wherein each Rₐ₅ is identical or different and independently represents deuterium, halogen (e.g., fluorine, chlorine, bromine, or iodine), hydroxyl, mercapto, nitro, amino, cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), deuterated C₁₋₆ alkoxy, halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), C₂₋₆ alkenyl (e.g., vinyl), or C₂₋₆ alkynyl (e.g., ethynyl), and m represents an integer of 0, 1, 2, or 3. In some sub-embodiments of the present disclosure, m represents an integer of 0, 1, or 2. In some sub-embodiments of the present disclosure, each Rₐ₅ is identical or different and independently represents deuterium, halogen (e.g., fluorine, chlorine, bromine, or iodine), hydroxyl, mercapto, nitro, amino, cyano, C₁₋₄ alkyl (e.g., C₁₋₃ alkyl, such as methyl, ethyl, propyl, isopropyl, butyl, sec-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₄ alkoxy (e.g., C₁₋₃ alkoxy, such as methoxy, ethoxy, propoxy, isopropoxy, n-butoxy, sec-butoxy, or tert-butoxy) , or halogenated C₁₋₄ alkoxy (e.g., halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-).

In embodiments of the present disclosure, the number of substituents is not theoretically limited in any way, or is automatically limited by the size of the building units.

In some embodiments of the present disclosure, R represents 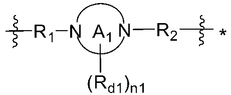
wherein ring A₁ represents heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
R₁ represents N(R_{w}), where R_{w} represents hydrogen or C₁₋₃ alkyl, and R₂ represents a bond, CH₂, or C(O); or
R₁ represents a bond, and R₂ represents N(R_{w}), where R_{w} represents hydrogen or C₁₋₃ alkyl, or R₂ represents N(R_{w})C(O) *, where R_{w} represents hydrogen or C₁₋₃ alkyl, or R₂ represents -N=CH-*; and
the symbol * indicates the point of attachment to R_{c}.

In some embodiments of the present disclosure, R₁ represents N(R_{w}), where R_{w} represents hydrogen or C₁₋₃ alkyl, and R₂ represents a bond.

In some embodiments of the present disclosure, R₁ represents N(R_{w}), where R_{w} represents hydrogen or C₁₋₃ alkyl, and R₂ represents CH₂ or C(O).

In some embodiments of the present disclosure, R₁ represents a bond, and R₂ represents N(R_{w}), where R_{w} represents hydrogen or C₁₋₃ alkyl.

In some embodiments of the present disclosure, R₁ represents a bond, and R₂ represents N(R_{w})C(O) *, where R_{w} represents hydrogen or C₁₋₃ alkyl. The symbol * indicates the point of attachment to R_{c}.

In some embodiments of the present disclosure, R₁ represents a bond, and R₂ represents -N=CH-*. The symbol * indicates the point of attachment to R_{c}.

In some embodiments of the present disclosure, R₁ represents N(R_{w}), where R_{w} represents hydrogen or C₁₋₃ alkyl, and R₂ represents CH₂ or C(O).

In some embodiments of the present disclosure, R represents 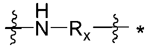 wherein R_{X} represents NH, -N=CH-*, or N(R_{w})C(O) *, where R_{w} represents hydrogen or C₁₋₃ alkyl, and the symbol * indicates the point of attachment to R_{c}.

In some embodiments of the present disclosure, R represents 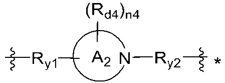
wherein ring A₂ represents heterocyclylene containing one nitrogen atom, and (R_{d4})ₙ₄ indicates that ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20; and
wherein R_{y1} represents 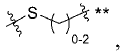 symbol ** indicates the point of attachment to ring A₂; and R_{y2} represents N(R_{w}) or N(R_{w})C(O) *, where R_{w} represents hydrogen or C₁₋₃ alkyl; and
the symbol * indicates the point of attachment to R_{c}.

In some embodiments of the present disclosure, R_{y1} represents S, and R_{y2} represents N(R_{w}) or N(R_{w})C(O) *, where R_{w} represents hydrogen or C₁₋₃ alkyl. The symbol * indicates the point of attachment to R_{c}.

In some embodiments of the present disclosure, R_{y1} represents -S-CH₂-, and R_{y2} represents N(R_{w}) or N(R_{w})C(O) *, where R_{w} represents hydrogen or C₁₋₃ alkyl. The symbol * indicates the point of attachment to R_{c}.

In some embodiments of the present disclosure, R_{y1} represents -S-CH₂-CH₂-, and R_{y2} represents N(R_{w}) or N(R_{w})C(O) *, where R_{w} represents hydrogen or C₁₋₃ alkyl. The symbol * indicates the point of attachment to R_{c}.

In some embodiments of the present disclosure, R represents one of the following formulae: 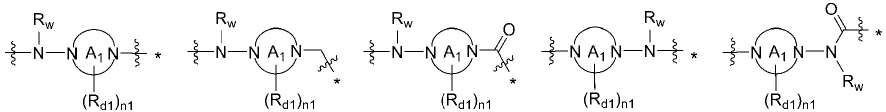 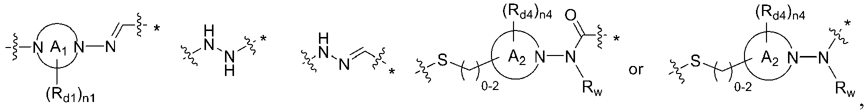
wherein each R_{w} independently represents hydrogen or C₁₋₃ alkyl;
each ring A₁ independently represents heterocyclylene containing at least two nitrogen atoms (including 4-to 30-membered, 4- to 20-membered, and 4- to 15-membered heterocyclylene), and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20 (e.g., an integer of 0-10);
ring A₂ represents heterocyclylene containing one nitrogen atom (including 4- to 30-membered, 4- to 20-membered, and 4- to 15-membered heterocyclylene), and (R_{d4})ₙ₄ indicates that ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20 (e.g., an integer of 0-10); and
symbol * indicates the point of attachment to R_{c}.

In some embodiments of the present disclosure, each ring A₁ herein independently represents heterocyclylene containing at least two nitrogen atoms, for example, 4- to 30-membered, 4- to 20-membered, or 4- to 15-membered heterocyclylene. Ring A₁ is optionally substituted with n1 R_{d1} groups, wherein each Rₐᵣ independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20 (e.g., an integer of 0-10).

In some embodiments of the present disclosure, each ring A₁ independently represents 4- to 30-membered heterocyclylene containing at least two nitrogen atoms (including 4- to 25-membered heterocyclene, 4- to 20-membered heterocyclene, 4- to 15-membered heterocyclene, and 5- to 20-membered heterocyclene). Ring A₁ is optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0, 1, 2, 3, 4, 5, 6, 7, 8, 9, 10, 11, 12, 13, 14, 15, 16, 17, 18, 19, or 20.

In some embodiments of the present disclosure, each ring A₁ independently represents 1,3-diazetidinylidene, imidazolidylene, pyrazolidylene, piperazinylene, diazacycloheptanylene, diazacyclooctylene, diazabicyclo[3.1.1]heptanylene, diazabicyclo[2.2.1]heptanylene, diazabicyclo[3.2.1]octanylene, diazabicyclo[2.2.2]octanylene, 2,6-diazaspiro[3.3]heptanylene, 2,7-diazaspiro[3.5]nonanylene, 2,8-diazaspiro[4.5]decanylene, 3,9-diazaspiro[5.5]undecanylene, or octahydropyrrolo[3,4-c]pyrrolylene, each being optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0, 1, 2, 3, 4, 5, 6, 7, 8, 9, 10, 11, 12, 13, 14, 15, 16, 17, 18, 19, or 20.

In some embodiments of the present disclosure, each ring A₂ herein independently represents heterocyclylene containing one nitrogen atom, e.g., 4- to 30-membered, 4- to 20-membered, or 4- to 15-membered heterocyclylene. Ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20 (e.g., an integer of 0-10).

In some embodiments of the present disclosure, each ring A₂ independently represents 4- to 30-membered heterocyclylene containing one nitrogen atom (including 4- to 25-membered heterocyclylene, 4- to 20-membered heterocyclylene, 4- to 15-membered heterocyclylene, and 5- to 20-membered heterocyclylene). Ring A₂ is optionally substituted with n4 R_{d4} groups, wherein each R_{d4} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n4 represents an integer of 0, 1, 2, 3, 4, 5, 6, 7, 8, 9, 10, 11, 12, 13, 14, 15, 16, 17, 18, 19, or 20.

In some embodiments of the present disclosure, each ring A₂ herein independently represents azetidinylene, pyrrolidinylene, piperidinylene, dihydroxypiperidinylene, difluoropiperidinylene, azepanylene, azacyclooctylene, monoazabridged-cycloalkylene (e.g., 6- to 20-membered monoazabridged-cycloalkylene, such as 6-azabicyclo[3.1.1]heptanylene, 3-azabicyclo[3.2.1]octanylene, and quinuclidinylene), or monoazaspirocycloalkylene (e.g., 5- to 20-membered monoazaspirocycloalkylene, such as 3-azaspiro[5.5]undecanylene and 7-azaspiro[3.5]nonanylene), each of which is optionally substituted with n4 R_{d4} groups, wherein each R_{d4} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n4 represents an integer of 0, 1, 2, 3, 4, 5, 6, 7, 8, 9, 10, 11, 12, 13, 14, 15, 16, 17, 18, 19, or 20.

In some embodiments of the present disclosure, R_{w} represents hydrogen or C₁₋₃ alkyl.

In some embodiments of the present disclosure, R_{w} represents hydrogen.

In some embodiments of the present disclosure, R_{w} represents C₁₋₃ alkyl, e.g., methyl, ethyl, propyl, and isopropyl; wherein said groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl.

In some embodiments of the present disclosure, examples of R include, but are not limited to: 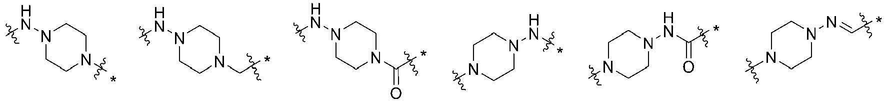 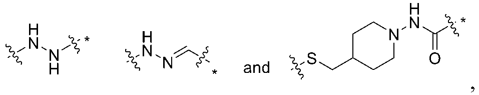 wherein the symbol * indicates the point of attachment to R_{c}.

In some embodiments of the present disclosure, R_{c} represents CR_{c1}R_{c2}, wherein R_{c1} and R_{c2} each independently represent H, deuterium, halogen (e.g., fluorine, chlorine, bromine, or iodine), optionally substituted linear or branched alkyl (e.g., optionally substituted linear or branched C₁₋₁₀ alkyl), optionally substituted cycloalkyl (e.g., optionally substituted C₃₋₃₀ cycloalkyl), optionally substituted heterocyclyl (e.g., optionally substituted 4- to 30-membered heterocyclyl), optionally substituted aryl (e.g., optionally substituted C₅₋₃₀ aryl), or optionally substituted heteroaryl (e.g., optionally substituted 5- to 30-membered heteroaryl).

In some embodiments of the present disclosure, R_{c} represents a bond. When R_{c} represents a bond, ring B is directly attached to R.

In some embodiments of the present disclosure, R_{c} represents CR_{c1}R_{c2}, wherein R_{c1} and R_{c2} each independently represent H, deuterium, halogen (e.g., fluorine, chlorine, bromine, or iodine), optionally substituted linear or branched C₁₋₁₀ alkyl (e.g., optionally substituted linear or branched C₁₋₆ alkyl or optionally substituted linear or branched C₁₋₃ alkyl), optionally substituted C₃₋₃₀ cycloalkyl (e.g., optionally substituted C₃₋₂₀ cycloalkyl, or optionally substituted C₃₋₁₅ cycloalkyl), optionally substituted C₅₋₃₀ aryl (e.g., optionally substituted C₅₋₂₀ aryl, or optionally substituted C₅₋₁₅ aryl), optionally substituted 4- to 30-membered heterocyclyl (e.g., optionally substituted 4- to 20-membered heterocyclyl, or optionally substituted 4- to 15-membered heterocyclyl), or optionally substituted 5- to 30-membered heteroaryl (e.g., optionally substituted 5- to 20-membered heteroaryl, or optionally substituted 5- to 15-membered heteroaryl). In some embodiments, examples of linear or branched C₁₋₁₀ alkyl include, but are not limited to, methyl, ethyl, propyl, isopropyl, butyl, isobutyl, sec-butyl, tert-butyl, pentyl, isopentyl, neopentyl, tert-pentyl, hexyl, heptyl, and octyl. In some embodiments, the linear or branched C₁₋₁₀ alkyl is optionally substituted with one or more (e.g., 1-20, 1-15, 1-10, 1-6, 1-4, 1-3, 2-6, or 1) substituents independently selected from the group consisting of deuterium, halogen, amino, hydroxyl, mercapto, cyano, nitro, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl. In some embodiments, examples of C₃₋₃₀ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₁₅ spiro-cycloalkyl, C₅-C₂₀ spiro-cycloalkyl, or C₅-C₂₅ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m*-menthanyl, or bridged cycloalkyl (e.g., C₆-C₁₅ bridged cycloalkyl, C₆-C₂₀ bridged cycloalkyl, or C₆-C₂₅ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl). In some embodiments, C₃₋₃₀ cycloalkyl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, nitro, amino, cyano, oxo, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl. In some embodiments, examples of 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, dihydropyranyl, pyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, and 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), and octahydropyrrolo[3,4-c]pyrrolyl. In some embodiments, 4- to 30-membered heterocyclyl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, amino, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, and C₂₋₆ alkynyl. In some embodiments, examples of C₅₋₃₀ aryl include, but are not limited to, phenyl and naphthyl. In some embodiments, C₅₋₃₀ aryl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, and C₂₋₆ alkynyl. In some embodiments, examples of 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. In some embodiments, 5- to 30-membered heteroaryl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl.

In some embodiments of the present disclosure, m1 represents an integer of 1, and ring B represents 4- to 30-membered heterocyclylene (e.g., 4- to 20-membered or 4- to 15-membered heterocyclylene), C₃₋₃₀ cycloalkylene (e.g., C₃₋₂₀ cycloalkylene or C₃₋₁₅ cycloalkylene), C₅₋₃₀ arylene (e.g., C₅₋₂₀ arylene or C₅₋₁₅ arylene), or 5- to 30-membered heteroarylene (e.g., 5- to 20-membered heteroarylene or 5- to 15-membered heteroarylene). Ring B is optionally substituted with n2 R_{d2} groups, wherein each R_{d2} independently represents deuterium, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), halogen (e.g., fluorine, chlorine, bromine, or iodine), amino, hydroxyl, mercapto, cyano, oxo, C₂₋₆ alkynyl (e.g., ethynyl), or C₂₋₆ alkenyl (e.g., vinyl), n2 represents an integer of 0, 1, 2, 3, 4, 5, 6, 7, 8, 9, 10, 11, 12, 13, 14, 15, 16, 17, 18, 19, or 20.

In some embodiments, ring B represents 4- to 30-membered heterocyclylene, examples of which include but are not limited to 4- to 20-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclylene, e.g., azetidinylene, oxetanylene, pyrrolidinylene, imidazolidylene, pyrazolidylene, oxetanylene, dihydropyranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azepanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6- to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene and 7-azaspiro[3.5]nonanylene), and octahydropyrrolo[3,4-c]pyrrolylene. The heterocyclylene is optionally substituted with 0-20 (e.g., 1-20, 1-15, 1-10, 1-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl.

In some embodiments, ring B represents C₃₋₃₀ cycloalkylene, examples of which include but are not limited to C₃₋₂₀ cycloalkylene, C₃₋₁₅ cycloalkylene, and C₃₋₁₁ cycloalkylene, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅-C₂₀ spiro-cycloalkylene and C₅₋₁₅ spiro-cycloalkylene, such as spiro[3.3]heptylene, spiro[2.5]octylene, spiro[3.5]nonylene, spiro[3.5]nonenylene, spiro[4.4]nonylene, spiro[4.5]decylene, spiro[4.5]decenylene, and spiro[5.5]undecylene), *p*-menthanylene, *m*-menthanylene, and bridged cycloalkylene (e.g., C₆-C₂₀ bridged cycloalkylene, such as adamantanylene, noradamantanylene, bornylene, norbornylene, bicyclo[2.2.1]heptylene, 2-oxobicyclo[2.2.1]heptylene, and bicyclo[2.2.1]heptentylene). The cycloalkylene is optionally substituted with 0-20 (e.g., 1-20, 1-15, 1-10, 1-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl.

In some embodiments, ring B represents C₅₋₃₀ arylene, examples of which include but are not limited to C₅₋₂₀ arylene, C₆₋₂₀ arylene, C₅₋₁₅ arylene, and C₆₋₁₅ arylene, e.g., phenylene and naphthylene. The arylene is optionally substituted with 0-20 (e.g., 1-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl.

In some embodiments, ring B represents 5- to 30-membered heteroarylene, examples of which include but are not limited to 5- to 20-membered heteroarylene, 5- to 15-membered, 5- to 10-membered, 5- to 9-membered, 5- to 8-membered, 5- to 7-membered, and 5- to 6-membered heteroarylene, e.g., furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, indolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, and 6,7-dihydro-5H-cyclopenta[4,5]thieno[2,3-d]pyrimidinylene. The heteroarylene is optionally substituted with 0-20 (e.g., 1-20, 1-15, 1-10, 1-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl.

In some embodiments of the present disclosure, when m1 represents 0, i.e., when ring B is absent, R_{c} is directly connected to ring C.

In some embodiments of the present disclosure, ring C represents C₃₋₃₀ cycloalkyl (e.g., C₃₋₂₀ cycloalkyl or C₃₋₁₅ cycloalkyl), 4- to 30-membered heterocyclyl (e.g., 4- to 20-membered heterocyclyl or 4- to 15-membered heterocyclyl), C₅₋₃₀ aryl (e.g., C₅₋₂₀ aryl or C₅₋₁₅ aryl), or 5- to 30-membered heteroaryl (e.g., 5- to 20-membered heteroaryl or 5- to 15-membered heteroaryl). Ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n4 represents an integer of 0, 1, 2, 3, 4, 5, 6, 7, 8, 9, 10, 11, 12, 13, 14, 15, 16, 17, 18, 19, or 20.

In some embodiments, ring C represents C₃₋₃₀ cycloalkyl, examples of which include but are not limited to C₃₋₂₀ cycloalkyl, C₃₋₁₅ cycloalkyl, and C₃₋₁₁ cycloalkyl, e.g., cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m*-menthanyl, and bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl). The cycloalkyl is optionally substituted with 0-20 (e.g., 1-20, 1-15, 1-10, 0-10, 1-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl.

In some embodiments, ring C represents 4- to 30-membered heterocyclyl, examples of which include but are not limited to 4- to 20-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclyl, e.g., azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl), azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), and octahydropyrrolo[3,4-c]pyrrolyl. The heterocyclyl is optionally substituted with 0-20 (e.g., 1-20, 1-15, 1-10, 0-10, 1-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl.

In some embodiments, ring C represents C₅₋₃₀ aryl, examples of which include but are not limited to C₅₋₂₀ aryl, C₆₋₂₀ aryl, C₅₋₁₅ aryl, and C₆₋₁₅ aryl, e.g., phenyl and naphthyl, which are optionally substituted with 0-20 (e.g., 1-7, 1-6, 0-7, 0-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl.

In some embodiments, ring C represents 5- to 30-membered heteroaryl, examples of which include but are not limited to 5- to 20-membered heteroaryl, 5- to 15-membered, 5- to 10-membered, 5- to 9-membered, 5-to 8-membered, 5- to 7-membered, and 5- to 6-membered heteroaryl, e.g., furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. The heteroaryl is optionally substituted with 0-20 (e.g., 1-20, 1-15, 1-10, 0-10, 1-6, 1-4, 1-3, 2-6, or 1) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl.

In some embodiments of the present disclosure, examples of the moiety 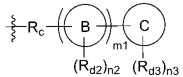 of the compound of Formula (I) include, but are not limited to:

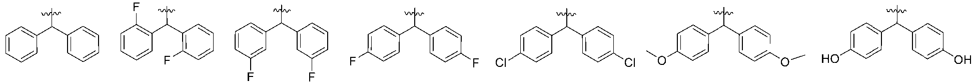

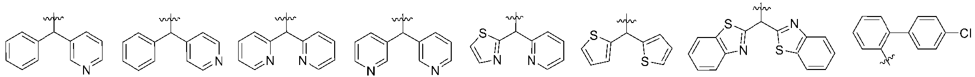

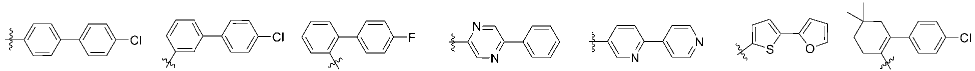

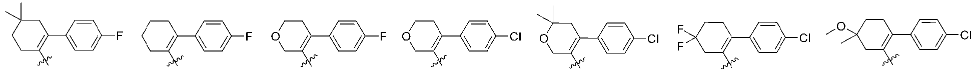

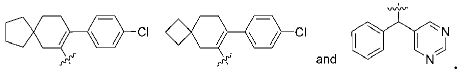

In some embodiments of the present disclosure, the compound of Formula (I) is also represented by Formula (I-1): 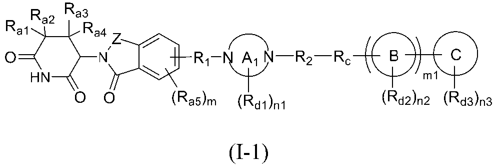
wherein Z, Rₐ₁, Rₐ₂, Rₐ₃, Rₐ₄, (Rₐ₅)ₘ, ring A₁, (R_{d1})ₙ₁, R_{c}, ring B, (R_{d2})ₙ₂, m1, ring C, and (R_{d3)n3} are as defined in the compounds of Formula (I) above and the various embodiments thereof; and
wherein (i) R₁ represents N(R_{w}), where R_{w} represents hydrogen or C₁₋₃ alkyl, and R₂ represents a bond, CH₂, or C(O); or
(ii) R₁ represents a bond, and R₂ represents N(R_{w}), where R_{w} represents hydrogen or C₁₋₃ alkyl, or R₂ represents N(R_{w})C(O) *, where R_{w} represents hydrogen or C₁₋₃ alkyl, or R₂ represents -N=CH-*; and the symbol * indicates the point of attachment to R_{c}.

In some embodiments of the present disclosure, the compound of Formula (I) is also represented by Formula (I-2): 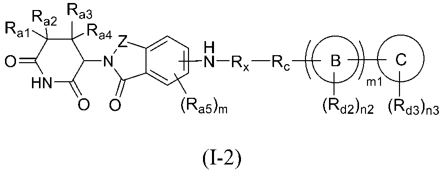
wherein Z, Rₐ₁, Rₐ₂, Rₐ₃, Rₐ₄, (Rₐ₅)ₘ, ring A₁, (R_{d1})ₙ₁, R_{c}, ring B, (R_{d2})ₙ₂, m1, ring C, and (R_{d3})ₙ₃ are as defined in the compounds of Formula (I) above and the various embodiments thereof; and
wherein R_{X} represents NH, -N=CH-*, or N(R_{w})C(O) *, where R_{w} represents hydrogen or C₁₋₃ alkyl, and symbol * indicates the point of attachment to R_{c}.

In some embodiments of the present disclosure, the compound of Formula (I) is also represented by Formula (I-3): 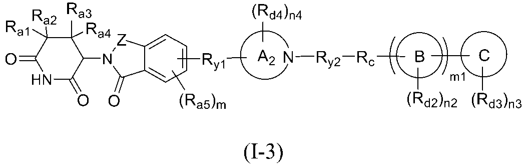
wherein Z, Rₐ₁, Rₐ₂, Rₐ₃, Rₐ₄, (Rₐ₅)ₘ, ring A₂, (R_{d4})ₙ₄, R_{c}, ring B, (R_{d2})ₙ₂, m1, ring C, and (R_{d3})ₙ₃ are as defined in the compounds of Formula (I) above and the various embodiments thereof; and
wherein R_{y1} represents 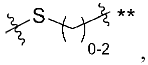 symbol ** indicates the point of attachment to ring A₂; and R₂ represents N(R_{w})C(O) *, where R_{w} represents hydrogen or C₁₋₃ alkyl, and symbol * indicates the point of attachment to R_{c}.

In some embodiments of the present disclosure, R_{y1} represents -S-, -S-CH₂-, or -S-CH₂-CH₂-.

In some embodiments of the present disclosure, the compound of Formula (I) is also represented by Formula (I-4): 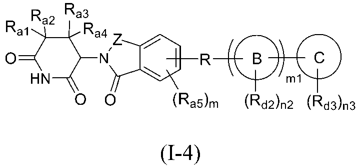 wherein Z, Rₐ₁, Rₐ₂, Rₐ₃, Rₐ₄, (Rₐ₅)ₘ, R, ring B, (R_{d2})ₙ₂, m1, ring C, and (R_{d3})ₙ₃ are as defined in the compounds of Formula (I) above and the various embodiments thereof.

In some embodiments of the present disclosure, the compound of Formula (I) is also represented by Formula (I-5): 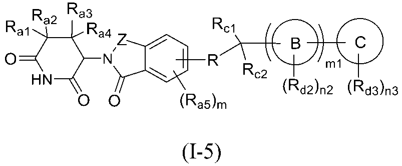
wherein Z, Rₐ₁, Rₐ₂, Rₐ₃, Rₐ₄, (Rₐ₅)ₘ, R, ring B, (R_{d2})ₙ₂, m1, ring C, and (R_{d3})ₙ₃ are as defined in the compounds of Formula (I) above and the various embodiments thereof;
R_{c1} represents H, deuterium, halogen (e.g., fluorine, chlorine, bromine, or iodine), or optionally substituted linear or branched C₁₋₁₀ alkyl (e.g., optionally substituted linear or branched C₁₋₆ alkyl or optionally substituted linear or branched C₁₋₃ alkyl); and
R_{c2} represents optionally substituted C₃₋₃₀ cycloalkyl (e.g., optionally substituted C₃₋₂₀ cycloalkyl or optionally substituted C₃₋₁₅ cycloalkyl), optionally substituted C₅₋₃₀ aryl (e.g., optionally substituted C₅₋₂₀ aryl or optionally substituted C₅₋₁₅ aryl), optionally substituted 4- to 30-membered heterocyclyl (e.g., optionally substituted 4- to 20-membered heterocyclyl or optionally substituted 4- to 15-membered heterocyclyl), or optionally substituted 5- to 30-membered heteroaryl (e.g., optionally substituted 5- to 20-membered heteroaryl or optionally substituted 5- to 15-membered heteroaryl).

In some embodiments of the compounds of Formula (I-5) of the present disclosure, R_{c1} represents optionally substituted linear or branched C₁₋₁₀ alkyl. Examples of linear or branched C₁₋₁₀ alkyl include, but are not limited to, methyl, ethyl, propyl, isopropyl, butyl, isobutyl, sec-butyl, tert-butyl, pentyl, isopentyl, neopentyl, tert-pentyl, hexyl, heptyl, and octyl. In some embodiments, linear or branched C₁₋₁₀ alkyl is optionally substituted with one or more (e.g., 1-20, 1-15, 1-10, 1-6, 1-4, 1-3, 2-6, or 1) substituents independently selected from the group consisting of deuterium, halogen, amino, hydroxyl, mercapto, cyano, nitro, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl.

In some embodiments of the compounds of Formula (I-5) of the present disclosure, R_{c2} represents optionally substituted C₃₋₃₀ cycloalkyl (e.g., optionally substituted C₃₋₂₀ cycloalkyl or optionally substituted C₃₋₁₅ cycloalkyl), optionally substituted C₅₋₃₀ aryl (e.g., optionally substituted C₅₋₂₀ aryl or optionally substituted C₅₋₁₅ aryl), optionally substituted 4- to 30-membered heterocyclyl (e.g., optionally substituted 4- to 20-membered heterocyclyl or optionally substituted 4- to 15-membered heterocyclyl) or optionally substituted 5- to 30-membered heteroaryl (e.g., optionally substituted 5- to 20-membered heteroaryl or optionally substituted 5- to 15-membered heteroaryl). In some embodiments, examples of C₃₋₃₀ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₁₅ spiro-cycloalkyl, C₅-C₂₀ spiro-cycloalkyl, or C₅-C₂₅ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m-*menthanyl, or bridged cycloalkyl (e.g., C₆-C₁₅ bridged cycloalkyl, C₆-C₂₀ bridged cycloalkyl, or C₆-C₂₅ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl). In some embodiments, C₃₋₃₀ cycloalkyl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, nitro, amino, cyano, oxo, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkynyl, and C₂₋₆ alkenyl. In some embodiments, examples of 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, dihydropyranyl, pyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), and octahydropyrrolo[3,4-c]pyrrolyl. In some embodiments, 4- to 30-membered heterocyclyl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, amino, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkynyl, and C₂₋₆ alkenyl. In some embodiments, examples of C₅₋₃₀ aryl include, but are not limited to, phenyl and naphthyl. In some embodiments, C₅₋₃₀ aryl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkynyl, and C₂₋₆ alkenyl. In some embodiments, examples of 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. In some embodiments, 5- to 30-membered heteroaryl is optionally substituted with one or more (e.g., 1-20, 1-15, 1-10, 1-6, 1-4, 1-3, 2-6, or 1) substituents independently selected from the group consisting of deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkynyl, and C₂₋₆ alkenyl.

In some embodiments of the present disclosure, the compound of Formula (I) is also represented by Formula (II-1), Formula (II-2), Formula (II-3), or Formula (II-4): 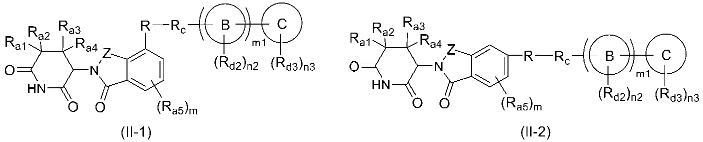 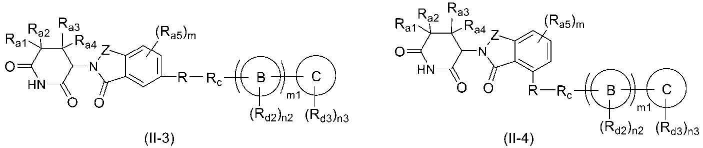 wherein Z, Rₐ₁, Rₐ₂, Rₐ₃, Rₐ₄, (Rₐ₅)ₘ, R, R_{c}, ring B, (R_{d2})ₙ₂, ring C, (R_{d3})ₙ₃, and m1 are as defined in the compounds of Formula (I) above and the various embodiments thereof.

Preferably the compounds of the present invention and their salts (especially pharmaceutically acceptable salts, such as hydrochloride, etc.), enantiomers, diastereomers, solvates, or polymorphs thereof in Table 1 below are provided:

**Table 1. The compounds of the present invention**

| Compound No. | Structure of the compounds | Compound No. | Structure of the compounds |
|---|---|---|---|
| GT-06422 | 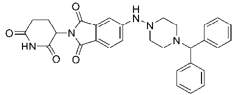 | GT-06866 | 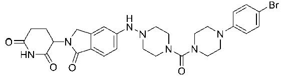 |
| GT-10308 | 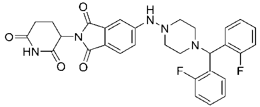 | GT-06867 | 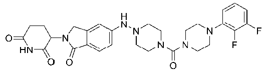 |
| GT-06401 | 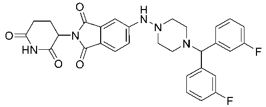 | GT-06868 | 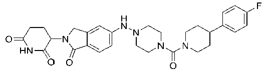 |
| GT-06541 | 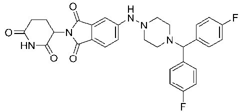 | GT-06869 | 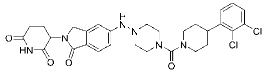 |
| GT-06542 | 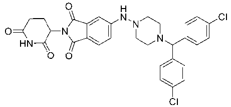 | GT-06870 | 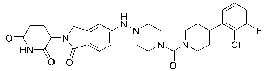 |
| GT-06543 | 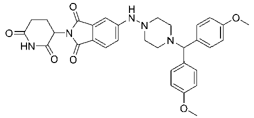 | GT-06871 | 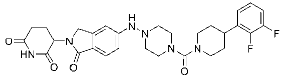 |
| GT-10084 | 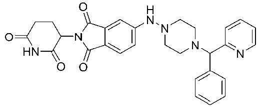 | GT-06911 | 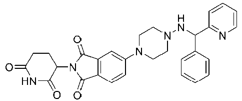 |
| | 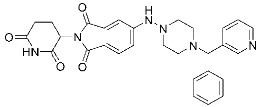 | GT-06902 | 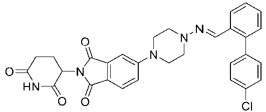 |
| GT-10494 | 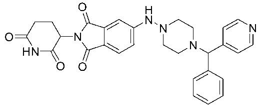 | GT-07209 | 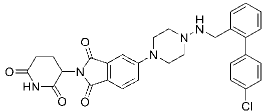 |
| GT-10509 | 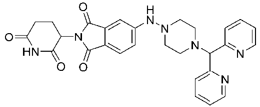 | GT-06903 | 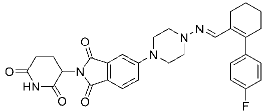 |
| GT-10166 | 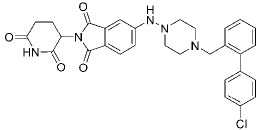 | GT-07211 | 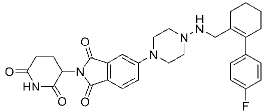 |
| GT-06441 | 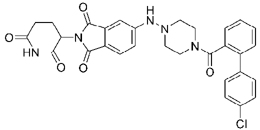 | GT-06904 | 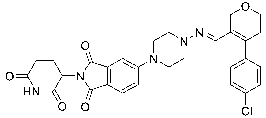 |
| GT-06544 | 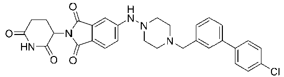 | GT-07212 | 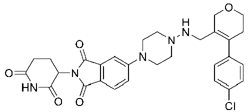 |
| GT-06545 | 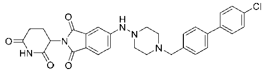 | GT-06905 | 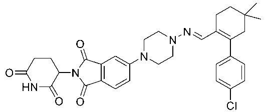 |
| GT-06436 | 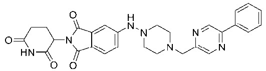 | GT-07214 | 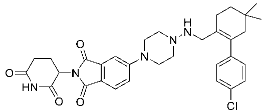 |
| GT-10207 | 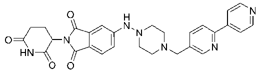 | GT-07057 | 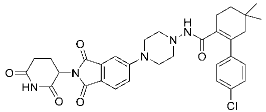 |
| GT-10297 | 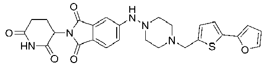 | GT-06906 | 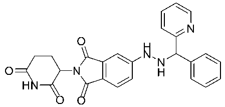 |
| GT-06469 | 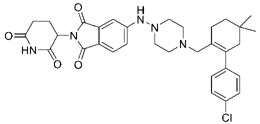 | GT-06907 | 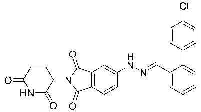 |
| GT-06473 | 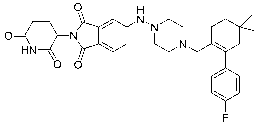 | GT-06908 | 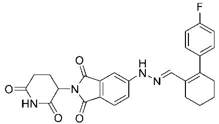 |
| GT-06509 | 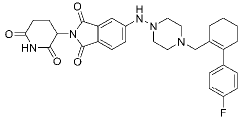 | GT-06909 | 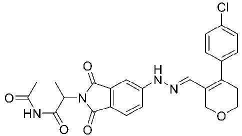 |
| GT-06546 | 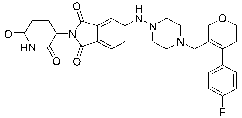 | GT-06910 | 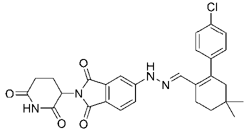 |
| GT-07045 | 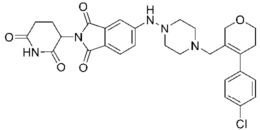 | GT-07058 | 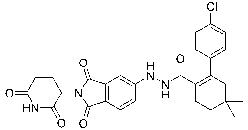 |
| GT-06547 | 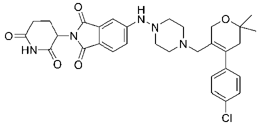 | GT-07066 | 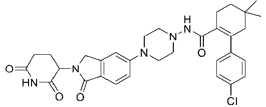 |
| GT-10411 | 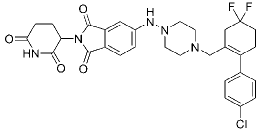 | GT-07059 | 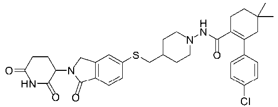 |
| GT-10417 | 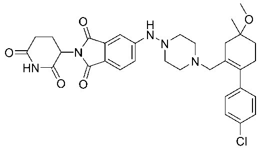 | GT-10649 | 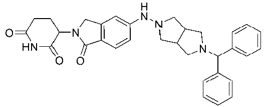 |
| | 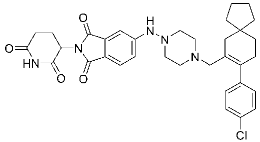 | GT-10650 | 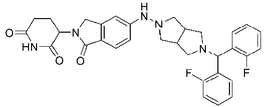 |
| | 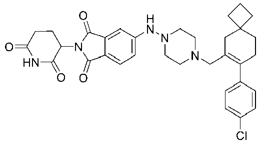 | GT-10651 | 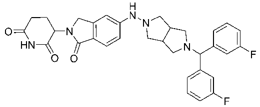 |
| GT-06465 | 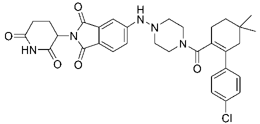 | GT-10652 | 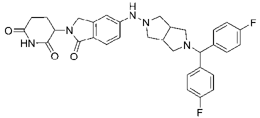 |
| GT-06460 | 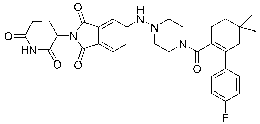 | GT-09971 | 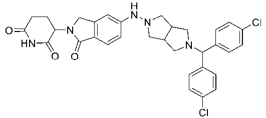 |
| GT-06423 | 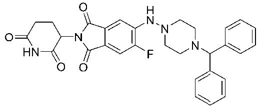 | | 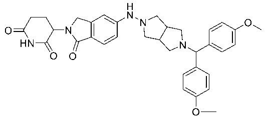 |
| GT-10309 | 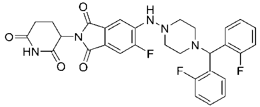 | GT-10653 | 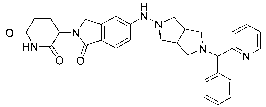 |
| GT-06402 | 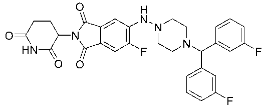 | GT-10475 | 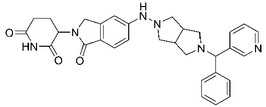 |
| GT-09963 | 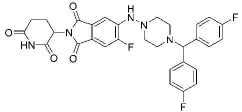 | GT-10655 | 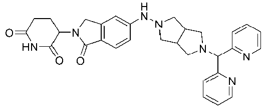 |
| GT-10079 | 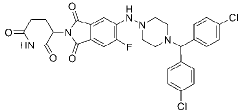 | GT-10141 | 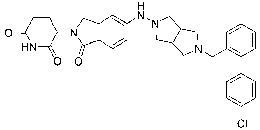 |
| | 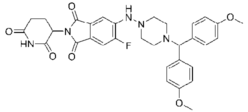 | GT-10142 | 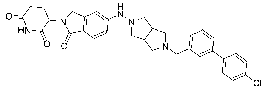 |
| GT-10085 | 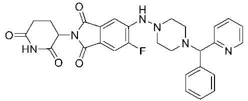 | GT-10130 | 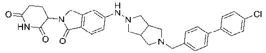 |
| GT-10091 | 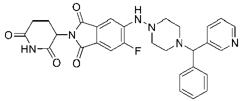 | GT-09992 | 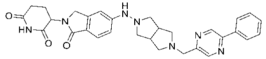 |
| GT-10495 | 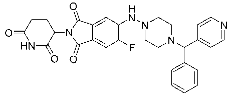 | GT-10124 | 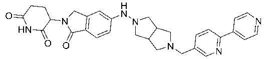 |
| GT-10510 | 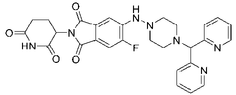 | GT-10135 | 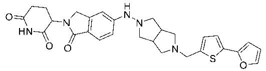 |
| GT-10167 | 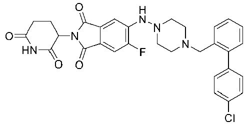 | GT-09972 | 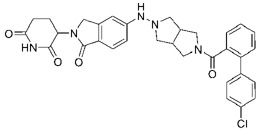 |
| GT-06442 | 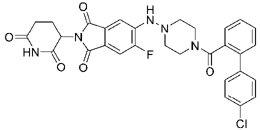 | GT-10143 | 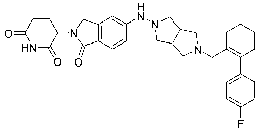 |
| GT-10173 | 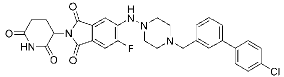 | GT-10144 | 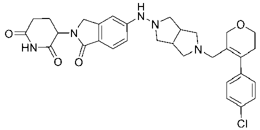 |
| GT-10178 | 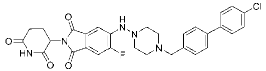 | GT-10145 | 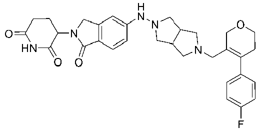 |
| GT-06437 | 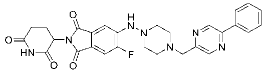 | GT-10146 | 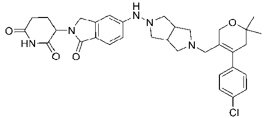 |
| GT-10208 | 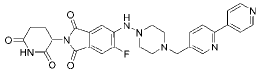 | GT-10147 | 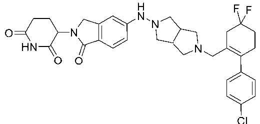 |
| GT-10298 | 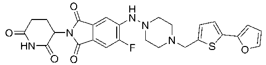 | GT-10148 | 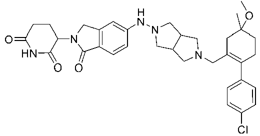 |
| GT-06470 | 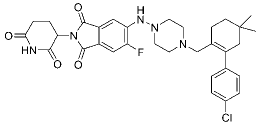 | GT-10065 | 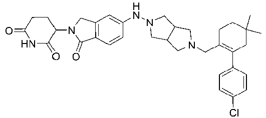 |
| GT-06474 | 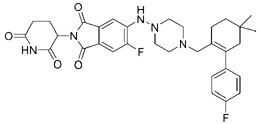 | GT-10149 | 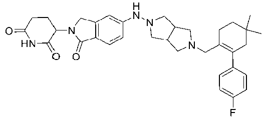 |
| GT-06510 | 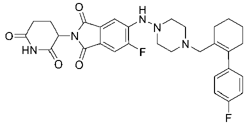 | GT-09980 | 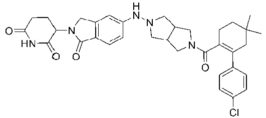 |
| GT-10315 | 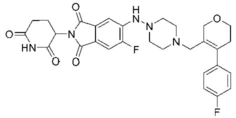 | GT-09987 | 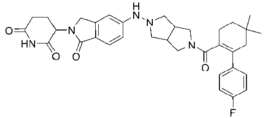 |
| GT-06961 | 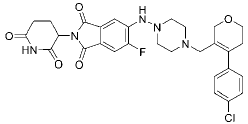 | GT-10615 | 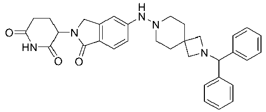 |
| GT-10320 | 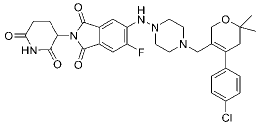 | GT-10616 | 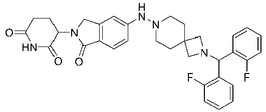 |
| GT-10492 | 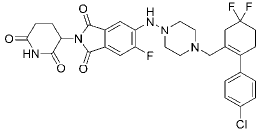 | GT-10617 | 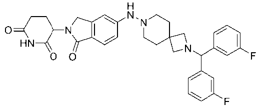 |
| GT-10493 | 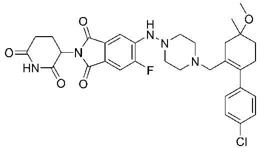 | GT-10618 | 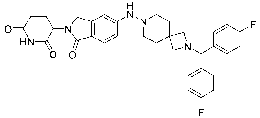 |
| | 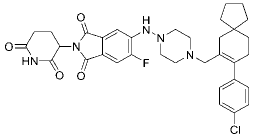 | GT-10619 | 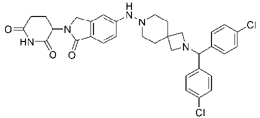 |
| | 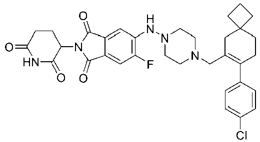 | | 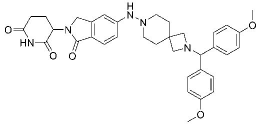 |
| GT-06466 | 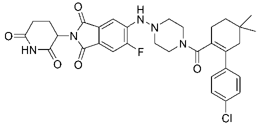 | GT-10620 | 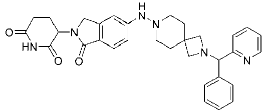 |
| GT-06461 | 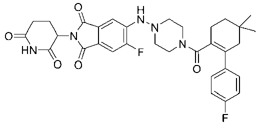 | GT-10621 | 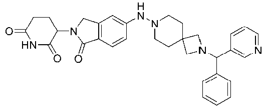 |
| GT-10310 | 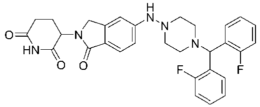 | GT-10622 | 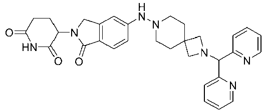 |
| GT-06398 | 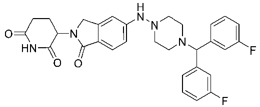 | GT-10241 | 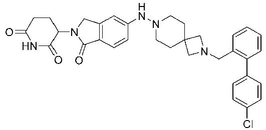 |
| GT-06426 | 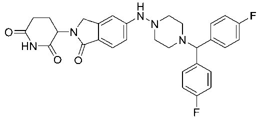 | GT-10242 | 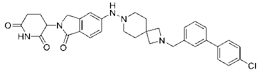 |
| GT-06427 | 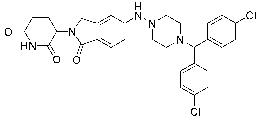 | GT-10132 | 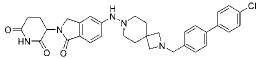 |
| GT-06428 | 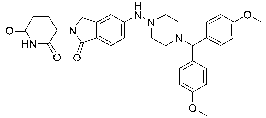 | GT-10026 | 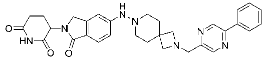 |
| GT-10086 | 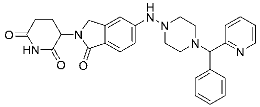 | GT-10126 | 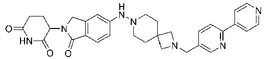 |
| | 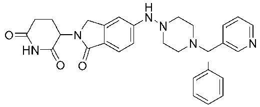 | GT-10137 | 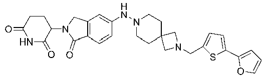 |
| | 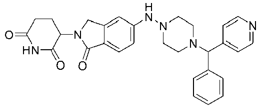 | GT-09974 | 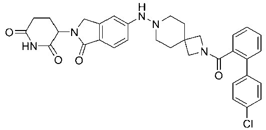 |
| GT-10511 | 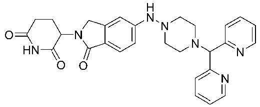 | GT-10243 | 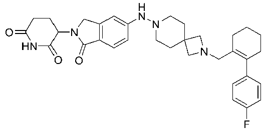 |
| GT-06444 | 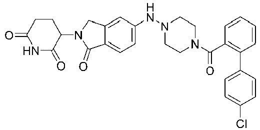 | GT-10244 | 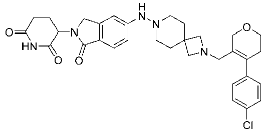 |
| GT-06499 | 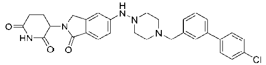 | GT-10245 | 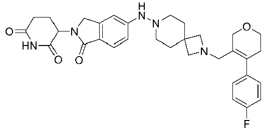 |
| GT-06500 | 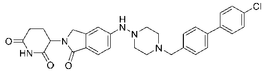 | GT-10246 | 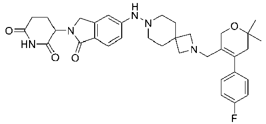 |
| GT-06439 | 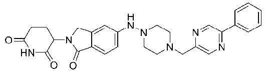 | GT-10247 | 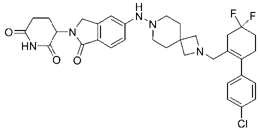 |
| GT-10209 | 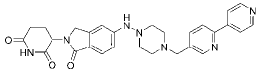 | GT-10248 | 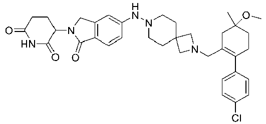 |
| GT-10299 | 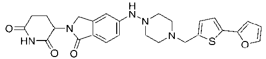 | GT-10067 | 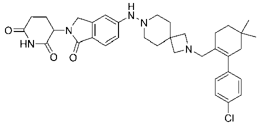 |
| GT-06536 | 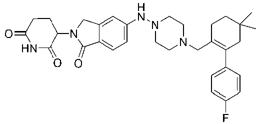 | GT-10249 | 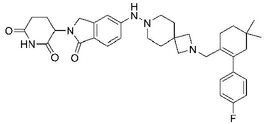 |
| GT-06462 | 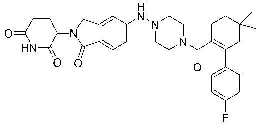 | GT-09982 | 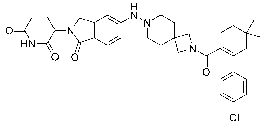 |
| GT-06505 | 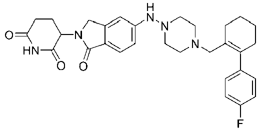 | GT-09989 | 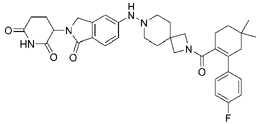 |
| GT-06501 | 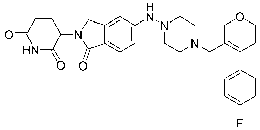 | GT-10568 | 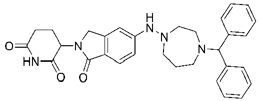 |
| GT-06511 | 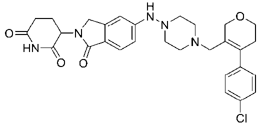 | GT-10545 | 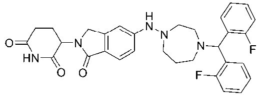 |
| GT-06502 | 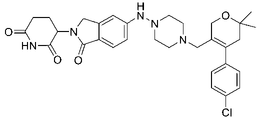 | GT-10546 | 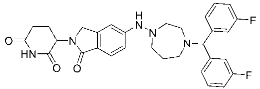 |
| GT-10412 | 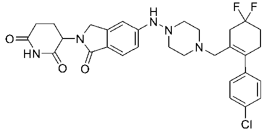 | GT-10547 | 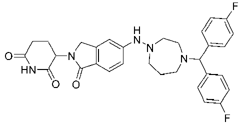 |
| GT-10482 | 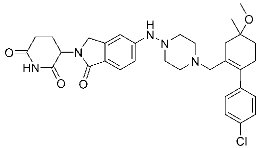 | GT-10548 | 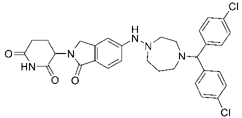 |
| | 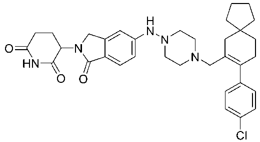 | | 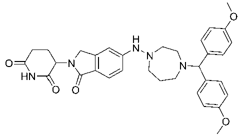 |
| | 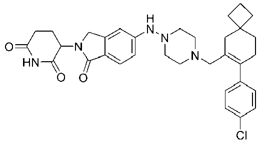 | GT-10569 | 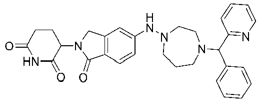 |
| GT-06420 | 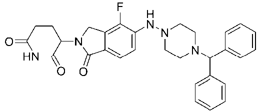 | GT-10645 | 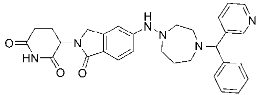 |
| GT-10311 | 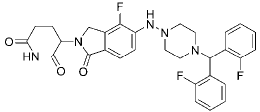 | GT-10647 | 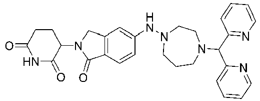 |
| GT-06399 | 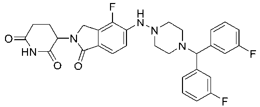 | GT-10251 | 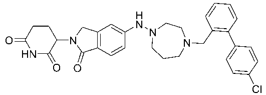 |
| GT-09964 | 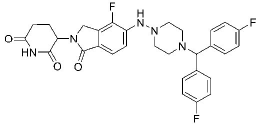 | GT-10252 | 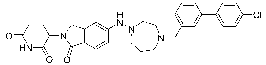 |
| GT-10080 | 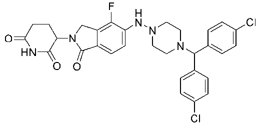 | GT-10133 | 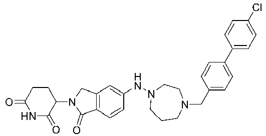 |
| | 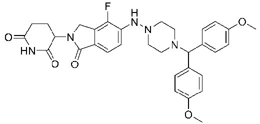 | GT-10028 | 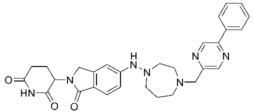 |
| GT-10087 | 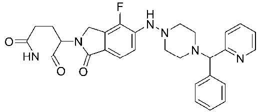 | GT-10127 | 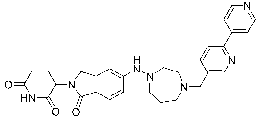 |
| GT-10092 | 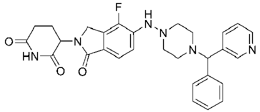 | GT-10138 | 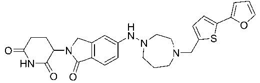 |
| | 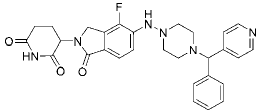 | GT-09975 | 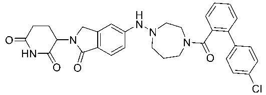 |
| GT-10512 | 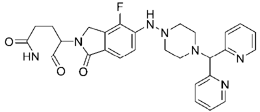 | GT-10253 | 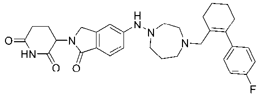 |
| GT-10168 | 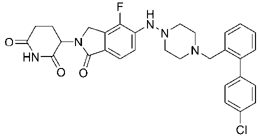 | GT-10254 | 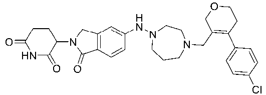 |
| GT-06445 | 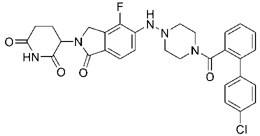 | GT-10255 | 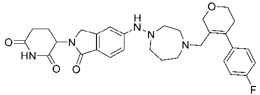 |
| GT-10174 | 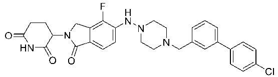 | GT-10256 | 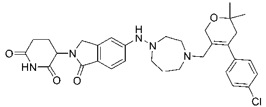 |
| GT-10179 | 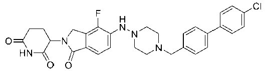 | GT-10257 | 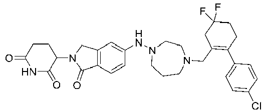 |
| GT-06440 | 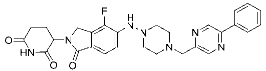 | GT-10258 | 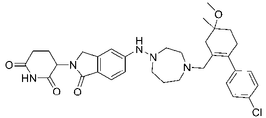 |
| GT-10210 | 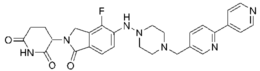 | GT-10068 | 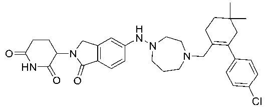 |
| GT-10300 | 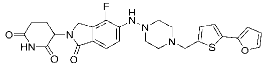 | GT-10259 | 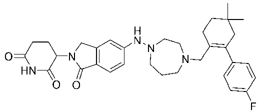 |
| GT-06471 | 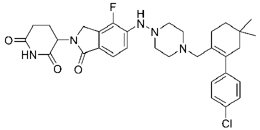 | GT-09983 | 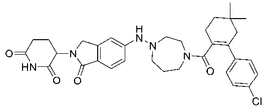 |
| GT-06467 | 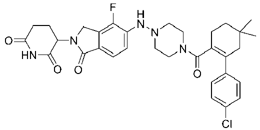 | GT-09990 | 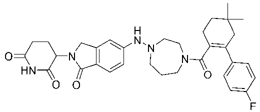 |
| GT-06475 | 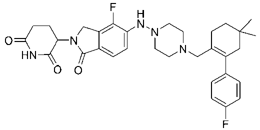 | GT-10459 | 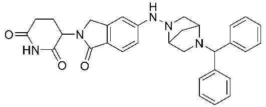 |
| GT-06463 | 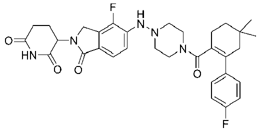 | GT-10460 | 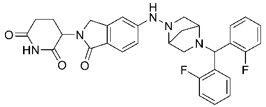 |
| GT-06506 | 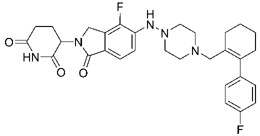 | GT-10461 | 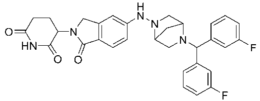 |
| GT-10316 | 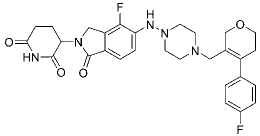 | GT-10462 | 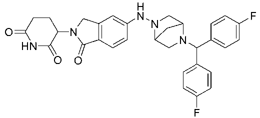 |
| GT-06962 | 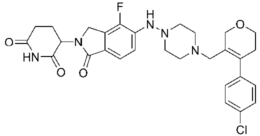 | GT-10463 | 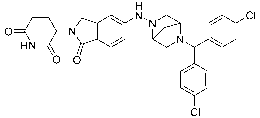 |
| GT-10321 | 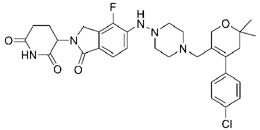 | | 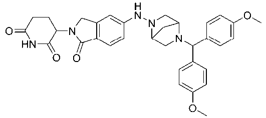 |
| GT-10413 | 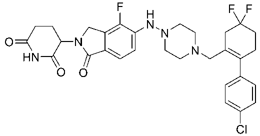 | GT-10464 | 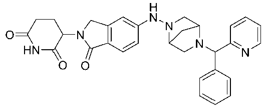 |
| GT-10483 | 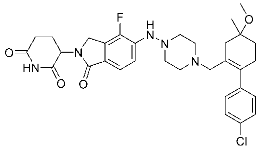 | GT-10521 | 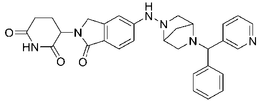 |
| | 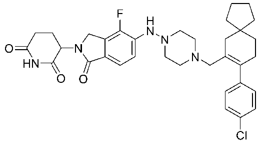 | GT-10520 | 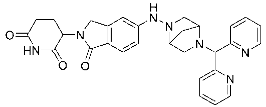 |
| | 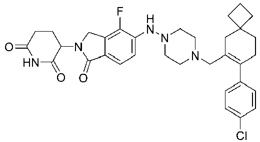 | GT-10287 | 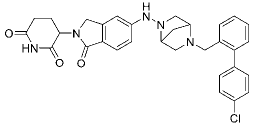 |
| GT-10078 | 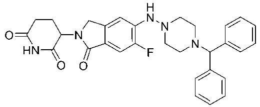 | GT-10288 | 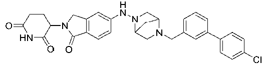 |
| GT-10312 | 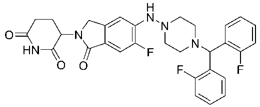 | GT-10134 | 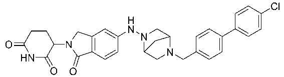 |
| GT-09960 | 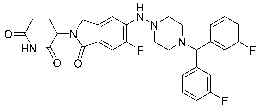 | GT-10056 | 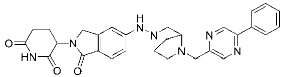 |
| GT-09965 | 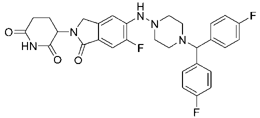 | GT-10129 | 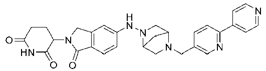 |
| GT-10081 | 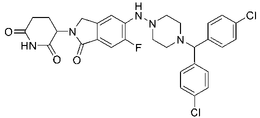 | GT-10139 | 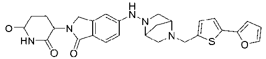 |
| | 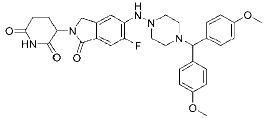 | GT-09977 | 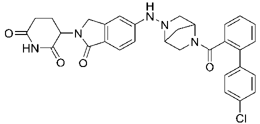 |
| GT-10088 | 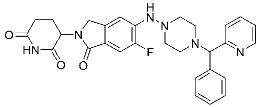 | GT-10289 | 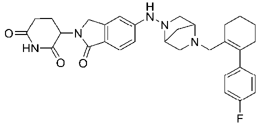 |
| GT-10093 | 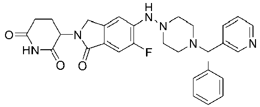 | GT-10290 | 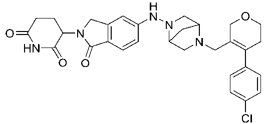 |
| | 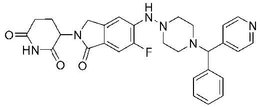 | GT-10291 | 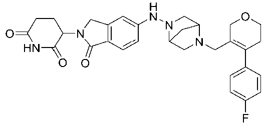 |
| GT-10513 | 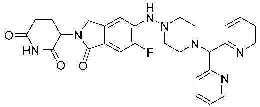 | GT-10292 | 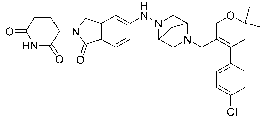 |
| GT-10169 | 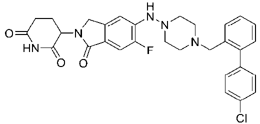 | GT-10293 | 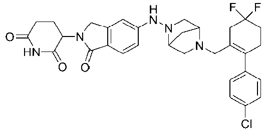 |
| GT-06447 | 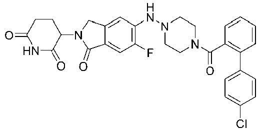 | GT-10294 | 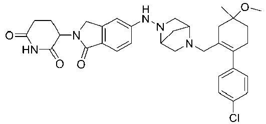 |
| GT-10175 | 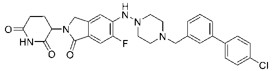 | GT-10069 | 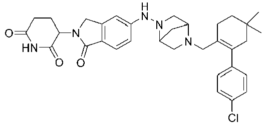 |
| GT-10180 | 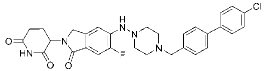 | GT-10295 | 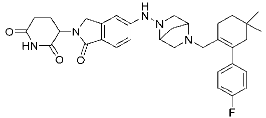 |
| GT-06446 | 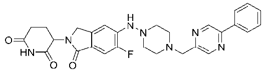 | GT-09985 | 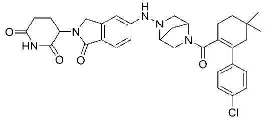 |
| GT-10211 | 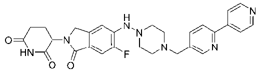 | GT-09994 | 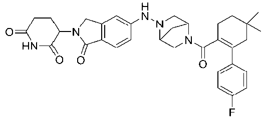 |
| GT-10301 | 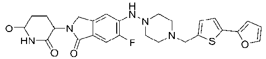 | GT-10648 | 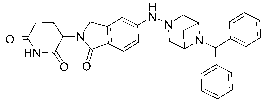 |
| GT-06537 | 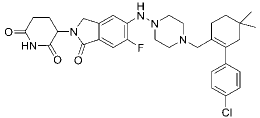 | GT-10563 | 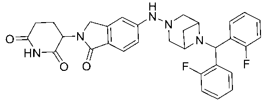 |
| GT-06539 | 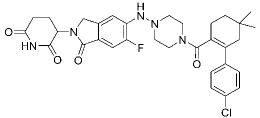 | GT-10564 | 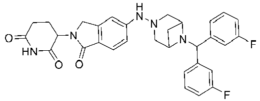 |
| GT-06538 | 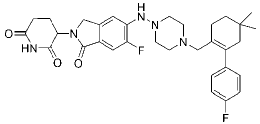 | GT-10565 | 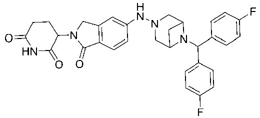 |
| GT-06540 | 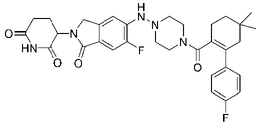 | GT-10566 | 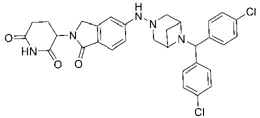 |
| GT-06507 | 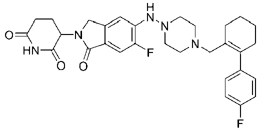 | | 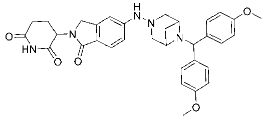 |
| GT-10317 | 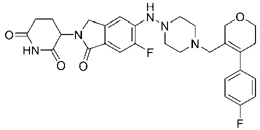 | GT-10478 | 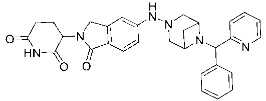 |
| GT-10306 | 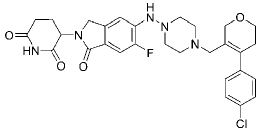 | GT-10479 | 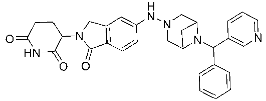 |
| GT-10408 | 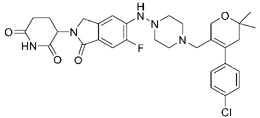 | GT-10519 | 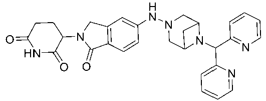 |
| GT-10414 | 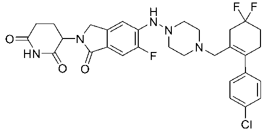 | | 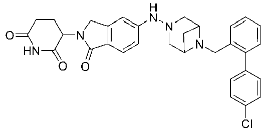 |
| GT-10484 | 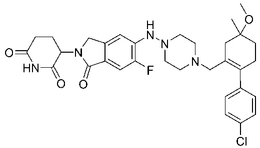 | | 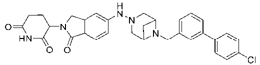 |
| | 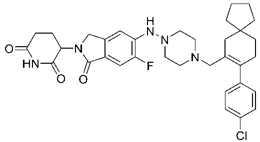 | | 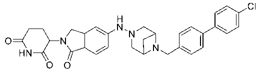 |
| | 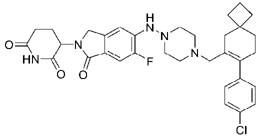 | GT-10029 | 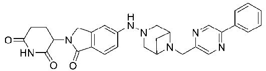 |
| GT-06421 | 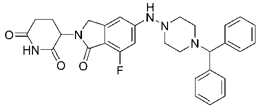 | GT-10128 | 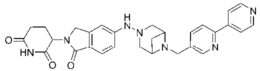 |
| GT-10313 | 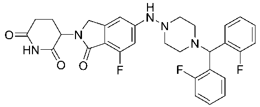 | | 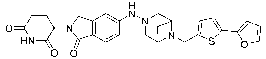 |
| GT-06400 | 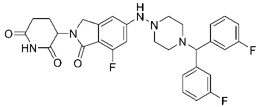 | GT-09976 | 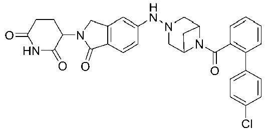 |
| GT-10076 | 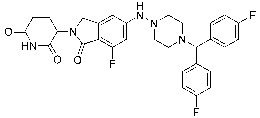 | | 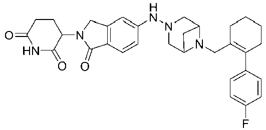 |
| GT-10082 | 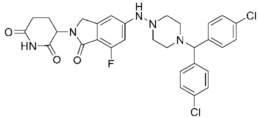 | | 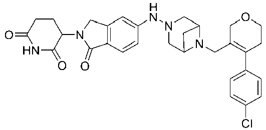 |
| | 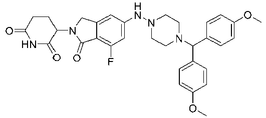 | | 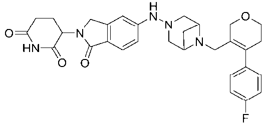 |
| GT-10089 | 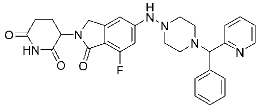 | | 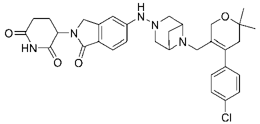 |
| GT-10496 | 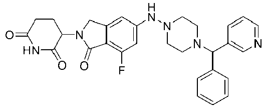 | | 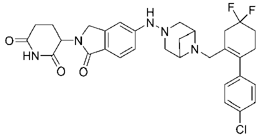 |
| | 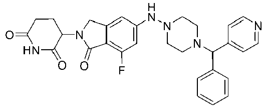 | | 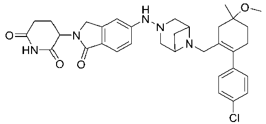 |
| GT-10514 | 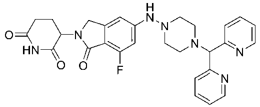 | | 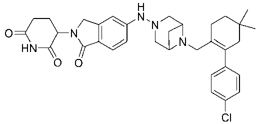 |
| GT-10170 | 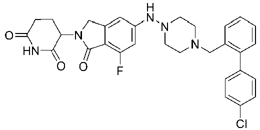 | | 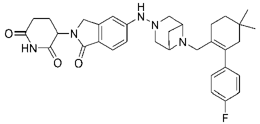 |
| GT-06443 | 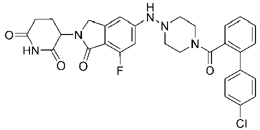 | GT-09984 | 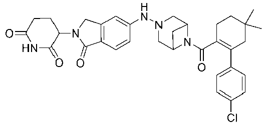 |
| GT-10176 | 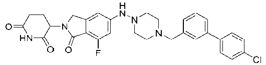 | GT-09991 | 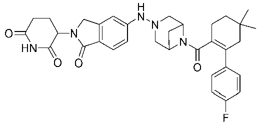 |
| GT-10181 | 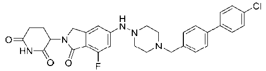 | GT-10330 | 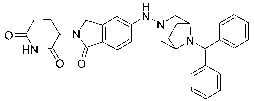 |
| GT-06438 | 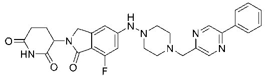 | GT-10337 | 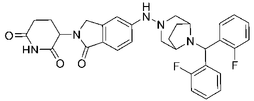 |
| GT-10212 | 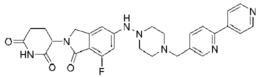 | GT-10339 | 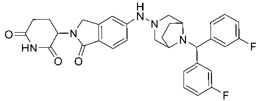 |
| GT-10302 | 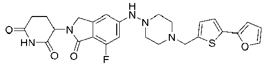 | GT-10654 | 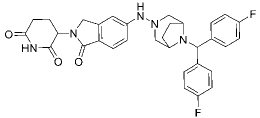 |
| GT-06472 | 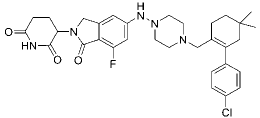 | GT-10340 | 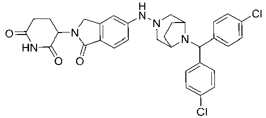 |
| GT-06468 | 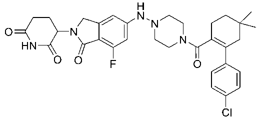 | | 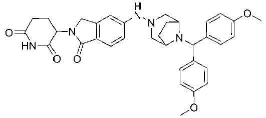 |
| GT-06476 | 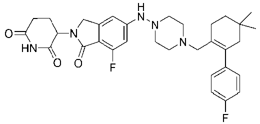 | GT-10430 | 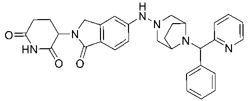 |
| GT-06464 | 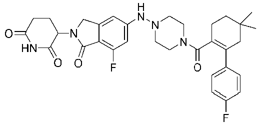 | GT-10518 | 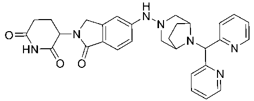 |
| GT-06508 | 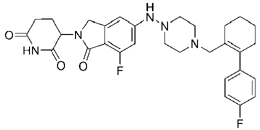 | GT-10431 | 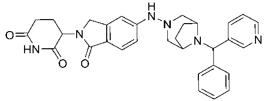 |
| GT-10318 | 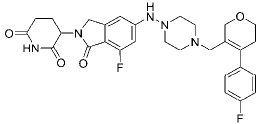 | GT-10232 | 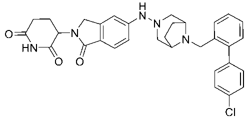 |
| GT-06963 | 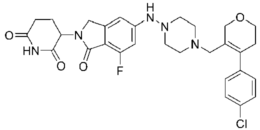 | GT-10233 | 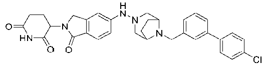 |
| GT-10409 | 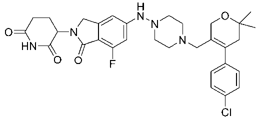 | GT-10131 | 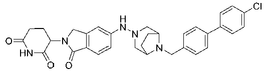 |
| GT-10415 | 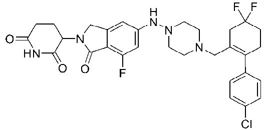 | GT-09993 | 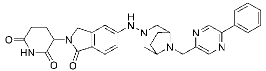 |
| GT-10485 | 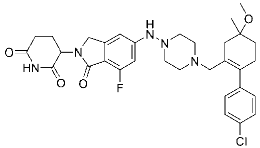 | GT-10125 | 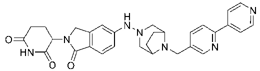 |
| | 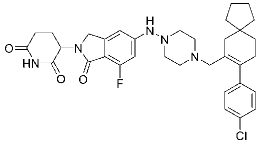 | GT-10136 | 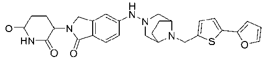 |
| | 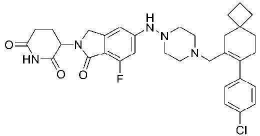 | GT-09973 | 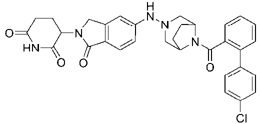 |
| GT-09962 | 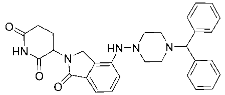 | GT-10234 | 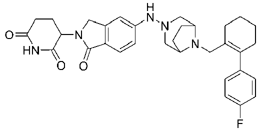 |
| GT-10314 | 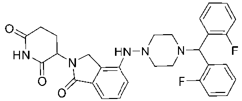 | GT-10235 | 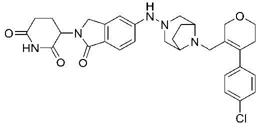 |
| GT-09961 | 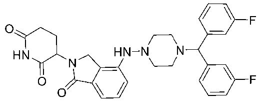 | GT-10236 | 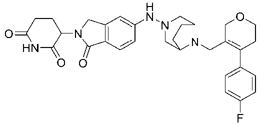 |
| GT-10077 | 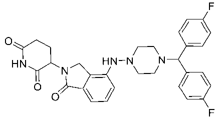 | GT-10237 | 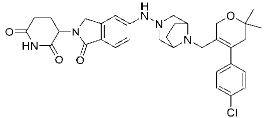 |
| GT-10083 | 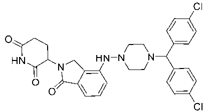 | GT-10238 | 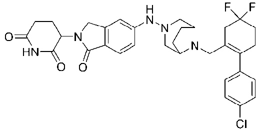 |
| | 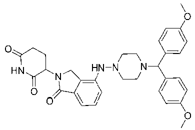 | GT-10239 | 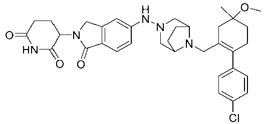 |
| GT-10090 | 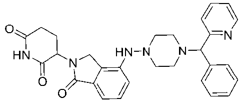 | GT-10066 | 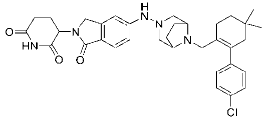 |
| GT-10094 | 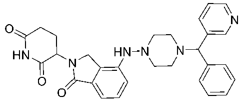 | GT-10240 | 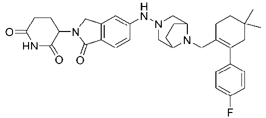 |
| | 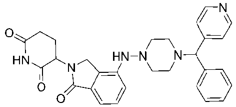 | GT-09981 | 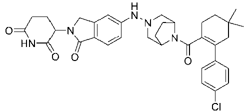 |
| GT-10515 | 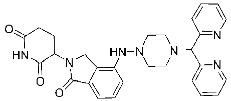 | GT-09988 | 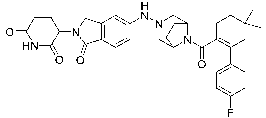 |
| GT-10171 | 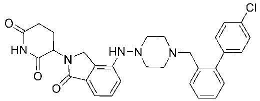 | GT-10331 | 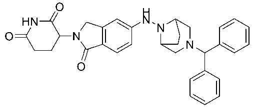 |
| GT-10304 | 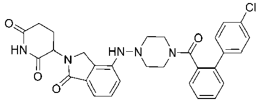 | GT-10334 | 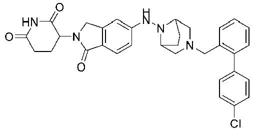 |
| GT-10177 | 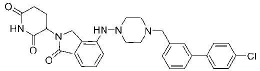 | GT-09978 | 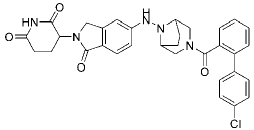 |
| GT-10182 | 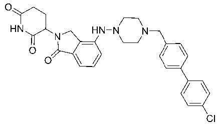 | GT-10061 | 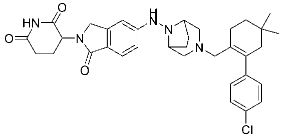 |
| GT-10183 | 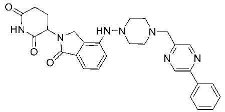 | GT-09986 | 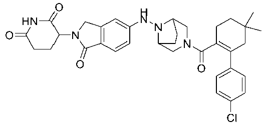 |
| GT-10296 | 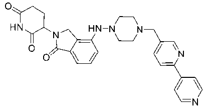 | GT-10477 | 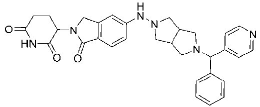 |
| GT-10303 | 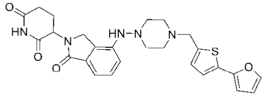 | GT-10332 | 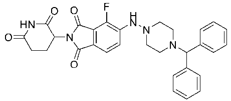 |
| GT-10487 | 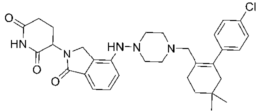 | GT-10335 | 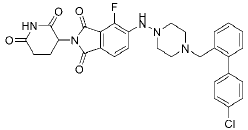 |
| GT-10489 | 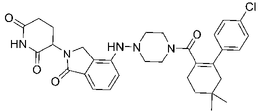 | GT-10057 | 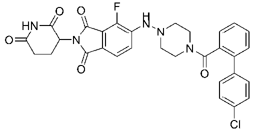 |
| GT-10488 | 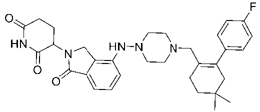 | GT-10062 | 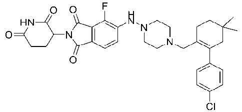 |
| GT-10490 | 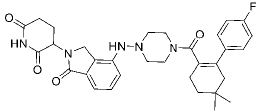 | GT-10059 | 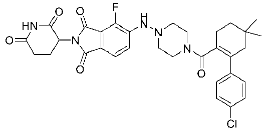 |
| GT-10305 | 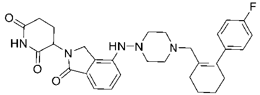 | GT-10333 | 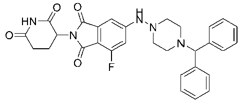 |
| GT-10319 | 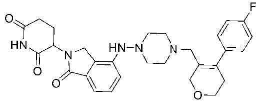 | GT-10336 | 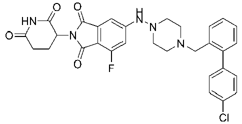 |
| GT-10307 | 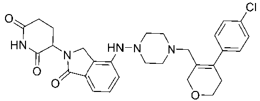 | GT-10058 | 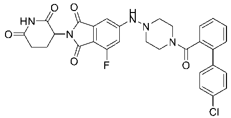 |
| GT-10410 | 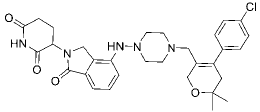 | GT-10063 | 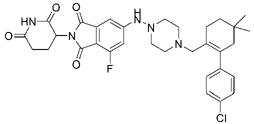 |
| GT-10416 | 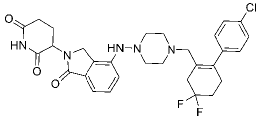 | GT-10060 | 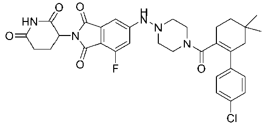 |
| GT-10486 | 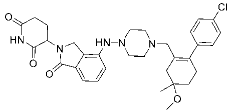 | GT-10172 | 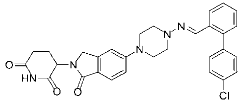 |
| | 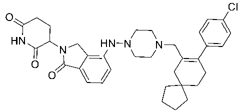 | GT-10491 | 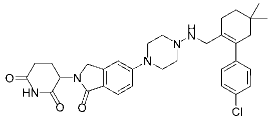 |
| | 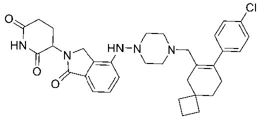 | GT-10070 | 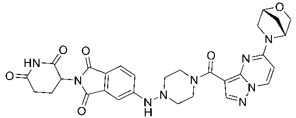 |
| GT-06863 | 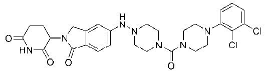 | GT-08034 | 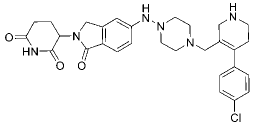 |
| GT-06864 | 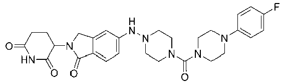 | GT-08040 | 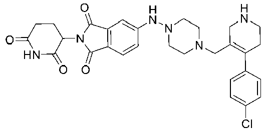 |
| GT-06865 | 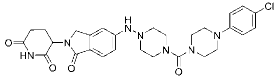 | GT-09709 | 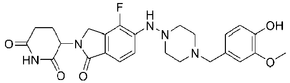 |
| GT-09741 | 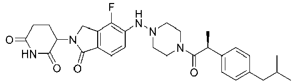 | | |

### Compounds of Formula (I')

The present disclosure provides a compound of Formula (I') 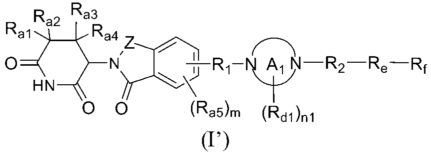
or salts (including pharmaceutically acceptable salts), stereoisomers (including enantiomers and diastereoisomers), solvates, isotopically enriched analogs, or polymorphs thereof,
wherein Z, Rₐ₁, Rₐ₂, Rₐ₃, Rₐ₄, (Rₐ₅)ₘ, R₁, R₂, ring A₁, (R_{d1})ₙ₁, Rₑ, and R_{f} are as defined in the compounds of Formula (I') above and the various embodiments thereof.

In some embodiments of the compound of Formula (I') of the present disclosure, Rₐ₁, Rₐ₂, Rₐ₃, and Rₐ₄ are identical or different and each independently represents H, deuterium (i.e., D), halogen (e.g., fluorine, chlorine, bromine, or iodine), C₁₋₆ alkyl (e.g., C₁₋₃ alkyl, such as methyl, ethyl, or propyl), halogenated C₁₋₆ alkyl (e.g., halogenated C₁₋₄ alkyl, such as trifluoromethyl), deuterated C₁₋₆ alkyl, C₁₋₆ alkoxy (e.g., C₁₋₄ alkoxy, such as methoxy), deuterated C₁₋₆ alkoxy, or halogenated C₁₋₆ alkoxy (e.g., halogenated C₁₋₄ alkoxy, such as trifluoromethoxy). In some sub-embodiments of the present disclosure, Rₐ₁, Rₐ₂, Rₐ₃, and Rₐ₄ each independently represent H.

In some embodiments of the compound of Formula (I') of the present disclosure, Z represents C(O), CH₂, or CD₂.

In some embodiments of the compound of Formula (I') of the present disclosure, Z represents C(O).

In some embodiments of the compound of Formula (I') of the present disclosure, Z represents CH₂.

In some embodiments of the compound of Formula (I') of the present disclosure, Z represents CD₂.

In some embodiments of the compound of Formula (I') of the present disclosure, (Rₐ₅)ₘ indicates that the isoindoline ring in Formula (I') to which it is attached is optionally substituted with m Rₐ₅ groups, wherein each Rₐ₅ is identical or different and independently represents deuterium, halogen (e.g., fluorine, chlorine, bromine, or iodine), hydroxyl, mercapto, nitro, amino, cyano, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), C₁₋₆ alkoxy (e.g., C₁₋₄ alkoxy, such as methoxy, ethoxy, propoxy, isopropoxy, n-butoxy, sec-butoxy, or tert-butoxy), deuterated C₁₋₆ alkoxy, halogenated C₁₋₆ alkoxy (e.g., halogenated C₁₋₄ alkoxy, such as F₃C-O-, FCH₂-O-, F₂CH-O-, ClCH₂-O-, Cl₂CHO-, CF₃CF₂-O-, CF₃CHF-O-, CHF₂CF₂-O-, CHF₂CHF-O-, CF₃CH₂-O-, or CH₂ClCH₂-O-), C₂₋₆ alkenyl (e.g., vinyl), C₂₋₆ alkynyl (e.g., ethynyl), or heterocyclyl (e.g., 4- to 30-membered heterocyclyl, 4- to 20-membered heterocyclyl, and 4- to 15-membered heterocyclyl), wherein said heterocyclyl is optionally substituted with one or more (e.g., 1-10, 1-6, 1-3, or 1) substituents independently selected from the group consisting of halogen, C₁₋₆ alkyl, and tert-butoxycarbonyl, and m represents an integer of 0, 1, 2, or 3. In some sub-embodiments of the present disclosure, m represents an integer of 0, 1, or 2. In some sub-embodiments of the present disclosure, each Rₐ₅ is identical or different and independently represents deuterium, halogen (e.g., fluorine, chlorine, bromine, or iodine), hydroxyl, mercapto, nitro, amino, cyano, C₁₋₄ alkyl (e.g., C₁₋₃ alkyl, such as methyl, ethyl, propyl, isopropyl, butyl, sec-butyl, or tert-butyl), halogenated C₁₋₄ alkyl (e.g., halogenated C₁₋₃ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₄ alkoxy (e.g., C₁₋₃ alkoxy, such as methoxy, ethoxy, propoxy, isopropoxy, n-butoxy, sec-butoxy, or tert-butoxy), halogenated C₁₋₄ alkoxy (e.g., halogenated C₁₋₃ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), or 4- to 15-membered heterocyclyl (e.g., piperazinyl), wherein said 4- to 15-membered heterocyclyl is optionally substituted with one or more (e.g., 1-10, 1-6, 1-3, or 1) substituents independently selected from the group consisting of halogen, C₁₋₆ alkyl, and tert-butoxycarbonyl.

In embodiments of the compound of Formula (I') of the present disclosure, the number of substituents is not theoretically limited in any way, or is automatically limited by the size of the building units.

In some embodiments of the compound of Formula (I') of the present disclosure, ring A₁ represents heterocyclylene containing at least two nitrogen atoms, e.g., 4- to 30-membered, 4- to 20-membered, or 4- to 15-membered heterocyclylene. Ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20 (e.g., an integer of 0-10).

In some embodiments of the compound of Formula (I') of the present disclosure, ring A₁ represents 4- to 30-membered heterocyclylene containing at least two nitrogen atoms (including 4- to 25-membered heterocyclene, 4- to 20-membered heterocyclene, 4- to 15-membered heterocyclene, and 5- to 20-membered heterocyclene). Ring A₁ is optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl (e.g., perdeuterated C₁₋₆ alkyl, perdeuterated C₁₋₅ alkyl, or perdeuterated C₁₋₄ alkyl, such as CD₃, CD₃CD₂-, CD₃CD₂CD₂- etc.), halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0, 1, 2, 3, 4, 5, 6, 7, 8, 9, 10, 11, 12, 13, 14, 15, 16, 17, 18, 19, or 20.

In some embodiments of the compound of Formula (I') of the present disclosure, ring A₁ represents 1,3-diazetidinylidene, imidazolidylene, pyrazolidylene, piperazinylene, diazacycloheptanylene, diazacyclooctylene, diazabicyclo[3.1.1]heptanylene, diazabicyclo[2.2.1]heptanylene, diazabicyclo[3.2.1]octanylene, diazabicyclo[2.2.2]octanylene, 2,6-diazaspiro[3.3]heptanylene, 2,7-diazaspiro[3.5]nonanylene, 2,8-diazaspiro[4.5]decanylene, 3,9-diazaspiro[5.5]undecanylene, or octahydropyrrolo[3,4-c]pyrrolylene, each being optionally substituted with n1 R_{d1} groups, wherein each R_{d1} independently represents deuterium, halogen (e.g., fluorine, chlorine, bromine, or iodine), hydroxyl, mercapto, nitro, amino, cyano, oxo, C₁₋₆ alkyl (e.g., C₁₋₅ alkyl, C₁₋₄ alkyl, or C₁₋₃ alkyl, such as methyl, ethyl, propyl, isopropyl, butyl, sec-butyl, tert-butyl, pentyl, or hexyl), deuterated C₁₋₆ alkyl, halogenated C₁₋₆ alkyl (e.g., halogenated C₁₋₄ alkyl, such as F₃C-, FCH₂-, F₂CH-, ClCH₂-, Cl₂CH-, CF₃CF₂-, CF₃CHF-, CHF₂CF₂-, CHF₂CHF-, CF₃CH₂-, or CH₂ClCH₂-), C₁₋₆ alkoxy (e.g., C₁₋₄ alkoxy, such as methoxy, ethoxy, propoxy, isopropoxy, n-butoxy, sec-butoxy, or tert-butoxy), or halogenated C₁₋₆ alkoxy (e.g., halogenated C₁₋₄ alkoxy, such as F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-), and n1 represents an integer of 0, 1, 2, 3, 4, 5, 6, 7, 8, 9, 10, 11, 12, 13, 14, 15, 16, 17, 18, 19, or 20.

In some embodiments of the compound of Formula (I') of the present disclosure, R₁ represents a bond, and R₂ represents N(R_{w}), where R_{w} represents hydrogen or C₁₋₃ alkyl (e.g., methyl, ethyl, and propyl); wherein said groups are optionally substituted with a substituent selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl.

In some embodiments of the compound of Formula (I') of the present disclosure, R₁ represents N(R_{w}), where R_{w} represents hydrogen or C₁₋₃ alkyl optionally substituted with one or more substituents independently selected from the group consisting of optionally substituted aryl (e.g., C₅₋₃₀ aryl, C₅₋₂₀ aryl, and C₅₋₁₅ aryl) and optionally substituted heteroaryl (e.g., 5- to 30-membered heteroaryl, 5- to 20-membered heteroaryl, and 5- to 15-membered heteroaryl), and R₂ represents a bond. In some embodiments of the compound of Formula (I') of the present disclosure, R_{w} represents hydrogen or C₁₋₃ alkyl (e.g., methyl, ethyl, and propyl), wherein the C₁₋₃ alkyl is optionally substituted with one or more substituents independently selected from the group consisting of:
phenyl or naphthyl, each of which is optionally substituted with one or more (e.g., 1-7, 1-5, 1-4, 1-3, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl; and
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, and 6,7-dihydro-5H-cyclopenta[4,5]thieno[2,3-d]pyrimidinyl, each of which is optionally substituted with one or more (e.g., 1-10, 1-7, 1-5, 1-4, 1-3, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl; and/or

In some embodiments of the compound of Formula (I') of the present disclosure, R₁ represents N(R_{w}), wherein R_{w} represents H or 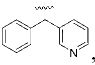 and R₂ represents a bond.

In some embodiments of the compound of Formula (I') of the present disclosure, Rₑ represents a bond. In some embodiments of the compound of Formula (I') of the present disclosure, Rₑ represents C(O)O.

In some embodiments of the compound of Formula (I') of the present disclosure, Rₑ represents the structure of the following formula: 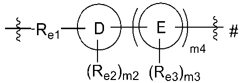
wherein Rₑ₁ represents C(O), CH₂, or halogenated CH₂;
ring D represents arylene, and (Rₑ₂)ₘ₂ indicates that the ring D is optionally substituted with m2 Rₑ₂ groups, wherein each Rₑ₂ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m2 represents an integer of 0-20; and
ring E represents nitrogen-containing heterocyclylene, m4 represents an integer of 0 or 1, and (Rₑ₃)ₘ₃ indicates that the ring E is optionally substituted with m3 Rₑ₃ groups, wherein each Rₑ₃ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m3 represents an integer of 0-20; and
symbol # indicates the point of attachment to R_{f}.

In some embodiments of the compound of Formula (I') of the present disclosure, ring D represents C₅₋₃₀ arylene, examples of which include but are not limited to C₅₋₂₀ arylene, C₆₋₂₀ arylene, C₅₋₁₅ arylene, and C₆₋₁₅ arylene, e.g., phenylene and naphthylene. The arylene is optionally substituted with m2 (e.g., an integer of 0-20, or an integer of 0-10, such as 1-6, 1-4, 1-3, 2-6, or 1) substituents R_{c2} independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl.

In some embodiments of the compound of Formula (I') of the present disclosure, m4 represents an integer of 1, and ring E represents 4- to 30-membered nitrogen-containing heterocyclylene, examples of which include but are not limited to 4- to 20-membered, 4- to 15-membered, 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered nitrogen-containing heterocyclylene, e.g., azetidinylene, pyrrolidinylene, imidazolidylene, pyrazolidylene, piperidinylene, piperazinylene, tetrahydropyridinylene, dihydroxypiperidinylene, difluoropiperidinylene, azepanylene, azacyclooctylene, diazacycloheptanylene (e.g., 1,4-diazacycloheptanylene, 4,5-diazacycloheptanylene, 1,3-diazacycloheptanylene), diazacyclooctylene, nitrogen-containing bridged heterocyclylene (e.g., 6- to 20-membered nitrogen-containing bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), and octahydropyrrolo[3,4-c]pyrrolylene. The heterocyclylene is optionally substituted with m3 (e.g., 0-20, such as 1-20, 1-15, 1-10, 1-6, 1-4, 1-3, 2-6, or 1) substituents Rₑ₃ independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl.

In some embodiments of the compound of Formula (I') of the present disclosure, Rₑ represents 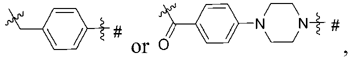 wherein symbol # indicates the point of attachment to R_{f} .

In some embodiments of the compound of Formula (I') of the present disclosure, R_{f} represents C₁₋₁₀ alkyl, examples of which include but are not limited to, e.g., C₁₋₁₀ alkyl, C₁₋₉ alkyl, C₁₋₈ alkyl, C₁₋₇ alkyl, C₁₋₆ alkyl, C₁₋₅ alkyl, C₁₋₄ alkyl, C₁₋₃ alkyl, C₁₋₂ alkyl, and methyl, e.g., methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The C₁₋₁₀ alkyl is optionally substituted with one or more (e.g., 1-20, such as 1-18, 1-15, 1-10, 1-6, 1-5, 1-4, 1-3, 1-3, 2-6, or 1) substituents independently selected from the group consisting of: halogen (e.g., fluorine, chlorine, bromine, or iodine), optionally substituted aryl (e.g., optionally substituted C₅₋₃₀ aryl, optionally substituted C₅₋₂₀ aryl, or optionally substituted C₅₋₁₅ aryl), optionally substituted heteroaryl (e.g., optionally substituted 5- to 30-membered heteroaryl, optionally substituted 5- to 20-membered heteroaryl, or optionally substituted 5- to 15-membered heteroaryl), optionally substituted heterocyclyl (e.g., optionally substituted 4- to 30-membered heterocyclyl, optionally substituted 4- to 20-membered heterocyclyl, or optionally substituted 4- to 15-membered heterocyclyl), optionally substituted cycloalkyl (e.g., optionally substituted C₃₋₃₀ cycloalkyl, optionally substituted C₃₋₂₀ cycloalkyl or optionally substituted C₃₋₁₅ cycloalkyl), and NR₃R₄, where R₃ and R₄ each independently represents H, C₁₋₃ alkyl, or optionally substituted cycloalkyl (e.g., optionally substituted C₃₋₃₀ cycloalkyl, optionally substituted C₃₋₂₀ cycloalkyl or optionally substituted C₃₋₁₅ cycloalkyl). In some embodiments, examples of aryl include, but are not limited to, C₅₋₃₀ aryl, C₅₋₂₀ aryl, C₆₋₂₀ aryl, C₅₋₁₅ aryl, and C₆₋₁₅ aryl, e.g., phenyl and naphthyl. In some embodiments, the aryl is optionally substituted with one or more (e.g., 1-20, 1-15, 1-10, 1-7, 1-6, 1-4, 1-3, 2-6, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl. In some embodiments, examples of heteroaryl include, but are not limited to, 5- to 30-membered heteroaryl, 5- to 20-membered heteroaryl, 5- to 15-membered, 5- to 10-membered, 5- to 9-membered, 5- to 8-membered, 5- to 7-membered, and 5- to 6-membered heteroaryl, e.g., furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. In some embodiments, the heteroaryl is optionally substituted with one or more (e.g., 1-20, 1-15, 1-10, 1-7, 1-6, 1-4, 1-3, 2-6, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl. In some embodiments, examples of heterocyclyl include, but are not limited to, 4- to 30-membered heterocyclyl, 4- to 20-membered heterocyclyl, 4- to 15-membered, 4- to 12-membered, 4- to 11-membered, 4- to 10-membered, 4- to 9-membered, 4- to 8-membered, 4- to 7-membered, 4-to 6-membered, 5- to 15-membered, and 5- to 9-membered heterocyclyl, e.g., azetidinyl, oxetanyl, pyrrolidinyl, imidazolidinyl, pyrazolidyl, tetrahydrofuranyl, tetrahydropyranyl, dihydropyranyl, pyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, and 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), and octahydropyrrolo[3,4-c]pyrrolyl. In some embodiments, the heterocyclyl is optionally substituted with one or more (e.g., 1-20, 1-15, 1-10, 1-6, 1-4, 1-3, 2-6, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and Rₕ. The Rₕ represents optionally substituted C₅₋₁₅ aryl, examples of which include but are not limited to C₅₋₁₂ aryl, C₆₋₁₂ aryl, and C₆₋₁₀ aryl, e.g., phenyl and naphthyl. The C₅₋₁₅ aryl is optionally substituted with one or more (e.g., 1-10, 1-6, 1-4, 1-3, 2-6, or 1) substituents independently selected from the group consisting of halogen, C₁₋₆ alkyl, and C₁₋₆ alkoxy. In some embodiments, examples of cycloalkyl include, but are not limited to, C₃₋₃₀ cycloalkyl, C₃₋₂₀ cycloalkyl, C₃₋₁₅ cycloalkyl, C₃₋₁₁ cycloalkyl, C₅₋₁₅ cycloalkyl, and C₇₋₁₅ cycloalkyl, e.g., cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₁₅ spiro-cycloalkyl, C₅-C₂₀ spiro-cycloalkyl, or C₅-C₂₅ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m*-menthanyl, or bridged cycloalkyl (e.g., C₆-C₁₅ bridged cycloalkyl, C₆-C₂₀ bridged cycloalkyl, C₆-C₂₅ bridged cycloalkyl, and C₇-C₁₅ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl). In some embodiments, the cycloalkyl is optionally substituted with one or more (e.g., 1-20, 1-15, 1-10, 1-6, 1-4, 1-3, 2-6, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and Rₕ, wherein Rₕ represents optionally substituted C₅₋₁₅ aryl, examples of which include but are not limited to C₅₋₁₂ aryl, C₆₋₁₂ aryl, and C₆₋₁₀ aryl, e.g., phenyl and naphthyl, wherein the C₅₋₁₅ aryl is optionally substituted with one or more (e.g., 1-10, 1-6, 1-5, 1-4, 1-3, 2-6, or 1) substituents independently selected from the group consisting of halogen, C₁₋₆ alkyl, and C₁₋₆ alkoxy.

In some embodiments, R₃ and R₄ each independently represent H, C₁₋₃ alkyl, or optionally substituted cycloalkyl (e.g., optionally substituted C₃₋₃₀ cycloalkyl, optionally substituted C₃₋₂₀ cycloalkyl, or optionally substituted C₃₋₁₅ cycloalkyl). In some embodiments, examples of cycloalkyl include, but are not limited to, C₃₋₃₀ cycloalkyl, C₃₋₂₀ cycloalkyl, C₃₋₁₅ cycloalkyl, C₅₋₁₅ cycloalkyl, and C₇₋₁₅ cycloalkyl, e.g., cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₁₅ spiro-cycloalkyl, C₅-C₂₀ spiro-cycloalkyl, or C₅-C₂₅ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m*-menthanyl, or bridged cycloalkyl (e.g., C₆-C₁₅ bridged cycloalkyl, C₆-C₂₀ bridged cycloalkyl, C₆-C₂₅ bridged cycloalkyl, and C₇-C₁₅ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl). In some embodiments, the cycloalkyl is optionally substituted with one or more (e.g., 1-20, 1-15, 1-10, 1-6, 1-4, 1-3, 2-6, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and Rₕ, wherein Rₕ represents optionally substituted C₅₋₁₅ aryl, examples of which include but are not limited to C₅₋₁₂ aryl, C₆₋₁₂ aryl, and C₆₋₁₀ aryl, e.g., phenyl and naphthyl, wherein said C₅₋₁₅ aryl is optionally substituted with one or more (e.g., 1-10, 1-6, 1-5, 1-4, 1-3, 2-6, or 1) substituents independently selected from the group consisting of halogen, C₁₋₆ alkyl, and C₁₋₆ alkoxy. In some embodiments, R₃ represents H, and R₄ represents bridged cycloalkyl (e.g., C₆-C₁₅ bridged cycloalkyl, C₆-C₂₀ bridged cycloalkyl, C₆-C₂₅ bridged cycloalkyl, and C₇-C₁₅ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl), which is optionally substituted with one or more (e.g., 1-20, 1-15, 1-10, 1-6, 1-4, 1-3, 2-6, or 1) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, and tert-butoxycarbonyl.

In some embodiments of the compound of Formula (I') of the present disclosure, examples of R_{f} include, but are not limited to:
methyl, isopropyl, tert-butyl, 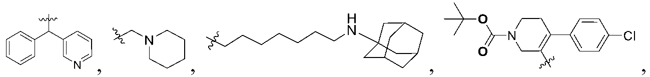 or

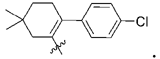

Preferably the compounds of the present invention and their salts (especially pharmaceutically acceptable salts, such as hydrochloride, etc.), enantiomers, diastereomers, solvates, or polymorphs thereof in Table 2 below are provided:

**Table 2. The compounds of the present invention**

| Compound No. | Structure of the compounds | Compound No. | Structure of the compounds |
|---|---|---|---|
| GT-08644 | 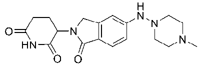 | GT-09135 | 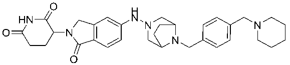 |
| GT-08881 | 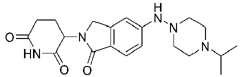 | GT-09197 | 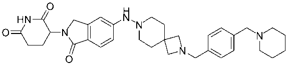 |
| GT-09018 | 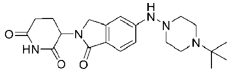 | GT-09198 | 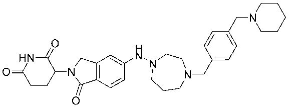 |
| GT-09057 | 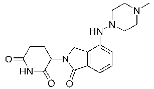 | GT-09199 | 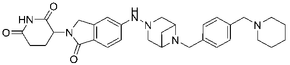 |
| GT-08880 | 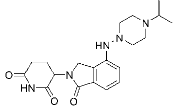 | GT-09200 | 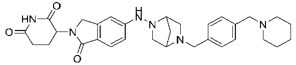 |
| GT-09138 | 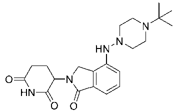 | GT-09201 | 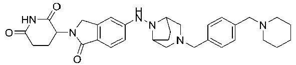 |
| GT-08531 | 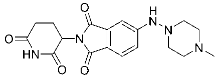 | GT-09202 | 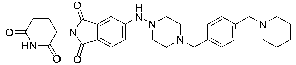 |
| GT-08710 | 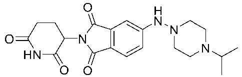 | GT-09203 | 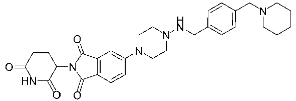 |
| GT-08711 | 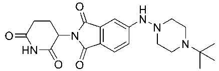 | GT-09213 | 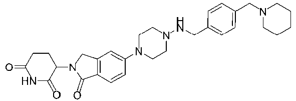 |
| GT-09058 | 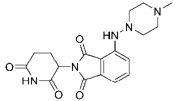 | GT-09104 | 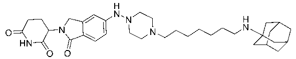 |
| GT-08866 | 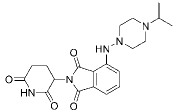 | GT-09105 | 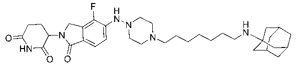 |
| GT-08865 | 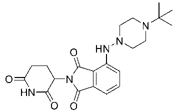 | GT-09106 | 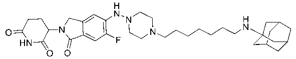 |
| GT-09917 | 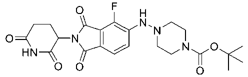 | GT-09107 | 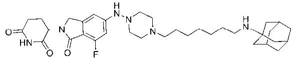 |
| GT-09918 | 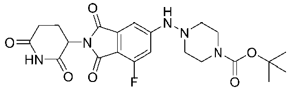 | GT-09108 | 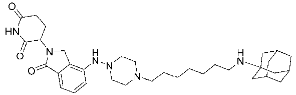 |
| GT-09919 | 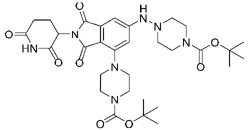 | GT-09109 | 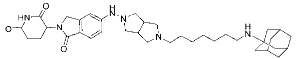 |
| GT-10465 | 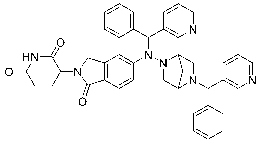 | GT-09110 | 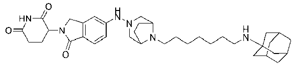 |
| GT-10476 | 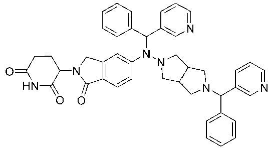 | GT-09111 | 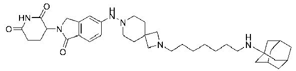 |
| GT-10480 | 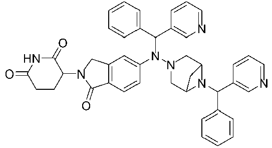 | GT-09112 | 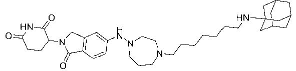 |
| GT-10646 | 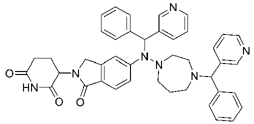 | GT-09113 | 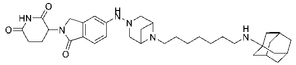 |
| GT-09129 | 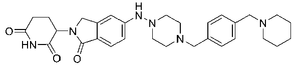 | GT-09114 | 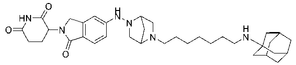 |
| GT-09130 | 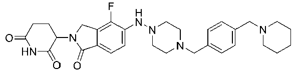 | GT-09115 | 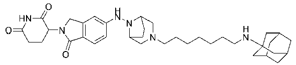 |
| GT-09131 | 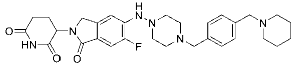 | GT-09116 | 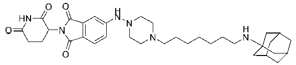 |
| GT-09132 | 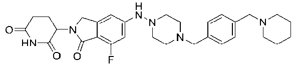 | GT-09117 | 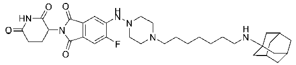 |
| GT-09133 | 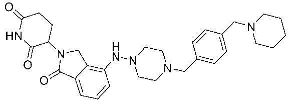 | GT-07990 | 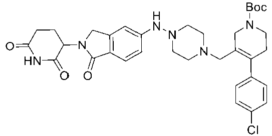 |
| GT-09134 | 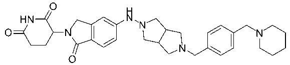 | GT-07996 | 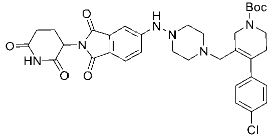 |
| GT-09708 | 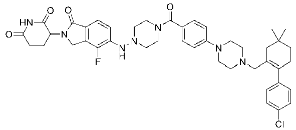 | | |

### II. Other Forms of Compounds (including salts, enantiomers, stereoisomers, solvates, isotopically enriched analogs, or polymorphs of compounds)

The compounds of the present disclosure have the structures of any one of Formula (I), Formula (I-1), Formula (I-2), Formula (I-3), Formula (I-4), Formula (I-5), Formula (II-1), Formula (II-2), Formula (II-3), Formula (II-4), or Formula (I'). Unless otherwise specified, all references to the compounds of the present disclosure also include compounds of any one of Formula (I), Formula (I-1), Formula (I-2), Formula (I-3), Formula (I-4), Formula (I-5), Formula (II-1), Formula (II-2), Formula (II-3), Formula (II-4), or Formula (I') and specific compounds within the scope of these general formulae.

It should be recognized that compounds of the present disclosure (including compounds of Formula (I), Formula (I-1), Formula (I-2), Formula (I-3), Formula (I-4), Formula (I-5), Formula (II-1), Formula (II-2), Formula (II-3), Formula (II-4), or Formula (I')) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorphs forms or salts of the compounds of the present disclosure (including compounds of Formula (I), Formula (I-1), Formula (I-2), Formula (I-3), Formula (I-4), Formula (I-5), Formula (II-1), Formula (II-2), Formula (II-3), Formula (II-4), or Formula (I')) are also provided. The salts may be pharmaceutically acceptable salts, including but not limited to hydrohalides (including hydrochlorides, hydrobromides), sulfates, maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, trifluoroacetate, glycolate, or 4-methylbenzenesulfonate etc. The compounds of the present disclosure can exist as non-solvated or solvated forms in pharmaceutically acceptable solvents such as water, ethanol, and the like. In some embodiments, the compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or ²H).

### III. Pharmaceutical Compositions/Formulations

In some embodiments, the present disclosure provides a pharmaceutical composition comprising as active ingredient the compound of the present disclosure (the compound of Formula (I), Formula (I-1), Formula (I-2), Formula (I-3), Formula (I-4), Formula (I-5), Formula (II-1), Formula (II-2), Formula (II-3), Formula (II-4), or Formula (I')) or a pharmaceutically acceptable salt, solvate, isotopically enriched analog, polymorph, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or pharmaceutically acceptable salts thereof may be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

### IV. Kits/Packaged Products

The compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or a pharmaceutically acceptable salt, solvate, isotopically enriched analog, polymorph, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Methods and Uses

The compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or a pharmaceutically acceptable salt, solvate, isotopically enriched analog, polymorph, stereoisomer (including enantiomer), or mixture of stereoisomers thereof can be used as a medicament. Especially the compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or a pharmaceutically acceptable salt, solvate, isotopically enriched analog, polymorph, stereoisomer (including enantiomer), or mixture of stereoisomers thereof can be used for the manufacture of a medicament for the prevention and/or treatment of diseases or disorders associated with cereblon protein.

The disease or disorder associated with cereblon protein is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

The disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type I diabetes mellitus, urticaria, inflammatory bowel disease (including Crohn's disease and ulcerative colitis); keratoconjunctivitis sicca; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

The present disclosure provides a method for preventing and/or treating a disease or disorder associated with cereblon protein in a subject, comprising administering to the subject a therapeutically effective amount of the compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or a pharmaceutically acceptable salt thereof, or the pharmaceutical composition of the present disclosure comprising as an active ingredient the compounds of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or a pharmaceutically acceptable salt thereof.

In the method for preventing and/or treating a disease or disorder associated with a cereblon protein, the compound of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or the pharmaceutical composition comprising as an active ingredient the compound of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The term "treatment" or "treating" refers to administering to a subject the compound of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure, or a pharmaceutically acceptable salt thereof, or the pharmaceutical composition comprising, as an active ingredient, the compound of Formula (I) (or a compound of Formula (I-1), Formula (I-2), Formula (I-3), Formula (I-4), Formula (I-5), Formula (II-1), Formula (II-2), Formula (II-3), Formula (II-4), or Formula (I')) of the present disclosure or a pharmaceutically acceptable salt thereof, to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

### VI. Preparation Method

The present disclosure also provides a method for preparing the compound of Formula (I), comprising using a compound of Formula (M1) and a compound of Formula (M2) as starting materials to obtain the compound of Formula (I): 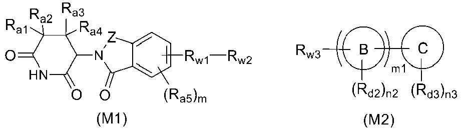 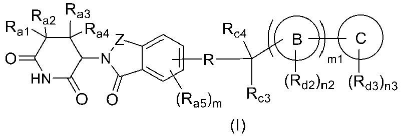
wherein Rₐ₁, Rₐ₂, Rₐ₃, Rₐ₄, (Rₐ₅)ₘ, Z, R, ring B, ring C, (R_{d2})ₙ₂, (R_{d3})ₙ₃, and m1 are as defined in the compounds of Formula (I) of the present disclosure and the respective sub-embodiments thereof; and
R_{w3} represents CHO, COOH, or 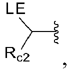 wherein R_{c2} is as defined in the compounds of Formula (I) of the present disclosure and the respective sub-embodiments thereof, and the group LE represents Cl, Br, I, OMs, OTs, or ONs;
   wherein (1) -R_{w1}-R_{w2} represents 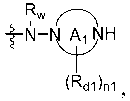 and R represents 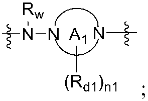 or
   (2) -R_{w1}-R_{w2} represents 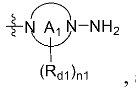 and R represents 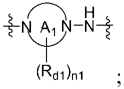 or
   (3) -R_{w1}-R_{w2} represents -NH-NH₂, and R represents -NH-NH-; or
   (4) -R_{w1}-R_{w2} represents 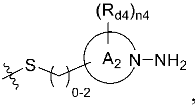 , and R represents 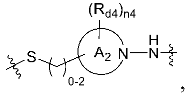
   wherein R_{w}, ring A₁, (R_{d1})ₙ₁, ring A₂, and (R_{d4})ₙ₄ are as defined in the compounds of Formula (I) of the present disclosure and the respective sub-embodiments thereof;
wherein when R_{w3} represents CHO, R_{c3} and R_{c4} represent H; or
when R_{w3} represents COOH, R_{c3} and R_{c4} together form a carbonyl group; or
when R_{w3} represents 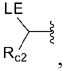 R_{c3} represents H, and R_{c4} represents R_{c2}.

In some embodiments of the method for preparing the compound of Formula (I) in the present disclosure, when R_{w3} represents CHO, the compound of Formula (M1) and the compound of Formula (M2) undergo a reductive amination reaction to prepare the compound of Formula (I), wherein R_{c3} and R_{c4} in the compound of Formula (I) represent H.

In some embodiments of the method for preparing the compound of Formula (I) in the present disclosure, the reductive amination reaction can be carried out in the presence of sodium triacetoxyborohydride and an organic solvent (e.g., 1,2-dichloroethane, N,N-dimethylformamide, or dichloromethane) at a temperature ranging from room temperature to 80 °C (e.g., 40 °C to 60 °C, 40 °C to 50 °C, or 50 °C to 60 °C). Alternatively, the reductive amination reaction can be carried out in the presence of sodium borohydride and 1,4-dioxane at a temperature ranging from room temperature to 80 °C (e.g., 40 °C to 60 °C, 40 °C to 50 °C, or 50 °C to 60 °C). In some embodiments, the molar ratio of the compound of Formula (M1) to the compound of Formula (M2) can be, for example, from 1.0 to 2:1, 1:1.0 to 2, 1:1.1 to 2, 1:1.1 to 1.5, 1:1.1 to 1.2, or 1:1.2 to 1.3, and the like.

In some embodiments of the method for preparing the compound of Formula (I) in the present disclosure, when R_{w3} represents COOH, the compound of Formula (M1) and the compound of Formula (M2) undergo an amide condensation reaction to prepare the compound of Formula (I), wherein R_{c3} and R_{c4} in the compound of Formula (I) together form a carbonyl group.

In some embodiments of the method for preparing the compound of Formula (I) in the present disclosure, the amide condensation reaction can be carried out in the presence of e.g., HATU/DIEA/DMF, or HATU/TEA/DMF, or HOAt/EDCI/TEA/DCM at room temperature. In some embodiments, the molar ratio of the compound of Formula (M1) to the compound of Formula (M2) can be, for example, from 1.0 to 2:1, 1:1.0 to 2, 1:1.1 to 2, 1:1.1 to 1.5, 1:1.1 to 1.2, or 1:1.2 to 1.3, and the like.

In some embodiments of the method for preparing the compound of Formula (I) in the present disclosure, when R_{w3} represents 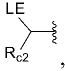 the compound of Formula (M1) and the compound of Formula (M2) undergo an aminoalkylation reaction to give the compound of Formula (I), wherein in the compound of Formula (I), R_{c3} represents H, and R_{c4} represents R_{c2} .

In some embodiments of the method for preparing the compound of Formula (I) of the present disclosure, the aminoalkylation reaction can be carried out, for example, in the presence of DIEA and sodium iodide, or triethylamine and sodium iodide, at a temperature ranging from room temperature to 80°C (e.g., 40°C to 60°C, 40°C to 50°C, or 50°C to 60°C). In some embodiments, the molar ratio of the compound of formula (M1) to the compound of formula (M2) can be, for example, 1.0 to 2:1, 1:1.0 to 2, 1:1.1 to 2, 1:1.1 to 1.5, 1:1.1 to 1.2, or 1:1.2 to 1.3, etc.

In some embodiments of the method for preparing the compound of Formula (I) in the present disclosure, when -R_{w1}-R_{w2} in the compound of Formula (M1) represents 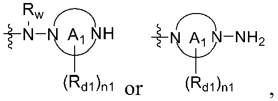 the compound of Formula (M1) is prepared by a reaction using the compound of Formula (M3) and the compound of Formula (M4) as starting materials, 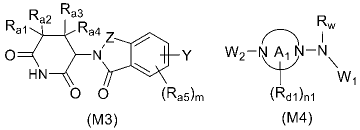
Y represents F or Br;
W₁ represents H, and W₂ represents an amino protecting group; or
W₁ represents an amino protecting group, and W₂ represents H.

In some embodiments of the method for preparing the compound of Formula (I) in the present disclosure, when Y represents F, the compound of Formula (M3) and the compound of Formula (M4) undergo a palladium-catalyzed coupling reaction to prepare the compound of Formula (M1). In some embodiments of the method for preparing the compound of Formula (I) in the present disclosure, the palladium-catalyzed coupling reaction can be carried out in the presence of cesium carbonate, a palladium catalyst, and N,N-dimethylformamide at a temperature of, for example, 50 °C to 100 °C (e.g., 80 °C).

In some embodiments of the method for preparing the compound of Formula (I) in the present disclosure, when Y represents Br, the compound of Formula (M3) and the compound of Formula (M4) undergo a substitution reaction to prepare the compound of Formula (M1). In some embodiments of the method for preparing the compound of Formula (I) in the present disclosure, the substitution reaction can be carried out in the presence of N,N-diisopropylethylamine and dimethyl sulfoxide at a temperature of, for example, 80 °C to 150 °C (e.g., 130 °C).

In some embodiments of the method for preparing the compound of Formula (I) in the present disclosure, the amino protecting group can be, for example, Boc and the like. The deprotection of the protecting groups can be achieved by techniques and methods well known to those skilled in the art. For example, the removal of the Boc protecting group can be achieved under acidic conditions, such as hydrochloric acid or trifluoroacetic acid.

### VII. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R_{c} represents a bond" means that R_{c} is a bond linker. In other words, when R_{c} represents a bond, the ring B in the structure of Formula (I) is directly connected to the ring A in the structure of Formula (I).

In this document, the term "optionally substituted with" used alone or in combination means that the referenced group may be unsubstituted or substituted with one or more substituents as defined here. Herein, the wording "optionally substituted with..." and "unsubstituted or substituted" can be used interchangeably. The term "substituted" generally represents the replacement of one or more hydrogen atoms in the referenced structure by identical or different specific substituents. The number of substituents is not theoretically limited in any way, or is automatically limited by the size of the building units (i.e., the total number of replaceable hydrogen atoms in the building units), or as clearly defined herein.

Herein, a bond interrupted by a wavy line shows the point of attachment of the depicted group to the rest of the molecule. For example, the monovalent group depicted below 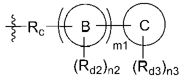 shows the point of attachment of R_{c} in said group to R in the structure of Formula (I).

As used herein, the term "one or more" in the expression "substituted with one or more substituents selected from the group consisting of ...", whether used alone or in combination, means that some or allof the hydrogen atoms of the referenced group are replaced by a substituent or substituents. The number of substituents includes but is not limited to 1 to 40, such as 1 to 30, 1 to 25, 1 to 20, 1 to 15, 1 to 10, 1 to 5, 1 to 4, 1 to 3, 1 to 2, or 1. The number of hydrogens to be replaced is in principle not limited in any way, or is automatically limited by the size of the building unit. For example, where the referenced group is a methyl group, the number of substituents may be 1 to 3.

As used herein, the term "deuterated" used alone or in combination, indicates that one or more hydrogen atoms in the referenced group are replaced by deuterium atoms.

As used herein, the term "oxo" or "oxo group" used alone or in combination, refers to=O.

As used herein, the term "C(O)" or "C(=O)" used alone or in combination, refers to a carbonyl group.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. Examples of the C₁₋₁₀ alkyl of the present disclosure may include a C₁₋₉ alkyl, C₁₋₈ alkyl, C₂₋₈ alkyl, C₁₋₇ alkyl, C₁₋₆ alkyl, C₁₋₅ alkyl, C₁₋₄ alkyl, C₁₋₃ alkyl, and C₁₋₂ alkyl. Representative examples include methyl, ethyl, n-propyl, isopropyl, n-butyl, isobutyl, sec-butyl, tert-butyl, pentyl, isopentyl, neopentyl, tert-pentyl, and hexyl. The term "C₁₋₃ alkyl" or "C₁-C₃ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted, and the substituent(s) may be one or more (e.g., 1-10, 1-6, 1-5, 1-4, or 1-3) groups each independently selected from the group consisting of e.g., halogen, hydroxyl, cyano, C₁₋₃ alkyl, C₁₋₃ alkoxy, halogenated C₁₋₆ alkoxy, halogenated C₁₋₆ alkyl (e.g., trifluoromethyl), C₃₋₆ cycloalkyl, 4- to 7-membered heterocyclyl, and combinations thereof.

As used herein, the term "halogenated alkyl", used alone or in combination, refers to a linear or branched alkyl group substituted with one or more halogens, wherein one or more hydrogen atom(s) of the alkyl group are replaced with one or more halogens. The term "halogenated Cₓ₋C_{y} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present disclosure refers to a linear or branched alkyl group containing from 1 to 10 carbon atoms substituted with one or more halogens. Examples of the halogenated C₁₋₁₀ alkyl group of the present disclosure include halogenated C₁₋₉ alkyl group, e.g., halogenated C₁₋₈ alkyl group, halogenated C₂₋₈ alkyl group, halogenated C₁₋₇ alkyl group, halogenated C₁₋₆ alkyl, halogenated C₁₋₅ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halogenated C₁₋₃ alkyl" or "halogenated C₁₋C₃ alkyl" of the present disclosure refers to an alkyl group containing from 1 to 3 carbon atoms substituted with one or more halogens, and its representative examples include halomethyl (e.g., trifluoromethyl), haloethyl, halo-n-propyl, and haloisopropyl.

As used herein, the term "deuterated alkyl", used alone or in combination, refers to a linear or branched alkyl group substituted with one or more deuterium atoms, wherein one or more hydrogen atoms of the alkyl group are replaced with one or more deuterium atoms. The term "deuterated Cₓ₋C_{y} alkyl" or "deuterated C_{x-y} alkyl" (x and y are each an integer) refers to a linear or branched alkyl containing from x to y carbon atoms substituted with one or more deuterium atoms. The term "deuterated C₁₋₁₀ alkyl" used alone or in combination in the present disclosure refers to a linear or branched alkyl group containing from 1 to 10 carbon atoms substituted with one or more deuterium atoms. Examples of the deuterated C₁₋₁₀ alkyl group of the present disclosure include deuterated C₁₋₉ alkyl group, e.g., deuterated C₁₋₈ alkyl group, deuterated C₂₋₈ alkyl group, deuterated C₁₋₇ alkyl group, deuterated C₁₋₆ alkyl, deuterated C₁₋₅ alkyl, or deuterated C₁₋₄ alkyl. Representative examples include perdeuterated methyl (CD₃), perdeuterated ethyl (CD₃CD₂), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, and perdeuterated hexyl. The term "deuterated C₁₋₃ alkyl" or "deuterated C₁₋C₃ alkyl" of the present disclosure refers to an alkyl group containing from 1 to 3 carbon atoms substituted with one or more deuterium atoms, and its representative examples include perdeuterated methyl (CD₃) and perdeuterated ethyl (CD₃CD₂).

As used herein, the term "alkoxy", used alone or in combination, refers to a linear or branched alkoxy group having structural formula of alkyl-O-. Optionally, the alkyl portion of the alkoxy group may contain 1-10 (e.g., 1-6, 1-4, or 1-3) carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "C₁-C₆ alkoxy" or "C₁₋₆ alkoxy" refers to a linear or branched alkoxy group containing from 1 to 6 carbon atoms. Representative examples of C₁₋₆ alkoxy include, but are not limited to, methoxy, ethoxy, n-propoxy, isopropoxy, butoxy, pentyloxy, and hexyloxy.

As used herein, the term "halogenated alkoxy", used alone or in combination, refers to an alkoxy group substituted with one or more halogen atoms. Optionally, the alkyl portion of the alkoxy group may contain 1-10 (e.g., 1-6, 1-4, or 1-3) carbon atoms. Examples of "halogenated alkoxy" include halogenated C₁₋₆ alkoxy and halogenated C₁₋₄ alkoxy. Representative examples include, but are not limited to, F₃C-O-, FCH₂-O-, F₂CH-O-, ClCH₂-O-, Cl₂CH-O-, CF₃CF₂-O-, CF₃CHF-O-, CHF₂CF₂-O-, CHF₂CHF-O-, CF₃CH₂-O-, or CH₂ClCH₂-O-.

As used herein, the term "heteroaryl", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, 6- to 9-membered, 6-to 10-membered, or 6- to 20-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic, tetracyclic, or polycyclic heteroaryl groups, which contain one aromatic ring having one or more (e.g., 1-4, 1-3, 1-2, or 1) heteroatoms independently selected from O, S and N, and the remaining rings which may be a saturated, partially unsaturated or aromatic ring and can be carbocyclic ring or contain one or more (e.g., 1-4, 1-3, 1-2, or 1) heteroatoms independently selected from O, S and N. Examples of monocyclic heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, chromanyl, and 6,7-dihydrothieno[3,2-d]pyrimidinyl. Examples of tricyclic or tetracyclic heteroaryl include, but are not limited to, acridinyl, benzindolyl, carbazolyl, dibenzofuranyl, xanthyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of e.g., deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "heteroarylene", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, 6- to 9-membered, 6- to 10-membered, or 6- to 20-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic, tetracyclic or polycyclic heteroarylene groups, which contain one aromatic ring having one or more (e.g., 1-4, 1-3, 1-2, or 1) heteroatoms independently selected from O, S and N, and the remaining ring(s) which may be a saturated, partially unsaturated or aromatic ring and can be carbocyclic ring or contain one or more (e.g., 1-4, 1-3, 1-2, or 1) heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, chromanylene, and 6,7-dihydrothieno[3,2-d]pyrimidinylene. Examples of tricyclic or polycyclic heteroarylene groups include, but are not limited to, acridinylene, benzindolylene, carbazolylene, dibenzofuranylene, xanthylene, 5,6,7,8-tetrahydrobenzo[4,5]thieno[2,3-d]pyrimidinylene, and 6,7-dihydro-5H-cyclopenta[4,5]thieno[2,3-d]pyrimidinylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of e.g., deuterium, hydroxyl, amino, mercapto, nitro, halogen, cyano, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "aryl", used alone or in combination, refers to a monovalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, from 6 to 10, or from 6 to 20) carbon atoms and optionally one or more fused rings, such as phenyl group, naphthyl group, and fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, tri-, or poly-substituted with a substituent(s) optionally selected from e.g., deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "arylene", used alone or in combination, refers to a divalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, from 6 to 10, or from 6 to 20) carbon atoms and optionally one or more fused rings, such as phenylene (e.g., 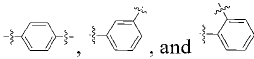 ), naphthylene, or fluorenylene. As used herein, the "arylene" is optionally substituted. A substituted arylene refers to an arylene group substituted one or more times (e.g., 1-4, 1-3, or 1-2 times) with a substituent(s). For example, arylene is mono-, di-, tri-, or poly-substituted with a substituent(s) optionally selected from e.g., deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "cycloalkyl", used alone or in combination, refers to a saturated or partially unsaturated (i.e., containing one or more double bonds, but not having a fully conjugated π-electron system) monocyclic or bicyclic or tricyclic or polycyclic cyclic hydrocarbon radical, containing a number of carbon atoms including, but not limited to, from 3 to 30 carbon atoms (i.e., C₃₋₃₀ cycloalkyl), or from 3 to 25 carbon atoms (i.e., C₃₋₂₅ cycloalkyl), or from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms ( i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl), or from 4 to 20 carbon atoms ( i.e., C₄₋₂₀ cycloalkyl), or from 4 to 15 carbon atoms ( i.e., C₄₋₁₅ cycloalkyl), or from 4 to 12 carbon atoms (i.e., C₄₋₁₂ cycloalkyl), or from 4 to 10 carbon atoms (i.e., C₄₋₁₀ cycloalkyl). The term "cycloalkyl" includes monocyclic, bicyclic, tricyclic, and polycyclic cyclic hydrocarbon radical having from 3 to 30 carbon atoms. Examples of the term "cycloalkyl" include, but are not limited to, monocyclic cycloalkyl, bridged cycloalkyl (e.g., C₅₋₃₀ bridged cycloalkyl or C₅₋₂₀ bridged cycloalkyl, C₅₋₁₅ bridged cycloalkyl, and C₇₋₁₅ bridged cycloalkyl), fused cycloalkyl (e.g., C₅₋₃₀ fused cycloalkyl, C₅₋₂₀ fused cycloalkyl, C₅₋₁₅ fused cycloalkyl, C₆₋₃₀ fused cycloalkyl, C₆₋₂₀ fused cycloalkyl, C₆₋₁₅ fused cycloalkyl, C₇₋₃₀ fused cycloalkyl, C₇₋₂₀ fused cycloalkyl, C₇₋₁₅ fused cycloalkyl, and C₈₋₁₅ fused cycloalkyl), and spiro-cycloalkyl (e.g., C₅₋₃₀ spiro-cycloalkyl, C₅₋₂₀ spiro-cycloalkyl, C₅₋₁₅ spiro-cycloalkyl, C₆₋₃₀ spiro-cycloalkyl, C₆₋₂₀ spiro-cycloalkyl, C₆₋₁₅ spiro-cycloalkyl, C₇₋₃₀ spiro-cycloalkyl, C₇₋₂₀ spiro-cycloalkyl, C₇₋₁₅ spiro-cycloalkyl, and C₈₋₁₅ spiro-cycloalkyl). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Examples of bridged cycloalkyl, fused cycloalkyl and spiro-cycloalkyl groups include, but are not limited to, decalinyl, octahydropentalenyl, octahydro-1H-indenyl, C₅₋₂₀ spiro-cycloalkyl, C₅₋₁₅ spiro-cycloalkyl, adamantanyl, noradamantanyl, bornyl, and norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents independently selected from e.g., deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof. Examples of the term "C₃₋₆ cycloalkyl" include, but are not limited to, cyclopropyl, cyclobutyl, cyclopentyl, cyclopentenyl, and cyclohexyl.

As used herein, the term "C_{x-y} spiro-cycloalkyl" (x and y each being an integer), used alone or in combination, refers to a spiro-cycloalkyl group containing from x to y carbon atoms. As used herein, the term "C₅₋₃₀ spiro-cycloalkyl", used alone or in combination, refers to a spiro-cycloalkyl group containing from 5 to 30 carbon atoms (e.g., but not limited to, 5-20, 5-15, 7-20, 7-15, 5-11, 5-10, and 7-9 carbon atoms). The term "C₅₋₃₀ spirocycloalkyl" includes "C₅₋₂₀ spirocycloalkyl", "C₅₋₁₅ spirocycloalkyl", "C₇₋₁₅ spirocycloalkyl", and "C₇₋₂₀ spirocycloalkyl", and representative examples of which include, but are not limited to, spiro[3.3]heptanyl, spiro[2.5]octanyl, spiro[3.5]nonanyl, spiro[3.5]nonenyl, spiro[4.4]nonanyl, spiro[4.5]decanyl, spiro[4.5]decenyl, and spiro[5.5]undecanyl. The "C₅₋₃₀ spiro-cycloalkyl" is optionally further substituted with one or more (e.g., 1-10, 1-6, 1-5, 1-4, or 1-3) substituents selected from the group consisting of e.g., deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "C_{x-y} bridged cycloalkyl" (where x and y are integers) used alone or in combination, refers to a bridged cycloalkyl group containing x to y carbon atoms. In the present invention, the term "C₅₋₃₀ bridged cycloalkyl" used alone or in combination, refers to a bridged cycloalkyl group containing from 5 to 30 carbon atoms (e.g., but not limited to, 5-20, 6-20, 7-20, 5-15, 7-15, 5-11, 5-10, and 7-9 carbon atoms). The term "C₅₋₃₀ bridged cycloalkyl" includes "C₅₋₂₀ bridged cycloalkyl", "C₆₋₂₀ bridged cycloalkyl", "C₇₋₂₀ bridged cycloalkyl", "C₅₋₁₅ bridged cycloalkyl", and "C₇₋₁₅ bridged cycloalkyl", representative examples of which include, but are not limited to, adamantanyl, noradamantanyl, bornyl, norbornanyl (also named as bicyclo[2.2.1]heptanyl by the IUPAC system), 2-oxobicyclo[2.2.1]heptanyl, bicyclo[2.2.1]heptenyl, and cubanyl. Said "C₅₋₃₀ bridged cycloalkyl" may be optionally substituted with 1 to 10 (e.g., 1-6, 1-5, 1-4, or 1-3) substituents selected from the group consisting of e.g., deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "cycloalkylene", used alone or in combination, refers to a saturated or partially unsaturated (i.e., containing one or more double bonds, but not having a fully conjugated π-electron system) monocyclic, bicyclic, tricyclic or polycyclic divalent cyclic hydrocarbon radical, containing a number of carbon atoms including, but not limited to, from 3 to 30 carbon atoms (i.e., C₃₋₃₀ cycloalkylene), or from 3 to 25 carbon atoms (i.e., C₃₋₂₅ cycloalkylene), from 3 to 20 carbon atoms (i.e., C₃₋₂₀ cycloalkylene), or from 3 to 15 carbon atoms (i.e., C₃₋₁₅ cycloalkylene), or from 3 to 12 carbon atoms (i.e., C₃₋₁₂ cycloalkylene), or from 3 to 11 carbon atoms (i.e., C₃₋₁₁ cycloalkylene), or from 3 to 10 carbon atoms (i.e., C₃₋₁₀ cycloalkylene), or from 3 to 8 carbon atoms ( i.e., C₃₋₈ cycloalkylene), or from 3 to 7 carbon atoms (i.e., C₃₋₇ cycloalkylene), or from 3 to 6 carbon atoms (i.e., C₃₋₆ cycloalkylene), or from 4 to 20 carbon atoms (i.e., C₄₋₂₀ cycloalkylene), or from 4 to 15 carbon atoms ( i.e., C₄₋₁₅ cycloalkylene), or from 4 to 12 carbon atoms (i.e., C₄₋₁₂ cycloalkylene), or from 4 to 10 carbon atoms (i.e., C₄₋₁₀ cycloalkylene). The term "cycloalkylene" includes monocyclic, bicyclic, tricyclic and polycyclic divalent cyclic hydrocarbon radical having from 3 to 30 carbon atoms. Examples of the term "cycloalkylene" include, but are not limited to, monocyclic cycloalkylene, bridged cycloalkylene (e.g., C₅₋₃₀ bridged cycloalkylene, C₅₋₂₀ bridged cycloalkylene, C₅₋₁₅ bridged cycloalkylene, and C₇₋₁₅ bridged cycloalkylene), fused cycloalkylene (e.g., C₅₋₃₀ fused cycloalkylene, C₅₋₂₀ fused cycloalkylene, C₅₋₁₅ fused cycloalkylene, C₆₋₃₀ fused cycloalkylene, C₆₋₂₀ fused cycloalkylene, C₆₋₁₅ fused cycloalkylene, C₇₋₃₀ fused cycloalkylene, C₇₋₂₀ fused cycloalkylene, C₇₋₁₅ fused cycloalkylene, and C₈₋₁₅ fused cycloalkylene), and spiro-cycloalkylene (e.g., C₅₋₃₀ spiro-cycloalkylene, C₅₋₂₀ spiro-cycloalkylene, C₅₋₁₅ spiro-cycloalkylene, C₆₋₃₀ spiro-cycloalkylene, C₆₋₂₀ spiro-cycloalkylene, C₆₋₁₅ spiro-cycloalkylene, C₇₋₃₀ spiro-cycloalkylene, C₇₋₂₀ spiro-cycloalkylene, C₇₋₁₅ spiro-cycloalkylene, and C₈₋₁₅ spiro-cycloalkylene). Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Examples of bridged cycloalkylene, fused cycloalkylene and spiro-cycloalkylene groups include, but are not limited to, decalinylene, octahydropentalenylene, octahydro-1H-indenylene, 2,3-dihydro-1H-indenylene, C₅₋₂₀ spiro-cycloalkylene (e.g., C₅₋₁₅ spiro-cycloalkylene), adamantanylene, noradamantanylene, and norbornylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more (e.g., 1-5, 1-4, 1-3, 1-2, or 1) substituents independently selected from the group consisting of e.g., deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "C_{x-y} spiro-cycloalkylene" (x and y each being an integer), used alone or in combination, refers to a spiro-cycloalkylene group containing from x to y carbon atoms. As used herein, the term "C₅₋₃₀ spiro-cycloalkylene", used alone or in combination, refers to a spiro-cycloalkylene group containing from 5 to 30 carbon atoms (e.g., 5-20, 5-15, 7-20, 7-15, 5-11, 5-10, or 7-9 carbon atoms). The term "C₅₋₃₀ spiro-cycloalkylene" includes "C₅₋₂₀ spiro-cycloalkylene", "C₅₋₁₅ spiro-cycloalkylene", "C₇₋₁₅ spiro-cycloalkylene", and "C₇₋₂₀ spiro-cycloalkylene", representative examples of which include, but are not limited to, spiro[3.3]heptylene, spiro[2.5]octylene, spiro[3.5]nonylene, spiro[3.5]nonenylene, spiro[4.4]nonylene, spiro[4.5]decylene, spiro[4.5]decenylene, and spiro[5.5]undecylene. The "C₅₋₃₀ spiro-cycloalkylene" is optionally further substituted with one or more (e.g., 1-10, 1-6, 1-5, 1-4, or 1-3) substituents independently selected from the group consisting of e.g., deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "C_{x-y} bridged cycloalkylene" (where x and y are integers) used alone or in combination, refers to a bridged cycloalkylene group containing x to y carbon atoms. In the present invention, the term "C₅₋₃₀ bridged cycloalkylene" used alone or in combination, refers to a bridged cycloalkylene group containing 5 to 30 (e.g., but not limited to, 5-20, 6-20, 7-20, 5-15, 7-15, 5-11, 5-10, and 7-9) carbon atoms. The term "C₅₋₃₀ bridged cycloalkylene" includes "C₅₋₂₀ bridged cycloalkylene", "C₆₋₂₀ bridged cycloalkylene", "C₇₋₂₀ bridged cycloalkylene", "C₅₋₁₅ bridged cycloalkylene", and "C₇₋₁₅ bridged cycloalkylene", representative examples of which include, but are not limited to, adamantanylene, noradamantanylene, bornylene, bicyclo[2.2.1]heptanylene, 2-oxobicyclo[2.2.1]heptanylene, bicyclo[2.2.1]heptenylene, and cubanylene. Said "C₅₋₃₀ bridged cycloalkylene" may be optionally substituted with one or more (e.g., 1-10, 1-6, 1-5, 1-4, or 1-3) substituents independently selected from the group consisting of deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "heterocyclyl" or "heterocyclic group", used alone or in combination, refers to a 4- to 30-membered (e.g., 4- to 30-membered, 4- to 25-membered, 4- to 20-membered, 4- to 15-membered, 4-to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated π-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl group include, but not limited to, monocyclic heterocyclyl (e.g., 4- to 30-membered monocyclic heterocyclyl, and 4- to 20-membered monocyclic heterocyclyl), bridged heterocyclyl (e.g., 5- to 30-membered bridged heterocyclyl, 5- to 20-membered bridged heterocyclyl, 7- to 20-membered bridged heterocyclyl, and 7- to 15-membered bridged heterocyclyl), fused heterocyclyl (e.g., 5- to 30-membered fused heterocyclyl, and 5- to 20-membered fused heterocyclyl), and spiro-heterocyclyl groups (e.g., 5- to 30-membered spiro-heterocyclyl, and 5- to 20-membered spiro-heterocyclyl). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptanyl), and diazacyclooctyl. Examples of bridged heterocyclyl, fused heterocyclyl and spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, quinuclidinyl, octahydro-1H-indolyl, pyrrolidinocyclopropyl, cyclopentylaziridinyl, pyrrolidinocyclobutyl, pyrrolidinopyrrolidinyl (e.g., hexahydropyrrolo[3,4-c]pyrrolyl), pyrrolidinopiperidinyl, pyrrolidinopiperazinyl, pyrrolidinomorpholinyl, piperidinomorpholinyl, and azaspirocycloalkyl (e.g., 5- to 20-membered azaspirocycloalkyl, such as 3-azaspiro[5.5]undecan-3-yl and 7-azaspiro[3.5]nonyl). The heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "nitrogen-containing heterocyclyl", used alone or in combination, refers to 4-to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated π-electron system) monocyclic, bicyclic, tricyclic or polycyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclyl group include, but are not limited to, nitrogen-containing monocyclic heterocyclyl (e.g., 4- to 30-membered nitrogen-containing monocyclic heterocyclyl, and 4- to 20-membered nitrogen-containing monocyclic heterocyclyl), nitrogen-containing bridged heterocyclyl (e.g., 5- to 30-membered nitrogen-containing bridged heterocyclyl, 5- to 20-membered nitrogen-containing bridged heterocyclyl, 5- to 15-membered nitrogen-containing bridged heterocyclyl, 7- to 20-membered nitrogen-containing bridged heterocyclyl, and 7- to 15-membered nitrogen-containing bridged heterocyclyl), nitrogen-containing fused heterocyclyl (e.g., 5- to 30-membered nitrogen-containing fused heterocyclyl, and 5- to 20-membered nitrogen-containing fused heterocyclyl), and nitrogen-containing spiro-heterocyclyl groups (e.g., 5-to 30-membered nitrogen-containing spiro-heterocyclyl, and 5- to 20-membered nitrogen-containing spiro-heterocyclyl). Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. Examples of nitrogen-containing bridged heterocyclyl, nitrogen-containing fused heterocyclyl and nitrogen-containing spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, quinuclidinyl, octahydro-1H-indolyl, pyrrolidinocyclopropyl, cyclopentylaziridinyl, pyrrolidinocyclobutyl, pyrrolidinopyrrolidinyl (e.g., hexahydropyrrolo[3,4-c]pyrrolyl), pyrrolidinopiperidinyl, pyrrolidinopiperazinyl, pyrrolidinomorpholinyl, piperidinomorpholinyl, and azaspirocycloalkyl (e.g., 5- to 20-membered azaspirocycloalkyl, such as 3-azaspiro[5.5]undecan-3-yl and 7-azaspiro[3.5]nonyl). The nitrogen-containing heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "heterocyclylene", used alone or in combination, refers to a 4- to 30-membered saturated or partially unsaturated (i.e., containing one or more double bonds, but not having a fully conjugated π-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but are not limited to, monocyclic heterocyclylene (e.g., 4- to 30-membered monocyclic heterocyclylene, and 4- to 20-membered monocyclic heterocyclylene), bridged heterocyclylene (e.g., 5- to 30-membered bridged heterocyclylene, 5- to 20-membered bridged heterocyclylene, 7- to 20-membered bridged heterocyclylene, and 7- to 15-membered bridged heterocyclylene), fused heterocyclylene (e.g., 5- to 30-membered fused heterocyclylene, and 5- to 20-membered fused heterocyclylene), and spiro-heterocyclylene groups (e.g., 5- to 30-membered spiro-heterocyclylene, and 5-to 20-membered spiro-heterocyclylene). Representative examples of monocyclic heterocyclylene include, but are not limited to, azetidinylene, oxetanylene, pyrrolidinylene, imidazolidylene, pyrazolidylene, tetrahydrofuranylene, dihydropyranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azepanylene, azacyclooctylene, dioxacyclohexylene, diazacycloheptanylene (e.g., 1,4-diazacycloheptanylene, 4,5-diazacycloheptanylene, 1,3-diazacycloheptanylene), and diazacyclooctylene. Examples of the bridged heterocyclylene, fused heterocyclylene and spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, quinuclidinylene, octahydro-1H-indolylene, pyrrolidinocyclopropylene, cyclopentylaziridinylene, pyrrolidinocyclobutylene, pyrrolidinopyrrolidinylene (e.g., hexahydropyrrolo[3,4-c]pyrrolylene), pyrrolidinopiperidinylene, pyrrolidinopiperazinylene, pyrrolidinomorpholinylene, piperidinomorpholinylene, and azaspirocycloalkylene (e.g., 5- to 20-membered azaspirocycloalkylene, such as 3-azaspiro[5.5]undecanylene and 7-azaspiro[3.5]nonylene). The heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "nitrogen-containing heterocyclylene", used alone or in combination, refers to a 4- to 30-membered (e.g., 4- to 30-membered, 4- to 25-membered, 4- to 20-membered, 4- to 15-membered, 4-to 14-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5-to 20-membered, or 5- to 15-membered) saturated or partially unsaturated (i.e., containing one or more double bonds, but not having a fully conjugated π-electron system) monocyclic, bicyclic, tricyclic or polycyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclylene group include, but are not limited to, nitrogen-containing monocyclic heterocyclylene (e.g., 4- to 30-membered nitrogen-containing monocyclic heterocyclylene, and 4- to 20-membered nitrogen-containing monocyclic heterocyclylene), nitrogen-containing bridged heterocyclylene (e.g., 5- to 30-membered nitrogen-containing bridged heterocyclylene, 5- to 20-membered nitrogen-containing bridged heterocyclylene, 7- to 20-membered nitrogen-containing bridged heterocyclylene, and 7- to 15-membered nitrogen-containing bridged heterocyclylene), nitrogen-containing fused heterocyclylene (e.g., 5- to 30-membered nitrogen-containing fused heterocyclylene, and 5- to 20-membered nitrogen-containing fused heterocyclylene), and nitrogen-containing spiro-heterocyclylene (e.g., 5- to 30-membered nitrogen-containing spiro-heterocyclylene, and 5- to 20-membered nitrogen-containing spiro-heterocyclylene). Representative examples of the nitrogen-containing monocyclic heterocyclylene include, but are not limited to, azetidinylene, pyrrolidinylene, imidazolidylene, pyrazolidylene, oxazolidinylene, thiazolidinylene, piperidinylene, piperazinylene, morpholinylene, thiomorpholinylene, azepanylene, azacyclooctylene, diazacycloheptanylene (e.g., 1,4-diazacycloheptanylene, 4,5-diazacycloheptanylene, 1,3-diazacycloheptanylene), and diazacyclooctylene. Examples of nitrogen-containing bridged heterocyclylene, nitrogen-containing fused heterocyclylene, and nitrogen-containing spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, quinuclidinylene, octahydro-1H-indolylene, pyrrolidinocyclopropylene, cyclopentylaziridinylene, pyrrolidinocyclobutylene, pyrrolidinopyrrolidinylene (e.g., hexahydropyrrolo[3,4-c]pyrrolylene), pyrrolidinopiperidinylene, pyrrolidinopiperazinylene, pyrrolidinomorpholinylene, piperidinomorpholinylene, and azaspirocycloalkylene (e.g., 5- to 20-membered azaspirocycloalkylene, such as 3-azaspiro[5.5]undecanylene and 7-azaspiro[3.5]nonanylene). The nitrogen-containing heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, and any combination thereof.

As used herein, the term "monoazabridged-cycloalkylene", used alone or in combination, refers to 5- to 30-membered (e.g., 5- to 30-membered, 5- to 25-membered, 5- to 20-membered, 5- to 15-membered, 5- to 14-membered, 5- to 12-membered, 5- to 11-membered, 5- to 10-membered, 5- to 9-membered, 5- to 8-membered, 5- to 7-membered, 5- to 6-membered, 7- to 30-membered, 7- to 20-membered, or 7- to 15-membered) saturated or partially unsaturated (i.e., containing one or more double bonds, but not having a fully conjugated π-electron system) bicyclic, tricyclic or polycyclic bivalent bridged cyclic hydrocarbon group containing one nitrogen atom. Examples of the "monoazabridged-cycloalkylene" include, but are not limited to, 6-azabicyclo[3.1.1]heptanylene, 3-azabicyclo[3.2.1]octanylene, and quinuclidinylene. The "monoazabridged-cycloalkylene" may be unsubstituted or substituted as explicitly defined (e.g., substituted with one or more substituents), wherein the substituents may be selected from deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, or any combination thereof. The number of substituents is in principle not limited in any way, or is automatically limited by the size of the building unit. For example, the number of substituents may be one or more, such as 1-20, 1-15, 1-10, 1-6, 1-4, 1-3, 2-6, or 1.

As used herein, the term "monoazaspirocycloalkylene", used alone or in combination, refers to 5- to 30-membered (e.g., 5- to 30-membered, 5- to 25-membered, 5- to 20-membered, 5- to 15-membered, 5- to 14-membered, 5- to 12-membered, 5- to 11-membered, 5- to 10-membered, 5- to 9-membered, 5- to 8-membered, 5- to 7-membered, 5- to 6-membered, 7- to 30-membered, 7- to 20-membered, or 7- to 15-membered) saturated or partially unsaturated (i.e., containing one or more double bonds, but not having a fully conjugated π-electron system) bicyclic, tricyclic or polycyclic bivalent spirocycloalkyl group containing one nitrogen atom. Examples of the "monoazaspirocycloalkylene" include, but are not limited to, 3-azaspiro[5.5]undecanylene and 7-azaspiro[3.5]nonanylene. The "monoazaspirocycloalkylene" may be unsubstituted or substituted as explicitly defined (e.g., substituted with one or more substituents), wherein the substituents may be selected from deuterium, hydroxyl, amino, mercapto, nitro, halogen, cyano, oxo, optionally deuterated C₁₋₆ alkyl, halogenated C₁₋₆ alkyl, optionally deuterated C₃₋₆ cycloalkyl, optionally deuterated C₁₋₆ alkoxy, halogenated C₁₋₆ alkoxy, optionally deuterated C₁₋₆ alkyl-NH-, NH₂-C₁₋₆ alkylene, optionally deuterated C₁₋₆ alkyl-NHC(O)-, optionally deuterated C₁₋₆ alkyl-C(O)NH-, C₂₋₆ alkynyl, C₂₋₆ alkenyl, or any combination thereof. The number of substituents is in principle not limited in any way, or is automatically limited by the size of the building unit. For example, the number of substituents may be one or more, such as 1-20, 1-15, 1-10, 1-6, 1-4, 1-3, 2-6, or 1.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynyl include C₂₋₈ alkynyl, C₂₋₆ alkynyl or C₂₋₄ alkynyl, representative examples of which include, but are not limited to, ethynyl, 1-propynyl, 1-butynyl, and 1,3-diynyl.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenyl group include C₂₋₈ alkenyl, C₂₋₆ alkenyl or C₂₋₄ alkenyl, representative examples of which include, but are not limited to, vinyl (e.g., CH₂=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane) has a definition known to those skilled in the art. As used herein, "camphanyl" or "bornyl" refers to a monovalent group of bornane, i.e., the group remaining after removal of any one hydrogen atom from bornane. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

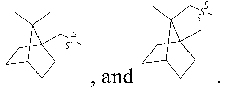

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after removal of any one hydrogen atom from bicyclo[2.2.1]heptane. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, and bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after removal of any one hydrogen atom from bicyclo[2.2.1]heptene. Representative examples of "bicyclo[2.2.1]heptenyl" include, but are not limited to, bicyclo[2.2.1]hept-5-en-2-yl, bicyclo[2.2.1]hept-5-en-3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is e.g., as follows: 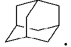 As used herein, "adamantanyl" refers to a monovalent group of adamantane, that is, the group remaining after removal of any one hydrogen atom from adamantane. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, the term "noradamantane" (also known as octahydro-2,5-methanopentalene) has the definition known to those skilled in the art, and its structural formula is, for example, as shown below: 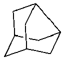 or 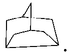 As used herein, "noradamantanyl" refers to a monovalent group derived from noradamantane, i.e., the group remaining after removal of any one hydrogen atom from noradamantane. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl, and 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (the corresponding English chemical name is adamantan-1-amine or Tricyclo[3.3.1.1^{3,7}]decan-1-amine; CAS No.: 768-94-5), with the following structural formula:

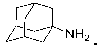

Salts or pharmaceutically acceptable salts, enantiomers, stereoisomers, solvates, prodrugs, polymorphs of the compounds of the present disclosure (including compounds of Formula (I), Formula (I-1), Formula (I-2), Formula (I-3), Formula (I-4), Formula (I-5), Formula (II-1), Formula (II-2), Formula (II-3), Formula (II-4), or Formula (I')) are also encompassed within the scope of the present disclosure.

In all embodiments of the present disclosure, the salts or pharmaceutically acceptable salts of the compounds of Formula (I) (including compounds of Formula (I-1), Formula (I-2), Formula (I-3), Formula (I-4), Formula (I-5), Formula (II-1), Formula (II-2), Formula (II-3), Formula (II-4), or Formula (I')) refer to non-toxic inorganic or organic acid and/or base addition salts. Examples include: sulfates, hydrohalides (including hydrochlorides, hydrobromides), citrates, maleates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, glycolate, trifluoroacetate, terephthalate, and 4-methylbenzenesulfonate, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are non-superimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, the term "prodrug" refers to a compound that can be converted in vivo, such as by hydrolysis in the blood, into its biologically active form. When a prodrug is administered to a patient, it releases the parent compound in the body. Prodrugs are generally prepared by modifying functional groups, and such modifications can be removed by conventional operations (e.g., acid/base hydrolysis) or by cleavage in vivo to release the parent drug. Prodrugs include, for example, forms in which the hydroxyl, amino, or mercapto groups of the compounds of the present invention are combined with any group that, when administered to a patient, can be cleaved to re-form the hydroxyl, amino, or mercapto groups. Accordingly, representative examples of prodrugs include, but are not limited to, acetate/amide, formate/amide, tert-butoxycarbonyl (Boc), and benzoate/amide derivatives of the hydroxyl, mercapto, and amino functional groups of the compounds of Formula (I) and Formula (II). In addition, in the case of carboxylic acids (-COOH), esters such as methyl esters, ethyl esters, and the like can be used. These esters may themselves be active and/or may be hydrolyzed under physiological conditions in the human body. Suitable pharmaceutically acceptable, in vivo hydrolyzable ester groups include those that are readily cleaved in the human body to release the parent acid or a salt thereof.

As used herein, "p-menthane" has the definitions known to those skilled in the art, and its structural formula is e.g., as follows: 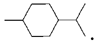 As used herein, "p-menthanyl" refers to a monovalent group ofp-menthane, that is, the group remaining after removal of any one hydrogen atom from p-menthane. Representative examples of "p-menthanyl" include, but are not limited to, 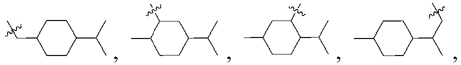 and

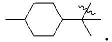

As used herein, "m-menthane" has the definitions known to those skilled in the art, and its structural formula is e.g., as follows: 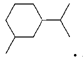 As used herein, "m-menthanyl" refers to a monovalent group of m-menthane, that is, the group remaining after removal of any one hydrogen atom from m-menthane. Representative examples of "m-menthanyl" include, but are not limited to,

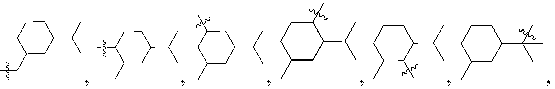

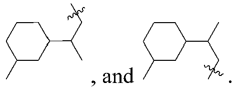

As used herein, "Quinuclidine" (also known as 1-azabicyclo[2.2.2]octane) has the definitions known to those skilled in the art, and its structural formula is e.g., as follows: 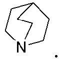 As used herein, "quinuclidinyl" refers to a monovalent group of Quinuclidine, that is, the group remaining after any hydrogen in Quinuclidine is removed. Representative examples of "quinuclidinyl" include, but are not limited to,

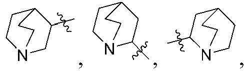

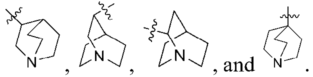

### Description of the Drawings

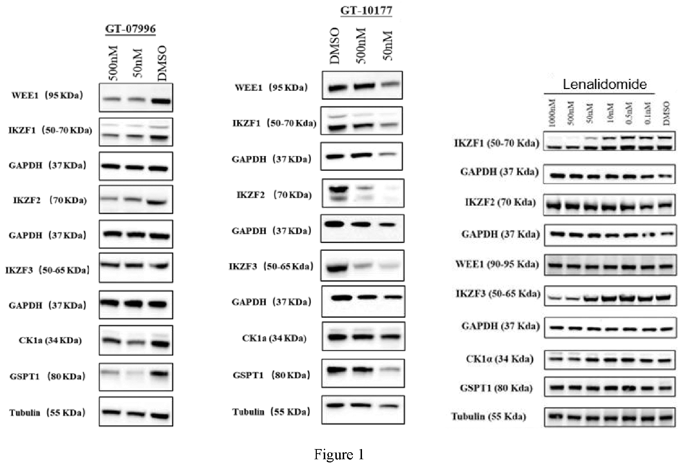

FIG. 1 shows the Western Blot results of target substrate protein degradation induced by the compounds of the present invention in hPBMCs.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:
- AcOH: acetic acid
- Boc: t-Butyloxy carbonyl
- DCM: dichloromethane
- DIEA or DIPEA: N, N-diisopropylethylamine
- DMF: N,N-dimethylformamide
- DMSO: dimethyl sulfoxide
- EA: ethyl acetate
- ESI: electrospray ionization
- equiv: equivalent
- EtOH: ethanol
- EtOAc: ethyl acetate
- HPLC: high performance liquid chromatography
- HRMS: high resolution mass spectrometry
- LC-MS: liquid chromatography-mass spectrometry
- LRMS: low resolution mass spectrometry
- LC: liquid chromatography
- Me: methyl
- MeCN: acetonitrile
- MeOH: methanol
- MS: mass spectrometry
- MsCl: methanesulfonyl chloride
- MsO-: methanesulfonyloxy
- Ms₂O: methanesulfonic anhydride
- ¹H NMR: Proton nuclear magnetic resonance
- MeO-: methoxy
- ONs: o-nitrobenzenesulfonyl
- rt: room temperature
- tBu: tert-butyl
- TEA: triethylamine
- TFA: trifluoroacetic acid
- T_{f}O-: trifluoromethanesulfonate
- TLC: thin layer chromatography
- TMS: tetramethylsilane
- TsO-: tosyloxy
- Xantphos: 4,5-bis(diphenylphosphino)-9,9-dimethylxanthene
- X-Phos: 2-dicyclohexylphosphino-2',4',6'-triisopropylbiphenyl

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). LC-MS spectra were recorded on a Sciex API 2000 mass spectrometer equipped with an Agilent 1100 binary pump, DAD and ELSD detectors, or on an Agilent 1260-6125B single quadrupole LC-MS system equipped with an Agilent 1260 quaternary pump, DAD and ELSD detectors. HPLC preparative purification was performed using a SHIMADZU LC-20AP system. HPLC purity was determined using either a SHIMADZU LC-30AP or Waters 1525 system. Supercritical fluid chromatography (SFC) chiral resolution/separation was performed on a SHIMADZU LC-20AP instrument equipped with a Daicel CHIRALPAK^{®}IE column. All reactions were carried out under ambient atmosphere unless otherwise specified. Reaction progress was monitored by TLC or LC-MS.

Solvents and reagents are processed as follows: DCM, DMF, anhydrous EtOH, and anhydrous MeOH used in the reactions were purchased from Sinopharm Group; preparative-grade CH₃CN and deionized water were used for HPLC preparation. Unless otherwise specified, all other raw materials, reagents, and chemicals were commercially available or could be synthesized according to methods known in the art.

The materials and reagents used in the following examples, unless otherwise specified, were either commercially available and used directly, or could be synthesized using or according to methods known in the art.

### General synthesis methods

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) of the present disclosure according to routine techniques in the art.

### Synthesis Scheme 1:

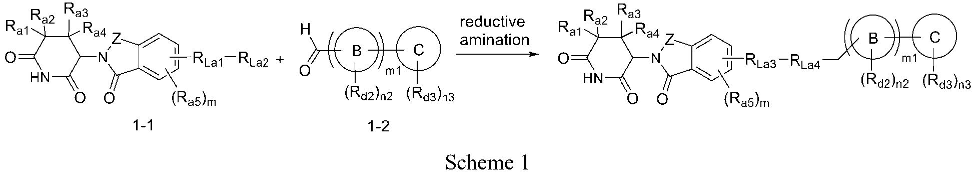

In Scheme 1, Z, Rₐ₁, Rₐ₂, Rₐ₃, Rₐ₄, (Rₐ₅)ₘ, ring B, (R_{d2})ₙ₂, m1, ring C, and (R_{d3})ₙ₃ are as defined in the compounds of Formula (I) of the present disclosure and the respective sub-embodiments thereof. In Scheme 1,
(i) R_{La1} and R_{La3} represent N(R_{w}), R_{La2} represents 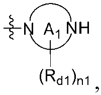 R_{La4} represents 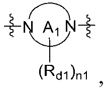 wherein R_{w}, ring A₁, and (R_{d1})ₙ₁ are as defined in the compounds of Formula (I) of the present disclosure and the respective sub-embodiments thereof; or
(ii) R_{La1} and R_{La3} represent 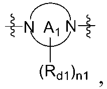 R_{La2} represents -NH₂, R_{La4} represents NH or -N=; or
(iii) R_{La1} and R_{La3} represents NH, R_{La2} represents -NH₂, R_{La4} represents NH or -N=; or
(iv) R_{La2} represents 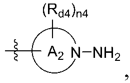 wherein ring A₂ and (R_{d4})ₙ₄ are as defined in the compounds of Formula (I) of the present disclosure and the respective sub-embodiments thereof, R_{La1} and R_{La3} represent 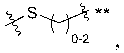 symbol ** indicates the point of attachment to ring A₂, and R_{La4} represents

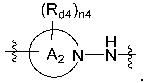

The reductive amination reaction in Scheme 1 can be carried out using conventional techniques and methods well known to those skilled in the art. For example, the reductive amination reaction can be conducted in the presence of sodium triacetoxyborohydride and N,N-dimethylformamide, or sodium cyanoborohydride and 1,2-dichloroethane, at a temperature ranging from room temperature to 80 °C (e.g., room temperature to 50 °C, 40 °C to 60 °C, 40 °C to 50 °C, or 50 °C to 60 °C). The molar ratio of substrate 1-1 to substrate 1-2 can be, for example, 1:1.1 to 3, 1:1.1 to 2, 1:1.1 to 1.5, 1:1.1 to 1.2, or 1:1.2 to 1.3, etc.

For example, the procedure of Scheme 1 can be as follows:
To a solution of substrate 1-1 (1.0 eq.) and aldehyde substrate 1-2 (2.0 eq.) in N,N-dimethylformamide was added AcOH (1 drop). The reaction mixture was stirred at 50 °C for 2 hours and then cooled to room temperature. To the reaction mixture was then added sodium triacetoxyborohydride (4.0 eq.), and the mixture was stirred for an additional 17 hours. After the completion of the reaction was confirmed by LC-MS, the reaction mixture was filtered, and the filtrate was purified by high-performance liquid chromatography (HPLC) to afford the target compound.

### Synthesis Scheme 2:

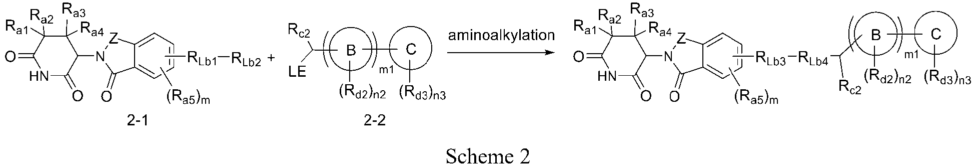

In Scheme 2, Z, Rₐ₁, Rₐ₂, Rₐ₃, Rₐ₄, (Rₐ₅)ₘ, R_{c2}, ring B, (R_{d2})ₙ₂, m1, ring C, and (R_{d3})ₙ₃ are as defined in the compounds of Formula (I) of the present disclosure and the respective sub-embodiments thereof. The group LE represents Cl, Br, I, OMs, OTs, or ONs. In Scheme 2,
(i) R_{Lb1} and R_{Lb3} represent NH, R_{Lb2} represents 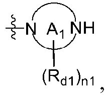 and R_{Lb4} represents 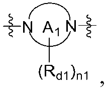 wherein ring A₁ and (R_{d1})ₙ₁ are as defined in the compounds of Formula (I) of the present disclosure and the respective sub-embodiments thereof; or
(ii) R_{Lb1} and R_{Lb3} represent NH, R_{Lb2} represents -NH₂, and R_{Lb4} represents NH; or
(iii) R_{Lb1} and R_{Lb3} represent 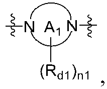 R_{Lb2} represents -NH₂, and R_{Lb4} represents NH, wherein ring A₁ and (R_{d1})ₙ₁ are as defined in the compounds of Formula (I) of the present disclosure and the respective sub-embodiments thereof; or
(iv) R_{Lb2} represents 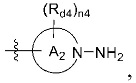 wherein ring A₂ and (R_{d4})ₙ₄ are as defined in the compounds of Formula (I) of the present disclosure and the respective sub-embodiments thereof, R_{Lb1} and R_{Lb3} represent 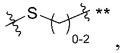 symbol ** indicates the point of attachment to ring A₂, and R_{Lb4} represents

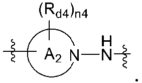

The aminoalkylation reaction in Scheme 2 can be carried out, for example, in the presence of DIEA and sodium iodide, or triethylamine and sodium iodide, at a temperature ranging from room temperature to 80 °C (e.g., 40 °C to 60 °C, 40 °C to 50 °C, or 50 °C to 60 °C). The molar ratio of substrate 2-1 to substrate 2-2 can be, for example, 1:1.1 to 2, 1:1.1 to 1.5, 1:1.1 to 1.2, or 1:1.2 to 1.3, etc.

For example, the procedure of Scheme 2 can be as follows:
To a solution of substrate 2-1 (1.0 eq.) and substrate 2-2 (1.2 eq.) in N,N-dimethylformamide were added triethylamine (3.0 eq.) and sodium iodide (1.0 eq.), and the reaction mixture was stirred at room temperature for 18 hours. After the completion of the reaction was confirmed by LC-MS, the reaction mixture was filtered, and the filtrate was purified by HPLC to afford the target compound.

### Synthesis Scheme 3:

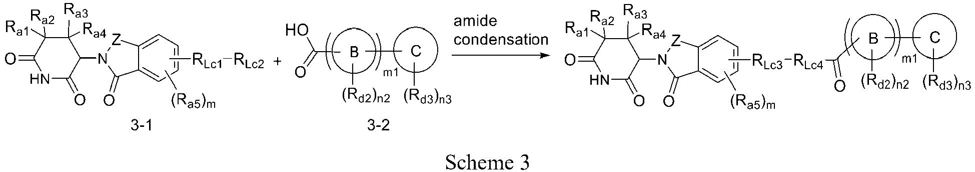

In Scheme 3, Z, Rₐ₁, Rₐ₂, Rₐ₃, Rₐ₄, (Rₐ₅)ₘ, ring B, (R_{d2})ₙ₂, m1, ring C, and (R_{d3})ₙ₃ are as defined in the compounds of Formula (I) of the present disclosure and the respective sub-embodiments thereof. In Scheme 3,
(i) R_{Lc1} and R_{Lc3} represent NH, R_{Lc2} represents 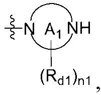 and R_{Lc4} represents 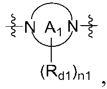 wherein ring A₁ and (R_{d1})ₙ₁ are as defined in the compounds of Formula (I) of the present disclosure and the respective sub-embodiments thereof; or
(ii) R_{Lc1} and R_{Lc3} represent NH, R_{Lc2} represents -NH₂, and R_{Lc4} represents NH; or
(iii) R_{Lc1} and R_{Lc3} represent 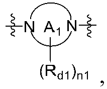 R_{Lc2} represents -NH₂, and R_{Lc4} represents NH; or
(iv) R_{Lc2} represents 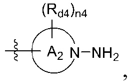 wherein ring A₂ and (R_{d4})ₙ₄ are as defined in the compounds of Formula (I) of the present disclosure and the respective sub-embodiments thereof, R_{Lc1} and R_{Lc3} represent 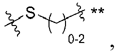 symbol ** indicates the point of attachment to ring A₂, and R_{Lc4} represents

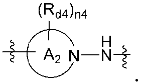

For example, the procedure of Scheme 3 can be as follows:
To a solution of substrate 3-1 (1.0 eq.) and substrate 3-2 (1.2 eq.) in N,N-dimethylformamide were added HATU (1.5 eq.) and triethylamine (3.0 eq.), and the reaction mixture was stirred at room temperature for 18 hours. After the completion of the reaction was confirmed by LC-MS, the reaction mixture was filtered, and the filtrate was purified by HPLC to afford the target compound.

### Synthesis Scheme 4: Urea Formation (Triphosgene)

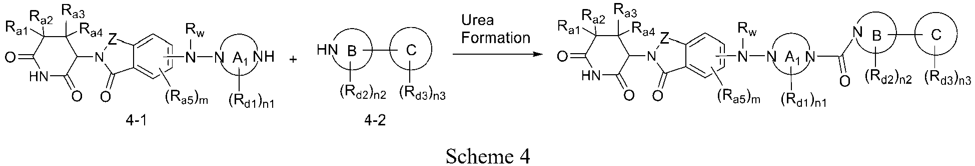

In Scheme 4, Z, Rₐ₁, Rₐ₂, Rₐ₃, Rₐ₄, (Rₐ₅)ₘ, R_{w}, ring A₁, (R_{d1})ₙ₁, (R_{d2})ₙ₂, m1, ring C, and (R_{d3})ₙ₃ are as defined in the compounds of Formula (I) of the present disclosure and the respective sub-embodiments thereof. Ring B represents nitrogen-containing heterocyclyl.

The procedure of Scheme 4 can be as follows:
To a solution of substrate 4-2 (1.0 eq.) and triphosgene (0.5 eq.) in dichloromethane was added triethylamine (3.0 eq.), and the reaction mixture was stirred at room temperature for 0.5 hours. A solution of substrate 4-1 in DMF was then added, and the resulting mixture was stirred for an additional 17 hours. After the completion of the reaction was confirmed by LC-MS, the reaction mixture was filtered, and the filtrate was purified by HPLC to give the target compound.

### Synthesis Scheme 5:

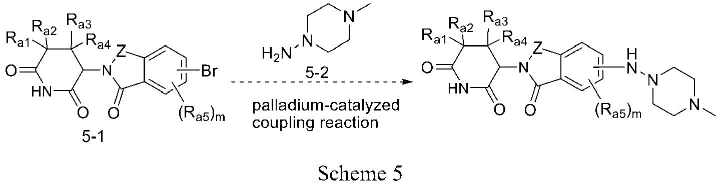

In Scheme 5, Z, Rₐ₁, Rₐ₂, Rₐ₃, Rₐ₄, and (Rₐ₅)ₘ are as defined in the compounds of Formula (I) of the present disclosure and the respective sub-embodiments thereof.

The palladium-catalyzed coupling reaction in Scheme 5 can be carried out by conventional techniques and methods well known to those skilled in the art.

General procedure for Scheme 5: to a solution of halide substrate 5-1 (1.0 eq.) and amine substrate 5-2 (1.2 eq.) in N,N-dimethylformamide were added palladium catalyst (0.2 eq.) and cesium carbonate (3.0 eq.). The reaction flask was purged with nitrogen three times, and the reaction mixture was stirred at 80°C for 18 hours. After the completion of the reaction was confirmed by LC-MS, the reaction mixture was filtered, and the filtrate was purified by HPLC (eluent (v/v): acetonitrile/0.05% aq. HCl = 5%-90%) to afford the target compound.

### Synthesis Scheme A1:

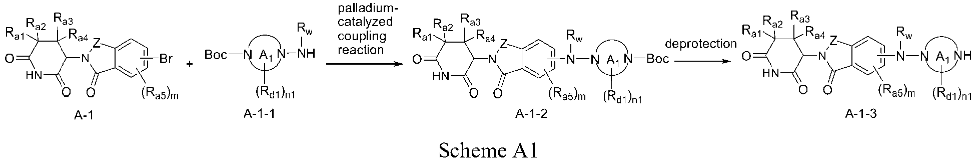

In Scheme A1, Z, Rₐ₁, Rₐ₂, Rₐ₃, Rₐ₄, (Rₐ₅)ₘ, R_{w}, ring A₁, and (R_{d1})ₙ₁ are as defined in the compounds of Formula (I) of the present disclosure and the respective sub-embodiments thereof.

The palladium-catalyzed coupling reaction and deprotection in Scheme A1 can be carried out by conventional techniques and methods well known to those skilled in the art.

The procedure of Scheme A1 can be as follows:
Step 1: At room temperature, to a 100 mL round-bottom flask were sequentially added compound A-1 (1.0 eq.), compound A-1-1 (1.2 eq.), cesium carbonate (2.0 eq.), palladium catalyst (0.025 eq.), and N,N-dimethylformamide. Under a nitrogen atmosphere, the reaction mixture was heated to 80 °C and stirred for 16 hours. After the completion of the reaction was confirmed by TLC, the reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound A-1-2 (gray solid, yield 73%).

Step 2: At room temperature, to a solution of compound A-1-2 in dichloromethane in a 100 mL round-bottom flask was added trifluoroacetic acid, and the reaction mixture was stirred for 1 hour. After the completion of the reaction was confirmed by TLC, the reaction mixture was concentrated under reduced pressure, and the resulting residue was lyophilized to afford compound A-1-3.

### Synthesis Scheme A2:

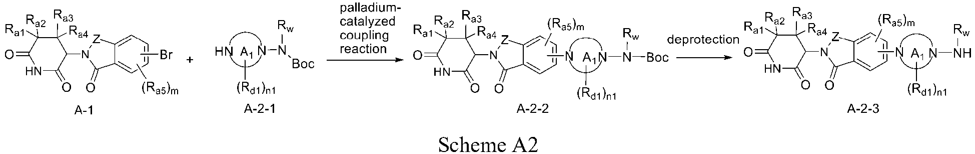

In Scheme A2, Z, Rₐ₁, Rₐ₂, Rₐ₃, Rₐ₄, (Rₐ₅)ₘ, R_{w}, ring A₁, and (R_{d1})ₙ₁ are as defined in the compounds of Formula (I) of the present disclosure and the respective sub-embodiments thereof.

The palladium-catalyzed coupling reaction and deprotection in Scheme A2 can be carried out by conventional techniques and methods well known to those skilled in the art.

The procedure of Scheme A2 can be as follows:
Step 1: At room temperature, to a 100 mL round-bottom flask were sequentially added compound A-1 (1.0 eq.), compound A-2-1 (1.2 eq.), cesium carbonate (2.0 eq.), palladium catalyst (0.025 eq.), and N,N-dimethylformamide. Under a nitrogen atmosphere, the reaction mixture was heated to 80 °C and stirred for 16 hours. After the completion of the reaction was confirmed by TLC, the reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound A-2-2 (gray solid, yield 73%).

Step 2: At room temperature, to a solution of compound A-2-2 in dichloromethane in a 100 mL round-bottom flask was added trifluoroacetic acid, and the reaction mixture was stirred for 1 hour. After the completion of the reaction was confirmed by TLC, the reaction mixture was concentrated under reduced pressure, and the resulting residue was lyophilized to afford compound A-2-3.

### Synthesis Scheme B1:

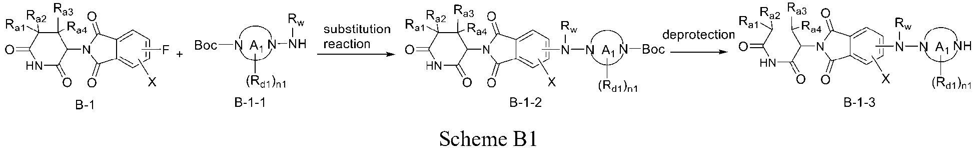

In Scheme B1, Rₐ₁, Rₐ₂, Rₐ₃, Rₐ₄, R_{w}, ring A₁, and (R_{d1})ₙ₁ are as defined in the compounds of Formula (I) of the present disclosure and the respective sub-embodiments thereof. X represents H or F.

The substitution reaction and deprotection in Scheme B1 can be carried out by conventional techniques and methods well known to those skilled in the art.

The procedure of Scheme B1 can be as follows:
Step 1: At room temperature, to a 100 mL round-bottom flask were sequentially added compound B-1 (1.0 eq.), compound B-1-1 (1.2 eq.), N,N-diisopropylethylamine (5.0 eq.), and dimethyl sulfoxide. Under a nitrogen atmosphere, the reaction mixture was heated to 130 °C and stirred for 16 hours. After the completion of the reaction was confirmed by TLC, the reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound B-1-2 (gray solid, yield 62%).

Step 2: At room temperature, to a solution of compound B-1-2 in dichloromethane in a 100 mL round-bottom flask was added trifluoroacetic acid, and the reaction mixture was stirred for 1 hour. After the completion of the reaction was confirmed by TLC, the reaction mixture was concentrated under reduced pressure, and the resulting residue was lyophilized to afford compound B-1-3.

### Synthesis Scheme B2:

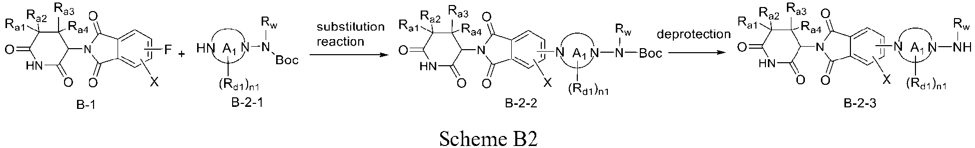

In Scheme B2, Rₐ₁, Rₐ₂, Rₐ₃, Rₐ₄, R_{w}, ring A₁, and (R_{d1})ₙ₁ are as defined in the compounds of Formula (I) of the present disclosure and the respective sub-embodiments thereof. X represents H or F.

The substitution reaction and deprotection in Scheme B2 can be carried out by conventional techniques and methods well known to those skilled in the art.

The procedure of Scheme B2 can be as follows:
Step 1: At room temperature, to a 100 mL round-bottom flask were sequentially added compound B-1 (1.0 eq.), compound B-2-1 (1.2 eq.), N,N-diisopropylethylamine (5.0 eq.), and dimethyl sulfoxide. Under a nitrogen atmosphere, the reaction mixture was heated to 130 °C and stirred for 16 hours. After the completion of the reaction was confirmed by TLC, the reaction mixture was cooled to room temperature, diluted with water, and extracted with ethyl acetate three times. The organic phases were combined. The combined organic phase was dried over anhydrous sodium sulfate and concentrated under reduced pressure. The residue was purified by column chromatography to afford compound B-2-2 (gray solid, yield 62%).

Step 2: At room temperature, to a solution of compound B-2-2 in dichloromethane in a 100 mL round-bottom flask was added trifluoroacetic acid, and the reaction mixture was stirred for 1 hour. After the completion of the reaction was confirmed by TLC, the reaction mixture was concentrated under reduced pressure, and the resulting residue was lyophilized to afford compound B-2-3.

### Synthesis Scheme C:

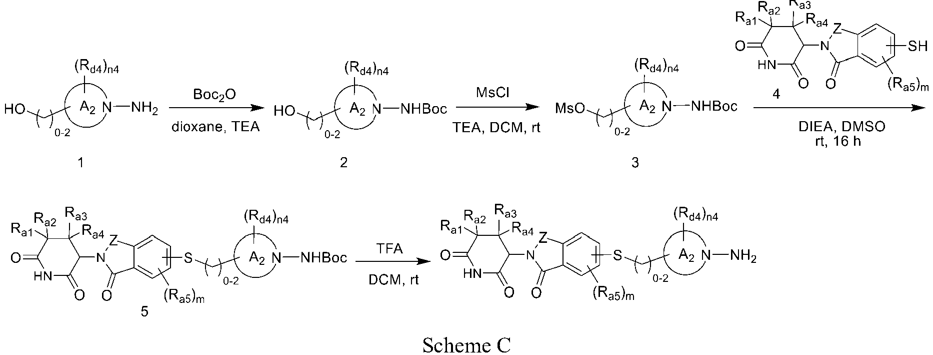

In Scheme C, Rₐ₁, Rₐ₂, Rₐ₃, Rₐ₄, Z, (Rₐ₅)ₘ, ring A₂, and (R_{d4})ₙ₄ are as defined in the compounds of Formula (I) of the present disclosure and the respective sub-embodiments thereof.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

It should be understood that, in each of the above synthetic schemes, when a group contains an active hydrogen or a reactive group that is not intended to participate in the scheme reaction, such active hydrogen or reactive group can be protected by techniques and methods well known to those skilled in the art, for example, using protecting groups. For example, when the reactive group is piperidinyl or piperazinyl, conventional protecting groups such as Boc and the like can be used to protect the hydrogen on the nitrogen. For example, when the reactive group is -CHO, conventional protecting groups can be used to protect the aldehyde group, such as by forming an acetal. The deprotection of the protecting groups can be achieved by techniques and methods well known to those skilled in the art. For example, the removal of the Boc protecting group can be achieved under acidic conditions, such as hydrochloric acid or trifluoroacetic acid. As another example, the removal of the acetal protecting group can be achieved under acidic conditions, such as sulfuric acid, hydrochloric acid, or trifluoroacetic acid.

### Examples

### Example A-1: Preparation of compound GT-04304

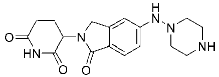

With reference to the method of Scheme A1, the target compound GT-04304 was obtained (gray solid, 6.5 g, yield 97 %). ¹H NMR (400 MHz, MeOD-d4) *δ* 7.57 (d, J = 8.4 Hz, 1H), 7.05 (s, 1H), 6.95 (dd, J = 8.4, 1.8 Hz, 1H), 5.08 (dd, J = 13.3, 5.2 Hz, 1H), 4.44 - 4.31 (m, 2H), 3.38 (t, J = 5.1 Hz, 4H), 3.03 (s, 4H), 2.86 (dd, J = 13.3, 5.3 Hz, 1H), 2.81 - 2.72 (m, 1H), 2.45 (dd, J = 13.1, 4.7 Hz, 1H), 2.17 - 2.09 (m, 1H). LCMS (ESI) calcd. for C₁₇H₂₂N₅O₃⁺ [M+H]⁺: 344.17, found, 344.2.

### Example A-2: Preparation of compound GT-06985

With reference to the method of Scheme A1, the target compound GT-06985 was obtained (gray solid, 11.7 g, yield 99 %). ¹H NMR (400 MHz, MeOD) *δ* 7.49 (d, J = 8.3 Hz, 1H), 7.45 - 7.36 (m, 1H), 5.09 (dd, J = 13.3, 5.1 Hz, 1H), 4.47 (q, J = 16.7 Hz, 2H), 3.44 - 3.36 (m, 4H), 3.09 (d, J = 4.6 Hz, 4H), 2.95 - 2.85 (m, 1H), 2.81 - 2.75 (m, 1H), 2.48 (qd, J = 13.2, 4.7 Hz, 1H), 2.19 - 2.10 (m, 1H). LCMS (ESI) calcd. for C₁₇H₂₁FN₅O₃⁺ [M+H]⁺: 362.16, found, 362.2.

### Example A-3: Preparation of compound GT-06988

With reference to the method of Scheme A₁, the target compound GT-06988 was obtained (gray solid, 7.9 g, yield 97 %). ¹H NMR (400 MHz, DMSO-d6) *δ* 10.96 (s, 1H), 8.74 (s, 2H), 7.73 (s, 1H), 7.36 (d, J = 10.7 Hz, 1H), 7.31 (d, J = 7.2 Hz, 1H), 5.05 (dd, J = 13.3, 5.0 Hz, 1H), 4.32 (d, J = 16.8 Hz, 1H), 4.20 (d, J = 16.9 Hz, 1H), 3.25 (s, 4H), 2.98 (s, 4H), 2.92 - 2.82 (m, 1H), 2.59 (d, J = 17.2 Hz, 1H), 2.36 (dd, J = 13.2, 4.4 Hz, 1H), 2.04 - 1.91 (m, 1H). LCMS (ESI) calcd. for C₁₇H₂₁FN₅O₃⁺ [M+H]⁺: 362.16, found, 362.2.

### Example A-4: Preparation of compound GT-06989

With reference to the method of Scheme A1, the target compound GT-06989 was obtained (gray solid, 5.4 g, yield 87 %). ¹H NMR (400 MHz, MeOD) *δ* 6.77 (s, 1H), 6.65 (d, J = 12.1 Hz, 1H), 5.03 (dd, J = 13.3, 5.1 Hz, 1H), 4.35 (q, J = 17.0 Hz, 2H), 3.38 (t, J = 4.8 Hz, 4H), 3.02 (s, 4H), 2.85 (dd, J = 13.3, 5.2 Hz, 1H), 2.79 - 2.69 (m, 1H), 2.42 (dd, J= 13.1, 4.7 Hz, 1H), 2.17 - 2.07 (m, 1H). LCMS (ESI) calcd. for C₁₇H₂₁FN₅O₃⁺ [M+H]⁺: 362.16, found, 362.2.

### Example A-5: Preparation of compound GT-07368

With reference to the method of Scheme A₁, the target compound GT-07368 was obtained (gray solid, 5.4 g, yield 98 %). ¹H NMR (400 MHz, DMSO-d6) δ 10.98 (s, 1H), 8.78 (s, 2H), 7.30 (t, *J* = 7.6 Hz, 1H), 7.17 (s, 1H), 7.08 (dd, *J* = 7.6, 4.4 Hz, 2H), 5.09 (dd, *J* = 13.2, 5.2 Hz, 1H), 4.36 *(d, J =* 17.6 Hz, 1H), 4.24 *(d, J =* 17.6 Hz, 1H), 3.24 (s, 4H), 2.93 (s, 4H), 2.89 (d, *J* = 5.2 Hz, 1H), 2.62 (d, *J* = 17.2 Hz, 1H), 2.40 (qd, *J =* 13.2, 4.4 Hz, 1H), 2.07 - 1.97 (m, 1H). LCMS (ESI) calcd. for C₁₇H₂₂N₅O₃⁺ [M+H]⁺: 344.17, found, 344.1.

### Example A-6: Preparation of compound GT-05579

With reference to the method of Scheme A1, the target compound GT-05579 was obtained (gray solid, 4.1 g, yield 97 %). ¹H NMR (400 MHz, DMSO-d6) δ 11.07 (s, 1H), 8.85 (s, 2H), 8.35 (s, 1H), 7.64 (*d, J =* 8.3 Hz, 1H), 7.23 (s, 1H), 7.06 (d, *J* = 7.8 Hz, 1H), 5.05 (dd, *J* = 12.9, 5.3 Hz, 1H), 3.68 (d, *J* = 4.3 Hz, 1H), 3.31 (d, *J* = 18.6 Hz, 4H), 2.88 (td, *J* = 14.6, 5.2 Hz, 4H), 2.56 (dd, *J* = 20.1, 10.7 Hz, 2H), 2.01 (dd, *J* = 9.9, 4.8 Hz, 1H). LCMS (ESI) calcd. for C₁₇H₂₀N₅O₄⁺ [M+H]⁺: 358.15, found, 358.1.

### Example A-7: Preparation of compound GT-04310

With reference to the method of Scheme B1, the target compound GT-04310 was obtained (pale yellow solid, 1.5 g, yield 96 %). ¹H NMR (400 MHz, DMSO-d6) δ 11.09 (s, 1H), 8.84 (s, 1H), 8.42 (s, 1H), 7.65 (d, *J* = 10.3 Hz, 1H), 7.57 (d, *J* = 7.0 Hz, 1H), 5.08 (dd, *J* = 12.8, 5.4 Hz, 1H), 3.30 (s, 4H), 2.97 (d, *J* = 33.3 Hz, 4H), 2.93 - 2.83 (m, 1H), 2.56 (dd, *J* = 22.6, 11.2 Hz, 2H), 2.05 - 1.97 (m, 1H). LCMS (ESI) calcd. for C₁₇H₁₉FN₅O₄⁺ [M+H]⁺: 376.14, found, 376.2.

### Example A-8: Preparation of compound GT-07007

With reference to the method of Scheme B2, the target compound GT-07007 was obtained (gray solid, 3.6 g, yield 92 %). ¹H NMR (400 MHz, DMSO-d6) δ 11.09 (s, 1H), 9.48 (s, 2H), 7.74 (d, *J* = 8.5 Hz, 1H), 7.41 (d, *J* = 2.0 Hz, 1H), 7.30 (dd, *J* = 8.5, 2.0 Hz, 1H), 5.09 (dd, *J* = 12.9, 5.4 Hz, 1H), 3.58 (s, 4H), 3.02 (s, 4H), 2.92 - 2.83 (m, 1H), 2.57 (dd, *J* = 19.3, 10.3 Hz, 2H), 2.08 - 1.99 (m, 1H). LCMS (ESI) calcd. for C₁₇H₂₀N₅O₄⁺ [M+H]⁺: 358.15, found, 358.2.

### Example A-9: Preparation of compound GT-07051

With reference to the method of Scheme B2, the target compound GT-07051 was obtained (gray solid, 1.6 g, yield 97 %). ¹H NMR (400 MHz, DMSO-d6) δ 10.95 (s, 1H), 9.65 (s, 2H), 7.55 (d, *J* = 8.4 Hz, 1H), 7.13 - 7.06 (m, 2H), 5.05 (dd, *J* = 13.3, 5.1 Hz, 1H), 4.34 (d, *J* = 17.0 Hz, 1H), 4.23 (t, *J* = 17.0 Hz, 1H), 3.46 - 3.30 (m, 4H), 3.11 (brs, 4H), 2.94 - 2.84 (m, 1H), 2.58 (d, *J* = 17.2 Hz, 1H), 2.43 - 2.31 (m, 1H), 1.98 - 1.90 (m, 1H). LCMS (ESI) calcd. for C₁₇H₂₂N₅O₃⁺ [M+H]⁺: 344.17, found, 344.2.

### Example A-10: Preparation of compound GT-07008

The target compound GT-07008 was prepared according to the method of Scheme C.

Step 1: to a solution of (1-aminopiperidin-4-yl)methanol (1.0 eq.) in 1,4-dioxane in a 100 mL single-neck flask were added sequentially (Boc)₂O (1.2 eq.) and triethylamine (3.0 eq.). The reaction mixture was stirred at room temperature for 16 hours. After the completion of the reaction was confirmed by TLC, the reaction mixture was concentrated under reduced pressure. The resulting residue was washed with water and extracted with ethyl acetate three times. The combined organic phase was dried over anhydrous sodium sulfate and concentrated under reduced pressure. The crude residue was purified by column chromatography (eluent: petroleum ether/ethyl acetate = 0-50%) to give tert-butyl (4-(hydroxymethyl)piperidin-1-yl)carbamate as a white solid (yield: 80%).

Step 2: To a solution of tert-butyl (4-(hydroxymethyl)piperidin-1-yl)carbamate (1.0 eq.) in dichloromethane in a 100 mL single-neck flask were added sequentially MsCl (1.2 eq.) and triethylamine (3.0 eq.). The reaction mixture was stirred at room temperature for 1 hour. After the completion of the reaction was confirmed by TLC, the reaction mixture was washed with water and extracted with dichloromethane three times. The combined organic phase was dried over anhydrous sodium sulfate and concentrated under reduced pressure. The crude residue was purified by column chromatography (eluent: petroleum ether/ethyl acetate = 0-40%) to give (1-((tert-butoxycarbonyl)amino)piperidin-4-yl)methyl methanesulfonate as a white solid (yield: 85%).

Step 3: To a solution of (1-((tert-butoxycarbonyl)amino)piperidin-4-yl)methyl methanesulfonate (1.3 eq.) and 3-(5-mercapto-1-oxoisoindolin-2-yl)piperidine-2,6-dione (1.0 eq.) in dimethyl sulfoxide in a 100 mL single-neck flask was added triethylamine (3.0 eq.). The reaction mixture was stirred at room temperature for 16 hours. After the completion of the reaction was confirmed by TLC, the reaction mixture was washed with water and extracted with ethyl acetate three times. The combined organic phase was dried over anhydrous sodium sulfate and concentrated under reduced pressure. The crude residue was purified by column chromatography (eluent: petroleum ether/ethyl acetate = 0-50%) to give tert-butyl (4-(((2-(2,6-dioxopiperidin-3-yl)-1-oxoisoindolin-5-yl)thio)methyl)piperidin-1-yl)carbamate as a white solid (yield: 50%).

Step 4: At room temperature, to a solution of tert-butyl (4-(((2-(2,6-dioxopiperidin-3-yl)-1-oxoisoindolin-5-yl)thio)methyl)piperidin-1-yl)carbamate in dichloromethane in a 100 mL single-neck flask was added trifluoroacetic acid. The reaction mixture was stirred for 1 hour. After the completion of the reaction was confirmed by TLC, the reaction mixture was concentrated under reduced pressure and lyophilized to afford compound GT-07008 (gray solid, 0.6 g, yield 93 %). ¹H NMR (400 MHz, MeOD-d4) δ 11.80 (s, 1H), 10.03 (s, 1H), 8.48 - 8.43 (m, 1H), 8.38 (s, 1H), 8.25 (d, *J* = 7.1 Hz, 1H), 5.91 (dd, *J* = 13.3, 5.1 Hz, 1H), 5.17 (dd, *J* = 50.0, 17.4 Hz, 2H), 4.26 (s, 4H), 3.86 (d, *J* = 6.1 Hz, 2H), 3.77 - 3.68 (m, 1H), 3.42 (d, *J* = 17.0 Hz, 1H), 3.30 - 3.12 (m, 2H), 2.89 - 2.68 (m, 3H), 2.27 - 2.16 (m, 2H). LCMS (ESI) calcd. for C₁₉H₂₅N₄O₃S⁺ [M+H]⁺: 389.16, found, 389.2.

### Example A-11: Preparation of compound GT-07966

With reference to the method of Scheme A1, the target compound GT-07966 was obtained (yellow solid, 1.56 g, yield 94 %). ¹H NMR(400 MHz, DMSO-d6) δ 10.93 (s, 1H), 8.99 (s, 2H), 7.56 (s, 1H), 7.46 (d, J = 8.4 Hz, 1H), 6.94(s, 1H), 6.87 (d, J = 8.4 Hz, 1H), 5.03 (dd, J = 13.2, 5.2 Hz, 1H), 4.31 (d, J = 16.8 Hz, 1H), 4.18 (d, J =16.8 Hz, 1H), 4.08 - 4.01 (m, 2H), 2.99 - 2.82 (m, 5H), 2.58 (d,J = 16.8 Hz, 1H), 2.33 (dd, J = 13.2, 4.4 Hz, 1H), 2.15 (d, J = 8.8 Hz, 2H), 1.98 - 1.89 (m, 3H). LCMS (ESI) calcd. for C ₁₉H₂₄N₅O₃⁺: 370.19, found, 370.1.

### Example A-12: Preparation of compound GT-08008

With reference to the method of Scheme A1, the target compound GT-08008 was obtained (yellow solid, 1.7 g, yield 83 %). ¹H NMR(400 MHz, DMSO-d6) δ 10.93 (s, 1H), 9.49 (s, 1H), 7.98 (s, 1H), 7.47 (d, J = 8.4 Hz, 2H), 7.03(s, 1H), 6.96 (d, J = 8.4 Hz, 1H), 5.03 (dd, J = 13.2, 5.2 Hz, 1H), 4.30 (dd, J = 11.2, 5.6 Hz, 3H), 4.18 (d, J = 16.8 Hz, 1H), 3.46 (d, J = 10.4 Hz, 2H), 3.34 (d, J = 11.2 Hz, 2H), 2.96 - 2.85 (m, 1H), 2.75 - 2.64 (m,1H), 2.58 (d, J = 16.8 Hz, 1H), 2.35 (ddd, J = 26.0, 13.2, 4.4 Hz, 2H), 1.98 - 1.91 (m, 1H). LCMS (ESI) calcd. for C ₁₈H₂₂N₅O₃⁺: 356.16, found, 356.1.

### Example A-13: Preparation of compound GT-08009

With reference to the method of Scheme A1, the target compound GT-08009 was obtained (yellow solid, 1.2 g, yield 75 %). ¹H NMR(400 MHz, DMSO-d6) δ 10.93 (s, 1H), 9.03 (s, 1H), 8.77 (s, 1H), 7.72 (s, 1H), 7.45 (d, J = 8.4Hz, 1H), 6.96 (s, 1H), 6.86 (d, J = 8.4 Hz, 1H), 5.03 (dd, J = 13.2, 5.2 Hz, 1H), 4.30 (d, J = 16.4 Hz, 2H),4.17 (d, J = 16.8 Hz, 1H), 3.63 (s, 1H), 3.15 (s, 2H), 3.07 - 2.77 (m, 2H), 2.58 (d, J = 16.8 Hz, 1H), 2.39 - 2.14 (m, 2H), 1.95 (dd, J = 9.2, 3.6 Hz, 1H), 1.75 (d, J = 10.8 Hz, 1H), 0.83 (dd, J = 9.6, 6.8 Hz, 1H). LCMS (ESI) calcd. for C ₁₈H₂₂N₅O₃⁺: 356.17, found, 356.1.

### Example A-14: Preparation of compound GT-08007

With reference to the method of Scheme A1, the target compound GT-08007 was obtained (brown solid, 1.1 g, yield 99 %). ¹H NMR (400 MHz, DMSO-d6) δ 10.94 (s, 1H), 8.91 (s, 2H), 7.73 (s, 1H), 7.45 (d, J = 8.4 Hz, 1H), 6.96 (s, 1H), 6.87 (d, J = 8.4 Hz, 1H), 5.03 (dd, J = 13.2, 5.2 Hz, 1H), 4.31 (d, J = 16.8 Hz, 1H), 4.18 (d, J = 16.8 Hz, 1H), 3.26 (d, J = 4.8 Hz, 4H), 3.12 (s, 2H), 2.91 (ddd, J = 18.4, 13.6, 6.4 Hz, 3H), 2.59 (d, J = 16.8 Hz, 1H), 2.35 (qd, J = 13.2, 4.4 Hz, 1H), 2.04 - 1.82 (m, 3H). LCMS (ESI) calcd. for C ₁₈H₂₄N₅O₃⁺ [M+H]⁺: 358.18, found, 358.2.

### Example A-15: Preparation of compound GT-08006

With reference to the method of Scheme A1, the target compound GT-08006 was obtained (brown solid, 980 mg, yield 95%). ¹H NMR (400 MHz, DMSO-d6) δ 10.99 (s, 1H), 9.02 (s, 2H), 7.49 (d, *J* = 8.4 Hz, 1H), 7.28 (d, *J* = 16.8 Hz, 1H), 6.94 (s, 1H), 6.88 (d, *J* = 8.4 Hz, 1H), 5.08 (dd, *J* = 13.2, 5.2 Hz, 1H), 4.35 (d, *J* = 16.8 Hz, 1H), 4.21 (d, *J* = 16.8 Hz, 1H), 3.80 (t, *J* = 6.0 Hz, 4H), 3.01 - 2.90 (m, 1H), 2.64 (d, *J* = 16.8 Hz, 5H), 2.39 (ddd, *J* = 26.0, 13.2, 4.4 Hz, 1H), 1.99 (dd, *J* = 17.6, 10.4 Hz, 5H). LCMS (ESI) calcd. for C₂₀H₂₆N₅O₃⁺ [M+H]⁺: 384.20, found, 384.3.

### Example A-16: Preparation of compound GT-07965

With reference to the method of Scheme A1, the target compound GT-07965 was obtained (off-white solid, 1.1 g, yield 99 %). ¹H NMR (400 MHz, DMSO-d6) δ 10.94 (s, 1H), 8.93 (s, 1H), 8.76 (s, 1H), 7.44 (d, *J* = 8.4 Hz, 1H), 7.24 (s, 1H), 6.95 (s, 1H), 6.89 (d, *J* = 8.0 Hz, 1H), 5.03 (dd, *J* = 13.2, 4.8 Hz, 1H), 4.29 (d, *J* = 16.8 Hz, 1H), 4.19 (s, 1H), 3.47 (s, 2H), 3.10 - 2.82 (m, 7H), 2.68 (s, 2H), 2.59 (d, *J* = 17.6 Hz, 1H), 2.34 (dt, *J* = 22.4, 11.2 Hz, 1H), 2.02 - 1.90 (m, 1H). LCMS (ESI) calcd. for C₁₉H₂₄N₅O₃⁺ [M+H]⁺: 370.18, found, 370.1.

### Example 1: Preparation of compound GT-06422

With reference to the method of Scheme 2, the target compound GT-06422 was obtained (white solid, 15 mg, yield 25 %). ¹H NMR (400 MHz, DMSO-d6) δ 11.66 (s, 1H), 11.07 (s, 1H), 7.95 - 7.79 (m, 3H), 7.63 (d, J = 8.2 Hz, 1H), 7.54 - 7.30 (m, 7H), 7.23 - 7.19 (m, 1H), 7.08 - 6.96 (m, 1H), 5.05 (dd, J = 12.6, 5.3 Hz, 1H), 3.30 - 3.15 (m, 6H), 3.15 - 3.01 (m, 2H), 2.96 - 2.78 (m, 2H), 2.58 (d, J = 17.6 Hz, 1H), 2.45 - 2.33 (m, 1H), 2.05 - 1.95 (m, 1H). LCMS (ESI) calcd. for C₃₀H₃₀N₅O₄⁺ [M+H]⁺: 524.23, found, 524.3.

### Example 2: Preparation of compound GT-06401

With reference to the method of Scheme 2, the target compound GT-06401 was obtained (white solid, 10 mg, yield 16 %). ¹H NMR (400 MHz, DMSO-d6) δ 10.99 (s, 1H), 7.87 - 7.73 (m, 1H), 7.68 - 7.53 (m, 2H), 7.48 - 7.39 (m, 2H), 7.36 - 7.23 (m, 2H), 7.22 - 7.05 (m, 3H), 6.97 - 6.93 (m, 1H), 4.97 (dd, J = 12.7, 5.5 Hz, 1H), 3.21 - 3.11 (m, 4H), 3.11 - 2.91 (m, 4H), 2.80 - 2.76 (m, 2H), 2.53 - 2.48 (m, 1H), 2.41 - 2.33 (m, 1H), 1.99 - 1.87 (m, 1H). LCMS (ESI) calcd. for C₃₀H₂₈F₂N₅O₄⁺ [M+H]⁺: 560.21, found, 560.2.

### Example 3: Preparation of compound GT-06509

With reference to the method of Scheme 2, the target compound GT-06509 was obtained (white solid, 41 mg, yield 66 %). ¹H NMR (400 MHz, DMSO-d6) δ 11.00 (s, 1H), 9.69 (s, 1H), 8.30 (s, 1H), 7.54 (d, J = 8.3 Hz, 1H), 7.19 - 7.12 (m, 4H), 6.95 (d, J = 7.6 Hz, 1H), 4.97 (dd, J = 12.7, 5.3 Hz, 1H), 3.61 - 3.48 (m, 2H), 3.05 - 2.73 (m, 8H), 2.51 (d, J = 18.1 Hz, 1H), 2.48 - 2.46 (m, 1H), 2.20 (brs, 5H), 1.99 - 1.86 (m, 1H), 1.64 (brs, 4H). LCMS (ESI) calcd. for C₃₀H₃₃FN₅O₄⁺ [M+H]⁺: 546.25, found, 546.3.

### Example 4: Preparation of compound GT-07045

With reference to the method of Scheme 1, the target compound GT-07045 was obtained (white solid, 34 mg, yield 53 %). ¹H NMR (400 MHz, DMSO-d6) δ 10.99 (s, 1H), 10.17 (s, 1H), 8.32 (s, 1H), 7.54 (d, *J* = 8.3 Hz, 1H), 7.43 (d, *J* = 8.2 Hz, 2H), 7.19 (d, *J* = 8.3 Hz, 2H), 7.12 (s, 1H), 6.95 (s, 1H), 4.97 (dd, *J* = 12.8, 5.4 Hz, 1H), 4.30 (s, 2H), 3.76 (t, *J* = 5.3 Hz, 2H), 3.61 (s, 2H), 3.39 - 3.31 (m, 2H), 3.07 - 3.01 (m, 2H), 2.98 - 2.85 (m, 4H), 2.81 - 2.76 (m, 1H), 2.49 - 2.43 (m, 2H), 2.30 (s, 2H), 1.96 - 1.89 (m, 1H). LCMS (ESI) calcd. for C₂₉H₃₁ClN₅O₅⁺ [M+H]⁺: 564.20, found, 564.2.

### Example 5: Preparation of compound GT-06423

With reference to the method of Scheme 2, the target compound GT-06423 was obtained (white solid, 14 mg, yield 24 %). ¹H NMR (400 MHz, DMSO-d6) δ 11.37 (s, 1H), 11.09 (s, 1H), 7.94 - 7.74 (m, 3H), 7.64 (d, J = 10.2 Hz, 1H), 7.60 - 7.26 (m, 8H), 5.07 (dd, J = 12.9, 5.3 Hz, 1H), 3.31 - 3.17 (m, 6H), 3.13 - 3.06 (m, 2H), 2.94 - 2.83 (m, 2H), 2.59 (d, J = 19.6 Hz, 1H), 2.49 - 2.42 (m, 1H), 2.07 - 1.96 (m, 1H). LCMS (ESI) calcd. for C₃₀H₂₉FN₅O₄⁺ [M+H]⁺: 542.22, found, 542.3.

### Example 6: Preparation of compound GT-06402

With reference to the method of Scheme 2, the target compound GT-06402 was obtained (white solid, 14 mg, yield 22 %). ¹H NMR (400 MHz, DMSO-d6) δ 11.09 (s, 1H), 7.96 - 7.78 (m, 1H), 7.64 (d, J = 10.3 Hz, 2H), 7.59 - 7.41 (m, 4H), 7.34 - 7.01 (m, 3H), 5.07 (dd, J = 12.6, 5.4 Hz, 1H), 3.29 - 3.17 (m, 4H), 3.17 - 2.95 (m, 4H), 2.95 - 2.81 (m, 2H), 2.61 - 2.56 (m, 2H), 2.49 - 2.38 (m, 1H), 2.06 - 1.94 (m, 1H). LCMS (ESI) calcd. for C₃₀H₂₇F₃N₅O₄⁺ [M+H]⁺: 578.20, found, 578.2.

### Example 7: Preparation of compound GT-06510

With reference to the method of Scheme 2, the target compound GT-06510 was obtained (white solid, 41 mg, yield 67 %). ¹H NMR (400 MHz, DMSO-d6) δ 11.08 (s, 1H), 9.68 (s, 1H), 8.41 (s, 1H), 7.64 (d, J = 10.3 Hz, 1H), 7.52 (d, J = 6.9 Hz, 1H), 7.27 - 7.20 (m, 3H), 5.06 (dd, J = 12.8, 5.4 Hz, 1H), 3.65 - 3.58 (m, 2H), 3.21 - 3.13 (m, 2H), 3.10 - 3.05 (m, 1H), 3.04 - 2.78 (m, 6H), 2.58 (d, J = 19.8 Hz, 1H), 2.39 - 2.20 (m, 5H), 2.06 - 1.96 (m, 1H), 1.71 (brs, 4H). LCMS (ESI) calcd. for C₃₀H₃₂F₂N₅O₄⁺ [M+H]⁺: 564.24, found, 564.3.

### Example 8: Preparation of compound GT-06961

With reference to the method of Scheme 1, the target compound GT-06961 was obtained (white solid, 15 mg, yield 24 %). ¹H NMR (400 MHz, DMSO-d6) δ 11.01 (s, 1H), 9.89 (s, 1H), 8.34 (s, 1H), 7.56 (d, J = 10.3 Hz, 1H), 7.43 (d, J = 7.7 Hz, 3H), 7.19 (d, J = 8.2 Hz, 2H), 4.99 (dd, J = 12.8, 5.4 Hz, 1H), 4.27 (s, 2H), 3.77 (t, J = 5.1 Hz, 2H), 3.62 (s, 2H), 3.44 - 3.38 (m, 2H), 3.16 - 3.03 (m, 2H), 3.03 - 2.84 (m, 4H), 2.83 - 2.77 (m, 1H), 2.53 - 2.46 (m, 2H), 2.30 (s, 2H), 2.00 - 1.86 (m, 1H). LCMS (ESI) calcd. for C₂₉H₃₀ClFN₅O₅⁺ [M+H]⁺: 582.19, found, 582.2.

### Example 9: Preparation of compound GT-06398

With reference to the method of Scheme 2, the target compound GT-06398 was obtained (white solid, 14 mg, yield 22 %). ¹H NMR (400 MHz, DMSO-d6) δ 10.86 (s, 1H), 7.88 - 7.78 (m, 1H), 7.70 - 7.59 (m, 2H), 7.53 - 7.41 (m, 2H), 7.38 (d, J = 8.4 Hz, 1H), 7.35 - 7.26 (m, 1H), 7.24 - 7.10 (m, 2H), 6.85 (s, 1H), 6.79 (d, J = 8.6 Hz, 1H), 4.95 (dd, J = 13.2, 5.2 Hz, 1H), 4.22 (d, J = 16.9 Hz, 1H), 4.09 (d, J = 16.9 Hz, 1H), 3.24 - 3.09 (m, 4H), 3.06 - 2.93 (m, 2H), 2.87 - 2.85 (m, 3H), 2.66 (s, 1H), 2.51 (d, J = 17.8 Hz, 1H), 2.32 - 2.22 (m, 1H), 1.95 - 1.81 (m, 1H). LCMS (ESI) calcd. for C₃₀H₃₀F₂N₅O₃⁺ [M+H]⁺: 546.23, found, 546.2.

### Example 10: Preparation of compound GT-06426

With reference to the method of Scheme 2, the target compound GT-06426 was obtained (white solid, 18 mg, yield 28 %). ¹H NMR (400 MHz, DMSO-d6) δ 10.86 (s, 1H), 7.96 - 7.75 (m, 3H), 7.66 - 7.58 (m, 1H), 7.38 (d, J = 8.1 Hz, 1H), 7.33 - 7.14 (m, 4H), 6.85 (s, 1H), 6.82 - 6.72 (m, 1H), 4.95 (dd, J = 13.3, 5.1 Hz, 1H), 4.22 (d, J = 17.0 Hz, 1H), 4.09 (d, J = 16.9 Hz, 1H), 3.19 - 3.08 (m, 4H), 3.07 - 2.95 (m, 4H), 2.87 - 2.77 (m, 2H), 2.51 (d, J = 17.2 Hz, 1H), 2.32 - 2.22 (m, 1H), 1.96 - 1.80 (m, 1H). LCMS (ESI) calcd. for C₃₀H₃₀F₂N₅O₃⁺ [M+H]⁺: 546.23, found, 546.2.

### Example 11: Preparation of compound GT-06427

With reference to the method of Scheme 2, the target compound GT-06427 was obtained (white solid, 23 mg, yield 34 %). ¹H NMR (400 MHz, DMSO-d6) δ 10.86 (s, 1H), 7.91 - 7.74 (m, 2H), 7.58 - 7.27 (m, 7H), 6.84 (s, 1H), 6.78 (d, J = 7.7 Hz, 1H), 4.95 (dd, J = 13.4, 5.0 Hz, 1H), 4.22 (d, J = 16.7 Hz, 1H), 4.09 (d, J = 17.0 Hz, 1H), 3.20 - 3.12 (m, 4H), 3.04 - 2.92 (m, 4H), 2.89 - 2.77 (m, 2H), 2.51 (d, J = 17.6 Hz, 1H), 2.31 - 2.22 (m, 1H), 1.91 - 1.84 (m, 1H). LCMS (ESI) calcd. for C₃₀H₃₀Cl₂N₅O₃⁺ [M+H]⁺: 578.17, found, 578.2.

### Example 12: Preparation of compound GT-06428

With reference to the method of Scheme 2, the target compound GT-06428 was obtained (white solid, 23 mg, yield 35%). ¹H NMR (400 MHz, DMSO-d6) δ 11.12 (s, 1H), 10.86 (s, 1H), 7.66 (d, J = 8.4 Hz, 4H), 7.39 (d, J = 8.2 Hz, 1H), 6.94 (d, J = 8.4 Hz, 4H), 6.86 (s, 1H), 6.79 (d, J = 8.6 Hz, 1H), 5.40 (d, J = 9.4 Hz, 1H), 4.96 (dd, J = 13.2, 5.2 Hz, 1H), 4.22 (d, J = 16.9 Hz, 1H), 4.09 (d, J = 17.0 Hz, 1H), 3.68 (s, 6H), 3.19 - 3.09 (m, 4H), 3.01 (s, 4H), 2.87 - 2.80 (m, 1H), 2.51 (d, J = 17.4 Hz, 1H), 2.31 - 2.25 (m, 1H), 1.95 - 1.79 (m, 1H). LCMS (ESI) calcd. for C₃₂H₃₅N₅NaO₅⁺ [M+Na]⁺: 592.25, found, 592.3.

### Example 13: Preparation of compound GT-06499

With reference to the method of Scheme 1, the target compound GT-06499 was obtained (white solid, 29 mg, yield 46%). ¹H NMR (400 MHz, DMSO-d6) δ 10.87 (s, 1H), 10.62 (s, 1H), 7.91 (s, 1H), 7.74 - 7.70 (m, 2H), 7.60 (s, 1H), 7.50 (d, J = 8.6 Hz, 4H), 7.40 (d, J = 8.3 Hz, 1H), 6.86 (s, 1H), 6.79 (d, J = 8.3 Hz, 1H), 4.96 (dd, J = 13.2, 5.1 Hz, 1H), 4.35 (d, J = 3.8 Hz, 2H), 4.23 (d, J = 16.9 Hz, 1H), 4.10 (d, J = 17.0 Hz, 1H), 3.26 - 3.22 (m, 4H), 3.10 - 3.03 (m, 2H), 2.95 - 2.78 (m, 3H), 2.52 (d, J = 16.4 Hz, 1H), 2.32 - 3.23 (m, 1H), 1.92 - 1.87 (m, 1H). LCMS (ESI) calcd. for C₃₀H₃₁ClN₅O₃⁺ [M+H]⁺: 544.21, found, 544.2.

### Example 14: Preparation of compound GT-06500

With reference to the method of Scheme 1, the target compound GT-06500 was obtained (white solid, 32 mg, yield 51%). ¹H NMR (400 MHz, DMSO-d6) δ 10.87 (s, 1H), 10.57 (s, 1H), 7.74 - 7.67 (m, 4H), 7.63 (d, J = 8.0 Hz, 2H), 7.48 (d, J = 8.6 Hz, 2H), 7.40 (d, J = 8.4 Hz, 1H), 6.86 (s, 1H), 6.80 (d, J = 8.3 Hz, 1H), 4.96 (dd, J = 13.3, 5.0 Hz, 1H), 4.32 (brs, 2H), 4.23 (d, J = 17.0 Hz, 1H), 4.10 (d, J = 17.0 Hz, 1H), 3.27 - 3.16 (m, 4H), 3.10 - 3.00 (m, 2H), 2.94 - 2.77 (m, 3H), 2.52 (d, J = 17.3 Hz, 1H), 2.34 - 2.21 (m, 1H), 1.94 - 1.83 (m, 1H). LCMS (ESI) calcd. for C₃₀H₃₁ClN₅O₃⁺ [M+H]⁺: 544.21, found, 544.2.

### Example 15: Preparation of compound GT-06505

With reference to the method of Scheme 2, the target compound GT-06505 was obtained (white solid, 20 mg, yield 32%). ¹H NMR (400 MHz, DMSO-d6) δ 10.94 (s, 1H), 7.44 (d, J = 8.4 Hz, 1H), 7.24 - 7.16 (m, 4H), 6.90 (s, 1H), 6.84 (d, J = 8.2 Hz, 1H), 5.02 (dd, J = 13.3, 5.2 Hz, 1H), 4.28 (d, J = 16.9 Hz, 1H), 4.15 (d, J = 16.9 Hz, 1H), 2.94 - 2.82 (m, 5H), 2.59 (d, J = 17.4 Hz, 1H), 2.32 - 2.25 (m, 7H), 1.98 - 1.93 (m, 1H), 1.71 - 1.64 (m, 8H). LCMS (ESI) calcd. for C₃₀H₃₅FN₅O₃⁺ [M+H]⁺: 532.27, found, 532.3.

### Example 16: Preparation of compound GT-06501

With reference to the method of Scheme 1, the target compound GT-06501 was obtained (white solid, 12 mg, yield 19%). ¹H NMR (400 MHz, DMSO-d6) δ 10.93 (s, 1H), 10.26 (s, 1H), 7.44 (d, J = 8.3 Hz, 1H), 7.35 - 7.21 (m, 4H), 6.89 (s, 1H), 6.83 (d, J = 8.9 Hz, 1H), 5.02 (dd, J = 13.2, 4.7 Hz, 1H), 4.37 (s, 2H), 4.28 (d, J = 16.8 Hz, 1H), 4.14 (d, J = 16.8 Hz, 1H), 3.84 (t, J = 5.1 Hz, 2H), 3.65 (s, 2H), 3.05 - 2.83 (m, 7H), 2.61 - 2.54 (m, 2H), 2.44 - 2.29 (m, 4H), 2.01 - 1.88 (m, 1H). LCMS (ESI) calcd. for C₂₉H₃₃FN₅O₄⁺ [M+H]⁺: 534.25, found, 534.3.

### Example 17: Preparation of compound GT-06511

With reference to the method of Scheme 1, the target compound GT-06511 was obtained (white solid, 22 mg, yield 34%). ¹H NMR (400 MHz, DMSO-d6) δ 10.93 (s, 1H), 10.18 (s, 1H), 7.51 (d, J = 8.4 Hz, 2H), 7.44 (d, J = 8.4 Hz, 1H), 7.27 (d, J = 8.4 Hz, 2H), 6.90 (s, 1H), 6.84 (d, J = 8.0 Hz, 1H), 5.02 (dd, J = 13.2, 5.0 Hz, 1H), 4.36 (s, 2H), 4.28 (d, J = 16.8 Hz, 1H), 4.14 (d, J = 16.9 Hz, 1H), 3.84 (t, J = 5.4 Hz, 2H), 3.72 - 3.62 (m, 2H), 3.17 - 2.76 (m, 8H), 2.58 (d, J = 18.9 Hz, 1H), 2.56 - 2.53 (m, 1H), 2.41 - 2.33 (m, 3H), 1.99 - 1.93 (m, 1H). LCMS (ESI) calcd. for C₂₉H₃₃ClN₅O₄⁺ [M+H]⁺: 550.22, found, 550.3.

### Example 18: Preparation of compound GT-06502

With reference to the method of Scheme 1, the target compound GT-06502 was obtained (white solid, 19 mg, yield 28%). ¹H NMR (400 MHz, DMSO-d6) δ 10.93 (s, 1H), 10.20 (s, 1H), 7.50 (d, J = 8.4 Hz, 2H), 7.44 (d, J = 8.4 Hz, 1H), 7.25 (d, J = 8.4 Hz, 2H), 6.90 (s, 1H), 6.84 (d, J = 8.4 Hz, 1H), 5.02 (dd, J = 13.3, 5.0 Hz, 1H), 4.37 (s, 2H), 4.28 (d, J = 17.0 Hz, 1H), 4.14 (d, J = 16.9 Hz, 1H), 3.67 (brs, 2H), 3.06 - 2.83 (m, 7H), 2.64 - 2.52 (m, 3H), 2.35 - 2.32 (m, 1H), 2.24 (s, 2H), 2.00 - 1.88 (m, 1H), 1.23 (s, 6H). LCMS (ESI) calcd. for C₃₁H₃₇ClN₅O₄⁺ [M+H]⁺: 578.25, found, 578.3.

### Example 19: Preparation of compound GT-06420

With reference to the method of Scheme 1, the target compound GT-06420 was obtained (white solid, 18 mg, yield 30%). ¹H NMR (400 MHz, DMSO-d6) δ 11.29 (s, 1H), 10.89 (s, 1H), 7.80 - 7.73 (m, 2H), 7.48 - 7.38 (m, 4H), 7.37 - 7.29 (m, 4H), 7.27 - 7.13 (m, 2H), 5.55 (s, 1H), 4.97 (dd, J = 13.3, 4.9 Hz, 1H), 4.38 (d, J = 16.9 Hz, 1H), 4.20 (d, J = 16.7 Hz, 1H), 3.53 - 3.44 (m, 1H), 3.18 - 3.07 (m, 4H), 3.04 - 2.98 (m, 2H), 2.87 - 2.78 (m, 2H), 2.52 (d, J = 18.1 Hz, 1H), 2.34 - 2.26 (m, 1H), 1.93 - 1.85 (m, 1H). LCMS (ESI) calcd. for C₃₀H₃₁FN₅O₃⁺ [M+H]⁺: 528.24, found, 528.3.

### Example 20: Preparation of compound GT-06399

With reference to the method of Scheme 1, the target compound GT-06399 was obtained (white solid, 11 mg, yield 17%). ¹H NMR (400 MHz, DMSO-d6) δ 10.89 (s, 1H), 7.83 - 7.72 (m, 1H), 7.70 - 7.51 (m, 2H), 7.51 - 7.38 (m, 2H), 7.36 - 7.28 (m, 2H), 7.26 - 7.05 (m, 3H), 4.97 (dd, J = 13.2, 4.9 Hz, 1H), 4.38 (d, J = 16.8 Hz, 1H), 4.20 (d, J = 16.7 Hz, 1H), 3.22 - 3.10 (m, 4H), 3.07 - 2.98 (m, 2H), 2.88 - 2.75 (m, 3H), 2.66 (s, 1H), 2.51 (d, J = 17.5 Hz, 1H), 2.36 - 2.24 (m, 1H), 1.94 - 1.83 (m, 1H). LCMS (ESI) calcd. for C₃₀H₂₉F₃N₅O₃⁺ [M+H]⁺: 564.22, found, 564.2.

### Example 21: Preparation of compound GT-06506

With reference to the method of Scheme 2, the target compound GT-06506 was obtained (white solid, 13 mg, yield 22%). ¹H NMR (400 MHz, DMSO-d6) δ 10.89 (s, 1H), 9.50 (s, 1H), 7.64 (s, 1H), 7.31 (d, J = 8.2 Hz, 1H), 7.19 - 7.13 (m, 4H), 4.97 (dd, J = 13.2, 5.1 Hz, 1H), 4.38 (d, J = 16.7 Hz, 1H), 4.20 (d, J = 16.8 Hz, 1H), 3.56 - 3.48 (m, 2H), 3.07 - 2.95 (m, 4H), 2.92 - 2.82 (m, 4H), 2.52 (d, J = 17.4 Hz, 1H), 2.38 - 2.24 (m, 2H), 2.19 (brs, 4H), 1.92 - 1.83 (m, 1H), 1.64 (brs, 4H). LCMS (ESI) calcd. for C₃₀H₃₄F₂N₅O₃⁺ [M+H]⁺: 550.26, found, 550.3.

### Example 22: Preparation of compound GT-06507

With reference to the method of Scheme 2, the target compound GT-06507 was obtained (white solid, 33 mg, yield 54%). ¹H NMR (400 MHz, DMSO-d6) δ 10.95 (s, 1H), 9.90 (s, 1H), 7.72 (s, 1H), 7.34 (d, J = 10.7 Hz, 1H), 7.24 (d, J = 8.5 Hz, 4H), 5.03 (dd, J = 13.2, 5.1 Hz, 1H), 4.29 (d, J = 16.9 Hz, 1H), 4.16 (d, J = 16.9 Hz, 1H), 3.63 - 3.58 (m, 2H), 3.15 - 2.95 m, 3H), 2.94 - 2.86 (m, 5H), 2.59 (d, J = 16.8 Hz, 1H), 2.35 - 2.21 (m, 6H), 1.97 - 1.93(m, 1H), 1.71 (brs, 4H). LCMS (ESI) calcd. for C₃₀H₃₄F₂N₅O₃⁺ [M+H]⁺: 550.26, found, 550.3.

### Example 23: Preparation of compound GT-06421

With reference to the method of Scheme 2, the target compound GT-06421 was obtained (white solid, 12 mg, yield 20%). ¹H NMR (400 MHz, DMSO-d6) δ 11.61 (s, 1H), 10.95 (s, 1H), 8.13 - 7.98 (m, 1H), 7.97 - 7.81 (m, 3H), 7.51 - 7.38 (m, 6H), 6.74 - 6.65 (m, 1H), 6.62 - 6.58 (m, 1H), 4.98 (dd, J = 13.3, 5.1 Hz, 1H), 4.31 (d, J = 17.3 Hz, 1H), 4.17 (d, J = 16.9 Hz, 1H), 3.60 - 3.52 (m, 1H), 3.26 - 3.14 (m, 4H), 3.11 - 3.05 (m, 3H), 2.97 - 2.80 (m, 2H), 2.58 (d, J = 17.7 Hz, 1H), 2.34 - 2.29 (m, 1H), 2.00 - 1.89 (m, 1H). LCMS (ESI) calcd. for C₃₀H₃₁FN₅O₃⁺ [M+H]⁺: 528.24, found, 528.2.

### Example 24: Preparation of compound GT-06400

With reference to the method of Scheme 2, the target compound GT-06400 was obtained (white solid, 24 mg, yield 38%). ¹H NMR (400 MHz, DMSO-d6) δ 10.95 (s, 1H), 8.17 - 8.13 (m, 1H), 7.94 - 7.79 (m, 1H), 7.77 - 7.62 (m, 1H), 7.59 - 7.35 (m, 4H), 7.31 - 7.12 (m, 2H), 6.68 (s, 1H), 6.65 - 6.50 (m, 1H), 4.98 (dd, J = 13.2, 5.0 Hz, 1H), 4.31 (d, J = 17.2 Hz, 1H), 4.17 (d, J = 16.9 Hz, 1H), 3.28 - 3.17 (m, 4H), 3.13 - 3.04 (m, 2H), 2.94 - 2.84 (m, 3H), 2.74 (s, 1H), 2.58 (d, J = 16.3 Hz, 1H), 2.36 - 2.25 (m, 1H), 2.01 - 1.88 (m, 1H). LCMS (ESI) calcd. for C₃₀H₂₉F₃N₅O₃⁺ [M+H]⁺: 564.22, found, 564.3.

### Example 25: Preparation of compound GT-06508

With reference to the method of Scheme 2, the target compound GT-06508 was obtained (white solid, 38 mg, yield 62%). ¹H NMR (400 MHz, DMSO-d6) δ 10.87 (s, 1H), 10.19 (s, 1H), 7.97 (s, 1H), 7.16 (dd, J = 13.1, 4.4 Hz, 3H), 6.59 (s, 1H), 6.52 (d, J = 12.1 Hz, 1H), 4.91 (dd, J = 13.3, 5.1 Hz, 1H), 4.22 (d, J = 17.3 Hz, 1H), 4.08 (d, J = 17.3 Hz, 1H), 3.54 - 3.45 (m, 2H), 3.13 - 2.95 (m, 3H), 2.91 - 2.73 (m, 5H), 2.56 - 2.46 (m, 1H), 2.36 - 2.12 (m, 6H), 1.96 - 1.82 (m, 1H), 1.63 (s, 4H). LCMS (ESI) calcd. for C₃₀H₃₄F₂N₅O₃⁺ [M+H]⁺: 550.26, found, 550.3.

### Example 26: Preparation of compound GT-06863

With reference to the method of Scheme 4, the target compound GT-06863 was obtained (white solid, 25 mg, yield 36%). ¹H NMR (400 MHz, DMSO-d6) δ 10.86 (s, 1H), 7.38 (d, *J* = 8.4 Hz, 1H), 7.26 (d, *J* = 4.4 Hz, 2H), 7.14 - 7.05 (m, 1H), 6.87 (s, 1H), 6.79 (d, *J* = 8.1 Hz, 1H), 4.96 (dd, *J* = 13.2, 5.0 Hz, 1H), 4.24 (d, *J* = 16.9 Hz, 1H), 4.10 (d, *J* = 16.9 Hz, 1H), 3.34 - 3.14 (m, 9H), 2.98 - 2.87 (m, 4H), 2.88 - 2.77 (m, 1H), 2.70 - 2.61 (m, 3H), 2.52 (d, *J* = 16.8 Hz, 1H), 2.34 - 2.21 (m, 1H), 1.92 - 1.87 (m, 1H). LCMS (ESI) calcd. for C₂₈H₃₂Cl₂N₇O₄⁺ [M+H]⁺: 600.19, found, 600.2.

### Example 27: Preparation of compound GT-06864

With reference to the method of Scheme 4, the target compound GT-06864 was obtained (white solid, 26 mg, yield 41%). ¹H NMR (400 MHz, DMSO-d6) δ 10.87 (s, 1H), 7.39 (d, J = 8.4 Hz, 1H), 7.29 (s, 2H), 7.14 (t, J = 8.7 Hz, 2H), 6.91 (s, 1H), 6.83 (d, J = 8.1 Hz, 1H), 4.96 (dd, J = 13.2, 5.1 Hz, 1H), 4.30 - 4.09 (m, 5H), 3.39 (brs. 4H), 3.32 - 3.26 (m, 2H), 3.20 (brs, 4H), 2.90 - 2.78 (m, 1H), 2.75 - 2.66 (m, 3H), 2.52 (d, J = 16.9 Hz, 1H), 2.35 - 2.21 (m, 1H), 1.91 - 1.87 (m, 1H). LCMS (ESI) calcd. for C₂₈H₃₃FN₇O₄⁺ [M+H]⁺: 550.26, found, 550.3.

### Example 28: Preparation of compound GT-06865

With reference to the method of Scheme 4, the target compound GT-06865 was obtained (white solid, 35 mg, yield 53%). ¹H NMR (400 MHz, DMSO-d6) δ 10.87 (s, 1H), 7.39 (d, J = 8.3 Hz, 1H), 7.22 (d, J = 8.9 Hz, 2H), 6.99 (d, J = 8.9 Hz, 2H), 6.90 (s, 1H), 6.82 (d, J = 8.3 Hz, 1H), 4.96 (dd, J = 13.3, 5.1 Hz, 1H), 4.27 - 4.01 (m, 5H), 3.33 - 3.23 (m, 6H), 3.12 (brs, 4H), 2.90 - 2.77 (m, 1H), 2.76 - 2.60 (m, 3H), 2.52 (d, J = 16.8 Hz, 1H), 2.33 - 2.24 (m, 1H), 1.97 - 1.87 (m, 1H). LCMS (ESI) calcd. for C₂₈H₃₃ClN₇O₄⁺ [M+H]⁺: 566.23, found, 566.2.

### Example 29: Preparation of compound GT-06866

With reference to the method of Scheme 4, the target compound GT-06866 was obtained (white solid, 36 mg, yield 51%). ¹H NMR (400 MHz, DMSO-d6) δ 10.94 (s, 1H), 7.47 (d, J = 8.3 Hz, 1H), 7.42 (d, J = 8.8 Hz, 2H), 7.03 (d, J = 8.8 Hz, 2H), 7.00 (s, 1H), 6.91 (d, J = 8.0 Hz, 1H), 5.03 (dd, J = 13.2, 5.0 Hz, 1H), 4.34 - 4.20 (m, 5H), 3.36 (brs, 6H), 3.20 (brs, 4H), 2.99 - 2.88 (m, 1H), 2.86 - 2.71 (m, 3H), 2.59 (d, J = 17.5 Hz, 1H), 2.41 - 2.33 (m, 1H), 1.98 - 1.90 (m, 1H). LCMS (ESI) calcd. for C₂₈H₃₃BrN₇O₄⁺ [M+H]⁺: 610.18, found, 610.2.

### Example 30: Preparation of compound GT-06867

With reference to the method of Scheme 4, the target compound GT-06867 was obtained (white solid, 25 mg, yield 38%). ¹H NMR (400 MHz, DMSO-d6) δ 10.94 (s, 1H), 7.46 (d, J = 8.3 Hz, 1H), 7.14 - 7.08 (m, 1H), 7.04 - 6.98 (m, 2H), 6.90 - 6.86 (m, 2H), 5.03 (dd, J = 13.3, 5.1 Hz, 1H), 4.37 - 4.19 (m, 5H), 3.35 (brs, 6H), 3.06 (brs, 4H), 2.97 - 2.85 (m, 1H), 2.81 - 2.72 (m, 3H), 2.59 (d, J = 16.6 Hz, 1H), 2.39 - 2.32 (m, 1H), 1.99 - 1.90 (m, 1H). LCMS (ESI) calcd. for C₂₈H₃₂F₂N₇O₄⁺ [M+H]⁺: 568.25, found, 568.3.

### Example 31: Preparation of compound GT-06868

With reference to the method of Scheme 4, the target compound GT-06868 was obtained (white solid, 15 mg, yield 24%). ¹H NMR (400 MHz, DMSO-d6) δ 10.86 (s, 1H), 7.38 (d, J = 8.4 Hz, 1H), 7.22 (dd, J = 8.4, 5.7 Hz, 2H), 7.04 (t, J = 8.8 Hz, 2H), 6.86 (s, 1H), 6.78 (d, J = 8.4 Hz, 1H), 4.96 (dd, J = 13.3, 5.0 Hz, 1H), 4.24 (d, J = 16.8 Hz, 1H), 4.10 (d, J = 16.9 Hz, 1H), 3.65 (d, J = 12.7 Hz, 2H), 3.27 - 3.15 (m, 5H), 2.90 - 2.72 (m, 4H), 2.69 - 2.61 (m, 4H), 2.52 (d, J = 16.6 Hz, 1H), 2.33 - 2.20 (m, 1H), 1.93 - 1.82 (m, 1H), 1.70 - 1.67 (m, 2H), 1.54 - 1.45 (m, 2H). LCMS (ESI) calcd. for C₂₉H₃₄FN₆O₄⁺ [M+H]⁺: 549.26, found, 549.3.

### Example 32: Preparation of compound GT-06869

With reference to the method of Scheme 4, the target compound GT-06869 was obtained (white solid, 18 mg, yield 26%). ¹H NMR (400 MHz, DMSO-d6) δ 10.86 (s, 1H), 7.44 (dd, J = 7.7, 1.6 Hz, 1H), 7.38 (d, J = 8.3 Hz, 1H), 7.35 - 7.26 (m, 2H), 6.86 (s, 1H), 6.78 (d, J = 8.1 Hz, 1H), 4.96 (dd, J = 13.2, 5.0 Hz, 1H), 4.24 (d, J = 16.9 Hz, 1H), 4.10 (d, J = 16.9 Hz, 1H), 3.68 (d, J = 13.2 Hz, 2H), 3.27 - 3.19 (m, 5H), 3.11 (t, J = 12.0 Hz, 2H), 2.86 - 2.78 (m, 3H), 2.80 - 2.62 (m, 3H), 2.52 (d, J = 16.7 Hz, 1H), 2.32 - 2.22 (m, 1H), 1.91 - 1.84 (m, 1H), 1.72 - 1.69 (m, 2H), 1.57 - 1.49 (m, 2H). LCMS (ESI) calcd. for C₂₉H₃₃Cl₂N₆O₄⁺ [M+H]⁺: 599.19, found, 599.2.

### Example 33: Preparation of compound GT-06870

With reference to the method of Scheme 4, the target compound GT-06870 was obtained (white solid, 19 mg, yield 28%). ¹H NMR (400 MHz, DMSO-d6) δ 10.86 (s, 1H), 7.38 (d, J = 8.4 Hz, 1H), 7.34 - 7.25 (m, 1H), 7.24 - 7.15 (m, 2H), 6.86 (s, 1H), 6.78 (d, J = 8.3 Hz, 1H), 4.95 (dd, J = 13.3, 5.1 Hz, 1H), 4.24 (d, J = 17.0 Hz, 1H), 4.10 (d, J = 17.0 Hz, 1H), 3.68 (d, J = 13.1 Hz, 2H), 3.35 - 3.14 (m, 5H), 3.07 (t, J = 12.0 Hz, 1H), 2.87 - 2.77 (m, 3H), 2.72 - 2.61 (m, 3H), 2.51 (d, J = 16.6 Hz, 1H), 2.32 - 2.22 (m, 1H), 1.92 - 1.86 (m, 1H), 1.71 - 1.68 (m, 2H), 1.58 - 1.50 (m, 2H). LCMS (ESI) calcd. for C₂₉H₃₃ClFN₆O₄⁺ [M+H]⁺: 583.22, found, 583.3.

### Example 34: Preparation of compound GT-06871

With reference to the method of Scheme 4, the target compound GT-06871 was obtained (white solid, 22 mg, yield 33%). ¹H NMR (400 MHz, DMSO-d6) δ 10.86 (s, 1H), 7.38 (d, J = 8.3 Hz, 1H), 7.23 - 7.18 (m, 1H), 7.14 - 7.08 (m, 2H), 6.86 (s, 1H), 6.78 (d, J = 8.3 Hz, 1H), 4.96 (dd, J = 13.3, 5.1 Hz, 1H), 4.24 (d, J = 16.9 Hz, 1H), 4.10 (d, J = 16.9 Hz, 1H), 3.66 (d, J = 12.9 Hz, 2H), 3.25 - 3.16 (m, 4H), 3.00 - 2.95 (m, 1H), 2.88 - 2.78 (m, 4H), 2.69 - 2.61 (m, 3H), 2.52 (d, J = 17.0 Hz, 1H), 2.32 - 2.22 (m, 1H), 1.93 - 1.86 (m, 1H), 1.73 - 1.66 (m, 2H), 1.63 - 1.54 (m, 2H). LCMS (ESI) calcd. for C₂₉H₃₃F₂N₆O₄⁺ [M+H]⁺: 567.25, found, 567.3.

### Example 35: Preparation of compound GT-06436

With reference to the method of Scheme 2, the target compound GT-06436 was obtained (pale yellow solid, 43 mg, yield 64 %). ¹H NMR (400 MHz, DMSO-d6) δ 11.08 (s, 1H), 9.37 (s, 1H), 8.98 (s, 1H), 8.21 (dd, *J* = 7.9, 1.6 Hz, 2H), 7.67 - 7.55 (m, 4H), 7.25 (s, 1H), 7.06 (s, 1H), 5.06 (dd, *J* = 12.9, 5.3 Hz, 1H), 4.65 (d, *J* = 28.9 Hz, 2H), 3.51 (d, *J* = 33.9 Hz, 4H), 3.38 (s, 1H), 3.10 (d, *J* = 31.6 Hz, 4H), 2.88 (dd, *J* = 10.1, 7.0 Hz, 1H), 2.59 (d, *J* = 17.2 Hz, 1H), 2.54 (d, *J* = 4.5 Hz, 1H), 2.09 - 1.95 (m, 1H). LCMS (ESI) calcd. for C₂₈H₂₈N₇O₄⁺ [M+H]⁺: 526.22, found, 526.2.

### Example 36: Preparation of compound GT-06437

With reference to the method of Scheme 2, the target compound GT-06437 was obtained (pale yellow solid, 37 mg, yield 53 %). ¹H NMR (400 MHz, DMSO-d6) δ 11.09 (s, 1H), 10.94 (s, 1H), 9.37 (s, 1H), 8.97 (s, 1H), 8.21 (dd, *J* = 7.9, 1.6 Hz, 2H), 7.66 (d, *J* = 10.3 Hz, 1H), 7.58 (d, *J* = 7.4 Hz, 3H), 5.08 (dd, *J* = 12.8, 5.4 Hz, 1H), 4.65 (d, *J* = 30.2 Hz, 2H), 3.55 (s, 6H), 3.14 (s, 4H), 2.99 - 2.80 (m, 1H), 2.59 (d, *J* = 18.0 Hz, 1H), 2.09 - 1.96 (m, 1H). LCMS (ESI) calcd. for C₂₈H₂₇FN₇O₄⁺ [M+H]⁺: 544.21, found, 544.3.

### Example 37: Preparation of compound GT-06438

With reference to the method of Scheme 2, the target compound GT-06438 was obtained (gray solid, 42 mg, yield 62 %). ¹H NMR (400 MHz, DMSO-d6) δ 11.03 (d, *J* = 59.2 Hz, 2H), 9.37 (d, *J* = 1.3 Hz, 1H), 8.98 (d, *J* = 1.1 Hz, 1H), 8.22 (dd, *J* = 7.9, 1.6 Hz, 2H), 7.68 - 7.50 (m, 3H), 6.77 - 6.53 (m, 2H), 4.99 (dd, *J* = 13.3, 5.0 Hz, 1H), 4.60 (s, 2H), 4.25 (dd, *J* = 55.3, 17.3 Hz, 2H), 3.60 (dd, *J* = 20.2, 16.2 Hz, 5H), 3.09 (dd, *J* = 29.4, 21.6 Hz, 4H), 2.95 - 2.85 (m, 1H), 2.59 (d, *J* = 16.9 Hz, 1H), 2.33 (tt, *J* = 13.3, 6.8 Hz, 1H), 2.04 - 1.88 (m, 1H). LCMS (ESI) C₂₈H₂₉FN₇O₃⁺ [M+H]⁺: 530.23, found, 530.3.

### Example 38: Preparation of compound GT-06439

With reference to the method of Scheme 2, the target compound GT-06439 was obtained (orange solid, 42 mg, yield 64 %). ¹H NMR (400 MHz, DMSO-d6) δ 10.98 (d, *J* = 32.6 Hz, 2H), 9.38 (d, *J* = 1.4 Hz, 1H), 8.98 (d, *J* = 1.4 Hz, 1H), 8.22 (dd, *J* = 7.9, 1.7 Hz, 2H), 7.62 - 7.55 (m, 3H), 7.47 (d, *J* = 8.3 Hz, 1H), 7.01 - 6.83 (m, 2H), 5.04 (dd, *J* = 13.3, 5.1 Hz, 1H), 4.62 (s, 2H), 4.24 (dd, *J* = 52.7, 17.0 Hz, 2H), 3.57 (d, *J* = 11.5 Hz, 3H), 3.13 (d, *J* = 11.2 Hz, 2H), 2.92 (ddd, *J* = 17.4, 16.5, 9.6 Hz, 3H), 2.59 (d, *J* = 16.6 Hz, 1H), 2.35 (dd, *J* = 12.9, 4.4 Hz, 1H), 2.08 - 1.89 (m, 1H). LCMS (ESI) calcd. for C₂₈H₃₀N₇O₃⁺ [M+H]⁺: 512.24, found, 512.3.

### Example 39: Preparation of compound GT-06440

With reference to the method of Scheme 2, the target compound GT-06440 was obtained (gray solid, 31 mg, yield 46 %). ¹H NMR (400 MHz, DMSO-d6) δ 10.97 (s, 2H), 9.38 (d, *J* = 1.4 Hz, 1H), 8.97 (d, *J* = 1.4 Hz, 1H), 8.22 (dd, *J* = 7.9, 1.7 Hz, 2H), 7.62 - 7.54 (m, 3H), 7.43 (d, *J* = 8.2 Hz, 1H), 7.32 (t, *J* = 7.7 Hz, 1H), 5.05 (dd, *J* = 13.3, 5.1 Hz, 1H), 4.61 (s, 2H), 4.46 (d, *J* = 16.8 Hz, 1H), 4.28 (d, *J* = 16.8 Hz, 1H), 3.56 (s, 2H), 3.42 (s, 3H), 3.11 (s, 4H), 2.97 - 2.85 (m, 1H), 2.59 (d, *J* = 17.2 Hz, 1H), 2.40 (qd, *J* = 13.5, 4.5 Hz, 1H), 2.03 - 1.92 (m, 1H). LCMS (ESI) calcd. for C₂₈H₂₉FN₇O₃⁺ [M+H]⁺: 530.23, found, 530.3.

### Example 40: Preparation of compound GT-06441

With reference to the method of Scheme 3, the target compound GT-06441 was obtained (pale yellow solid, 38 mg, yield 48 %). ¹H NMR (400 MHz, DMSO-d6) δ 11.06 (s, 1H), 7.60 (d, J = 8.3 Hz, 1H), 7.57 - 7.52 (m, 3H), 7.52 - 7.49 (m, 1H), 7.45 (dd, J = 13.4, 8.4 Hz, 3H), 7.39 (d, J = 6.4 Hz, 1H), 7.15 (s, 1H), 7.02 (d, J = 7.7 Hz, 1H), 5.04 (dd, J = 12.8, 5.4 Hz, 1H), 3.40 (s, 4H), 3.21 (s, 2H), 2.94 - 2.78 (m, 2H), 2.58 (d, J = 18.1 Hz, 3H), 2.47 (dd, J = 9.5, 4.5 Hz, 1H), 2.04 - 1.94 (m, 1H). LCMS (ESI) calcd. for C₃₀H₂₈ClFN₅O₄⁺ [M+H]⁺: 572.18, found, 572.2.

### Example 41: Preparation of compound GT-06442

With reference to the method of Scheme 3, the target compound GT-06442 was obtained (pale yellow solid, 41 mg, yield 50 %). ¹H NMR (400 MHz, DMSO-d6) δ 11.08 (s, 1H), 8.12 (s, 1H), 7.62 (d, *J* = 10.3 Hz, 1H), 7.55 (dd, *J* = 7.2, 5.4 Hz, 3H), 7.45 (dd, *J* = 18.9, 7.6 Hz, 4H), 7.39 - 7.35 (m, 1H), 5.06 (dd, *J* = 12.8, 5.4 Hz, 1H), 3.35 (s, 3H), 3.27 (d, *J* = 6.9 Hz, 1H), 2.88 (ddd, *J* = 16.8, 13.8, 5.4 Hz, 2H), 2.74 (s, 1H), 2.65 - 2.51 (m, 4H), 2.50 - 2.44 (m, 1H), 2.05 - 1.95 (m, 1H). LCMS (ESI) calcd. for C₃₀H₂₆ClFN₅O₅⁺ [M+H]⁺: 590.16, found, 590.2.

### Example 42: Preparation of compound GT-06443

With reference to the method of Scheme 3, the target compound GT-06443 was obtained (white solid, 44 mg, yield 55 %). ¹H NMR (400 MHz, DMSO-d6) δ 10.86 (s, 1H), 7.47 (dt, *J* = 7.4, 3.1 Hz, 3H), 7.44 - 7.33 (m, 4H), 7.31 (d, *J* = 6.5 Hz, 1H), 6.59 (s, 1H), 6.45 (d, *J* = 12.4 Hz, 1H), 4.90 (dd, *J* = 13.3, 5.1 Hz, 1H), 4.22 (d, *J* = 17.2 Hz, 1H), 4.07 (d, *J* = 17.2 Hz, 1H), 3.31 (s, 4H), 3.22 (s, 1H), 3.10 (s, 1H), 2.89 - 2.70 (m, 2H), 2.50 (d, *J* = 16.5 Hz, 2H), 2.40 - 2.15 (m, 2H), 1.91 - 1.81 (m, 1H). LCMS (ESI) calcd. for C₃₀H₂₈ClFN₅O₄⁺ [M+H]⁺: 576.18, found, 576.2.

### Example 43: Preparation of compound GT-06444

With reference to the method of Scheme 3, the target compound GT-06444 was obtained (white solid, 36 mg, yield 47 %). ¹H NMR (400 MHz, DMSO-d6) δ 10.93 (s, 1H), 7.57 - 7.52 (m, 3H), 7.52 - 7.40 (m, 5H), 7.40 - 7.36 (m, 1H), 6.89 (s, 1H), 6.81 (d, *J* = 8.3 Hz, 1H), 5.01 (dd, *J* = 13.3, 5.1 Hz, 1H), 4.21 (dd, *J* = 54.5, 16.9 Hz, 2H), 3.48 (s, 2H), 3.18 (s, 1H), 2.89 (ddd, *J* = 18.7, 13.5, 5.2 Hz, 2H), 2.68 (s, 1H), 2.58 (d, *J* = 16.9 Hz, 2H), 2.48 - 2.26 (m, 2H), 1.99 - 1.90 (m, 1H). LCMS (ESI) calcd. for C₃₀H₂₉ClN₅O₄⁺ [M+H]⁺: 558.19, found, 558.2.

### Example 44: Preparation of compound GT-06445

With reference to the method of Scheme 3, the target compound GT-06445 was obtained (white solid, 32 mg, yield 40 %). ¹H NMR (400 MHz, DMSO-d6) δ 10.88 (s, 1H), 7.50 - 7.45 (m, 3H), 7.43 - 7.38 (m, 2H), 7.38 - 7.33 (m, 2H), 7.32 - 7.28 (m, 2H), 7.17 (t, *J* = 7.7 Hz, 1H), 4.96 (dd, *J* = 13.3, 5.0 Hz, 1H), 4.37 (d, *J* = 16.7 Hz, 1H), 4.19 (d, *J* = 16.7 Hz, 1H), 3.13 (s, 2H), 2.93 - 2.74 (m, 2H), 2.73 - 2.56 (m, 2H), 2.51 (d, *J* = 16.4 Hz, 2H), 2.41 - 2.24 (m, 2H), 1.93 - 1.82 (m, 1H). LCMS (ESI) calcd. for C₃₀H₂₈ClFN₅O₄⁺ [M+H]⁺: 576.18, found, 576.2.

### Example 45: Preparation of compound GT-06446

With reference to the method of Scheme 2, the target compound GT-06446 was obtained (white solid, 23 mg, yield 33 %). ¹H NMR (400 MHz, DMSO-d6) δ 10.96 (s, 2H), 9.37 (d, *J* = 1.3 Hz, 1H), 8.98 (d, *J* = 1.2 Hz, 1H), 8.22 (dd, *J* = 7.9, 1.6 Hz, 2H), 7.68 - 7.49 (m, 3H), 7.33 (dd, *J* = 29.4, 8.9 Hz, 2H), 5.05 (dd, *J* = 13.3, 5.1 Hz, 1H), 4.61 (s, 2H), 4.26 (dd, *J* = 50.0, 16.9 Hz, 2H), 3.56 (s, 2H), 3.40 (s, 3H), 3.13 (s, 4H), 2.89 (d, *J* = 13.1 Hz, 1H), 2.59 (d, *J* = 16.5 Hz, 1H), 2.37 (dd, *J* = 13.1, 4.4 Hz, 1H), 2.03 - 1.90 (m, 1H). LCMS (ESI) calcd. for C₂₈H₂₉FN₇O₃⁺ [M+H]⁺: 530.23, found, 530.3.

### Example 46: Preparation of compound GT-06447

With reference to the method of Scheme 3, the target compound GT-06447 was obtained (white solid, 26 mg, yield 33 %). ¹H NMR (400 MHz, DMSO-d6) δ 10.95 (s, 1H), 7.58 - 7.52 (m, 3H), 7.49 (dd, *J* = 12.6, 6.8 Hz, 2H), 7.43 (d, *J* = 8.5 Hz, 2H), 7.37 (d, *J* = 6.7 Hz, 1H), 7.34 (s, 1H), 7.23 (d, *J* = 7.2 Hz, 1H), 5.03 (dd, *J* = 13.2, 5.1 Hz, 1H), 4.24 (dd, *J* = 53.3, 17.0 Hz, 2H), 3.38 (s, 2H), 3.21 (s, 2H), 3.01 - 2.81 (m, 2H), 2.64 (t, *J* = 33.9 Hz, 4H), 2.33 (dd, *J* = 13.1, 4.5 Hz, 2H), 2.01 - 1.89 (m, 1H). LCMS (ESI) calcd. for C₃₀H₂₈ClFN₅O₄⁺ [M+H]⁺: 576.18, found, 576.3.

### Example 47: Preparation of compound GT-06460

With reference to the method of Scheme 3, the target compound GT-06460 was obtained (white solid, 44 mg, yield 58 %). ¹H NMR (400 MHz, DMSO-d6) δ 11.06 (s, 1H), 7.96 (s, 1H), 7.59 (d, *J* = 8.3 Hz, 1H), 7.27 (d, *J* = 5.6 Hz, 2H), 7.18 (t, *J* = 8.9 Hz, 2H), 7.12 (s, 1H), 7.00 (d, *J* = 7.5 Hz, 1H), 5.03 (dd, *J* = 12.8, 5.4 Hz, 1H), 4.16 (s, 1H), 3.43 (dd, *J* = 28.9, 7.6 Hz, 2H), 3.32 (t, *J* = 6.5 Hz, 2H), 2.85 (ddd, *J* = 74.1, 39.8, 33.0 Hz, 3H), 2.57 (d, *J* = 17.2 Hz, 2H), 2.41 (dd, *J* = 34.8, 11.0 Hz, 3H), 2.11 (d, *J* = 17.9 Hz, 1H), 2.02 - 1.87 (m, 2H), 1.46 (d, *J* = 9.9 Hz, 2H), 0.99 (d, *J* = 13.1 Hz, 6H). LCMS (ESI) calcd. for C₃₂H₃₅FN₅O₅⁺ [M+H]⁺: 588.26, found, 588.3.

### Example 48: Preparation of compound GT-06461

With reference to the method of Scheme 3, the target compound GT-06461 was obtained (white solid, 48 mg, yield 62 %). ¹H NMR (400 MHz, DMSO-d6) δ 11.00 (s, 1H), 8.02 (s, 1H), 7.53 (d, *J* = 10.3 Hz, 1H), 7.36 (d, *J* = 7.0 Hz, 1H), 7.23 - 7.15 (m, 2H), 7.09 (t, *J* = 8.9 Hz, 2H), 4.98 (dd, *J* = 12.7, 5.4 Hz, 1H), 4.11 (s, 1H), 3.64 - 3.29 (m, 2H), 3.21 (d, *J* = 25.2 Hz, 1H), 2.93 - 2.58 (m, 3H), 2.50 (d, *J* = 19.5 Hz, 3H), 2.41 - 2.20 (m, 3H), 2.06 (s, 1H), 1.96 - 1.78 (m, 2H), 1.39 (d, *J* = 9.8 Hz, 2H), 0.92 (d, *J* = 10.6 Hz, 6H). LCMS (ESI) calcd. for C₃₂H₃₄F₂N₅O₅⁺ [M+H]⁺: 606.25, found, 606.3.

### Example 49: Preparation of compound GT-06462

With reference to the method of Scheme 3, the target compound GT-06462 was obtained (white solid, 40 mg, yield 54 %). ¹H NMR (400 MHz, DMSO-d6) δ 10.93 (s, 1H), 7.41 (d, *J* = 8.4 Hz, 1H), 7.27 (dd, *J* = 8.7, 5.6 Hz, 2H), 7.17 (t, *J* = 8.9 Hz, 3H), 6.86 (s, 1H), 6.79 (d, *J* = 8.3 Hz, 1H), 5.01 (dd, *J* = 13.3, 5.1 Hz, 1H), 4.20 (dd, *J* = 54.5, 16.9 Hz, 2H), 3.32 - 3.01 (m, 3H), 2.95 - 2.67 (m, 2H), 2.58 (d, *J* = 16.8 Hz, 2H), 2.33 (ddd, *J* = 23.6, 17.6, 12.0 Hz, 5H), 2.17 - 1.83 (m, 4H), 1.51 - 1.36 (m, 2H), 0.99 (d, *J* = 13.0 Hz, 6H). LCMS (ESI) calcd. for C₃₂H₃₇FN₅O₄⁺ [M+H]⁺: 574.28, found, 574.3 .

### Example 50: Preparation of compound GT-06463

With reference to the method of Scheme 3, the target compound GT-06463 was obtained (white solid, 35 mg, yield 46 %). ¹H NMR (400 MHz, DMSO-d6) δ 10.95 (s, 1H), 7.36 (d, *J* = 8.2 Hz, 1H), 7.33 - 7.23 (m, 3H), 7.18 (dd, *J* = 17.3, 8.4 Hz, 3H), 5.03 (dd, *J* = 13.3, 5.0 Hz, 1H), 4.43 (d, *J* = 16.7 Hz, 1H), 4.25 (d, *J* = 16.7 Hz, 1H), 3.37 (s, 3H), 3.02 - 2.83 (m, 2H), 2.58 (d, *J* = 13.4 Hz, 3H), 2.46 - 2.28 (m, 4H), 2.00 (t, *J* = 42.5 Hz, 4H), 1.54 - 1.40 (m, 2H), 0.99 (d, *J* = 11.2 Hz, 6H). LCMS (ESI) calcd. for C₃₂H₃₆F₂N₅O₄⁺ [M+H]⁺: 592.27, found, 592.3.

### Example 51: Preparation of compound GT-06464

With reference to the method of Scheme 3, the target compound GT-06464 was obtained (white solid, 46 mg, yield 61 %). ¹H NMR (400 MHz, DMSO-d6) δ 10.94 (s, 1H), 7.55 (s, 1H), 7.33 - 7.23 (m, 2H), 7.17 (t, *J* = 8.9 Hz, 2H), 6.64 (s, 1H), 6.50 (d, *J* = 12.3 Hz, 1H), 4.97 (dd, *J* = 13.3, 5.1 Hz, 1H), 4.28 (d, *J* = 17.2 Hz, 1H), 4.14 (d, *J* = 17.2 Hz, 1H), 3.35 - 3.25 (m, 3H), 2.98 - 2.72 (m, 2H), 2.63 (dd, *J* = 45.2, 12.5 Hz, 3H), 2.47 - 2.25 (m, 4H), 1.97 (dd, *J* = 46.3, 41.1 Hz, 4H), 1.53 - 1.39 (m, 2H), 0.99 (d, *J* = 13.3 Hz, 6H). LCMS (ESI) calcd. for C₃₂H₃₆F₂N₅O₄⁺ [M+H]⁺: 592.27, found, 592.3.

### Example 52: Preparation of compound GT-06465

With reference to the method of Scheme 3, the target compound GT-06465 was obtained (white solid, 14 mg, yield 41 %). ¹H NMR (400 MHz, DMSO-d6) δ 10.99 (s, 1H), 7.93 (s, 1H), 7.52 (d, *J* = 8.4 Hz, 1H), 7.34 (d, *J* = 8.5 Hz, 2H), 7.18 (d, *J* = 8.1 Hz, 2H), 7.05 (s, 1H), 6.93 (d, *J* = 8.2 Hz, 1H), 4.96 (dd, *J* = 12.8, 5.3 Hz, 1H), 4.11 (s, 1H), 2.86 - 2.58 (m, 3H), 2.58 - 2.46 (m, 3H), 2.39 - 2.23 (m, 3H), 2.11 - 1.78 (m, 4H), 1.38 (s, 2H), 0.92 (d, *J* = 10.3 Hz, 6H). LCMS (ESI) calcd. for C₃₂H₃₅ClN₅O₅⁺ [M+H]⁺: 604.23, found, 604.3.

### Example 53: Preparation of compound GT-06466

With reference to the method of Scheme 3, the target compound GT-06466 was obtained (white solid, 16 mg, yield 46 %). ¹H NMR (400 MHz, DMSO-d6) δ 11.01 (s, 1H), 8.01 (s, 1H), 7.54 (d, *J* = 10.3 Hz, 1H), 7.41 - 7.31 (m, 3H), 7.17 (d, *J* = 8.3 Hz, 2H), 4.99 (dd, *J* = 12.8, 5.4 Hz, 1H), 4.13 (s, 1H), 3.56 - 3.30 (m, 2H), 2.92 - 2.61 (m, 3H), 2.53 (dd, *J* = 32.1, 16.3 Hz, 3H), 2.31 (dd, *J* = 55.2, 10.1 Hz, 3H), 2.19 - 1.75 (m, 4H), 1.38 (s, 2H), 0.92 (d, *J* = 3.9 Hz, 6H). LCMS (ESI) calcd. for C₃₂H₃₄ClFN₅O₅⁺ [M+H]⁺: 622.22, found, 622.3.

### Example 54: Preparation of compound GT-06467

With reference to the method of Scheme 3, the target compound GT-06467 was obtained (white solid, 8 mg, yield 22 %). ¹H NMR (400 MHz, DMSO-d6) δ 10.88 (s, 1H), 7.31 (dd, *J* = 15.6, 8.4 Hz, 3H), 7.23 - 7.07 (m, 4H), 4.96 (dd, *J* = 13.3, 5.1 Hz, 1H), 4.36 (d, *J* = 16.8 Hz, 1H), 4.18 (d, *J* = 16.8 Hz, 1H), 3.32 (s, 3H), 2.95 - 2.69 (m, 2H), 2.54 (t, *J* = 22.2 Hz, 4H), 2.41 - 2.17 (m, 4H), 2.10 - 1.82 (m, 3H), 1.39 (d, *J* = 10.8 Hz, 2H), 0.92 (d, *J* = 8.0 Hz, 6H). LCMS (ESI) calcd. for C₃₂H₃₆ClFN₅O₄ ⁺ [M+H]⁺: 608.24, found, 608.3.

### Example 55: Preparation of compound GT-06468

With reference to the method of Scheme 3, the target compound GT-06468 was obtained (white solid, 15 mg, yield 44 %). ¹H NMR (400 MHz, DMSO-d6) δ 10.87 (s, 1H), 7.51 (s, 1H), 7.33 (d, *J* = 8.5 Hz, 2H), 7.18 (d, *J* = 8.3 Hz, 2H), 6.58 (s, 1H), 6.44 (d, *J* = 12.3 Hz, 1H), 4.90 (dd, *J* = 13.3, 5.1 Hz, 1H), 4.21 (d, *J* = 17.2 Hz, 1H), 4.07 (d, *J* = 17.2 Hz, 1H), 3.29 - 2.97 (m, 3H), 2.88 - 2.67 (m, 2H), 2.56 (dd, *J* = 44.4, 12.2 Hz, 3H), 2.38 - 2.18 (m, 4H), 1.91 (dd, *J* = 44.6, 39.7 Hz, 4H), 1.39 (d, *J* = 9.8 Hz, 2H), 0.92 (d, *J* = 10.8 Hz, 6H). LCMS (ESI) calcd. for C₃₂H₃₆ClFN₅O₄ ⁺ [M+H]⁺: 608.24, found, 608.3.

### Example 56: Preparation of compound GT-06469

With reference to the method of Scheme 1, the target compound GT-06469 was obtained (yellow solid, 10 mg, yield 13 %). ¹H NMR (400 MHz, DMSO-d6) δ 10.99 (s, 1H), 9.87 (s, 1H), 8.32 (s, 1H), 7.54 (d, *J* = 8.3 Hz, 1H), 7.40 (d, *J* = 8.1 Hz, 2H), 7.15 - 7.07 (m, 3H), 6.95 (d, *J* = 6.6 Hz, 1H), 4.97 (dd, *J* = 12.7, 5.2 Hz, 1H), 3.55 (s, 2H), 3.05 - 2.74 (m, 8H), 2.62 - 2.46 (m, 2H), 2.27 (s, 2H), 2.05 - 1.85 (m, 4H), 1.41 (s, 2H), 0.90 (s, 6H). LCMS (ESI) calcd. for C₃₂H₃₇ClN₅O₄⁺ [M+H]⁺: 590.25, found, 590.3.

### Example 57: Preparation of compound GT-06470

With reference to the method of Scheme 1, the target compound GT-06470 was obtained (yellow solid, 18 mg, yield 28 %). ¹H NMR (400 MHz, DMSO-d6) δ 11.08 (s, 1H), 9.82 (s, 1H), 8.42 (s, 1H), 7.64 (d, *J* = 10.3 Hz, 1H), 7.50 (dd, *J* = 17.3, 7.6 Hz, 3H), 7.16 (d, *J* = 8.3 Hz, 2H), 5.06 (dd, *J* = 12.8, 5.4 Hz, 1H), 3.63 (s, 2H), 3.17 (d, *J* = 11.5 Hz, 3H), 3.12 - 2.79 (m, 6H), 2.56 (dd, *J* = 15.6, 12.3 Hz, 2H), 2.33 (s, 2H), 2.06 - 1.98 (m, 3H), 1.49 (t, *J* = 5.9 Hz, 2H), 0.97 (s, 6H). LCMS (ESI) calcd. for C₃₂H₃₆ClFN₅O₄⁺ [M+H]⁺: 608.24, found, 608.3.

### Example 58: Preparation of compound GT-06471

With reference to the method of Scheme 1, the target compound GT-06471 was obtained (yellow solid, 13 mg, yield 19 %). ¹H NMR (400 MHz, DMSO-d6) δ 10.96 (s, 1H), 9.70 (s, 1H), 7.73 (s, 1H), 7.48 (d, *J* = 8.3 Hz, 2H), 7.38 (d, *J* = 8.2 Hz, 1H), 7.27 (t, *J* = 7.8 Hz, 1H), 7.17 (d, *J* = 8.3 Hz, 2H), 5.04 (dd, *J* = 13.3, 5.1 Hz, 1H), 4.45 (d, *J* = 16.8 Hz, 1H), 4.27 (d, *J* = 16.8 Hz, 1H), 3.61 (s, 2H), 3.31 - 3.25 (m, 1H), 3.09 (d, *J* = 12.2 Hz, 2H), 2.89 (dd, *J* = 22.5, 8.7 Hz, 5H), 2.70 - 2.52 (m, 2H), 2.45 - 2.28 (m, 3H), 2.06 - 1.90 (m, 3H), 1.49 (t, *J* = 6.0 Hz, 2H), 0.98 (s, 6H). LCMS (ESI) calcd. for C₃₂H₃₈ClFN₅O₃⁺ [M+H]⁺: 594.26, found, 594.3.

### Example 59: Preparation of compound GT-06472

With reference to the method of Scheme 1, the target compound GT-06472 was obtained (yellow solid, 13 mg, yield 20 %). ¹H NMR (400 MHz, DMSO-d6) δ 10.95 (s, 1H), 9.94 (s, 1H), 7.99 (s, 1H), 7.46 (t, *J* = 9.6 Hz, 2H), 7.16 (d, *J* = 8.3 Hz, 2H), 6.71 - 6.54 (m, 2H), 4.98 (dd, *J* = 13.3, 5.1 Hz, 1H), 4.29 (d, *J* = 17.1 Hz, 1H), 4.15 (d, *J* = 17.1 Hz, 1H), 3.61 (s, 2H), 3.28 - 3.23 (m, 1H), 3.08 - 2.84 (m, 7H), 2.63 - 2.53 (m, 2H), 2.32 (d, *J* = 12.6 Hz, 3H), 2.05 (s, 2H), 1.99 - 1.91 (m, 1H), 1.49 (t, *J* = 6.2 Hz, 2H), 0.97 (s, 6H). LCMS (ESI) calcd. for C₃₂H₃₈ClFN₅O₃⁺ [M+H]⁺: 594.26, found, 594.3.

### Example 60: Preparation of compound GT-06473

With reference to the method of Scheme 1, the target compound GT-06473 was obtained (yellow solid, 14 mg, yield 21 %). ¹H NMR (400 MHz, DMSO-d6) δ 11.08 (d, *J* = 8.3 Hz, 1H), 9.92 (s, 1H), 8.38 (s, 1H), 7.61 (d, *J* = 8.3 Hz, 1H), 7.27 - 6.94 (m, 6H), 5.04 (dd, *J* = 12.8, 5.4 Hz, 1H), 3.70 - 3.52 (m, 2H), 3.37 (s, 1H), 3.32 (s, 2H), 3.12 - 2.83 (m, 6H), 2.56 (dd, *J* = 17.4, 12.2 Hz, 2H), 2.34 (s, 2H), 2.09 - 1.95 (m, 3H), 1.49 (t, *J* = 6.4 Hz, 2H), 0.98 (s, 6H). LCMS (ESI) calcd. for C₃₂H₃₇FN₅O₄⁺ [M+H]⁺: 574.28, found, 574.3.

### Example 61: Preparation of compound GT-06474

With reference to the method of Scheme 1, the target compound GT-06474 was obtained (yellow solid, 14 mg, yield 20 %). ¹H NMR (400 MHz, DMSO-d6) δ 11.08 (s, 1H), 9.69 (s, 1H), 8.41 (s, 1H), 7.64 (d, *J* = 10.3 Hz, 1H), 7.52 (d, *J* = 6.8 Hz, 1H), 7.27 - 7.12 (m, 4H), 5.06 (dd, *J* = 12.8, 5.4 Hz, 1H), 3.64 (s, 2H), 3.31 - 3.23 (m, 2H), 3.14 (d, *J* = 11.5 Hz, 2H), 3.08 - 2.82 (m, 5H), 2.68 - 2.53 (m, 2H), 2.31 (s, 2H), 2.08 - 1.96 (m, 3H), 1.48 (d, *J* = 6.1 Hz, 2H), 0.98 (s, 6H). LCMS (ESI) calcd. for C₃₂H₃₆F₂N₅O₄⁺ [M+H]⁺: 592.27, found, 592.3.

### Example 62: Preparation of compound GT-06475

With reference to the method of Scheme 1, the target compound GT-06475 was obtained (yellow solid, 12 mg, yield 18 %). ¹H NMR (400 MHz, DMSO-d6) δ 10.96 (s, 1H), 9.75 (s, 1H), 7.73 (s, 1H), 7.38 (d, *J* = 8.2 Hz, 1H), 7.22 (dt, *J* = 13.9, 8.6 Hz, 5H), 5.04 (dd, *J* = 13.3, 5.1 Hz, 1H), 4.45 (d, *J* = 16.9 Hz, 1H), 4.27 (d, *J* = 16.8 Hz, 1H), 3.61 (d, *J* = 3.8 Hz, 2H), 3.33 - 3.32 (m, 1H), 3.10 (t, *J* = 11.5 Hz, 2H), 2.92 (dd, *J* = 19.3, 11.8 Hz, 5H), 2.57 (dd, *J* = 17.7, 10.6 Hz, 2H), 2.45 - 2.28 (m, 3H), 2.06 (s, 2H), 2.00 - 1.91 (m, 1H), 1.49 (t, *J* = 6.1 Hz, 2H), 0.98 (s, 6H). LCMS (ESI) calcd. for C₃₂H₃₈F₂N₅O₃⁺ [M+H]⁺: 578.29, found, 578.3.

### Example 63: Preparation of compound GT-06476

With reference to the method of Scheme 1, the target compound GT-06476 was obtained (yellow solid, 10 mg, yield 14 %). ¹H NMR (400 MHz, DMSO-d6) δ 10.94 (s, 1H), 9.94 (s, 1H), 7.98 (s, 1H), 7.21 (dt, *J* = 14.0, 8.5 Hz, 4H), 6.74 - 6.51 (m, 2H), 4.98 (dd, *J* = 13.3, 5.0 Hz, 1H), 4.29 (d, *J* = 17.2 Hz, 1H), 4.15 (d, *J* = 17.2 Hz, 1H), 3.60 (s, 2H), 3.31 - 3.28 (m, 1H), 3.14 - 2.81 (m, 7H), 2.62 - 2.52 (m, 2H), 2.38 - 2.25 (m, 3H), 2.05 (s, 2H), 1.98 - 1.89 (m, 1H), 1.49 (t, *J* = 6.1 Hz, 2H), 0.98 (s, 6H). LCMS (ESI) calcd. for C₃₂H₃₈F₂N₅O₃⁺ [M+H]⁺: 578.29, found, 578.3.

### Example 64: Preparation of compound GT-06536

With reference to the method of Scheme 1, the target compound GT-06536 was obtained (yellow solid, 14 mg, yield 20 %). ¹H NMR (400 MHz, DMSO-d6) δ 10.94 (d, *J* = 7.6 Hz, 1H), 9.85 (s, 1H), 7.60 (d, *J* = 19.5 Hz, 1H), 7.44 (d, *J* = 8.4 Hz, 1H), 7.25 (t, *J* = 8.8 Hz, 2H), 7.17 (dd, *J* = 8.4, 5.7 Hz, 2H), 6.90 (s, 1H), 6.84 (d, *J* = 8.7 Hz, 1H), 5.02 (dd, *J* = 13.2, 5.0 Hz, 1H), 4.21 (dd, *J* = 52.6, 16.8 Hz, 2H), 3.60 (s, 2H), 3.30 - 3.26 (m, 1H), 3.05 - 2.80 (m, 7H), 2.65 - 2.52 (m, 2H), 2.33 (s, 3H), 2.05 (s, 2H), 1.99 - 1.92 (m, 1H), 1.49 (t, *J* = 6.0 Hz, 2H), 0.98 (s, 6H). LCMS (ESI) calcd. for C₃₂H₃₉FN₅O₃⁺ [M+H]⁺: 560.30, found, 560.4.

### Example 65: Preparation of compound GT-06537

With reference to the method of Scheme 1, the target compound GT-06537 was obtained (yellow solid, 15 mg, yield 19 %). ¹H NMR (400 MHz, DMSO-d6) δ 10.88 (s, 1H), 9.73 (s, 1H), 7.65 (s, 1H), 7.39 (t, *J* = 9.4 Hz, 2H), 7.27 (d, *J* = 10.7 Hz, 1H), 7.16 (d, *J* = 7.2 Hz, 1H), 7.08 (t, *J* = 9.1 Hz, 2H), 4.96 (dd, *J* = 13.3, 5.0 Hz, 1H), 4.15 (dd, *J* = 50.2, 16.9 Hz, 2H), 3.54 (s, 2H), 3.34 (s, 1H), 3.05 (s, 2H), 2.94 - 2.75 (m, 5H), 2.50 (dd, *J* = 16.8, 10.8 Hz, 2H), 2.29 (dd, *J* = 15.7, 11.4 Hz, 3H), 1.97 (s, 2H), 1.93 - 1.83 (m, 1H), 1.42 (t, *J* = 6.1 Hz, 2H), 0.90 (s, 6H). LCMS (ESI) calcd. for C₃₂H₃₈ClFN₅O₃⁺ [M+H]⁺: 594.26, found, 594.3.

### Example 66: Preparation of compound GT-06538

With reference to the method of Scheme 1, the target compound GT-06538 was obtained (yellow solid, 18 mg, yield 21 %). ¹H NMR (400 MHz, DMSO-d6) δ 10.88 (s, 1H), 9.62 (s, 1H), 7.64 (s, 1H), 7.28 (d, *J* = 10.7 Hz, 1H), 7.17 (dd, *J* = 10.0, 7.8 Hz, 3H), 7.10 (t, *J* = 7.0 Hz, 2H), 4.96 (dd, *J* = 13.2, 5.1 Hz, 1H), 4.15 (dd, *J* = 49.8, 16.9 Hz, 2H), 3.54 (s, 2H), 3.23 - 3.19 (m, 1H), 3.02 (s, 2H), 2.93 - 2.77 (m, 5H), 2.56 - 2.44 (m, 2H), 2.24 (d, *J* = 7.6 Hz, 2H), 1.98 (s, 2H), 1.93 - 1.84 (m, 1H), 1.42 (t, *J* = 6.2 Hz, 2H), 0.90 (s, 6H). LCMS (ESI) calcd. for C₃₂H₃₈F₂N₅O₃ ⁺ [M+H]⁺: 578.29, found, 578.3.

### Example 67: Preparation of compound GT-06539

With reference to the method of Scheme 3, the target compound GT-06539 was obtained (white solid, 10 mg, yield 14 %). ¹H NMR (400 MHz, DMSO-d6) δ 10.95 (s, 1H), 7.40 (d, *J* = 8.4 Hz, 2H), 7.32 (d, *J* = 10.7 Hz, 1H), 7.29 - 7.19 (m, 4H), 5.03 (dd, *J* = 13.3, 5.0 Hz, 1H), 4.23 (dd, *J* = 52.6, 17.0 Hz, 2H), 3.44 (s, 2H), 3.00 (s, 2H), 2.90 (dd, *J* = 11.1, 6.2 Hz, 1H), 2.59 (d, *J* = 15.6 Hz, 4H), 2.47 - 2.28 (m, 4H), 2.13 (s, 1H), 1.99 - 1.91 (m, 2H), 1.47 (d, *J* = 9.9 Hz, 2H), 0.99 (d, *J* = 8.2 Hz, 6H). LCMS (ESI) calcd. for C₃₂H₃₆ClFN₅O₄⁺ [M+H]⁺: 608.24, found, 608.3.

### Example 68: Preparation of compound GT-06540

With reference to the method of Scheme 3, the target compound GT-06540 was obtained (white solid, 30 mg, yield 39 %). ¹H NMR (400 MHz, DMSO-d6) δ 10.94 (s, 1H), 7.36 - 7.28 (m, 2H), 7.26 (dd, *J* = 8.5, 5.7 Hz, 2H), 7.18 (dd, *J* = 16.6, 7.8 Hz, 3H), 5.02 (dd, *J* = 13.2, 5.1 Hz, 1H), 4.22 (dd, *J* = 52.8, 16.9 Hz, 2H), 3.42 (s, 2H), 3.29 (d, *J* = 6.1 Hz, 2H), 2.92 - 2.84 (m, 1H), 2.58 (d, *J* = 16.8 Hz, 4H), 2.48 - 2.28 (m, 4H), 2.12 (s, 1H), 1.93 (d, *J* = 15.0 Hz, 2H), 1.46 (dd, *J* = 11.4, 5.8 Hz, 2H), 0.99 (d, *J* = 11.1 Hz, 6H). LCMS (ESI) calcd. for C₃₂H₃₆F₂N₅O₄⁺ [M+H]⁺: 592.27, found, 592.3.

### Example 69: Preparation of compound GT-06541

With reference to the method of Scheme 2, the target compound GT-06541 was obtained (white solid, 13 mg, yield 19 %). ¹H NMR (400 MHz, DMSO-d6) δ 11.88 (s, 1H), 11.07 (s, 1H), 8.44 (s, 1H), 7.92 (s, 3H), 7.62 (d, *J* = 8.2 Hz, 1H), 7.27 (d, *J* = 35.7 Hz, 5H), 7.01 (s, 1H), 5.69 (s, 1H), 5.04 (dd, *J* = 12.7, 5.3 Hz, 1H), 3.32 - 3.04 (m, 7H), 2.89 (dd, *J* = 22.2, 9.2 Hz, 2H), 2.58 (d, *J* = 17.9 Hz, 2H), 2.06 - 1.88 (m, 1H). LCMS (ESI) calcd. for C₃₀H₂₈F₂N₅O₄⁺ [M+H]⁺: 560.21, found, 560.2.

### Example 70: Preparation of compound GT-06542

With reference to the method of Scheme 2, the target compound GT-06542 was obtained (white solid, 22 mg, yield 30 %). ¹H NMR (400 MHz, DMSO-d6) δ 12.06 (s, 1H), 11.07 (s, 1H), 8.46 (s, 1H), 7.84 (d, *J* = 39.2 Hz, 3H), 7.65 - 7.47 (m, 5H), 7.22 (s, 1H), 7.02 (s, 1H), 5.69 (s, 1H), 5.05 (dd, *J* = 12.7, 5.4 Hz, 1H), 3.23 (s, 4H), 3.06 (s, 3H), 2.98 - 2.81 (m, 2H), 2.58 (d, *J* = 18.1 Hz, 2H), 2.06 - 1.94 (m, 1H). LCMS (ESI) calcd. for C₃₀H₂₈Cl₂N₅O₄⁺ [M+H]⁺: 592.15, found, 592.2.

### Example 71: Preparation of compound GT-06543

With reference to the method of Scheme 2, the target compound GT-06543 was obtained (white solid, 4 mg, yield 5 %). ¹H NMR (400 MHz, DMSO-d6) δ 11.45 (s, 1H), 11.07 (s, 1H), 8.46 (s, 1H), 7.76 (d, *J* = 8.2 Hz, 4H), 7.63 (d, *J* = 8.2 Hz, 1H), 7.23 (d, *J* = 8.5 Hz, 1H), 7.01 (d, *J* = 8.3 Hz, 5H), 5.48 (d, *J* = 8.1 Hz, 1H), 5.05 (dd, *J* = 12.9, 5.2 Hz, 1H), 3.75 (s, 6H), 3.19 (d, *J* = 10.3 Hz, 4H), 3.10 (s, 3H), 2.97 - 2.78 (m, 2H), 2.68 - 2.54 (m, 2H), 2.03 - 1.94 (m, 1H). LCMS (ESI) calcd. for C₃₂H₃₄N₅O₆⁺ [M+H]⁺: 584.25, found, 584.3.

### Example 72: Preparation of compound GT-06544

With reference to the method of Scheme 1, the target compound GT-06544 was obtained (yellow solid, 27 mg, yield 39 %). ¹H NMR (400 MHz, DMSO-d6) δ 11.08 (s, 1H), 10.91 (s, 1H), 8.44 (s, 1H), 8.00 (s, 1H), 7.79 (d, *J* = 8.5 Hz, 3H), 7.64 (d, *J* = 8.3 Hz, 1H), 7.58 (t, *J* = 5.9 Hz, 4H), 7.24 (s, 1H), 7.04 (s, 1H), 5.05 (dd, *J* = 12.8, 5.3 Hz, 1H), 4.42 (s, 2H), 3.40 (s, 2H), 3.32 - 3.19 (m, 2H), 3.13 (d, *J* = 10.8 Hz, 2H), 3.03 (s, 2H), 2.89 (dd, *J* = 15.6, 10.1 Hz, 1H), 2.60 (dd, *J* = 36.5, 18.6 Hz, 2H), 2.08 - 1.95 (m, 1H). LCMS (ESI) calcd. for C₃₀H₂₉ClN₅O₄⁺ [M+H]⁺: 558.19, found, 558.2.

### Example 73: Preparation of compound GT-06545

With reference to the method of Scheme 1, the target compound GT-06545 was obtained (yellow solid, 24 mg, yield 35 %). ¹H NMR (400 MHz, DMSO-d6) δ 11.09 (d, *J* = 8.4 Hz, 1H), 10.81 (s, 1H), 8.44 (s, 1H), 7.84 - 7.69 (m, 6H), 7.64 (d, *J* = 8.3 Hz, 1H), 7.55 (d, *J* = 8.6 Hz, 2H), 7.24 (s, 1H), 7.04 (s, 1H), 5.06 (dd, *J* = 12.9, 5.3 Hz, 1H), 4.40 (s, 2H), 3.14 (d, *J* = 11.4 Hz, 2H), 3.07 - 2.92 (m, 2H), 2.89 (dd, *J* = 15.6, 10.4 Hz, 1H), 2.65 - 2.52 (m, 2H), 2.10 - 1.91 (m, 1H). LCMS (ESI) calcd. for C₃₀H₂₉ClN₅O₄⁺ [M+H]⁺: 558.19, found, 558.2.

### Example 74: Preparation of compound GT-06546

With reference to the method of Scheme 1, the target compound GT-06546 was obtained (yellow solid, 26 mg, yield 38 %). ¹H NMR (400 MHz, DMSO-d6) δ 11.07 (s, 1H), 10.15 (s, 1H), 8.38 (s, 1H), 7.62 (t, *J* = 8.9 Hz, 1H), 7.28 (d, *J* = 7.3 Hz, 3H), 7.23 - 7.13 (m, 1H), 7.03 (s, 1H), 5.04 (dd, *J* = 12.7, 5.3 Hz, 1H), 4.36 (s, 2H), 3.84 (t, *J* = 5.3 Hz, 2H), 3.67 (s, 2H), 3.40 (s, 2H), 3.32 - 3.19 (m, 2H), 3.18 - 2.85 (m, 6H), 2.58 (d, *J* = 18.3 Hz, 2H), 2.38 (s, 1H), 2.04 - 1.96 (m, 1H). LCMS (ESI) calcd. for C₂₉H₃₁FN₅O₅⁺ [M+H]⁺: 548.23, found, 548.2.

### Example 75: Preparation of compound GT-06547

With reference to the method of Scheme 1, the target compound GT-06547 was obtained (yellow solid, 26 mg, yield 36 %). ¹H NMR (400 MHz, DMSO-d6) δ 11.00 (s, 1H), 10.00 (s, 1H), 8.30 (s, 1H), 7.54 (d, *J* = 8.3 Hz, 1H), 7.43 (d, *J* = 8.3 Hz, 2H), 7.17 (d, *J* = 8.2 Hz, 2H), 7.12 (s, 1H), 6.94 (d, *J* = 7.6 Hz, 1H), 4.97 (dd, *J* = 12.8, 5.4 Hz, 1H), 4.29 (s, 2H), 3.62 (s, 2H), 2.93 (s, 6H), 2.79 (dd, *J* = 16.7, 5.0 Hz, 2H), 2.51 (d, *J* = 18.3 Hz, 3H), 2.17 (s, 2H), 1.94 - 1.88 (m, 1H), 1.16 (s, 6H). LCMS (ESI) calcd. for C₃₁H₃₅ClN₅O₅⁺ [M+H]⁺: 592.23, found, 592.3.

### Example 76: Preparation of compound GT-07057

With reference to the method of Scheme 3, the target compound GT-07057 was obtained (white solid, 29 mg, yield 43 %). ¹H NMR (500 MHz, DMSO-d6) δ 11.08 (s, 1H), 7.66 (d, *J* = 8.5 Hz, 1H), 7.37 (d, *J* = 8.4 Hz, 2H), 7.33 - 7.30 (m, 2H), 7.25 - 7.20 (m, 2H), 5.07 (dd, *J* = 12.8, 5.4 Hz, 1H), 4.24 - 3.90 (m, 6H), 2.92 - 2.84 (m, 1H), 2.65 - 2.55 (m, 4H), 2.27 (brs, 2H), 2.14 - 1.97 (m, 3H), 1.44 - 1.39 (m, 2H), 0.98 (s, 6H). LCMS (ESI) calcd. for C₃₂H₃₅ClN₅O₅⁺ [M+H]⁺: 604.23, found, 604.3.

### Example 77: Preparation of compound GT-07058

With reference to the method of Scheme 3, the target compound GT-07058 was obtained (white solid, 48 mg, yield 68 %). ¹H NMR (500 MHz, DMSO-d6) δ 11.07 (s, 1H), 9.80 (d, J = 1.8 Hz, 1H), 8.81 (d, J = 1.8 Hz, 1H), 7.48 - 7.40 (m, 3H), 7.25 (d, J = 8.5 Hz, 2H), 6.86 (d, J = 1.3 Hz, 1H), 6.21 (s, 1H), 5.04 (dd, J = 12.8, 5.4 Hz, 1H), 2.94 - 2.79 (m, 1H), 2.59 - 2.56 (m, 1H), 2.48 - 2.46 (m, 1H), 2.35 (t, J = 6.2 Hz, 2H), 2.10 (s, 2H), 2.03 - 1.97 (m, 1H), 1.46 (t, J = 6.4 Hz, 2H), 1.02 (s, 6H). LCMS (ESI) calcd. for C₂₈H₂₈ClN₄O₅⁺ [M+H]⁺: 535.17, found, 535.2.

### Example 78: Preparation of compound GT-07059

With reference to the method of Scheme 3, the target compound GT-07059 was obtained (white solid, 26 mg, yield 39 %). ¹H NMR (500 MHz, DMSO-d6) δ 10.98 (s, 1H), 7.62 (d, J = 8.0 Hz, 1H), 7.54 (s, 1H), 7.41 (d, J = 8.0 Hz, 1H), 7.36 (d, J = 8.5 Hz, 2H), 7.20 (d, J = 8.5 Hz, 2H), 5.09 (dd, J = 13.3, 5.1 Hz, 1H), 4.41 (d, J = 17.4 Hz, 1H), 4.29 (d, J = 17.4 Hz, 1H), 3.03 - 2.86 (m, 4H), 2.64 - 2.58 (m, 2H), 2.42 - 2.36 (m, 2H), 2.27 (s, 2H), 2.21 - 2.15 (m, 1H), 2.08 (d, J = 1.7 Hz, 2H), 2.04 - 1.94 (m, 1H), 1.80 (d, J = 12.5 Hz, 2H), 1.57 (s, 1H), 1.41 (t, J = 6.3 Hz, 2H), 1.35 (d, J = 9.6 Hz, 2H), 0.98 (s, 6H). LCMS (ESI) calcd. for C₃₄H₄₀ClN₄O₄S⁺ [M+H]⁺: 635.25, found, 635.3.

### Example 79: Preparation of compound GT-07066

With reference to the method of Scheme 7, the target compound GT-07066 was obtained (white solid, 26 mg, yield 38 %). ¹H NMR (500 MHz, DMSO-d6) δ 10.94 (s, 1H), 8.70 (s, 1H), 7.50 (d, J = 8.5 Hz, 1H), 7.40 - 7.34 (m, 2H), 7.30 (d, J = 8.6 Hz, 1H), 7.26 - 7.19 (m, 1H), 7.08 - 6.99 (m, 2H), 5.04 (dd, J = 13.3, 5.1 Hz, 1H), 4.31 (d, J = 16.9 Hz, 1H), 4.19 (d, J = 16.9 Hz, 1H), 3.33 - 3.20 (m, 3H), 2.96 - 2.83 (m, 1H), 2.63 - 2.54 (m, 4H), 2.52 - 2.50 (m, 1H), 2.44 - 2.31 (m, 2H), 2.27 (brs, 2H), 2.08 - 2.05(m, 2H), 2.01 - 1.89 (m, 1H), 1.43 - 1.35 (m, 2H), 0.98 (s, 6H). LCMS (ESI) calcd. for C₃₂H₃₇ClN₅O₄⁺ [M+H]⁺: 590.25, found, 590.3.

### Example 80: Preparation of compound GT-06911

With reference to the method of Scheme 2, the target compound GT-06911 was obtained (white solid, 14 mg, yield 35 %). ¹H NMR (400 MHz, DMSO-d6) δ 11.10 (s, 1H), 8.80 (d, J = 4.0 Hz, 1H), 8.03 - 7.89 (m, 1H), 7.89 - 7.61 (m, 3H), 7.60 - 7.32 (m, 6H), 7.32 - 7.00 (m, 2H), 5.09 (dd, J = 12.9, 5.4 Hz, 1H), 3.81 (s, 3H), 3.30 - 3.06 (m, 6H), 2.87 (t, 1H), 2.70 - 2.53 (m, 2H), 2.08 - 1.97 (m, 1H). LCMS (ESI) calcd. for C₂₉H₂₉N₆O₄⁺ [M+H]+: 525.22, found, 525.3.

### Example 81: Preparation of compound GT-06902

With reference to the method of Scheme 1, the target compound GT-06902 was obtained (white solid, 16 mg, yield 40 %). ¹H NMR (400 MHz, DMSO-d6) δ 11.09 (s, 1H), 7.90 (d, J = 6.1, 3.2 Hz, 1H), 7.70 (d, J = 8.5 Hz, 1H), 7.56 - 7.49 (m, 3H), 7.43 - 7.35 (m, 5H), 7.32 - 7.24 (m, 2H), 5.08 (dd, J = 12.9, 5.3 Hz, 1H), 3.68 - 3.59 (m, 4H), 3.20 - 3.11 (m, 4H), 2.88 (td, J = 14.2, 7.5 Hz, 1H), 2.66 - 2.52 (m, 2H), 2.13 - 1.88 (m, 1H). LCMS (ESI) calcd. for C₃₀H₂₇ClN₅O₄⁺ [M+H]⁺: 556.17, found, 556.2.

### Example 82: Preparation of compound GT-07209

With reference to the method of Scheme 1, the target compound GT-07209 was obtained (white solid, 5 mg, yield 6 %). ¹H NMR (400 MHz, DMSO-d6) δ 11.08 (s, 1H), 7.93 - 7.87 (m, 1H), 7.69 (d, J = 8.5 Hz, 1H), 7.57 - 7.46 (m, 3H), 7.42 - 7.35 (m, 4H), 7.32 - 7.25 (m, 2H), 5.07 (dd, J = 13.0, 5.4 Hz, 1H), 3.63 (s, 4H), 3.27 - 3.20 (m, 2H), 3.16 (s, 4H), 2.94 - 2.76 (m, 2H), 2.59 (d, J = 16.6 Hz, 1H), 2.08 - 1.98 (m, 1H). LCMS (ESI) calcd. for C₃₀H₂₉ClN₅O₄⁺ [M+H]⁺: 558.19, found, 558.2

### Example 83: Preparation of compound GT-06903

With reference to the method of Scheme 1, the target compound GT-06903 was obtained (white solid, 22 mg, yield 56 %). ¹H NMR (400 MHz, DMSO-d6) δ 11.10 (s, 1H), 7.74 - 7.61 (m, 1H), 7.46 - 7.31 (m, 3H), 7.31 - 7.21 (m, 4H), 5.09 (dd, *J* = 12.8, 5.4 Hz, 1H), 3.70 - 3.52 (m, 5H), 3.21 (d, *J* = 25.8 Hz, 2H), 3.14 - 2.64 (m, 5H), 2.64 - 2.54 (m, 2H), 2.50 - 2.30 (m, 3H), 2.06 - 2.00 (m, 1H), 1.80 - 1.60 (m, 3H). LCMS (ESI) calcd. for C₃₀H₃₁FN₅O₄⁺ [M+H]⁺: 544.24, found, 554.3.

### Example 84: Preparation of compound GT-07211

With reference to the method of Scheme 1, the target compound GT-07211 was obtained (white solid, 22 mg, yield 27%). ¹H NMR (400 MHz, DMSO-d6) δ 11.08 (s, 1H), 10.70 (s, 1H), 7.69 (dd, J = 14.0, 8.4 Hz, 1H), 7.45 - 7.19 (m, 6H), 5.08 (dd, J = 12.9, 5.5 Hz, 1H), 3.55 (s, 6H), 3.11 - 2.80 (m, 5H), 2.66 - 2.52 (m, 2H), 2.42 - 2.32 (m, 1H), 2.26 (s, 3H), 2.09 - 1.96 (m, 1H), 1.69 (s, 4H). LCMS (ESI) calcd. for C₃₀H₃₃FN₅O₄⁺ [M+H]⁺: 546.25, found, 546.3.

### Example 85: Preparation of compound GT-06904

With reference to the method of Scheme 1, the target compound GT-06904 was obtained (white solid, 20 mg, yield 49 %). ¹H NMR (400 MHz, DMSO-d6) δ 11.09 (s, 1H), 7.69 (d, *J* = 8.5 Hz, 1H), 7.46 (d, *J* = 8.5 Hz, 2H), 7.39 - 7.31 (m, 3H), 7.30 - 7.22 (m, 2H), 5.07 (dd, *J* = 12.9, 5.4 Hz, 1H), 4.37 (s, 2H), 3.82 (t, *J* = 5.5 Hz, 2H), 3.62 - 3.54 (m, 4H), 3.07 - 2.96 (m, 4H), 2.93 - 2.83 (m, 1H), 2.66 - 2.53 (m, 2H), 2.48 (s, 2H), 2.06 - 1.98 (m, 1H). LCMS (ESI) calcd. for C₂₉H₂₉ClN₅O₅⁺ [M+H]⁺: 562.19, found, 562.2.

### Example 86: Preparation of compound GT-07212

With reference to the method of Scheme 1, the target compound GT-07212 was obtained (white solid, 8 mg, yield 10 %). ¹H NMR (400 MHz, DMSO-d6) δ 11.08 (d, J = 3.8 Hz, 1H), 7.78 - 7.65 (m, 1H), 7.52 - 7.24 (m, 6H), 5.14 - 4.94 (m, 1H), 4.32 (d, J = 34.9 Hz, 2H), 3.81 (t, J = 5.4 Hz, 2H), 3.66 - 3.54 (m, 6H), 3.15 - 2.97 (m, 4H), 2.91 - 2.81 (m, 1H), 2.64 - 2.53 (m, 2H), 2.48 - 2.31 (m, 2H), 2.03 (t, J = 12.3 Hz, 1H). LCMS (ESI) calcd. for C₂₉H₃₁ClN₅O₅⁺ [M+H]⁺: 564.20, found, 564.2

### Example 87: Preparation of compound GT-06905

With reference to the method of Scheme 1, the target compound GT-06905 was obtained (white solid, 13 mg, yield 31 %). ¹H NMR (400 MHz, DMSO-d6) δ 11.10 (s, 1H), 7.70 (d, *J* = 8.5 Hz, 1H), 7.47 - 7.42 (m, 2H), 7.37 (d, *J* = 2.1 Hz, 1H), 7.30 - 7.25 (m, 2H), 7.25 - 7.20 (m, 2H), 5.09 (dd, *J* = 12.9, 5.4 Hz, 1H), 3.62 - 3.55 (m, 4H), 3.05 - 2.94 (m, 4H), 2.93 - 2.83 (m, 1H), 2.67 - 2.54 (m, 2H), 2.40 (s, 2H), 2.18 (s, 2H), 2.04 (dd, *J* = 9.0, 3.7 Hz, 1H), 1.47 (t, *J* = 6.5 Hz, 2H), 0.99 (s, 6H). LCMS (ESI) calcd. for C₃₂H₃₅ClN₅O₄⁺ [M+H]⁺: 588.24, found, 588.3.

### Example 88: Preparation of compound GT-07214

With reference to the method of Scheme 1, the target compound GT-07214 was obtained (white solid, 18 mg, yield 25 %). ¹H NMR (400 MHz, DMSO-d6) δ 11.08 (s, 1H), 10.83 (s, 1H), 7.69 (dd, J = 14.3, 8.5 Hz, 1H), 7.41 (ddd, J = 24.0, 15.3, 5.1 Hz, 3H), 7.32 - 7.18 (m, 3H), 5.08 (dd, J = 12.9, 5.6 Hz, 1H), 3.57 (d, J = 4.8 Hz, 4H), 3.09 - 2.79 (m, 5H), 2.63 - 2.53 (m, 3H), 2.37 (d, J = 5.1 Hz, 1H), 2.21 (d, J = 43.8 Hz, 2H), 2.02 (d, J = 11.7 Hz, 3H), 1.44 (s, 2H), 0.97 (d, J = 5.0 Hz, 6H). LCMS (ESI) calcd. for C₃₂H₃₇ClN₅O₄⁺ [M+H]⁺: 590.25, found, 590.3.

### Example 89: Preparation of compound GT-06907

With reference to the method of Scheme 1, the target compound GT-06907 was obtained (pale yellow solid, 28 mg, yield 43 %). ¹H NMR (500 MHz, ) δ 11.13 (s, 1H), 11.03 (s,1H), 8.07 (d, *J* = 7.6 Hz,1H), 7.83 (s,1H), 7.65 (d, *J* = 8.3 Hz,2H), 7.51 (d, *J* = 8.4 Hz,2H), 7.42 - 7.39 (m,1H), 7.39 - 7.37 (m,1H), 7.35 (s,1H), 7.34 (s,1H), 7.26 (d, *J* = 7.4 Hz,1H), 7.24 (s,1H), 5.02 (dd, *J* = 12.8, 5.4 Hz,1H), 2.82 (d, *J* = 2.0 Hz,1H), 2.51 (t, *J* = 15.2 Hz,2H), 1.96 (d, *J* = 5.0 Hz,1H). LCMS (ESI) calcd. for C₂₆H₂₀ClN₄O₄⁺ [M+H]⁺: 487.12, found, 487.2.

### Example 90: Preparation of compound GT-06908

With reference to the method of Scheme 1, the target compound GT-06908 was obtained (pale yellow solid, 21 mg, yield 33 %). ¹H NMR (500 MHz, ) δ 11.01 (s,1H), 10.79 (s,1H), 7.60 (d, *J* = 8.3 Hz,1H), 7.52 (s,1H), 7.25 - 7.20 (m,2H), 7.18 (d, *J* = 8.9 Hz,2H), 4.99 (dd, *J* = 12.8, 5.4 Hz,1H), 3.26 (s,1H), 2.81 (d, *J* = 2.0 Hz,1H), 2.58 - 2.45 (m,2H), 2.40 (d, *J* = 4.6 Hz,1H), 2.36 (s,2H), 1.95 (d, *J* = 5.4 Hz,1H), 1.66 (s,4H). LCMS (ESI) calcd. for C₂₆H₂₄FN₄O₄⁺ [M+H]⁺: 475.18, found, 475.3.

### Example 91: Preparation of compound GT-06909

With reference to the method of Scheme 1, the target compound GT-06909 was obtained (pale yellow solid, 10 mg, yield 15 %). ¹H NMR (500 MHz, ) δ 11.10 (s, 1H), 10.90 (s, 1H), 7.69 (d, *J* = 8.3 Hz,1H), 7.61 (s,1H), 7.50 (d, *J* = 8.4 Hz,2H), 7.26 (d, *J* = 8.4 Hz,2H), 7.20 (s, 1H), 5.09 (dd, *J* = 12.8, 5.4 Hz,1H), 2.92 - 2.89 (m,1H), 2.60 (d, *J* = 19.8 Hz,1H), 2.54 (s, 2H), 2.23 (s, 2H), 2.06 - 2.02 (m, 1H), 1.54 (t, *J* = 6.5 Hz, 2H), 1.25 (s, 2H), 1.02 (s, 6H). LCMS (ESI) calcd. for C₂₈H₂₈ClN₄O₄⁺ [M+H]⁺: 519.18, found, 519.2.

### Example 92: Preparation of compound GT-06906

With reference to the method of Scheme 1, the target compound GT-06906 was obtained (pale yellow solid, 6 mg, yield 10 %). LCMS (ESI) calcd. for C₂₅H₂₂N₅O₄⁺ [M+H]⁺: 456.17, found, 456.2.

### Example 93: Preparation of compound GT-06910

With reference to the method of Scheme 1, the target compound GT-06910 was obtained (pale yellow solid, 10 mg, yield 12 %). LCMS (ESI) calcd. for C₂₈H₂₈ClN₄O₄⁺ [M+H]⁺: 519.18, found, 519.2.

### Example 94: Preparation of compound GT-06962

With reference to the method of Scheme 1, the target compound GT-06962 was obtained (17 mg, white solid, yield 27%). ¹H NMR (400 MHz, MeOD) δ 7.49 (d, *J* = 8.5 Hz, 2H), 7.45 (d, *J* = 8.2 Hz, 1H), 7.37 - 7.31 (m, 1H), 7.24 (d, *J* = 8.4 Hz, 2H), 5.08 (dd, *J* = 13.3, 5.1 Hz, 1H), 4.45 (q, *J* = 16.7 Hz, 2H), 4.33 (s, 2H), 3.94 (t, *J* = 5.5 Hz, 2H), 3.79 (s, 2H), 3.52 (d, *J* = 8.9 Hz, 2H), 3.20 - 3.09 (m, 2H), 3.03 - 2.93 (m, 4H), 2.87 (dd, *J* = 13.4, 5.2 Hz, 1H), 2.80 - 2.71 (m, 1H), 2.55 - 2.42 (m, 3H), 2.17 - 2.11 (m, 1H). LCMS (ESI) calcd. for C₂₉H₃₂ClFN₅O₄⁺ [M+H]⁺: 558.21, found, 568.3.

### Example 95: Preparation of compound GT-06963

With reference to the method of Scheme 1, the target compound GT-06963 was obtained (17 mg, white solid, yield 27%). ¹H NMR (400 MHz, MeOD) δ 7.47 (d, *J* = 8.2 Hz, 2H), 7.23 (d, *J* = 8.2 Hz, 2H), 6.72 (s, 1H), 6.60 (d, *J* = 12.7 Hz, 1H), 5.03 (dd, *J* = 13.8, 5.5 Hz, 1H), 4.42 - 4.25 (m, 4H), 4.01 - 3.87 (m, 2H), 3.85 - 3.71 (m, 2H), 3.54 - 3.45 (m, 2H), 3.19 - 3.07 (m, 2H), 3.05 - 2.84 (m, 5H), 2.80 - 2.71 (m, 1H), 2.54 - 2.45 (m, 2H), 2.44 - 2.37 (m, 1H), 2.18 - 2.03 (m, 1H). LCMS (ESI) calcd. for C₂₉H₃₂ClFN₅O₄⁺ [M+H]⁺: 558.21, found, 568.3.

### Example 96: Preparation of compound GT-07990

With reference to the method of Scheme 1, the target compound GT-07990 was obtained (27 mg, white solid, yield 48%). LCMS (ESI) calcd. for C₃₄H₄₂ClN₆O₅⁺ [M+H]⁺: 649.29, found, 649.2.

### Example 97: Preparation of compound GT-07996

With reference to the method of Scheme 1, the target compound GT-07996 was obtained (33 mg, white solid, yield 58%). LCMS (ESI) calcd. for C₃₄H₄₀ClN₆O₆⁺ [M+H]⁺: 663.27, found, 663.2.

### Example 98: Preparation of compound GT-08034

With reference to the method of Scheme 1, the target compound GT-08034 was obtained (10 mg, white solid, yield 55%). ¹H NMR (400 MHz, MeOD) δ 7.55 - 7.47 (m, 3H), 7.36 - 7.28 (m, 2H), 6.99 (s, 1H), 6.92 - 6.88 (m, 1H), 5.28 - 5.00 (m, 3H), 4.40 - 4.31 (m, 1H), 4.14 (s, 1H), 3.84 (s, 1H), 3.67 - 3.61 (m, 2H), 3.53 - 3.48 (m, 1H), 3.30 (s, 4H), 3.17 - 3.10 (m, 2H), 3.02 - 2.83 (m, 2H), 2.82 - 2.68 (m, 2H), 2.55 - 2.36 (m, 1H), 2.20 - 2.09 (m, 1H), 2.09 - 1.98 (m, 2H). LCMS (ESI) calcd. for C₂₉H₃₄ClFN₆O₃⁺ [M+H]⁺: 549.24, found, 549.2.

### Example 99: Preparation of compound GT-08040

With reference to the method of Scheme 1, the target compound GT-08040 was obtained (15 mg, white solid, yield 66%). ¹H NMR (400 MHz, MeOD) δ 7.59 (d, *J* = 8.2 Hz, 1H), 7.52 (d, *J* = 8.3 Hz, 2H), 7.33 - 7.23 (m, 3H), 7.06 (d, *J* = 8.0 Hz, 1H), 5.04 (dd, *J* = 13.3, 6.2 Hz, 1H), 4.13 (s, 2H), 3.84 (s, 2H), 3.67 - 3.56 (m, 2H), 3.56 - 3.46 (m, 2H), 3.12 (s, 4H), 2.99 (s, 2H), 2.88 - 2.64 (m, 5H), 2.14 - 2.04 (m, 1H). LCMS (ESI) calcd. for C₂₉H₃₂ClN₆O₄⁺ [M+H]⁺: 563.22, found, 563.2.

### Example 100: Preparation of compound GT-08865

With reference to the method of Scheme 5, the target compound GT-08865 was obtained (46 mg, white solid, yield 46%). ¹H NMR (400 MHz, DMSO-d6) δ 11.10 (s, 1H), 7.62 (t, *J* = 7.5 Hz, 1H), 7.50 (d, *J* = 8.3 Hz, 1H), 7.28 (s, 1H), 7.11 (d, *J* = 6.7 Hz, 1H), 5.16 - 4.98 (m, 1H), 2.84 (d, *J* = 36.1 Hz, 6H), 2.59 (d, *J* = 18.7 Hz, 5H), 2.08 - 1.98 (m, 1H), 1.04 (s, 9H). LCMS (ESI) calcd. for C₂₁H₂₈N₅O₄⁺ [M+H]⁺: 414.21, found, 414.3.

### Example 101: Preparation of compound GT-08531

With reference to the method of Scheme 5, the target compound GT-08531 was obtained (630 mg, white solid, yield 35%). ¹H NMR (400 MHz, DMSO-d6) δ 11.06 (s, 1H), 8.05 (s, 1H), 7.60 (d, *J* = 8.3 Hz, 1H), 7.14 (s, 1H), 7.01 (d, *J* = 7.8 Hz, 1H), 5.04 (dd, *J* = 12.9, 5.4 Hz, 1H), 2.92 - 2.83 (m, 1H), 2.73 (dd, *J* = 45.1, 17.9 Hz, 4H), 2.57 (dd, *J* = 19.7, 5.9 Hz, 4H), 2.46 (d, *J* = 4.3 Hz, 2H), 2.20 (s, 3H), 2.03 - 1.96 (m, 1H). LCMS (ESI) calcd. for C₁₈H₂₂N₅O₄⁺ [M+H]⁺: 372.17, found, 372.2.

### Example 102: Preparation of compound GT-08644

With reference to the method of Scheme 5, the target compound GT-08644 was obtained (110 mg, white solid, yield 38%). ¹H NMR (400 MHz, DMSO-d6) δ 10.93 (s, 1H), 7.43 (d, *J* = 8.3 Hz, 1H), 7.25 (s, 1H), 6.90 (s, 1H), 6.85 - 6.78 (m, 1H), 5.02 (dd, *J* = 13.3, 5.1 Hz, 1H), 4.30 (d, *J* = 16.8 Hz, 1H), 4.16 (d, *J* = 16.8 Hz, 1H), 2.95 - 2.85 (m, 1H), 2.72 (s, 4H), 2.59 (d, *J* = 17.5 Hz, 2H), 2.45 (s, 2H), 2.34 (ddd, *J* = 26.4, 13.3, 4.4 Hz, 2H), 2.20 (s, 3H), 1.99 - 1.91 (m, 1H). LCMS (ESI) calcd. for C₁₈H₂₄N₅O₃⁺ [M+H]⁺: 358.19, found, 358.2.

### Example 103: Preparation of compound GT-08711

With reference to the method of Scheme 5, the target compound GT-08711 was obtained (43 mg, white solid, yield 21%).¹H NMR (400 MHz, DMSO-d6) δ 11.06 (s, 1H), 8.01 (s, 1H), 7.60 (d, *J* = 8.3 Hz, 1H), 7.16 (s, 1H), 7.03 (s, 1H), 5.04 (dd, *J* = 12.9, 5.4 Hz, 1H), 2.93 - 2.82 (m, 2H), 2.70 (d, *J* = 23.7 Hz, 4H), 2.65 - 2.52 (m, 4H), 2.15 - 1.55 (m, 2H), 1.04 (s, 9H). LCMS (ESI) calcd. for C₂₁H₂₈N₅O₄⁺ [M+H]⁺: 414.21, found, 414.3.

### Example 104: Preparation of compound GT-09057

With reference to the method of Scheme 5, the target compound GT-09057 was obtained (63 mg, white solid, yield 41%). ¹H NMR (400 MHz, DMSO-d6) δ 11.01 (s, 1H), 9.71 (s, 1H), 7.31 (t, *J* = 7.7 Hz, 1H), 7.14 (s, 1H), 7.09 (s, 1H), 7.07 (d, *J* = 1.2 Hz, 1H), 5.10 (dd, *J* = 13.3, 5.0 Hz, 1H), 4.35 (d, *J* = 17.4 Hz, 1H), 4.22 (d, *J* = 17.4 Hz, 1H), 3.46 - 3.43 (m, 2H), 3.21 (d, *J* = 8.7 Hz, 2H), 3.15 (d, *J* = 12.9 Hz, 2H), 2.99 - 2.89 (m, 1H), 2.84 (s, 3H), 2.79 (s, 2H), 2.62 (d, *J* = 17.1 Hz, 1H), 2.39 (ddd, *J* = 26.5, 13.3, 4.4 Hz, 1H), 2.07 - 1.98 (m, 1H). LCMS (ESI) calcd. for C₁₈H₂₄N₅O₃⁺ [M+H]⁺: 358.19, found, 358.2.

### Example 105: Preparation of compound GT-09058

With reference to the method of Scheme 5, the target compound GT-09058 was obtained (190 mg, white solid, yield 50%). ¹H NMR (400 MHz, DMSO-d6) δ 11.11 (s, 1H), 9.75 (s, 1H), 7.95 (s, 1H), 7.65 (dd, *J* = 8.4, 7.2 Hz, 1H), 7.54 (d, *J* = 8.5 Hz, 1H), 7.15 (d, *J* = 6.7 Hz, 1H), 5.07 (dd, *J* = 12.7, 5.4 Hz, 1H), 3.47 (s, 2H), 3.27 (s, 2H), 3.09 (s, 4H), 2.93 (d, *J* = 5.9 Hz, 1H), 2.83 (s, 3H), 2.59 (d, *J* = 19.1 Hz, 1H), 2.54 (s, 1H), 2.10 - 1.99 (m, 1H). LCMS (ESI) calcd. for C₁₈H₂₂N₅O₄⁺ [M+H]⁺: 372.17, found, 372.2.

### Example 106: Preparation of compound GT-09917

With reference to the method of Scheme 5, the target compound GT-09917 was obtained (51 mg, white solid, yield 31%). ¹H NMR (400 MHz, DMSO-d6) δ 11.09 (s, 1H), 8.15 (s, 1H), 7.57 (d, *J* = 8.2 Hz, 1H), 7.51 - 7.39 (m, 1H), 5.05 (dd, *J* = 12.8, 5.4 Hz, 1H), 3.35 - 3.30 (m, 4H), 2.88 - 2.82 (m, 1H), 2.73 (s, 4H), 2.63 - 2.51 (m, 2H), 2.02 (d, *J* = 5.7 Hz, 1H), 1.41 (d, *J* = 8.2 Hz, 9H). LCMS (ESI) calcd. for C₂₂H₂₇FN₅O₆⁺ [M+H]⁺: 476.19, found, 476.2.

### Example 107: Preparation of compound GT-09918

With reference to the method of Scheme 5, the target compound GT-09918 was obtained (53 mg, white solid, yield 34%). ¹H NMR (400 MHz, DMSO-d6) δ 11.08 (s, 1H), 8.51 (s, 1H), 7.00 (s, 1H), 6.79 (s, 1H), 5.05 (dd, *J* = 12.8, 5.4 Hz, 1H), 3.36 (s, 4H), 2.88 (ddd, *J* = 16.8, 14.0, 5.5 Hz, 4H), 2.58 (dd, *J* = 17.2, 2.4 Hz, 2H), 2.46 (dd, *J* = 13.3, 4.3 Hz, 1H), 2.05 - 1.97 (m, 1H), 1.42 (s, 9H). LCMS (ESI) calcd. for C₂₂H₂₇FN₅O₆⁺ [M+H]⁺: 476.19, found, 476.2.

### Example 108: Preparation of compound GT-09919

With reference to the method of Scheme 5, the target compound GT-09919 was obtained (32 mg, white solid, yield 19%). ¹H NMR (400 MHz, DMSO-d6) δ 11.03 (s, 1H), 8.03 (s, 1H), 6.79 (s, 1H), 6.47 (s, 1H), 5.00 (dd, *J* = 12.8, 5.4 Hz, 1H), 3.47 (d, *J* = 11.2 Hz, 4H), 3.35 (s, 4H), 3.30 (s, 4H), 3.16 (s, 4H), 2.90 - 2.83 (m, 1H), 2.61 - 2.52 (m, 2H), 1.97 (dd, *J* = 10.8, 5.7 Hz, 1H), 1.42 (d, *J* = 3.1 Hz, 18H). LCMS (ESI) calcd. for C₃₁H₄₄N₇O₈⁺ [M+H]⁺: 642.32, found, 642.4.

### Example 109: Preparation of compound GT-08710

With reference to the method of Scheme 5, the target compound GT-08710 was obtained (68 mg, white solid, yield 35%).¹H NMR (400 MHz, DMSO-d6) δ 11.06 (s, 1H), 8.06 (s, 1H), 7.60 (d, *J* = 8.3 Hz, 1H), 7.15 (s, 1H), 7.02 (d, *J* = 7.1 Hz, 1H), 5.04 (dd, *J* = 12.9, 5.3 Hz, 1H), 2.95 - 2.51 (m, 12H), 1.99 (dd, *J* = 9.5, 6.3 Hz, 1H), 1.00 (d, *J* = 5.3 Hz, 6H). LCMS (ESI) calcd. for C₂₀H₂₆N₅O₄⁺ [M+H]⁺: 400.20, found, 400.3.

### Example 110: Preparation of compound GT-08881

With reference to the method of Scheme 5, the target compound GT-08881 was obtained (53 mg, white solid, yield 36%). ¹H NMR (400 MHz, DMSO-d6) δ 10.93 (s, 1H), 7.43 (d, *J* = 8.3 Hz, 1H), 7.27 (s, 1H), 6.90 (s, 1H), 6.83 (d, *J* = 8.2 Hz, 1H), 5.02 (dd, *J* = 13.3, 5.1 Hz, 1H), 4.29 (d, *J* = 16.8 Hz, 1H), 4.16 (d, *J* = 16.9 Hz, 1H), 2.96 - 2.82 (m, 2H), 2.74 (s, 5H), 2.58 (d, *J* = 16.4 Hz, 4H), 2.33 (dd, *J* = 13.1, 4.4 Hz, 1H), 1.93 (dd, *J* = 12.0, 6.9 Hz, 1H), 1.02 (s, 6H). LCMS (ESI) calcd. for C₂₀H₂₇N₅O₃⁺ [M+H]⁺: 385.21, found, 385.3.

### Example 111: Preparation of compound GT-08880

With reference to the method of Scheme 5, the target compound GT-08880 was obtained (16 mg, white solid, yield 20%). ¹H NMR (400 MHz, DMSO-d6) δ 11.01 (s, 1H), 7.28 (t, *J* = 7.5 Hz, 1H), 7.04 (dd, *J* = 15.3, 7.4 Hz, 2H), 6.76 (s, 1H), 5.09 (dd, *J* = 13.1, 4.8 Hz, 1H), 4.28 (dd, *J* = 39.5, 17.3 Hz, 2H), 3.00 - 2.85 (m, 2H), 2.82 - 2.53 (m, 8H), 2.44 - 2.25 (m, 2H), 2.08 - 2.00 (m, 1H), 0.99 (s, 6H). LCMS (ESI) calcd. for C₂₀H₂₈N₅O₃⁺ [M+H]⁺: 386.22, found, 386.3.

### Example 112: Preparation of compound GT-08866

With reference to the method of Scheme 5, the target compound GT-08866 was obtained (142 mg, white solid, yield 38%). ¹H NMR (400 MHz, DMSO-d6) δ 11.10 (s, 1H), 7.61 (t, *J* = 7.6 Hz, 1H), 7.49 (d, *J* = 8.4 Hz, 1H), 7.33 (s, 1H), 7.10 (d, *J* = 6.8 Hz, 1H), 5.05 (dd, *J* = 12.3, 4.9 Hz, 1H), 2.81 (dd, *J* = 41.7, 21.4 Hz, 5H), 2.69 - 2.51 (m, 7H), 2.07 - 1.97 (m, 1H), 0.98 (d, *J* = 6.2 Hz, 6H). LCMS (ESI) calcd. for C₂₀H₂₆N₅O₄⁺ [M+H]⁺: 400.20, found, 400.3.

### Example 113: Preparation of compound GT-09018

With reference to the method of Scheme 5, the target compound GT-09018 was obtained (8 mg, white solid, yield 18%). ¹H NMR (400 MHz, DMSO-d6) δ 10.92 (s, 1H), 8.20 (s, 1H), 7.42 (d, *J* = 8.4 Hz, 1H), 7.19 (s, 1H), 6.90 (s, 1H), 6.82 (d, *J* = 8.3 Hz, 1H), 5.01 (dd, *J* = 13.3, 5.1 Hz, 1H), 4.29 (d, *J* = 16.8 Hz, 1H), 4.15 (d, *J* = 16.8 Hz, 1H), 2.89 (s, 2H), 2.76 - 2.56 (m, 8H), 2.33 (qd, *J* = 13.7, 9.0 Hz, 1H), 2.01 - 1.90 (m, 1H), 1.03 (s, 9H). LCMS (ESI) calcd. for C₂₁H₃₀N₅O₃⁺ [M+H]⁺: 400.23, found, 400.3.

### Example 114: Preparation of compound GT-09138

With reference to the method of Scheme 5, the target compound GT-09138 was obtained (13 mg, white solid, yield 20%). ¹H NMR (400 MHz, MeOD) δ 7.35 (t, *J* = 7.7 Hz, 1H), 7.25 (d, *J* = 3.4 Hz, 1H), 7.23 (dd, *J* = 4.7, 0.9 Hz, 1H), 5.14 (dd, *J* = 13.2, 5.2 Hz, 1H), 4.44 (s, 2H), 3.64 (d, *J* = 12.2 Hz, 2H), 3.42 - 3.32 (m, 4H), 3.02 - 2.86 (m, 3H), 2.84 - 2.75 (m, 1H), 2.57 - 2.45 (m, 1H), 2.26 - 2.12 (m, 1H), 1.47 (s, 9H). LCMS (ESI) calcd. for C₂₁H₃₀N₅O₃⁺ [M+H]⁺: 400.23, found, 400.3.

### Example 115: Preparation of compound GT-09129

With reference to the method of Scheme 2, the target compound GT-09129 was obtained (24 mg, white solid, yield 39%). ¹H NMR (400 MHz, MeOD) δ 7.73 (d, *J* = 8.2 Hz, 2H), 7.68 (d, *J* = 8.2 Hz, 2H), 7.56 (d, *J* = 8.4 Hz, 1H), 7.04 (s, 1H), 6.96 (dd, *J* = 8.4, 1.7 Hz, 1H), 5.08 (dd, *J* = 13.3, 5.1 Hz, 1H), 4.46 (s, 2H), 4.43 - 4.28 (m, 4H), 3.54 (d, *J* = 12.4 Hz, 2H), 3.48 - 3.41 (m, 4H), 3.29 - 3.16 (m, 3H), 3.06 - 2.93 (m, 4H), 2.82 - 2.72 (m, 1H), 2.51 - 2.40 (m, 1H), 2.20 - 2.08 (m, 1H), 1.94 (d, *J* = 14.7 Hz, 2H), 1.88 - 1.75 (m, 3H), 1.57 - 1.52 (m, 1H). LCMS (ESI) calcd. for C₃₀H₃₉N₆O₃⁺ [M+H]⁺: 531.31, found, 531.3.

### Example 116: Preparation of compound GT-09130

With reference to the method of Scheme 2, the target compound GT-09130 was obtained (13 mg, white solid, yield 26%). ¹H NMR (400 MHz, MeOD) δ 7.73 (d, *J* = 8.1 Hz, 2H), 7.68 (d, *J* = 8.1 Hz, 2H), 7.48 (t, *J* = 7.3 Hz, 1H), 7.44 - 7.38 (m, 1H), 5.09 (dd, *J* = 13.3, 5.1 Hz, 1H), 4.53 - 4.45 (m, 4H), 4.36 (s, 2H), 3.55 (d, *J* = 11.3 Hz, 2H), 3.45 - 4.39 (m, 4H), 3.28 - 3.22 (m, 2H), 3.13 - 3.03 (m, 4H), 2.77 (d, *J* = 15.6 Hz, 1H), 2.56 - 2.41 (m, 1H), 2.37 - 2.32 (m, 1H), 2.24 - 2.10 (m, 1H), 1.94 (d, *J* = 14.2 Hz, 2H), 1.89 - 1.73 (m, 3H), 1.55 - 1.51 (m, 1H). LCMS (ESI) calcd. for C₃₀H₃₈FN₆O₃⁺ [M+H]⁺: 549.30, found, 549.3.

### Example 117: Preparation of compound GT-09131

With reference to the method of Scheme 2, the target compound GT-09131 was obtained (33 mg, white solid, yield 67%).¹H NMR (400 MHz, MeOD) δ 7.74 (d, *J* = 8.2 Hz, 2H), 7.69 (d, *J* = 8.2 Hz, 2H), 7.41 (d, *J* = 7.0 Hz, 1H), 7.35 (d, *J* = 10.5 Hz, 1H), 5.09 (dd, *J* = 13.3, 5.2 Hz, 1H), 4.47 (s, 2H), 4.43 - 4.29 (m, 4H), 3.55 (d, *J* = 12.2 Hz, 2H), 3.47 - 3.40 (m, 4H), 3.25 (d, *J* = 12.1 Hz, 2H), 3.16 - 3.06 (m, 2H), 3.04 - 2.95 (m, 2H), 2.82 - 2.72 (m, 1H), 2.53 - 2.42 (m, 1H), 2.40 - 2.33 (m, 1H), 2.24 - 2.10 (m, 1H), 1.94 (d, *J* = 14.5 Hz, 2H), 1.86 - 1.75 (m, 3H), 1.56 - 1.51 (m, 1H). LCMS (ESI) calcd. for C₃₀H₃₈FN₆O₃⁺ [M+H]⁺: 549.30, found, 549.3.

### Example 118: Preparation of compound GT-09132

With reference to the method of Scheme 2, the target compound GT-09132 was obtained (29 mg, white solid, yield 59%). ¹H NMR (400 MHz, MeOD) δ 7.73 (d, *J* = 8.2 Hz, 2H), 7.69 (d, *J* = 8.2 Hz, 2H), 6.78 (s, 1H), 6.67 (d, *J* = 11.9 Hz, 1H), 5.04 (dd, *J* = 13.3, 5.1 Hz, 1H), 4.46 (s, 2H), 4.42 - 4.27 (m, 4H), 3.53 (d, *J* = 12.3 Hz, 2H), 3.48 - 3.39 (m, 4H), 3.25 - 3.20 (m, 2H), 3.00 (t, *J* = 12.1 Hz, 4H), 2.80 - 2.71 (m, 1H), 2.53 - 2.28 (m, 2H), 2.20 - 2.07 (m, 1H), 1.94 (d, *J* = 14.5 Hz, 2H), 1.86 - 1.75 (m, 3H), 1.59 - 1.44 (m, 1H). LCMS (ESI) calcd. for C₃₀H₃₈FN₆O₃⁺ [M+H]⁺: 549.30, found, 549.3.

### Example 119: Preparation of compound GT-09133

With reference to the method of Scheme 2, the target compound GT-09133 was obtained (32 mg, white solid, yield 65%).¹H NMR (400 MHz, MeOD) δ 7.74 (d, *J* = 8.2 Hz, 2H), 7.68 (d, *J* = 8.3 Hz, 2H), 7.32 (d, *J* = 7.7 Hz, 1H), 7.23 (d, *J* = 6.5 Hz, 1H), 7.21 - 7.15 (m, 1H), 5.15 (dd, *J* = 13.2, 5.2 Hz, 1H), 4.56 - 4.45 (m, 4H), 4.35 (s, 2H), 3.57 - 3.49 (m, 2H), 3.47 - 3.41 (m, 4H), 3.02 - 2.96 (m, 4H), 2.82 - 2.77 (m, 1H), 2.58 - 2.50 (m, 1H), 2.48 - 2.34 (m, 2H), 2.31 - 2.27 (m, 1H), 2.24 - 2.10 (m, 1H), 1.92 (d, *J* = 15.2 Hz, 2H), 1.85 - 1.77 (m, 3H), 1.59 - 1.45 (m, 1H). LCMS (ESI) calcd. for C₃₀H₃₉N₆O₃⁺ [M+H]⁺: 531.31, found, 531.3.

### Example 120: Preparation of compound GT-09134

With reference to the method of Scheme 2, the target compound GT-09134 was obtained (24 mg, white solid, yield 49%). LCMS (ESI) calcd. for C₃₂H₄₁N₆O₃⁺ [M+H]⁺: 557.32, found, 557.3.

### Example 121: Preparation of compound GT-09135

With reference to the method of Scheme 2, the target compound GT-09135 was obtained (30 mg, white solid, yield 61%). ¹H NMR (400 MHz, MeOD) δ 7.79 (d, *J* = 8.2 Hz, 2H), 7.69 (d, *J* = 8.2 Hz, 2H), 7.57 (d, *J* = 8.3 Hz, 1H), 7.02 (s, 1H), 6.95 (d, *J* = 8.3 Hz, 1H), 5.08 (dd, *J* = 13.3, 5.1 Hz, 1H), 4.46 - 4.29 (m, 6H), 4.01 (s, 2H), 3.46 (d, *J* = 12.6 Hz, 2H), 3.19 - 3.11 (m, 4H), 3.06 - 2.94 (m, 2H), 2.93 - 2.83 (m, 1H), 2.80 - 2.74 (m, 1H), 2.51 - 2.44 (m, 4H), 2.41 - 2.33 (m, 1H), 2.19 - 2.11 (m, 2H), 1.93 (d, *J* = 15.0 Hz, 2H), 1.88 - 1.73 (m, 3H), 1.60 - 1.47 (m, 1H). LCMS (ESI) calcd. for C₃₂H₄₁N₆O₃⁺ [M+H]⁺: 557.32, found, 557.3.

### Example 122: Preparation of compound GT-09197

With reference to the method of Scheme 2, the target compound GT-09197 was obtained (16 mg, white solid, yield 33%). ¹H NMR (400 MHz, MeOD) δ 7.66 (s, 4H), 7.57 (d, *J* = 8.2 Hz, 1H), 7.08 (s, 1H), 6.98 (d, *J* = 8.2 Hz, 1H), 5.08 (dd, *J* = 13.3, 5.1 Hz, 1H), 4.63 (s, 2H), 4.42 - 4.30 (m, 2H), 4.45 - 4.27 (m, 4H), 4.05 (t, *J* = 10.8 Hz, 2H), 3.88 (s, 1H), 3.40 (d, *J* = 11.4 Hz, 2H), 3.03 - 2.81 (m, 5H), 2.73 (d, *J* = 15.6 Hz, 1H), 2.49 - 2.30 (m, 2H), 2.18 - 2.04 (m, 5H), 1.91 (d, *J* = 14.7 Hz, 2H), 1.79 (t, *J* = 12.8 Hz, 3H), 1.57 - 1.42 (m, 1H). LCMS (ESI) calcd. for C₃₃H₄₃N₆O₃⁺ [M+H]⁺: 571.34, found, 571.3.

### Example 123: Preparation of compound GT-09198

With reference to the method of Scheme 2, the target compound GT-09198 was obtained (26 mg, white solid, yield 53%).¹H NMR (400 MHz, MeOD) δ 7.75 (d, *J* = 8.1 Hz, 2H), 7.68 (d, *J* = 8.0 Hz, 2H), 7.56 (d, *J* = 8.5 Hz, 1H), 7.06 (s, 1H), 6.95 (d, *J* = 8.0 Hz, 1H), 5.08 (dd, *J* = 13.3, 5.1 Hz, 1H), 4.49 (s, 2H), 4.46 - 4.29 (m, 4H), 3.67 - 3.54 (m, 2H), 3.54 - 3.44 (m, 4H), 3.28 - 3.24 (m, 1H), 3.15 - 3.08 (m, 2H), 3.03 - 2.98 (m, 2H), 2.92 - 2.84 (m, 1H), 2.80 - 2.74 (m, 1H), 2.46 (dd, *J* = 13.2, 4.4 Hz, 1H), 2.42 - 2.33 (m, 1H), 2.24 - 2.09 (m, 3H), 1.92 (t, *J* = 13.8 Hz, 2H), 1.89 - 1.73 (m, 3H), 1.56 - 1.49 (m, 1H). LCMS (ESI) calcd. for C₃₁H₄₁N₆O₃⁺ [M+H]⁺: 545.32, found, 545.3.

### Example 124: Preparation of compound GT-09199

With reference to the method of Scheme 2, the target compound GT-09199 was obtained (33 mg, white solid, yield 67%).¹H NMR (400 MHz, MeOD) δ 7.68 - 7.52 (m, 5H), 7.12 - 7.03 (m, 1H), 6.99 - 6.92 (m, 1H), 5.08 (dd, *J* = 13.3, 5.0 Hz, 1H), 4.42 - 4.23 (m, 7H), 3.79 - 3.57 (m, 3H), 3.22 - 3.00 (m, 4H), 2.92 - 2.84 (m, 2H), 2.81 - 2.66 (m, 2H), 2.54 - 2.37 (m, 2H), 2.20 - 2.12 (m, 2H), 1.84 (brs, 4H), 1.67 (brs, 2H). LCMS (ESI) calcd. for C₃₁H₃₉N₆O₃⁺ [M+H]⁺: 543.31, found, 543.3.

### Example 125: Preparation of compound GT-09200

With reference to the method of Scheme 2, the target compound GT-09200 was obtained (33 mg, white solid, yield 67%). ¹H NMR (400 MHz, MeOD) δ 7.79 (d, *J* = 8.1 Hz, 2H), 7.68 (d, *J* = 8.1 Hz, 2H), 7.58 - 7.51 (m, 1H), 7.07 (s, 1H), 6.99 - 6.91 (m, 1H), 5.07 (dd, *J* = 13.3, 5.0 Hz, 1H), 4.56 (d, *J* = 13.2 Hz, 1H), 4.45 - 4.31 (m, 6H), 3.83 - 3.74 (m, 1H), 3.69 - 3.53 (m, 1H), 3.45 (d, *J* = 12.2 Hz, 3H), 3.03 - 2.97 (m, 3H), 2.86 (dd, *J* = 12.5, 3.9 Hz, 1H), 2.76 (d, *J* = 17.8 Hz, 1H), 2.50 - 2.34 (m, 4H), 2.26 - 2.07 (m, 2H), 1.94 (d, *J* = 14.2 Hz, 2H), 1.85 - 1.78 (m, 3H), 1.58 - 1.44 (m, 1H). LCMS (ESI) calcd. for C₃₁H₃₉N₆O₃⁺ [M+H]⁺: 543.31, found, 543.3.

### Example 126: Preparation of compound GT-09201

With reference to the method of Scheme 2, the target compound GT-09201 was obtained (34 mg, white solid, yield 69%).¹H NMR (400 MHz, MeOD) δ 7.82 (d, *J* = 8.0 Hz, 2H), 7.67 (d, *J* = 8.2 Hz, 2H), 7.54 (d, *J* = 8.4 Hz, 1H), 7.20 (s, 1H), 7.08 (d, *J* = 8.4 Hz, 1H), 5.08 (dd, *J* = 13.3, 5.1 Hz, 1H), 4.49 (s, 2H), 4.43 - 4.30 (m, 4H), 3.60 (d, *J* = 11.5 Hz, 2H), 3.52 (s, 2H), 3.45 (d, *J* = 12.1 Hz, 2H), 3.37 - 3.34 (m, 2H), 3.00 (dt, *J* = 12.5, 6.3 Hz, 2H), 2.91 - 2.82 (m, 1H), 2.81 - 2.70 (m, 1H), 2.51 - 2.42 (m, 1H), 2.39 - 2.24 (m, 3H), 2.19 - 2.06 (m, 3H), 1.93 (d, *J* = 14.5 Hz, 2H), 1.87 - 1.77 (m, 3H), 1.57 - 1.47(m, 1H). LCMS (ESI) calcd. for C₃₂H₄₁N₆O₃⁺ [M+H]⁺: 557.32, found, 557.3.

### Example 127: Preparation of compound GT-09202

With reference to the method of Scheme 2, the target compound GT-09202 was obtained (37 mg, white solid, yield 75%). ¹H NMR (400 MHz, MeOD) δ 7.74 (d, *J* = 8.2 Hz, 2H), 7.69 (d, *J* = 8.2 Hz, 2H), 7.61 (d, *J* = 8.3 Hz, 1H), 7.32 (s, 1H), 7.12 (d, *J* = 7.3 Hz, 1H), 5.06 (dd, *J* = 12.5, 5.5 Hz, 1H), 4.47 (s, 2H), 4.36 (s, 2H), 3.55 (d, *J* = 12.2 Hz, 2H), 3.48 - 3.41 (m, 4H), 3.25 (d, *J* = 12.6 Hz, 2H), 3.13 - 2.98 (m, 4H), 2.90 - 2.80 (m, 1H), 2.77 - 2.74 (m, 1H), 2.68 - 2.65 (m, 1H), 2.13 - 2.09 (m, 1H), 1.94 (d, *J* = 14.6 Hz, 2H), 1.85 - 1.75 (m, 3H), 1.55 - 1.49 (m, 1H). LCMS (ESI) calcd. for C₃₀H₃₇N₆O₄⁺ [M+H]⁺: 545.29, found, 545.3.

### Example 128: Preparation of compound GT-09203

With reference to the method of Scheme 2, the target compound GT-09203 was obtained (14 mg, white solid, yield 28%). ¹H NMR (400 MHz, MeOD) δ 7.80 - 7.72 (m, 5H), 7.47 (d, *J* = 2.2 Hz, 1H), 7.36 (dd, *J* = 8.5, 2.3 Hz, 1H), 5.09 (dd, *J* = 12.4, 5.4 Hz, 1H), 4.39 (s, 2H), 4.06 (d, *J* = 14.0 Hz, 2H), 3.92 - 3.85 (m, 2H), 3.74 - 3.65 (m, 4H), 3.50 (d, *J* = 12.1 Hz, 2H), 3.29 - 3.25 (m, 1H), 3.02 (td, *J* = 12.3, 2.8 Hz, 2H), 2.90 - 2.81 (m, 1H), 2.80 - 2.74 (m, 1H), 2.73 (s, 1H), 2.70 (s, 1H), 2.19 - 2.06 (m, 1H), 1.94 (d, *J* = 14.0 Hz, 2H), 1.89 - 1.76 (m, 3H), 1.62 - 1.47 (m, 1H). LCMS (ESI) calcd. for C₃₀H₃₇N₆O₄⁺ [M+H]⁺: 545.29, found, 545.3.

### Example 129: Preparation of compound GT-09213

With reference to the method of Scheme 2, the target compound GT-09213 was obtained (24 mg, white solid, yield 49%). ¹H NMR (400 MHz, MeOD) δ 7.76 (q, *J* = 8.3 Hz, 4H), 7.69 (d, *J* = 8.5 Hz, 1H), 7.20 (s, 1H), 7.18 (dd, *J* = 8.5, 2.2 Hz, 1H), 5.11 (dd, *J* = 13.3, 5.1 Hz, 1H), 4.51 - 4.32 (m, 5H), 3.96 - 3.85 (m, 5H), 3.66 - 3.58 (m, 4H), 3.50 (d, *J* = 12.3 Hz, 2H), 3.05 - 2.98 (m, 2H), 2.92 - 2.84 (m, 1H), 2.82 - 2.72 (m, 1H), 2.47 (dd, *J* = 13.2, 4.7 Hz, 1H), 2.43 - 2.33 (m, 1H), 2.23 - 2.10 (m, 1H), 1.94 (d, *J* = 14.0 Hz, 2H), 1.89 - 1.80 (m, 3H), 1.61 - 1.49 (m, 1H). LCMS (ESI) calcd. for C₃₀H₃₉N₆O₃⁺ [M+H]⁺: 531.31, found, 531.3.

### Example 130: Preparation of compound GT-09708

With reference to the method of Scheme 3, the target compound GT-09708 was obtained (36 mg, white solid, yield 48%). ¹H NMR (400 MHz, DMSO-d6) δ 10.96 (s, 1H), 7.43 (t, *J* = 8.8 Hz, 4H), 7.32 (d, *J* = 8.8 Hz, 2H), 7.19 (d, *J* = 8.4 Hz, 2H), 6.97 (d, *J* = 8.9 Hz, 2H), 5.04 (dd, *J* = 13.2, 5.1 Hz, 1H), 4.45 (d, *J* = 16.7 Hz, 1H), 4.27 (d, *J* = 16.7 Hz, 1H), 3.77 (d, *J* = 13.8 Hz, 2H), 3.70 - 3.52 (m, 4H), 3.37 - 3.28 (m, 4H), 3.20 (t, *J* = 12.0 Hz, 2H), 2.90 - 2.73 (m, 6H), 2.59 (d, *J* = 16.3 Hz, 1H), 2.39 (d, *J* = 12.7 Hz, 1H), 2.34 - 2.22 (m, 3H), 2.11 (s, 2H), 1.99 - 1.94 (m, 1H), 1.48 (t, *J* = 6.4 Hz, 2H), 1.02 (s, 6H). LCMS (ESI) calcd. for C₄₃H₅₀ClFN₇O₄⁺ [M+H]⁺: 782.36, found, 782.4.

### Example 131: Preparation of compound GT-09709

With reference to the method of Scheme 1, the target compound GT-09709 was obtained (20 mg, white solid, yield 28%). ¹H NMR (400 MHz, DMSO-d6) δ 10.96 (s, 1H), 9.38 (s, 1H), 7.76 (s, 1H), 7.41 (d, *J* = 8.2 Hz, 1H), 7.29 (t, *J* = 7.7 Hz, 2H), 6.94 (d, *J* = 8.0 Hz, 1H), 6.83 (d, *J* = 8.0 Hz, 1H), 5.04 (dd, *J* = 13.3, 5.0 Hz, 1H), 4.45 (d, *J* = 16.8 Hz, 1H), 4.27 (d, *J* = 16.8 Hz, 1H), 4.21 (s, 2H), 3.81 (s, 3H), 3.30 - 3.27 (m, 2H), 3.24 - 3.15 (m, 2H), 3.07 (s, 4H), 2.96 - 2.84 (m, 1H), 2.59 (d, *J* = 17.3 Hz, 1H), 2.44 - 2.35 (m, 1H), 1.98 - 1.94 (m, 1H). LCMS (ESI) calcd. for C₂₅H₂₉FN₅O₅⁺ [M+H]⁺: 498.21, found, 498.2.

### Example 132: Preparation of compound GT-09741

With reference to the method of Scheme 3, the target compound GT-09741 was obtained (19 mg, white solid, yield 33%). ¹H NMR (400 MHz, DMSO-d6) δ 10.95 (s, 1H), 7.36 (d, *J* = 7.7 Hz, 1H), 7.27 (t, *J* = 7.7 Hz, 1H), 7.17 (d, *J* = 8.1 Hz, 2H), 7.11 (d, *J* = 8.1 Hz, 2H), 5.03 (dd, *J* = 13.3, 5.1 Hz, 1H), 4.43 (d, *J* = 16.8 Hz, 1H), 4.25 (d, *J* = 16.8 Hz, 1H), 4.10 (q, *J* = 6.4 Hz, 1H), 3.67 - 3.58 (m, 1H), 2.95 - 2.83 (m, 1H), 2.75 - 2.54 (m, 5H), 2.42 - 2.40 (m, 4H), 2.37 - 2.29 (m, 1H), 2.02 - 1.90 (m, 1H), 1.85 - 1.78 (m, 1H), 1.34 (d, *J* = 7.1 Hz, 1H), 1.28 (d, *J* = 6.8 Hz, 3H), 0.86 (dd, *J* = 6.6, 1.3 Hz, 6H). LCMS (ESI) calcd. for C₃₀H₃₇FN₅O₄⁺ [M+H]⁺: 550.28, found, 550.3.

### Example 133: Preparation of compound GT-09971

With reference to the method of Scheme 2, the target compound GT-09971 was obtained (9 mg, white solid, yield 23%).¹H NMR (400 MHz, MeOD) δ 7.69 - 7.66 (m, 4H), 7.57 - 7.51 (m, 5H), 7.07 - 7.03 (m, 2H), 5.82 (s, 1H), 5.17 - 5.06 (m, 1H), 4.46 - 4.35 (m, 2H), 3.80 - 3.60 (m, 2H), 3.27 - 3.18 (m, 3H), 3.12 - 3.02 (m, 3H), 2.99 - 2.96 (m, 1H), 2.92 - 2.89 (m, 1H), 2.82 - 2.76 (m, 1H), 2.70 - 2.60 (m, 1H), 2.52 - 2.39 (m, 1H), 2.23 - 2.11 (m, 1H). LCMS (ESI) calcd. for C₃₂H₃₂Cl₂N₅O₃⁺ [M+H]⁺: 604.19, found, 604.2.

### Example 134: Preparation of compound GT-09972

With reference to the method of Scheme 3, the target compound GT-09972 was obtained (25 mg, white solid, yield 49%). ¹H NMR (400 MHz, DMSO-d6) δ 10.94 (s, 1H), 7.56 - 7.52 (m, 3H), 7.50 - 7.40 (m, 6H), 6.82 (s, 1H), 6.74 (d, *J* = 8.1 Hz, 1H), 5.03 (dd, *J* = 13.3, 5.1 Hz, 1H), 4.31 (dd, *J* = 16.7, 6.2 Hz, 1H), 4.18 (d, *J* = 17.0 Hz, 1H), 2.96 - 2.86 (m, 2H), 2.78 - 2.65 (m, 5H), 2.62 - 2.55 (m, 4H), 2.43 - 2.32 (m, 2H), 2.02 - 1.92 (m, 1H). LCMS (ESI) calcd. for C₃₂H₃₁ClN₅O₄⁺ [M+H]⁺: 584.21, found, 584.2.

### Example 135: Preparation of compound GT-09973

With reference to the method of Scheme 3, the target compound GT-09973 was obtained (35 mg, white solid, yield 68%). ¹H NMR (400 MHz, DMSO-d6) δ 10.92 (s, 1H), 7.57 - 7.51 (m, 3H), 7.50 - 7.40 (m, 6H), 6.84 (s, 1H), 6.77 (d, *J* = 8.0 Hz, 1H), 5.00 (dd, *J* = 13.2, 5.0 Hz, 1H), 4.53 (s, 1H), 4.27 (dd, *J* = 16.9, 3.2 Hz, 1H), 4.14 (dd, *J* = 17.0, 3.3 Hz, 1H), 3.54 - 3.42 (m, 2H), 2.97 - 2.81 (m, 2H), 2.71 - 2.65 (m, 2H), 2.63 - 2.54 (m, 3H), 2.35 - 2.24 (m, 1H), 1.99 - 1.79 (m, 2H), 1.69 (brs, 1H). LCMS (ESI) calcd. for C₃₂H₃₁ClN₅O₄⁺ [M+H]⁺: 584.21, found, 584.2.

### Example 136: Preparation of compound GT-09974

With reference to the method of Scheme 3, the target compound GT-09974 was obtained (29 mg, white solid, yield 57%). ¹H NMR (400 MHz, DMSO-d6) δ 10.92 (s, 1H), 7.56 - 7.52 (m, 3H), 7.49 - 7.39 (m, 7H), 6.87 (s, 1H), 6.79 (d, *J* = 8.1 Hz, 1H), 5.01 (dd, *J* = 13.3, 5.1 Hz, 1H), 4.28 (d, *J* = 16.9 Hz, 1H), 4.14 (d, *J* = 16.9 Hz, 1H), 3.54 - 3.43 (m, 4H), 3.21 (s, 2H), 2.96 - 2.83 (m, 1H), 2.59 - 2.54 (m, 2H), 2.38 - 2.25 (m, 2H), 1.97 - 1.91 (m, 1H), 1.55 (brs, 2H), 1.44 (brs, 2H). LCMS (ESI) calcd. for C₃₃H₃₃ClN₅O₄⁺ [M+H]⁺: 598.22, found, 598.3.

### Example 137: Preparation of compound GT-09975

With reference to the method of Scheme 3, the target compound GT-09975 was obtained (26 mg, white solid, yield 50%). ¹H NMR (400 MHz, DMSO-d6) δ 10.93 (s, 1H), 7.56 - 7.51 (m, 3H), 7.51 - 7.38 (m, 6H), 6.90 (s, 1H), 6.83 (s, 1H), 5.02 (dd, *J* = 13.3, 5.1 Hz, 1H), 4.30 (d, *J* = 16.8 Hz, 1H), 4.19 (d, *J* = 16.8 Hz, 1H), 3.61 - 3.57 (m, 4H), 3.26 - 3.11 (m, 1H), 3.05 - 2.75 (m, 4H), 2.58 (d, *J* = 16.9 Hz, 2H), 2.40 - 2.26 (m, 1H), 1.97 - 1.93 (m, 1H), 1.69 (brs, 1H). LCMS (ESI) calcd. for C₃₁H₃₁ClN₅O₄⁺ [M+H]⁺: 572.21, found, 572.2.

### Example 138: Preparation of compound GT-09976

With reference to the method of Scheme 3, the target compound GT-09976 was obtained (32 mg, white solid, yield 62%). ¹H NMR (400 MHz, DMSO-d6) δ 10.92 (s, 1H), 7.57 - 7.53 (m, 5H), 7.49 - 7.42 (m, 4H), 6.86 (s, 1H), 6.79 (d, *J* = 8.3 Hz, 1H), 5.01 (dd, *J* = 13.3, 5.0 Hz, 1H), 4.28 (d, *J* = 16.9 Hz, 1H), 4.15 (d, *J* = 17.0 Hz, 1H), 3.60 (s, 1H), 3.23 (s, 2H), 3.12 - 2.99 (m, 1H), 2.94 - 2.83 (m, 1H), 2.70 - 2.63 (m, 1H), 2.60 - 2.55 (m, 2H), 2.37 - 2.28 (m, 1H), 2.04 - 1.87 (m, 2H), 1.64 (s, 1H). LCMS (ESI) calcd. for C₃₁H₂₉ClN₅O₄⁺ [M+H]⁺: 570.19, found, 570.2.

### Example 139: Preparation of compound GT-09977

With reference to the method of Scheme 3, the target compound GT-09977 was obtained (29 mg, white solid, yield 56%). ¹H NMR (400 MHz, DMSO-d6) δ 10.92 (s, 1H), 7.58 - 7.51 (m, 4H), 7.50 - 7.36 (m, 5H), 6.91 (s, 1H), 6.81 (d, *J* = 8.4 Hz, 1H), 5.08 - 4.96 (m, 1H), 4.33 - 4.24 (m, 1H), 4.15 (d, *J* = 16.3 Hz, 1H), 3.68 - 3.63 (m, 4H), 3.21 - 3.08 (m, 1H), 2.97 - 2.81 (m, 2H), 2.58 (d, *J* = 18.1 Hz, 2H), 2.37 - 2.29 (m, 1H), 2.02 - 1.87 (m, 1H), 1.82 - 1.64 (m, 1H). LCMS (ESI) calcd. for C₃₁H₂₉ClN₅O₄⁺ [M+H]⁺: 570.19, found, 570.2.

### Example 140: Preparation of compound GT-09978

With reference to the method of Scheme 3, the target compound GT-09978 was obtained (27 mg, white solid, yield 52%). ¹H NMR (400 MHz, DMSO-d6) δ 10.92 (s, 1H), 7.60 - 7.44 (m, 7H), 7.39 (d, *J* = 8.4 Hz, 2H), 6.95 (s, 1H), 6.88 (t, *J* = 8.7 Hz, 1H), 5.01 (dd, *J* = 13.3, 5.1 Hz, 1H), 4.28 (d, *J* = 16.8 Hz, 1H), 4.13 (dd, *J* = 17.0, 3.4 Hz, 1H), 3.56 - 3.51 (m, 4H), 3.30 - 3.18 (m, 2H), 2.98 - 2.84 (m, 2H), 2.58 (d, *J* = 16.8 Hz, 2H), 2.36 - 2.30 (m, 1H), 2.07 - 1.88 (m, 2H), 1.66 - 1.53 (m, 1H). LCMS (ESI) calcd. for C₃₂H₃₁ClN₅O₄⁺ [M+H]⁺: 584.21, found, 584.3.

### Example 141: Preparation of compound GT-09980

With reference to the method of Scheme 3, the target compound GT-09980 was obtained (14 mg, white solid, yield 26%). ¹H NMR (400 MHz, DMSO-d6) δ 10.94 (s, 1H), 7.43 (s, 1H), 7.40 (d, *J* = 8.0 Hz, 2H), 7.29 (d, *J* = 8.4 Hz, 2H), 6.78 (s, 1H), 6.74 - 6.67 (m, 1H), 5.03 (dd, *J* = 13.4, 4.3 Hz, 1H), 4.30 (d, *J* = 16.7 Hz, 1H), 4.17 (d, *J* = 16.8 Hz, 1H), 3.32 - 3.20 (m, 5H), 2.95 - 2.84 (m, 2H), 2.72 - 2.63 (m, 2H), 2.63 - 2.55 (m, 4H), 2.39 - 2.27 (m, 4H), 1.98 - 1.91 (m, 1H), 1.45 (t, *J* = 6.3 Hz, 2H), 0.99 (s, 6H). LCMS (ESI) calcd. for C₃₄H₃₉ClN₅O₄⁺ [M+H]⁺: 616.27, found, 616.3.

### Example 142: Preparation of compound GT-09981

With reference to the method of Scheme 3, the target compound GT-09981 was obtained (24 mg, white solid, yield 44%). ¹H NMR (400 MHz, DMSO-d6) δ 10.92 (s, 1H), 7.42 - 7.38 (m, 3H), 7.28 (d, *J* = 8.5 Hz, 2H), 6.82 (s, 1H), 6.75 (d, *J* = 7.6 Hz, 1H), 5.00 (dd, *J* = 13.2, 5.0 Hz, 1H), 4.37 (d, *J* = 4.3 Hz, 1H), 4.27 (dd, *J* = 16.9, 3.5 Hz, 1H), 4.13 (dd, *J* = 16.9, 2.5 Hz, 1H), 3.75 (s, 1H), 3.32 - 3.21 (m, 2H), 2.95 - 2.73 (m, 3H), 2.57 (d, *J* = 17.3 Hz, 2H), 2.41 - 2.27 (m, 3H), 2.24 - 2.18 (m, 1H), 2.00 - 1.88 (m, 2H), 1.78 (brs, 1H), 1.65 (brs, 1H), 1.51 - 1.43 (m, 2H), 1.27 - 1.16 (m, 1H), 0.99 (d, *J* = 4.2 Hz, 6H). LCMS (ESI) calcd. for C₃₄H₃₉ClN₅O₄⁺ [M+H]⁺: 616.27, found, 616.3.

### Example 143: Preparation of compound GT-09982

With reference to the method of Scheme 3, the target compound GT-09982 was obtained (18 mg, white solid, yield 34%). ¹H NMR (400 MHz, DMSO-d6) δ 10.93 (s, 1H), 7.43 - 7.36 (m, 3H), 7.28 (d, *J* = 8.4 Hz, 2H), 6.85 (s, 1H), 6.77 (d, *J* = 8.3 Hz, 1H), 5.01 (dd, *J* = 13.4, 5.1 Hz, 1H), 4.28 (d, *J* = 16.8 Hz, 1H), 4.14 (d, *J* = 16.9 Hz, 1H), 3.25 - 3.15 (m, 3H), 2.94 - 2.85 (m, 1H), 2.58 (d, *J* = 17.7 Hz, 2H), 2.35 - 2.24 (m, 5H), 2.14 (s, 3H), 2.01 - 1.86 (m, 1H), 1.43 - 1.40 (m, 5H), 1.35 - 1.19 (m, 2H), 0.98 (s, 6H). LCMS (ESI) calcd. for C₃₅H₄₁ClN₅O₄⁺ [M+H]⁺: 630.28, found, 630.3.

### Example 144: Preparation of compound GT-09983

With reference to the method of Scheme 3, the target compound GT-09983 was obtained (19 mg, white solid, yield 35%). ¹H NMR (400 MHz, DMSO-d6) δ 10.92 (s, 1H), 7.44 - 7.36 (m, 3H), 7.32 - 7.25 (m, 2H), 6.84 (d, *J* = 6.5 Hz, 1H), 6.76 (t, *J* = 8.1 Hz, 1H), 5.01 (dd, *J* = 13.2, 5.0 Hz, 1H), 4.28 (d, *J* = 16.8 Hz, 1H), 4.14 (d, *J* = 16.8 Hz, 1H), 3.34 - 3.18 (m, 3H), 3.03 - 2.83 (m, 2H), 2.80 - 2.65 (m, 2H), 2.58 (d, *J* = 15.9 Hz, 2H), 2.39 - 2.32 (m, 4H), 2.23 - 2.08 (m, 1H), 1.95 - 1.91 (m, 2H), 1.74 (brs, 1H), 1.65 - 1.51 (m, 1H), 1.51 - 1.37 (m, 2H), 0.99 (s, 6H). LCMS (ESI) calcd. for C₃₃H₃₉ClN₅O₄⁺ [M+H]⁺: 604.27, found, 604.3.

### Example 145: Preparation of compound GT-09984

With reference to the method of Scheme 3, the target compound GT-09984 was obtained (37 mg, white solid, yield 68%). ¹H NMR (400 MHz, DMSO-d6) δ 10.92 (s, 1H), 7.43 - 7.40 (m, 3H), 7.29 (d, *J* = 8.5 Hz, 2H), 6.83 (s, 1H), 6.77 (d, *J* = 8.4 Hz, 1H), 5.01 (dd, *J* = 13.2, 5.1 Hz, 1H), 4.27 (d, *J* = 17.0 Hz, 1H), 4.13 (d, *J* = 16.8 Hz, 1H), 3.99 (s, 1H), 3.67 (s, 1H), 3.34 - 2.99 (m, 3H), 2.89 - 2.84 (m, 1H), 2.57 (d, *J* = 19.2 Hz, 2H), 2.41 - 2.18 (m, 4H), 2.00 - 1.85 (m, 3H), 1.48 - 1.41 (m, 2H), 1.29 (brs, 1H), 0.96 (d, *J* = 3.3 Hz, 6H). LCMS (ESI) calcd. for C₃₃H₃₇ClN₅O₄⁺ [M+H]⁺: 602.25, found, 602.3.

### Example 146: Preparation of compound GT-09985

With reference to the method of Scheme 3, the target compound GT-09985 was obtained (36 mg, white solid, yield 66%). ¹H NMR (400 MHz, DMSO-d6) δ 10.92 (s, 1H), 7.50 - 7.32 (m, 4H), 7.28 (d, *J* = 8.4 Hz, 1H), 6.88 (s, 1H), 6.79 (d, *J* = 8.0 Hz, 1H), 5.01 (dd, *J* = 13.0, 4.7 Hz, 1H), 4.27 (d, *J* = 17.1 Hz, 1H), 4.14 (d, *J* = 16.7 Hz, 1H), 3.40 - 3.22 (m, 4H), 2.99 - 2.81 (m, 2H), 2.65 - 2.52 (m, 2H), 2.43 - 2.29 (m, 3H), 2.28 - 2.13 (m, 2H), 2.01 - 1.85 (m, 2H), 1.76 - 1.65 (m, 1H), 1.50 - 1.39 (m, 2H), 0.99 (s, 6H). LCMS (ESI) calcd. for C₃₃H₃₇ClN₅O₄⁺ [M+H]⁺: 602.25, found, 602.3.

### Example 147: Preparation of compound GT-09986

With reference to the method of Scheme 3, the target compound GT-09986 was obtained (25 mg, white solid, yield 46%). ¹H NMR (400 MHz, DMSO-d6) δ 10.92 (s, 1H), 7.46 - 7.34 (m, 4H), 7.21 (d, *J* = 8.0 Hz, 1H), 7.02 - 6.82 (m, 2H), 5.01 (dd, *J* = 13.3, 5.1 Hz, 1H), 4.28 (d, *J* = 16.7 Hz, 1H), 4.13 (d, *J* = 16.8 Hz, 1H), 3.36 - 3.24 (m, 2H), 3.24 - 3.08 (m, 2H), 3.00 (s, 1H), 2.95 - 2.81 (m, 1H), 2.58 (d, *J* = 16.4 Hz, 2H), 2.38 - 2.28 (m, 4H), 2.20 - 2.06 (m, 1H), 2.03 - 1.88 (m, 3H), 1.72 (brs, 1H), 1.52 - 1.34 (m, 3H), 0.98 (d, *J* = 14.1 Hz, 6H). LCMS (ESI) calcd. for C₃₄H₃₉ClN₅O₄⁺ [M+H]⁺: 616.27, found, 616.3.

### Example 148: Preparation of compound GT-09987

With reference to the method of Scheme 3, the target compound GT-09987 was obtained (25 mg, white solid, yield 47%). ¹H NMR (400 MHz, DMSO-d6) δ 10.94 (s, 1H), 7.42 (d, *J* = 8.4 Hz, 1H), 7.36 - 7.25 (m, 2H), 7.17 (t, *J* = 7.9 Hz, 2H), 6.87 - 6.61 (m, 2H), 5.03 (dd, *J* = 13.2, 5.0 Hz, 1H), 4.30 (d, *J* = 17.0 Hz, 1H), 4.16 (d, *J* = 16.9 Hz, 1H), 3.38 - 3.31 (m, 5H), 2.98 - 2.84 (m, 2H), 2.60 - 2.51 (m, 4H), 2.44 - 2.20 (m, 5H), 2.05 - 1.88 (m, 2H), 1.45 (t, *J* = 6.0 Hz, 2H), 0.99 (s, 6H). LCMS (ESI) calcd. for C₃₄H₃₉FN₅O₄⁺ [M+H]⁺: 600.30, found, 600.4.

### Example 149: Preparation of compound GT-09988

With reference to the method of Scheme 3, the target compound GT-09988 was obtained (32 mg, white solid, yield 61%). ¹H NMR (400 MHz, DMSO-d6) δ 10.92 (s, 1H), 7.41 (d, *J* = 8.3 Hz, 1H), 7.30 (dd, *J* = 8.5, 5.7 Hz, 2H), 7.16 (t, *J* = 8.8 Hz, 2H), 6.82 (s, 1H), 6.75 (d, *J* = 8.2 Hz, 1H), 5.01 (dd, *J* = 13.3, 5.1 Hz, 1H), 4.36 (s, 1H), 4.27 (dd, *J* = 16.9, 3.6 Hz, 1H), 4.13 (dd, *J* = 17.0, 2.6 Hz, 1H), 3.76 (s, 1H), 2.89 - 2.83 (m, 1H), 2.80 (d, *J* = 9.8 Hz, 2H), 2.59 - 2.52 (m, 3H), 2.46 - 2.39 (m, 2H), 2.39 - 2.27 (m, 2H), 2.26 - 2.13 (m, 1H), 2.01 - 1.86 (m, 2H), 1.77 (brs, 1H), 1.64 (brs, 1H), 1.54 - 1.39 (m, 2H), 1.20 - 1.16 (m, 1H), 0.99 (d, *J* = 3.8 Hz, 6H). LCMS (ESI) calcd. for C₃₄H₃₉FN₅O₄⁺ [M+H]⁺: 600.30, found, 600.4.

### Example 150: Preparation of compound GT-09989

With reference to the method of Scheme 3, the target compound GT-09989 was obtained (28 mg, white solid, yield 54%). ¹H NMR (400 MHz, DMSO-d6) δ 10.93 (s, 1H), 7.42 (d, *J* = 8.3 Hz, 1H), 7.30 (dd, *J* = 8.7, 5.6 Hz, 2H), 7.18 (t, *J* = 8.9 Hz, 2H), 6.88 (s, 1H), 6.80 (d, *J* = 8.4 Hz, 1H), 5.01 (dd, *J* = 13.2, 5.1 Hz, 1H), 4.28 (d, *J* = 17.0 Hz, 1H), 4.15 (d, *J* = 16.9 Hz, 1H), 3.45 - 3.37 (m, 4H), 3.32 (s, 2H), 3.22 (s, 2H), 2.94 - 2.85 (m, 1H), 2.58 (d, *J* = 16.5 Hz, 2H), 2.33 (dd, *J* = 12.9, 4.5 Hz, 1H), 2.26 (brs, 2H), 2.13 (s, 2H), 1.99 - 1.87 (m, 1H), 1.50 - 1.39 (m, 4H), 1.33 (brs, 1H), 0.98 (s, 6H). LCMS (ESI) calcd. for C₃₅H₄₁FN₅O₄⁺ [M+H]⁺: 614.31, found, 614.4.

### Example 151: Preparation of compound GT-09990

With reference to the method of Scheme 3, the target compound GT-09990 was obtained (26 mg, white solid, yield 49%). ¹H NMR (400 MHz, DMSO-d6) δ 10.93 (s, 1H), 7.41 (d, *J* = 8.4 Hz, 1H), 7.33 - 7.28 (m, 2H), 7.19 - 7.13 (m, 2H), 6.84 (d, *J* = 8.6 Hz, 1H), 6.78 (d, *J* = 9.3 Hz, 1H), 5.01 (dd, *J* = 13.3, 4.9 Hz, 1H), 4.28 (d, *J* = 16.8 Hz, 1H), 4.14 (d, *J* = 16.8 Hz, 1H), 3.34 - 3.26 (m, 2H), 3.00 - 2.89 (m, 1H), 2.83 - 2.66 (m, 2H), 2.62 - 2.55 (m, 4H), 2.44 - 2.28 (m, 4H), 2.19 - 2.12 (m, 1H), 2.02 - 1.85 (m, 2H), 1.75 (brs, 1H), 1.63 - 1.36 (m, 3H), 0.98 (s, 6H). LCMS (ESI) calcd. for C₃₃H₃₉FN₅O₄⁺ [M+H]⁺: 588.30, found, 588.3.

### Example 152: Preparation of compound GT-09991

With reference to the method of Scheme 3, the target compound GT-09991 was obtained (29 mg, white solid, yield 55%). ¹H NMR (400 MHz, DMSO-d6) δ 10.92 (s, 1H), 7.41 (d, *J* = 8.4 Hz, 1H), 7.30 (dd, *J* = 8.7, 5.6 Hz, 2H), 7.19 (t, *J* = 8.9 Hz, 2H), 6.83 (s, 1H), 6.77 (d, *J* = 8.3 Hz, 1H), 5.01 (dd, *J* = 13.3, 5.1 Hz, 1H), 4.27 (d, *J* = 16.9 Hz, 1H), 4.13 (d, *J* = 16.9 Hz, 1H), 3.98 (s, 1H), 3.67 (s, 1H), 3.24 (brs, 2H), 3.18 - 2.98 (m, 2H), 2.96 - 2.83 (m, 1H), 2.57 (d, *J* = 17.8 Hz, 2H), 2.41 - 2.29 (m, 2H), 2.29 - 2.16 (m, 1H), 1.98 - 1.83 (m, 3H), 1.47 - 1.41 (m, 2H), 1.30 (s, 1H), 0.96 (d, *J* = 3.2 Hz, 6H). LCMS (ESI) calcd. for C₃₃H₃₇FN₅O₄⁺ [M+H]⁺: 586.28, found, 586.3.

### Example 153: Preparation of compound GT-09994

With reference to the method of Scheme 3, the target compound GT-09994 was obtained (33 mg, white solid, yield 62%). ¹H NMR (400 MHz, DMSO-d6) δ 10.93 (s, 1H), 7.45 - 7.34 (m, 2H), 7.32 - 7.28 (m, 1H), 7.24 (t, *J* = 8.7 Hz, 1H), 7.17 (t, *J* = 8.8 Hz, 1H), 6.88 (s, 1H), 6.79 (d, *J* = 7.9 Hz, 1H), 5.09 - 4.96 (m, 1H), 4.27 (d, *J* = 15.1 Hz, 1H), 4.14 (d, *J* = 16.7 Hz, 1H), 3.32 - 3.23 (m, 5H), 3.01 - 2.80 (m, 2H), 2.74 - 2.62 (m, 2H), 2.41 - 2.32 (m, 3H), 2.28 - 2.13 (m, 2H), 2.02 - 1.83 (m, 2H), 1.53 - 1.38 (m, 2H), 1.00 (d, *J* = 1.6 Hz, 6H) LCMS (ESI) calcd. for C₃₃H₃₇FN₅O₄⁺ [M+H]⁺: 586.28, found, 586.3.

### Example 154: Preparation of compound GT-09992

With reference to the method of Scheme 2, the target compound GT-09992 was obtained (73 mg, white solid, yield 77%). ¹H NMR (400 MHz, DMSO-d6) δ 10.95 (s, 1H), 9.42 (d, *J* = 13.9 Hz, 1H), 8.97 (d, *J* = 13.9 Hz, 1H), 8.24 - 8.21 (m, 3H), 7.63 - 7.51 (m, 4H), 7.44 (d, *J* = 8.5 Hz, 1H), 6.95 (s, 2H), 5.01 (dd, *J* = 13.3, 5.1 Hz, 1H), 4.76 (d, *J* = 3.5 Hz, 1H), 4.66 (d, *J* = 19.9 Hz, 1H), 4.26 (d, *J* = 16.9 Hz, 1H), 4.13 (d, *J* = 16.6 Hz, 1H), 3.59 - 3.44 (m, 2H), 3.15 - 2.83 (m, 7H), 2.68 - 2.56 (m, 3H), 2.26 (dd, *J* = 13.1, 4.3 Hz, 1H), 1.99 - 1.87 (m, 1H). LCMS (ESI) calcd. for C₃₀H₃₂N₇O₃⁺ [M+H]⁺: 538.26, found, 538.3.

### Example 155: Preparation of compound GT-09993

With reference to the method of Scheme 2, the target compound GT-09993 was obtained (33 mg, white solid, yield 68%). ¹H NMR (400 MHz, DMSO-d6) δ 10.95 (s, 1H), 9.37 (s, 1H), 9.00 (s, 1H), 8.21 (dd, *J* = 7.9, 1.5 Hz, 2H), 7.61 - 7.56 (m, 3H), 7.48 (d, *J* = 8.3 Hz, 1H), 6.99 (s, 1H), 6.91 (d, *J* = 8.1 Hz, 1H), 5.03 (dd, *J* = 13.3, 5.0 Hz, 1H), 4.50 (s, 2H), 4.33 (d, *J* = 16.9 Hz, 1H), 4.21 (d, *J* = 16.9 Hz, 1H)), 4.17 (s, 2H), 3.23 - 3.10 (m, 2H), 3.01 (d, *J* = 11.7 Hz, 2H), 2.96 - 2.84 (m, 1H), 2.60 (d, *J* = 17.4 Hz, 2H), 2.45 - 2.25 (m, 5H), 2.01 - 1.95 (m, 1H). LCMS (ESI) calcd. for C₃₀H₃₂N₇O₃⁺ [M+H]⁺: 538.26, found, 538.3.

### Example 156: Preparation of compound GT-10026

With reference to the method of Scheme 2, the target compound GT-10026 was obtained (15 mg, white solid, yield 32%). ¹H NMR (400 MHz, DMSO-d6) δ 10.94 (s, 1H), 9.30 (d, *J* = 1.2 Hz, 1H), 8.87 (d, *J* = 1.2 Hz, 1H), 8.19 (dd, *J* = 7.7, 1.5 Hz, 2H), 7.59 - 7.54 (m, 3H), 7.47 (d, *J* = 8.3 Hz, 1H), 7.02 (s, 1H), 6.93 (d, *J* = 7.7 Hz, 1H), 5.03 (dd, *J* = 13.3, 5.0 Hz, 1H), 4.72 (d, *J* = 5.8 Hz, 2H), 4.31 (d, *J* = 17.0 Hz, 1H), 4.18 (d, *J* = 16.9 Hz, 1H), 4.13 - 4.00 (m, 4H), 2.97 - 2.84 (m, 2H), 2.58 (d, *J* = 17.3 Hz, 2H), 2.38 - 2.30 (m, 2H), 2.13 - 1.99 (m, 2H), 2.00 - 1.88 (m, 2H). LCMS (ESI) calcd. for C₃₁H₃₄N₇O₃⁺ [M+H]⁺: 552.27, found, 552.3.

### Example 157: Preparation of compound GT-10028

With reference to the method of Scheme 2, the target compound GT-10028 was obtained (23 mg, white solid, yield 48%). ¹H NMR (400 MHz, DMSO-d6) δ 11.19 (s, 1H), 10.93 (s, 1H), 9.36 (d, *J* = 1.2 Hz, 1H), 9.02 (d, *J* = 1.2 Hz, 1H), 8.21 (dd, *J* = 7.7, 1.6 Hz, 2H), 7.59 - 7.55 (m, 3H), 7.45 (d, *J* = 8.4 Hz, 1H), 6.95 (s, 1H), 6.88 (d, *J* = 8.1 Hz, 1H), 5.02 (dd, *J* = 13.3, 5.1 Hz, 1H), 4.66 (s, 2H), 4.29 (d, *J* = 16.9 Hz, 1H), 4.16 (d, *J* = 16.9 Hz, 1H), 3.51 - 3.41 (m, 4H), 3.31 - 3.25 (m, 1H), 3.18 - 3.14 (m, 1H), 3.08 - 2.96 (m, 1H), 2.96 - 2.83 (m, 1H), 2.58 (d, *J* = 17.2 Hz, 2H), 2.40 - 2.29 (m, 1H), 2.10 (brs, 2H), 2.00 - 1.86 (m, 1H) LCMS (ESI) calcd. for C₂₉H₃₂N₇O₃⁺ [M+H]⁺: 526.26, found, 526.3.

### Example 158: Preparation of compound GT-10029

With reference to the method of Scheme 2, the target compound GT-10029 was obtained (26 mg, white solid, yield 55%). ¹H NMR (400 MHz, DMSO-d6) δ 10.93 (s, 1H), 9.36 (d, *J* = 1.2 Hz, 1H), 9.02 (d, *J* = 1.2 Hz, 1H), 8.21 (dd, *J* = 7.7, 1.6 Hz, 2H), 7.59 - 7.55 (m, 3H), 7.45 (d, *J* = 8.4 Hz, 1H), 6.95 (s, 1H), 6.88 (d, *J* = 8.1 Hz, 1H), 5.03 (dd, *J* = 13.3, 5.0 Hz, 1H), 4.88 - 4.76 (m, 1H), 4.59 - 4.45 (m, 2H), 4.31 (d, *J* = 17.0 Hz, 1H), 4.18 (d, *J* = 16.4 Hz, 1H), 3.80 (s, 1H), 3.71 - 3.53 (m, 2H), 3.34 - 3.25 (m, 2H), 2.97 - 2.82 (m, 2H), 2.59 (d, *J* = 17.1 Hz, 2H), 2.40 - 2.21 (m, 1H), 2.00 - 1.89 (m, 1H). LCMS (ESI) calcd. for C₂₉H₃₀N₇O₃⁺ [M+H]⁺: 524.24, found, 524.3.

### Example 159: Preparation of compound GT-10056

With reference to the method of Scheme 2, the target compound GT-10056 was obtained (23 mg, white solid, yield 48%). ¹H NMR (400 MHz, DMSO-d6) δ 11.06 (s, 1H), 8.04 (s, 1H), 7.59 (d, *J* = 8.3 Hz, 1H), 7.57 - 7.51 (m, 3H), 7.48 (d, *J* = 5.9 Hz, 1H), 7.46 - 7.41 (m, 2H), 7.38 (d, *J* = 6.4 Hz, 1H), 7.14 (s, 1H), 7.01 (d, *J* = 8.2 Hz, 1H), 5.03 (dd, *J* = 12.8, 5.4 Hz, 1H), 3.45 - 3.38 (m, 2H), 3.34 - 3.26 (m, 4H), 3.26 - 3.15 (m, 2H), 2.91 - 2.78 (m, 2H), 2.59 (s, 1H), 2.61 - 2.58 (m, 1H), 2.49 - 2.39 (m, 2H), 2.01 - 1.95 (m, 1H). LCMS (ESI) calcd. for C₂₉H₃₀N₇O₃⁺ [M+H]⁺: 524.24, found, 524.3.

### Example 160: Preparation of compound GT-10057

With reference to the method of Scheme 3, the target compound GT-10057 was obtained (11 mg, white solid, yield 21%). ¹H NMR (400 MHz, DMSO-d6) δ 11.08 (s, 1H), 8.11 (s, 1H), 7.54 (dd, *J* = 8.3, 2.6 Hz, 4H), 7.48 (t, *J* = 6.9 Hz, 2H), 7.42 (d, *J* = 8.3 Hz, 2H), 7.37 (t, *J* = 7.1 Hz, 2H), 5.04 (dd, *J* = 12.7, 5.4 Hz, 1H), 3.43 - 3.40 (m, 2H), 3.26 - 3.19 (m, 2H), 2.91 - 2.82 (m, 2H), 2.79 - 2.70 (m, 1H), 2.57 (d, *J* = 21.0 Hz, 2H), 2.48 - 2.37 (m, 2H), 2.06 - 1.93 (m, 1H). LCMS (ESI) calcd. for C₃₀H₂₆ClFN₅O₅⁺ [M+H]⁺: 590.16, found, 590.2.

### Example 161: Preparation of compound GT-10058

With reference to the method of Scheme 3, the target compound GT-10058 was obtained (6 mg, white solid, yield 12%). ¹H NMR (400 MHz, DMSO-d6) δ 11.07 (s, 1H), 8.42 (s, 1H), 7.56 - 7.51 (m, 4H), 7.48 - 7.44 (m, 2H), 7.38 (d, *J* = 7.4 Hz, 2H), 6.96 (s, 1H), 6.75 (s, 1H), 5.03 (dd, *J* = 12.8, 5.4 Hz, 1H), 3.44 - 3.40 (m, 2H), 3.26 - 3.14 (m, 2H), 3.10 - 2.89 (m, 2H), 2.88 - 2.79 (m, 1H), 2.59 - 2.56 (m, 2H), 2.48 - 2.36 (m, 2H), 2.02 - 1.95 (m, 1H). LCMS (ESI) calcd. for C₃₀H₂₆ClFN₅O₅⁺ [M+H]⁺: 590.16, found, 590.2.

### Example 162: Preparation of compound GT-10059

With reference to the method of Scheme 3, the target compound GT-10059 was obtained (7 mg, white solid, yield 13%). ¹H NMR (400 MHz, DMSO-d6) δ 11.09 (s, 1H), 8.07 (s, 1H), 7.52 (d, *J* = 8.2 Hz, 1H), 7.41 - 7.35 (m, 3H), 7.24 (d, *J* = 8.4 Hz, 2H), 5.04 (dd, *J* = 12.8, 5.4 Hz, 1H), 3.31 - 3.15 (m, 4H), 2.94 - 2.81 (m, 2H), 2.60 - 2.55 (m, 2H), 2.40 - 2.21 (m, 3H), 2.21 - 2.06 (m, 2H), 2.06 - 1.85 (m, 3H), 1.57 - 1.38 (m, 2H), 0.99 (d, *J* = 9.2 Hz, 6H). LCMS (ESI) calcd. for C₃₂H₃₄ClFN₅O₅⁺ [M+H]⁺: 622.22, found, 622.3.

### Example 163: Preparation of compound GT-10060

With reference to the method of Scheme 3, the target compound GT-10060 was obtained (14 mg, white solid, yield 26%). ¹H NMR (400 MHz, DMSO-d6) δ 11.08 (s, 1H), 8.37 (s, 1H), 7.40 (d, *J* = 8.5 Hz, 2H), 7.28 - 7.24 (m, 2H), 6.94 (s, 1H), 6.75 (s, 1H), 5.03 (dd, *J* = 12.7, 5.4 Hz, 1H), 3.31 - 3.13 (m, 4H), 3.01 - 2.74 (m, 3H), 2.57 (d, *J* = 18.6 Hz, 2H), 2.37 - 2.33 (m, 3H), 2.22 - 2.06 (m, 1H), 2.04 - 1.78 (m, 3H), 1.51 - 1.41 (m, 2H), 0.98 (d, *J* = 11.1 Hz, 6H). LCMS (ESI) calcd. for C₃₂H₃₄ClFN₅O₅⁺ [M+H]⁺: 622.22, found, 622.2.

### Example 164: Preparation of compound GT-10061

With reference to the method of Scheme 1, the target compound GT-10061 was obtained (20 mg, white solid, yield 38%). ¹H NMR (400 MHz, DMSO-d6) δ 10.94 (s, 1H), 9.29 (s, 1H), 7.50 (d, *J* = 7.9 Hz, 2H), 7.44 (d, *J* = 8.3 Hz, 1H), 7.19 (d, *J* = 8.1 Hz, 2H), 6.94 (s, 1H), 6.90 (d, *J* = 8.4 Hz, 1H), 5.03 (dd, *J* = 13.2, 5.0 Hz, 1H), 4.30 (d, *J* = 16.8 Hz, 1H), 4.17 (d, *J* = 17.0 Hz, 1H), 3.61 (s, 2H), 3.28 - 3.11 (m, 3H), 3.03 (brs, 2H), 2.97 - 2.84 (m, 2H), 2.59 (d, *J* = 17.7 Hz, 2H), 2.36 - 2.31 (m, 2H), 2.13 (s, 4H), 2.02 (d, *J* = 17.3 Hz, 2H), 2.01 - 1.88 (m, 2H), 1.48 (s, 2H), 0.97 (s, 6H). LCMS (ESI) calcd. for C₃₄H₄₁ClN₅O₃⁺ [M+H]⁺: 602.29, found, 602.4.

### Example 165: Preparation of compound GT-10062

With reference to the method of Scheme 1, the target compound GT-10062 was obtained (9 mg, white solid, yield 17%). ¹H NMR (400 MHz, DMSO-d6) δ 11.09 (s, 1H), 8.43 (s, 1H), 7.54 (d, *J* = 8.1 Hz, 1H), 7.47 - 7.43 (m, 3H), 7.16 (d, *J* = 9.2 Hz, 2H), 5.05 (dd, *J* = 12.7, 5.4 Hz, 1H), 3.60 (s, 2H), 3.18 - 3.13 (m, 3H), 3.05 - 2.81 (m, 5H), 2.55 (d, *J* = 4.7 Hz, 2H), 2.38 - 2.27 (m, 2H), 2.10 - 1.93 (m, 4H), 1.54 - 1.42 (m, 2H), 0.97 (s, 6H) LCMS (ESI) calcd. for C₃₂H₃₆ClFN₅O₄⁺ [M+H]⁺: 608.24, found, 608.3.

### Example 166: Preparation of compound GT-10063

With reference to the method of Scheme 1, the target compound GT-10063 was obtained (12 mg, white solid, yield 23%). ¹H NMR (400 MHz, DMSO-d6) δ 11.08 (s, 1H), 9.97 (s, 1H), 8.81 (s, 1H), 7.47 (d, *J* = 8.3 Hz, 2H), 7.16 (d, *J* = 8.2 Hz, 2H), 7.00 (s, 1H), 6.82 (s, 1H), 5.04 (dd, *J* = 12.8, 5.4 Hz, 1H), 3.61 (s, 2H), 3.19 - 3.06 (m, 2H), 3.06 - 2.93 (m, 4H), 2.92 - 2.78 (m, 2H), 2.55 (d, *J* = 4.8 Hz, 2H), 2.33 (s, 2H), 2.10 - 1.95 (m, 4H), 1.47 (t, *J* = 6.6 Hz, 2H), 0.97 (s, 6H). LCMS (ESI) calcd. for C₃₂H₃₆ClFN₅O₄⁺ [M+H]⁺: 608.24, found, 608.3.

### Example 167: Preparation of compound GT-10065

With reference to the method of Scheme 1, the target compound GT-10065 was obtained (17 mg, white solid, yield 32%). ¹H NMR (400 MHz, DMSO-d6) δ 10.97 (s, 1H), 7.52 - 7.46 (m, 2H), 7.46 - 7.41 (m, 1H), 7.21 (d, *J* = 8.4 Hz, 2H), 7.17 (s, 1H), 6.69 (d, *J* = 8.1 Hz, 1H), 5.06 (dd, *J* = 13.1, 5.1 Hz, 1H), 4.30 (d, *J* = 16.7 Hz, 1H), 4.18 (d, *J* = 16.6 Hz, 1H), 3.76 - 3.60 (m, 3H), 3.00 - 2.80 (m, 5H), 2.65 - 2.55 (m, 2H), 2.45 - 2.41 (m, 2H), 2.33 - 2.27 (m, 4H), 2.05 (s, 2H), 2.02 - 1.97 (m, 2H), 1.48 (t, *J* = 6.3 Hz, 3H), 0.98 (s, 6H). LCMS (ESI) calcd. for C₃₄H₄₁ClN₅O₃⁺ [M+H]⁺: 602.29, found, 602.4.

### Example 168: Preparation of compound GT-10066

With reference to the method of Scheme 1, the target compound GT-10066 was obtained (14 mg, white solid, yield 26%). ¹H NMR (400 MHz, DMSO-d6) δ 10.93 (s, 1H), 7.48 - 7.43 (m, 4H), 7.23 (d, *J* = 8.3 Hz, 2H), 6.93 (s, 1H), 5.02 (dd, *J* = 13.2, 4.9 Hz, 1H), 4.28 (d, *J* = 17.0 Hz, 1H), 4.15 (d, *J* = 17.0 Hz, 1H), 3.79 (s, 2H), 3.55 (d, *J* = 4.8 Hz, 2H), 3.19 (d, *J* = 11.4 Hz, 2H), 2.92 - 2.87 (m, 2H), 2.63 - 2.54 (m, 2H), 2.41 - 2.31 (m, 3H), 2.10 (s, 2H), 2.04 - 1.91 (m, 3H), 1.50 (t, *J* = 5.9 Hz, 2H), 1.31 - 1.25 (m, 2H), 0.98 (s, 6H). LCMS (ESI) calcd. for C₃₄H₄₁ClN₅O₃⁺ [M+H]⁺: 602.29, found, 602.3.

### Example 169: Preparation of compound GT-10067

With reference to the method of Scheme 1, the target compound GT-10067 was obtained (16 mg, white solid, yield 31%). ¹H NMR (400 MHz, DMSO-d6) δ 10.93 (s, 1H), 7.47 - 7.42 (m, 3H), 7.21 (d, *J* = 8.3 Hz, 2H), 6.88 (s, 1H), 6.81 (d, *J* = 7.5 Hz, 1H), 5.02 (dd, *J* = 13.3, 5.1 Hz, 1H), 4.29 (d, *J* = 17.0 Hz, 1H), 4.15 (d, *J* = 16.9 Hz, 1H), 4.00 - 3.87 (m, 2H), 3.67 (d, *J* = 5.6 Hz, 2H), 3.63 - 3.50 (m, 4H), 3.35 - 3.24 (m, 2H), 2.97 - 2.84 (m, 1H), 2.60 (s, 1H), 2.55 - 2.51 (m, 2H), 2.37 - 2.30 (m, 1H), 2.18 (brs, 2H), 2.05 (brs, 2H), 1.99 - 1.87 (m, 2H), 1. 63 - 1.56(m, 1H), 1.50 - 1.38 (m, 2H), 0.96 (s, 6H). LCMS (ESI) calcd. for C₃₅H₄₃ClN₅O₃⁺ [M+H]⁺: 616.30, found, 616.4.

### Example 170: Preparation of compound GT-10068

With reference to the method of Scheme 1, the target compound GT-10068 was obtained (14 mg, white solid, yield 26%). ¹H NMR (400 MHz, DMSO-d6) δ 10.94 (s, 1H), 9.93 (s, 1H), 7.47 (d, *J* = 8.4 Hz, 2H), 7.43 (d, *J* = 8.3 Hz, 1H), 7.17 (d, *J* = 8.4 Hz, 2H), 6.86 (s, 1H), 6.79 (d, *J* = 7.9 Hz, 1H), 5.02 (dd, *J* = 13.1, 5.2 Hz, 1H), 4.29 (d, *J* = 16.8 Hz, 1H), 4.15 (d, *J* = 16.8 Hz, 1H), 3.60 (s, 2H), 3.18 - 3.02 (m, 3H), 2.93 - 2.80 (m, 4H), 2.59 (d, *J* = 17.4 Hz, 2H), 2.40 - 2.22 (m, 4H), 2.05 (s, 2H), 2.00 - 1.83 (m, 2H), 1.78 - 1.65 (m, 1H), 1.47 (t, *J* = 6.2 Hz, 2H), 0.96 (s, 6H). LCMS (ESI) calcd. for C₃₃H₄₁ClN₅O₃⁺ [M+H]⁺: 590.29, found, 590.3.

### Example 171: Preparation of compound GT-10069

With reference to the method of Scheme 1, the target compound GT-10069 was obtained (16 mg, white solid, yield 30%). ¹H NMR (400 MHz, DMSO-d6) δ 10.93 (s, 1H), 7.72 (s, 1H), 7.47 (d, *J* = 8.4 Hz, 2H), 7.19 (d, *J* = 8.4 Hz, 1H), 6.93 (s, 1H), 6.84 (d, *J* = 7.5 Hz, 1H), 5.03 (dd, *J* = 12.6, 5.7 Hz, 1H), 4.30 (d, *J* = 16.8 Hz, 1H), 4.16 (d, *J* = 17.0 Hz, 1H), 3.71 - 3.66 (m, 1H), 3.55 - 3.43 (m, 2H), 3.32 - 3.13 (m, 4H), 3.01 - 2.84 (m, 2H), 2.63 - 2.53 (m, 2H), 2.44 - 2.20 (m, 4H), 2.13 - 2.01 (m, 2H), 2.01 - 1.93 (m, 1H), 1.51 - 1.41 (m, 2H), 0.99 (s, 3H), 0.97 (s, 3H). LCMS (ESI) calcd. for C₃₃H₃₉ClN₅O₃⁺ [M+H]⁺: 588.27, found, 588.3.

### Example 172: Preparation of compound GT-10070

With reference to the method of Scheme 3, the target compound GT-10070 was obtained (14 mg, white solid, yield 28%). ¹H NMR (400 MHz, DMSO-d6) δ 11.07 (s, 1H), 8.69 (d, *J* = 7.7 Hz, 1H), 8.17 (d, *J* = 7.9 Hz, 1H), 8.07 (s, 1H), 7.63 (d, *J* = 8.4 Hz, 1H), 7.22 (s, 1H), 7.08 (s, 1H), 5.05 (dd, *J* = 13.0, 5.3 Hz, 1H), 4.74 (s, 1H), 4.42 - 4.10 (m, 1H), 3.81 (brs, 2H), 3.76 - 3.65 (m, 2H), 3.56 (s, 4H), 3.13 - 2.95 (m, 2H), 2.97 - 2.85 (m, 2H), 2.58 (d, *J* = 16.2 Hz, 2H), 2.07 - 1.87 (m, 4H). LCMS (ESI) calcd. for C₂₉H₃₀N₉O₆⁺ [M+H]⁺: 600.23, found, 600.3.

### Example 173: Preparation of compound GT-10124

With reference to the method of Scheme 2, the target compound GT-10124 was obtained (15 mg, white solid, yield 31%). ¹H NMR (400 MHz, DMSO-d6) δ 9.11 (s, 1H), 9.02 - 8.95 (m, 3H), 8.89 - 8.84 (m, 2H), 8.47 (d, *J* = 8.1 Hz, 1H), 8.40 (s, 1H), 7.63 (d, *J* = 8.4 Hz, 1H), 7.10 (d, *J* = 7.8 Hz, 1H), 5.10 (dd, *J* = 13.3, 5.1 Hz, 1H), 4.75 (s, 2H), 4.48 - 4.41 (m, 2H), 3.90 (s, 1H), 3.75 - 3.52 (m, 4H), 3.22 - 3.17 (m, 2H), 2.93 - 2.82 (m, 2H), 2.82 - 2.72 (m, 1H), 2.50 - 2.31 (m, 3H), 2.20 - 2.10 (m, 1H). LCMS (ESI) calcd. for C₃₀H₃₂N₇O₃⁺ [M+H]⁺: 538.26, found, 538.3.

### Example 174: Preparation of compound GT-10125

With reference to the method of Scheme 2, the target compound GT-10125 was obtained (19 mg, white solid, yield 40%). ¹H NMR (400 MHz, DMSO-d6) δ 9.13 (s, 1H), 9.00 (d, *J* = 6.9 Hz, 2H), 8.85 (d, *J* = 6.9 Hz, 2H), 8.47 (d, *J* = 1.3 Hz, 2H), 7.57 (d, *J* = 8.4 Hz, 1H), 7.02 (s, 1H), 6.95 (d, *J* = 8.2 Hz, 1H), 5.08 (dd, *J* = 13.3, 5.1 Hz, 1H), 4.51 (s, 2H), 4.38 (d, *J* = 9.0 Hz, 2H), 4.10 (s, 3H), 3.23 (d, *J* = 12.3 Hz, 2H), 3.19 - 3.08 (m, 3H), 2.94 (d, *J* = 12.3 Hz, 1H), 2.87 (dd, *J* = 13.3, 5.3 Hz, 1H), 2.80 - 2.73 (m, 1H), 2.39 - 2.33 (m, 1H), 2.18 - 2.09 (m, 2H). LCMS (ESI) calcd. for C₃₀H₃₂N₇O₃⁺ [M+H]⁺: 538.26, found, 538.3.

### Example 175: Preparation of compound GT-10126

With reference to the method of Scheme 2, the target compound GT-10126 was obtained (15 mg, white solid, yield 32%). ¹H NMR (400 MHz, DMSO-d6) δ 9.06 (d, *J* = 1.9 Hz, 1H), 8.99 (d, *J* = 6.9 Hz, 2H), 8.83 (d, *J* = 6.9 Hz, 2H), 8.44 (d, *J* = 8.1 Hz, 1H), 8.35 (dd, *J* = 8.2, 2.2 Hz, 1H), 7.75 (dd, *J* = 8.3, 4.3 Hz, 1H), 7.47 (d, *J* = 7.0 Hz, 1H), 7.33 - 7.29 (m, 1H), 5.13 (dd, *J* = 13.4, 5.0 Hz, 1H), 4.70 (s, 2H), 4.53 (d, *J* = 11.5 Hz, 1H), 4.48 (dd, *J* = 9.8, 2.2 Hz, 2H), 4.32 - 4.29 (m, 1H), 4.20 (s, 1H), 4.01 (s, 3H), 3.58 - 3.42 (m, 4H), 2.92 - 2.84 (m, 1H), 2.83 - 2.72 (m, 1H), 2.37 - 2.32 (m, 3H), 2.22 - 2.15 (m, 1H). LCMS (ESI) calcd. for C₃₁H₃₄N₇O₃⁺ [M+H]⁺: 552.27, found, 552.3.

### Example 176: Preparation of compound GT-10127

With reference to the method of Scheme 2, the target compound GT-10127 was obtained (10 mg, white solid, yield 21%). ¹H NMR (400 MHz, DMSO-d6) δ 9.10 (d, *J* = 1.5 Hz, 1H), 8.99 (d, *J* = 6.8 Hz, 2H), 8.89 - 8.83 (m, 2H), 8.46 (d, *J* = 8.2 Hz, 1H), 8.44 - 8.38 (m, 1H), 7.59 - 7.51 (m, 1H), 7.08 (s, 1H), 6.96 (d, *J* = 8.4 Hz, 1H), 5.08 (dd, *J* = 13.3, 5.1 Hz, 1H), 4.67 (s, 2H), 4.38 (d, *J* = 8.2 Hz, 2H), 3.61 (s, 4H), 3.46 - 3.37 (m, 2H), 3.28 - 3.24 (m, 1H), 3.17 - 3.06 (m, 2H), 2.91 - 2.84 (m, 1H), 2.82 - 2.72 (m, 1H), 2.46 (dd, *J* = 13.2, 4.7 Hz, 1H), 2.38 (dd, *J* = 12.3, 6.8 Hz, 1H), 2.14 - 2.07 (m, 1H). LCMS (ESI) calcd. for C₂₉H₃₂N₇O₃⁺ [M+H]⁺: 526.26, found, 526.3.

### Example 177: Preparation of compound GT-10128

With reference to the method of Scheme 2, the target compound GT-10128 was obtained (13 mg, white solid, yield 27%). ¹H NMR (400 MHz, DMSO-d6) δ 9.12 (s, 1H), 8.99 (d, *J* = 6.8 Hz, 2H), 8.85 (d, *J* = 6.3 Hz, 2H), 8.46 (s, 2H), 7.55 (d, *J* = 8.4 Hz, 1H), 7.07 (s, 1H), 6.97 (d, *J* = 8.5 Hz, 1H), 5.06 (dd, *J* = 13.3, 5.1 Hz, 1H), 4.59 - 4.51 (m, 1H), 4.43 (t, *J* = 8.4 Hz, 1H), 4.41 - 4.34 (m, 2H), 3.76 (d, *J* = 10.3 Hz, 1H), 3.70 (d, *J* = 14.4 Hz, 1H), 3.63 (d, *J* = 12.9 Hz, 1H), 3.43 (d, *J* = 11.9 Hz, 1H), 2.92 - 2.85 (m, 2H), 2.82 - 2.65 (m, 2H), 2.51 - 2.44 (m, 3H), 2.20 - 2.09 (m, 1H). LCMS (ESI) calcd. for C₂₉H₃₀N₇O₃⁺ [M+H]⁺: 524.24, found, 524.3.

### Example 178: Preparation of compound GT-10129

With reference to the method of Scheme 2, the target compound GT-10129 was obtained (11 mg, white solid, yield 23%). ¹H NMR (400 MHz, DMSO-d6) δ 9.12 (s, 1H), 8.99 (d, *J* = 6.8 Hz, 2H), 8.85 (d, *J* = 6.3 Hz, 2H), 8.46 (s, 2H), 7.55 (d, *J* = 8.4 Hz, 1H), 7.07 (s, 1H), 6.97 (d, *J* = 8.5 Hz, 1H), 5.08 (dd, *J* = 13.3, 5.1 Hz, 1H), 4.42 (s, 2H), 4.40 - 4.30 (m, 4H), 3.77 (s, 1H), 2.87 (dd, *J* = 13.4, 5.2 Hz, 1H), 2.82 - 2.73 (m, 1H), 2.54 - 2.41 (m, 3H), 2.39 - 2.31 (m, 2H), 2.20 - 2.09 (m, 2H). LCMS (ESI) calcd. for C₂₉H₃₀N₇O₃⁺ [M+H]⁺: 524.24, found, 524.3.

### Example 179: Preparation of compound GT-10130

With reference to the method of Scheme 1, the target compound GT-10130 was obtained (10 mg, white solid, yield 20%). ¹H NMR (400 MHz, DMSO-d6) δ 7.79 (d, *J* = 8.3 Hz, 2H), 7.71 - 7.63 (m, 4H), 7.55 (d, *J* = 8.9 Hz, 1H), 7.48 (d, *J* = 8.5 Hz, 2H), 6.95 - 6.91 (m, 2H), 5.03 (dd, *J* = 13.3, 5.1 Hz, 1H), 4.56 (s, 2H), 4.31 (s, 2H), 3.87 - 3.82 (m, 2H), 3.13 - 3.05 (m, 4H), 3.01 2.98 (m, 2H), 2.92 - 2.83 (m, 1H), 2.78 (dd, *J* = 10.1, 7.5 Hz, 1H), 2.72 - 2.57 (m, 1H), 2.36 (dd, *J* = 13.1, 4.9 Hz, 1H), 2.12 - 2.03 (m, 1H). LCMS (ESI) calcd. for C₃₂H₃₃ClN₅O₃⁺ [M+H]⁺: 570.23, found, 570.3.

### Example 180: Preparation of compound GT-10131

With reference to the method of Scheme 1, the target compound GT-10131 was obtained (15 mg, white solid, yield 30%). ¹H NMR (400 MHz, MeOD) δ 7.78 (d, *J* = 8.2 Hz, 2H), 7.68 - 7.64 (m, 4H), 7.58 (d, *J* = 8.3 Hz, 1H), 7.48 (d, *J* = 8.6 Hz, 2H), 7.03 (s, 1H), 6.97 (d, *J* = 8.8 Hz, 1H), 5.09 (dd, *J* = 13.3, 5.1 Hz, 1H), 4.38 - 4.33 (m, 4H), 4.06 (s, 2H), 3.26 - 3.22 (m, 2H), 3.21 - 3.16 (m, 2H), 3.01 - 2.97 (m, 2H), 2.79 - 2.75 (m, 1H), 2.52 - 2.48 (m, 3H), 2.42 - 2.35 (m, 1H), 2.17 - 2.07 (m, 1H). LCMS (ESI) calcd. for C₃₂H₃₃ClN₅O₃⁺ [M+H]⁺: 570.23, found, 570.3.

### Example 181: Preparation of compound GT-10132

With reference to the method of Scheme 1, the target compound GT-10132 was obtained (16 mg, white solid, yield 32%). ¹H NMR (400 MHz, DMSO-d6) δ 7.75 (d, *J* = 8.3 Hz, 2H), 7.64 (d, *J* = 8.6 Hz, 2H), 7.60 (d, *J* = 8.3 Hz, 2H), 7.55 (d, *J* = 8.4 Hz, 1H), 7.47 (d, *J* = 8.6 Hz, 2H), 7.02 (s, 1H), 6.93 (dd, *J* = 8.4, 1.6 Hz, 1H), 5.08 (dd, *J* = 13.3, 5.2 Hz, 1H), 4.46 (s, 2H), 4.36 (d, *J* = 7.5 Hz, 2H), 4.08 (d, *J* = 10.9 Hz, 2H), 4.01 (d, *J* = 10.8 Hz, 2H), 2.94 - 2.85 (m, 6H), 2.45 (dd, *J* = 13.1, 4.7 Hz, 1H), 2.18 - 2.12 (m, 1H), 2.11 - 1.95 (m, 4H). LCMS (ESI) calcd. for C₃₂H₃₅ClN₅O₃⁺ [M+H]⁺: 584.24, found, 584.3.

### Example 182: Preparation of compound GT-10133

With reference to the method of Scheme 1, the target compound GT-10133 was obtained (24 mg, white solid, yield 47%). ¹H NMR (400 MHz, DMSO-d6) δ 7.77 (d, *J* = 8.3 Hz, 2H), 7.69 - 7.64 (m, 4H), 7.56 (d, *J* = 8.4 Hz, 1H), 7.51 - 7.44 (m, 2H), 7.01 (s, 1H), 6.94 (d, *J* = 8.4 Hz, 1H), 5.07 (dd, *J* = 13.4, 5.1 Hz, 1H), 4.49 (s, 2H), 4.39 - 4.32 (m, 2H), 3.60 - 3.58 (m, 2H), 3.55 - 3.43 (m, 2H), 3.29 - 3.24 (m, 2H), 3.17 - 2.99 (m, 2H), 2.91 - 2.84 (m, 1H), 2.82 - 2.71 (m, 1H), 2.45 - 2.37 (m, 1H), 2.29 - 2.17 (m, 1H), 2.15 - 2.05 (m, 2H). LCMS (ESI) calcd. for C₃₁H₃₃ClN₅O₃⁺ [M+H]⁺: 558.23, found, 558.3.

### Example 183: Preparation of compound GT-10134

With reference to the method of Scheme 1, the target compound GT-10134 was obtained (21 mg, white solid, yield 42%). ¹H NMR (400 MHz, DMSO-d6) δ 7.77 (d, *J* = 8.2 Hz, 2H), 7.71 - 7.64 (m, 4H), 7.55 (d, *J* = 8.4 Hz, 1H), 7.48 (d, *J* = 8.6 Hz, 2H), 7.03 (s, 1H), 6.95 (d, *J* = 8.3 Hz, 1H), 5.08 (dd, *J* = 13.4, 5.1 Hz, 1H), 4.53 (d, *J* = 13.1 Hz, 1H), 4.43 - 4.28 (m, 4H), 3.80 (s, 1H), 3.65 - 3.55 (m, 1H), 3.34 - 3.31 (m, 2H), 3.17 - 3.05 (m, 1H), 2.93 - 2.83 (m, 1H), 2.82 - 2.69 (m, 1H), 2.51 - 2.44 (m, 1H), 2.41 (s, 2H), 2.20 - 2.09 (m, 1H). LCMS (ESI) calcd. for C₃₁H₃₁ClN₅O₃⁺ [M+H]⁺: 556.21, found, 556.3.

### Example 184: Preparation of compound GT-10135

With reference to the method of Scheme 1, the target compound GT-10135 was obtained (21 mg, white solid, yield 45%). ¹H NMR (400 MHz, DMSO-d6) δ 7.60 - 7.50 (m, 2H), 7.34 (s, 2H), 6.98 - 6.91 (m, 2H), 6.73 (d, *J* = 3.4 Hz, 1H), 6.53 (dd, *J* = 3.4, 1.9 Hz, 1H), 5.07 (dd, *J* = 13.2, 5.1 Hz, 1H), 4.77 (s, 2H), 4.37 - 4.22 (m, 2H), 3.91 - 3.86 (m, 2H), 3.63 - 3.48 (m, 1H), 3.23 - 3.04 (m, 4H), 3.05 - 2.97 (m, 2H), 2.88 (dd, *J* = 12.9, 4.9 Hz, 1H), 2.82 - 2.72 (m, 1H), 2.69 - 2.63 (m, 1H), 2.41 (dd, *J* = 13.2, 4.6 Hz, 1H), 2.20 - 2.05 (m, 1H). LCMS (ESI) calcd. for C₂₈H₃₀N₅O₄S⁺ [M+H]⁺: 532.20, found, 532.3.

### Example 185: Preparation of compound GT-10136

With reference to the method of Scheme 1, the target compound GT-10136 was obtained (16 mg, white solid, yield 34%). ¹H NMR (400 MHz, DMSO-d6) δ 7.58 (d, *J* = 8.4 Hz, 1H), 7.55 (d, *J* = 1.4 Hz, 1H), 7.35 (d, *J* = 3.8 Hz, 1H), 7.31 (d, *J* = 3.7 Hz, 1H), 7.02 (s, 1H), 6.96 (d, *J* = 8.3 Hz, 1H), 6.71 (d, *J* = 3.4 Hz, 1H), 6.53 (dd, *J* = 3.4, 1.9 Hz, 1H), 5.09 (dd, *J* = 13.3, 5.1 Hz, 1H), 4.52 (s, 2H), 4.45 - 4.30 (m, 2H), 4.08 (s, 2H), 3.20 (d, *J* = 11.2 Hz, 2H), 3.01 (d, *J* = 12.3 Hz, 2H), 2.91 - 2.83 (m, 1H), 2.81 - 2.69 (m, 1H), 2.50 - 2.37 (m, 5H), 2.18 - 2.12 (m, 1H). LCMS (ESI) calcd. for C₂₈H₃₀N₅O₄S⁺ [M+H]⁺: 532.20, found, 532.3.

### Example 186: Preparation of compound GT-10137

With reference to the method of Scheme 1, the target compound GT-10137 was obtained (21 mg, white solid, yield 45%). ¹H NMR (400 MHz, DMSO-d6) δ 7.59 (d, *J* = 8.4 Hz, 1H), 7.55 - 7.52 (m, 1H), 7.29 (s, 2H), 7.09 (s, 1H), 6.98 (dd, *J* = 8.4, 1.6 Hz, 1H), 6.69 (d, *J* = 3.4 Hz, 1H), 6.53 (dd, *J* = 3.4, 1.9 Hz, 1H), 5.09 (dd, *J* = 13.3, 5.1 Hz, 1H), 4.65 (s, 2H), 4.38 (q, *J* = 17.0 Hz, 2H), 4.12 - 4.03 (m, 4H), 2.99 - 2.82 (m, 4H), 2.80 - 2.70 (m, 1H), 2.51 - 2.40 (m, 1H), 2.18 - 2.00 (m, 6H). LCMS (ESI) calcd. for C₂₉H₃₂N₅O₄S⁺ [M+H]⁺: 546.22, found, 546.3.

### Example 187: Preparation of compound GT-10138

With reference to the method of Scheme 1, the target compound GT-10138 was obtained (16 mg, white solid, yield 34%). ¹H NMR (400 MHz, DMSO-d6) δ 7.58 - 7.53 (m, 2H), 7.34 (d, *J* = 3.8 Hz, 1H), 7.31 (d, *J* = 3.8 Hz, 1H), 7.01 (s, 1H), 6.93 (d, *J* = 8.3 Hz, 1H), 6.72 (d, *J* = 3.4 Hz, 1H), 6.53 (dd, *J* = 3.4, 1.9 Hz, 1H), 5.08 (dd, *J* = 13.3, 5.2 Hz, 1H), 4.68 (s, 2H), 4.41 - 4.31 (m, 2H), 3.67 - 3.61 (m, 2H), 3.56 - 3.45 (m, 2H), 3.30 - 3.23 (m, 2H), 3.13 - 3.02 (m, 2H), 2.93 - 2.83 (m, 1H), 2.82 - 2.71 (m, 1H), 2.43 - 2.39 (m, 1H), 2.27 - 2.01 (m, 3H). LCMS (ESI) calcd. for C₂₇H₃₀N₅O₄S⁺ [M+H]⁺: 520.20, found, 520.3.

### Example 188: Preparation of compound GT-10139

With reference to the method of Scheme 1, the target compound GT-10139 was obtained (23 mg, white solid, yield 49%). ¹H NMR (400 MHz, DMSO-d6) δ 7.58 - 7.54 (m, 2H), 7.35 (d, *J* = 3.7 Hz, 1H), 7.30 (d, *J* = 3.7 Hz, 1H), 7.04 (s, 1H), 6.95 (d, *J* = 8.4 Hz, 1H), 6.71 (d, *J* = 3.3 Hz, 1H), 6.53 (dd, *J* = 3.4, 1.8 Hz, 1H), 5.08 (dd, *J* = 13.2, 5.4 Hz, 1H), 4.71 (d, *J* = 14.0 Hz, 1H), 4.57 (d, *J* = 14.1 Hz, 1H), 4.40 - 4.34 (m, 3H), 3.80 (s, 1H), 3.75 (s, 1H), 3.58 - 3.44 (m, 1H), 3.37 (d, *J* = 9.9 Hz, 1H), 3.21 - 3.09 (m, 1H), 2.86 (dd, *J* = 13.3, 5.1 Hz, 1H), 2.76 (d, *J* = 17.3 Hz, 1H), 2.53 - 2.29 (m, 3H), 2.20 - 2.05 (m, 1H). LCMS (ESI) calcd. for C₂₇H₂₈N₅O₄S⁺ [M+H]⁺: 518.19, found, 518.2.

### Example 189: Preparation of compound GT-10141

With reference to the method of Scheme 1, the target compound GT-10141 was obtained (15 mg, white solid, yield 40%). ¹H NMR (400 MHz, DMSO-d6) δ 7.72 (dd, *J* = 7.0, 2.0 Hz, 1H), 7.63 - 7.59 (m, 2H), 7.56 (d, *J* = 8.5 Hz, 2H), 7.44 (dd, *J* = 5.2, 1.9 Hz, 1H), 7.41 (d, *J* = 8.5 Hz, 2H), 6.90 (d, *J* = 10.3 Hz, 1H), 6.79 (s, 1H), 6.75 (dd, *J* = 8.4, 1.8 Hz, 1H), 5.12 (dd, *J* = 13.4, 5.2 Hz, 1H), 4.55 (s, 2H), 4.45 - 4.33 (m, 3H), 3.67 (dd, *J* = 11.3, 7.9 Hz, 2H), 3.26 - 3.17 (m, 1H), 3.01 (d, *J* = 9.5 Hz, 1H), 2.87 - 2.83 (m, 1H), 2.83 - 2.75 (m, 1H), 2.74 - 2.64 (m, 3H), 2.61 - 2.54 (m, 2H), 2.48 (dd, *J* = 13.1, 4.7 Hz, 1H), 2.23 - 2.13 (m, 1H). LCMS (ESI) calcd. for C₃₂H₃₃ClN₅O₃⁺ [M+H]⁺: 570.23, found, 570.3.

### Example 190: Preparation of compound GT-10142

With reference to the method of Scheme 1, the target compound GT-10142 was obtained (19 mg, white solid, yield 51%). ¹H NMR (400 MHz, DMSO-d6) δ 7.86 (s, 1H), 7.80 (dt, *J* = 7.5, 1.5 Hz, 1H), 7.69 (d, *J* = 8.5 Hz, 2H), 7.65 - 7.53 (m, 3H), 7.51 - 7.46 (m, 2H), 6.97 - 6.90 (m, 2H), 5.09 (dd, *J* = 13.2, 5.2 Hz, 1H), 4.58 (s, 2H), 4.31 (s, 2H), 3.85 (dd, *J* = 11.0, 7.9 Hz, 2H), 3.16 - 3.04 (m, 4H), 3.02 - 2.94 (m, 2H), 2.88 (dd, *J* = 13.4, 5.1 Hz, 1H), 2.82 - 2.73 (m, 1H), 2.68 - 2.64 (m, 2H), 2.45 - 2.30 (m, 1H), 2.19 - 2.08 (m, 1H). LCMS (ESI) calcd. for C₃₂H₃₃ClN₅O₃⁺ [M+H]⁺: 570.23, found, 570.3.

### Example 191: Preparation of compound GT-10143

With reference to the method of Scheme 1, the target compound GT-10143 was obtained (12 mg, white solid, yield 33%). ¹H NMR (400 MHz, DMSO-d6) δ 7.55 (d, *J* = 8.3 Hz, 1H), 7.24 - 7.16 (m, 5H), 6.76 (s, 1H), 5.12 (dd, *J* = 13.5, 5.1 Hz, 1H), 4.39 - 4.30 (m, 2H), 3.84 (dd, *J* = 11.3, 8.0 Hz, 2H), 3.77 (s, 2H), 3.65 (s, 1H), 3.45 - 3.37 (m, 1H), 3.06 (d, *J* = 9.1 Hz, 1H), 3.00 - 2.88 (m, 4H), 2.84 - 2.75 (m, 1H), 2.62 - 2.57 (m, 4H), 2.46 (dd, *J* = 13.1, 4.8 Hz, 1H), 2.37 (brs, 3H), 2.29 (brs, 3H), 2.23 - 2.12 (m, 1H). LCMS (ESI) calcd. for C₃₂H₃₇FN₅O₃⁺ [M+H]⁺: 558.29, found, 558.3.

### Example 192: Preparation of compound GT-10144

With reference to the method of Scheme 1, the target compound GT-10144 was obtained (14 mg, white solid, yield 37%). ¹H NMR (400 MHz, DMSO-d6) δ 7.58 (d, *J* = 8.4 Hz, 1H), 7.49 (d, *J* = 8.3 Hz, 2H), 7.29 (d, *J* = 8.2 Hz, 2H), 6.81 (s, 1H), 6.78 (d, *J* = 8.5 Hz, 1H), 5.11 (dd, *J* = 13.2, 5.1 Hz, 1H), 4.41 - 4.35 (m, 4H), 3.94 (t, *J* = 5.4 Hz, 2H), 3.91 - 3.84 (m, 3H), 3.36 (brs, 1H), 3.21 - 3.13 (m, 1H), 2.99 - 2.94 (m, 3H), 2.81 (brs, 1H), 2.78 - 2.69 (m, 1H), 2.68 - 2.58 (m, 3H), 2.54 - 2.43 (m, 4H), 2.19 - 2.13 (m, 1H). LCMS (ESI) calcd. for C₃₁H₃₅ClN₅O₄⁺ [M+H]⁺: 576.24, found, 576.3.

### Example 193: Preparation of compound GT-10145

With reference to the method of Scheme 1, the target compound GT-10145 was obtained (11 mg, white solid, yield 30%). ¹H NMR (400 MHz, DMSO-d6) δ 7.55 (d, *J* = 8.4 Hz, 1H), 7.31 (d, *J* = 8.8 Hz, 2H), 7.22 (d, *J* = 8.8 Hz, 2H), 6.79 (s, 1H), 6.76 (d, *J* = 8.5 Hz, 1H), 5.12 (dd, *J* = 13.2, 5.0 Hz, 1H), 4.39 - 4.33 (m, 4H), 3.94 (t, *J* = 5.4 Hz, 2H), 3.87 - 3.83 (m, 3H), 3.36 (d, *J* = 5.2 Hz, 1H), 3.14 (d, *J* = 9.5 Hz, 1H), 2.96 - 2.87 (m, 3H), 2.81 - 2.78 (m, 2H), 2.68 - 2.56 (m, 3H), 2.54 - 2.42 (m, 4H), 2.22 - 2.11 (m, 1H). LCMS (ESI) calcd. for C₃₁H₃₅FN₅O₄⁺ [M+H]⁺: 560.27, found, 560.3.

### Example 194: Preparation of compound GT-10146

With reference to the method of Scheme 1, the target compound GT-10146 was obtained (20 mg, white solid, yield 50%). ¹H NMR (400 MHz, DMSO-d6) δ 7.58 (d, *J* = 8.3 Hz, 1H), 7.49 (d, *J* = 8.4 Hz, 2H), 7.26 (d, *J* = 8.3 Hz, 2H), 6.78 (s, 1H), 6.76 (d, *J* = 8.4 Hz, 1H), 5.11 (dd, *J* = 13.2, 5.2 Hz, 1H), 4.42 - 4.32 (m, 4H), 3.89 (s, 2H), 3.83 (dd, *J* = 11.2, 7.6 Hz, 2H), 3.36 (brs, 1H), 3.06 (d, *J* = 8.8 Hz, 1H), 2.95 - 2.92 (m, 3H), 2.83 - 2.73 (m, 1H), 2.68 - 2.55 (m, 4H), 2.47 (dd, *J* = 13.2, 4.6 Hz, 1H), 2.39 - 2.36 (m, 2H), 2.20 - 2.15 (m, 1H), 1.32 (s, 6H). LCMS (ESI) calcd. for C₃₃H₃₉ClN₅O₄⁺ [M+H]⁺: 604.27, found, 604.3.

### Example 195: Preparation of compound GT-10147

With reference to the method of Scheme 1, the target compound GT-10147 was obtained (16 mg, white solid, yield 40%). ¹H NMR (400 MHz, DMSO-d6) δ 7.58 (d, *J* = 8.0 Hz, 1H), 7.50 (d, *J* = 8.5 Hz, 2H), 7.25 (d, *J* = 8.3 Hz, 2H), 6.76 - 6.74 (m, 2H), 5.12 (dd, *J* = 13.2, 5.1 Hz, 1H), 4.38 (t, *J* = 10.0 Hz, 3H), 3.88 - 3.84 (m, 3H), 3.37 (d, *J* = 6.3 Hz, 1H), 3.04 (d, *J* = 8.5 Hz, 1H), 2.96 - 2.93 (m, 4H), 2.90 - 2.77 (m, 4H), 2.68 - 2.61 (m, 5H), 2.50 - 2.43 (m, 1H), 2.32 - 2.21 (m, 2H), 2.21 - 2.11 (m, 1H). LCMS (ESI) calcd. for C₃₂H₃₅ClFN₅O₃⁺ [M+H]⁺: 610.24, found, 610.3.

### Example 196: Preparation of compound GT-10148

With reference to the method of Scheme 1, the target compound GT-10148 was obtained (13 mg, white solid, yield 32%). ¹H NMR (400 MHz, DMSO-d6) δ 7.58 (d, *J* = 8.4 Hz, 1H), 7.46 (d, *J* = 8.5 Hz, 2H), 7.22 (d, *J* = 8.4 Hz, 2H), 6.78 (s, 1H), 6.76 (d, *J* = 8.4 Hz, 1H), 5.12 (dd, *J* = 13.2, 5.1 Hz, 1H), 4.42 - 4.32 (m, 2H), 3.87 - 3.81 (m, 2H), 3.77 (s, 2H), 3.35 - 3.31 (m, 1H), 3.29 (s, 3H), 3.17 - 3.08 (m, 1H), 2.97 - 2.93 (m, 3H), 291 - 2.88 (m, 2H), 2.81 - 2.71 (m, 3H), 2.66 - 2.54 (m, 3H), 2.50 - 2.45 (m, 1H), 2.35 - 2.31 (m, 2H), 2.22 - 2.13 (m, 1H), 2.05 - 2.00 (m, 1H), 1.80 - 1.71 (m, 1H), 1.34 (s, 3H). LCMS (ESI) calcd. for C₃₄H₄₁ClN₅O₄⁺ [M+H]⁺: 618.28, found, 618.3.

### Example 197: Preparation of compound GT-10149

With reference to the method of Scheme 1, the target compound GT-10149 was obtained (7 mg, white solid, yield 19%). ¹H NMR (400 MHz, DMSO-d6) δ 7.54 (d, *J* = 8.3 Hz, 1H), 7.24 - 7.13 (m, 5H), 6.77 - 6.74 (m, 1H), 5.12 (dd, *J* = 13.4, 5.2 Hz, 1H), 4.35 - 4.32 (m, 2H), 3.86 - 3.76 (m, 3H), 3.26 (dd, *J* = 11.8, 6.4 Hz, 1H), 3.02 - 2.97 (m, 4H), 2.85 - 2.77 (m, 1H), 2.63 - 2.55 (m, 4H), 2.49 - 2.41 (m, 1H), 2.33 (brs, 3H), 2.19 - 2.12 (m, 3H), 1.64 - 1.58 (m, 2H), 1.05 (s, 6H). LCMS (ESI) calcd. for C₃₄H₄₁FN₅O₃⁺ [M+H]⁺: 586.32, found, 586.4.

### Example 198: Preparation of compound GT-10232

With reference to the method of Scheme 1, the target compound GT-10232 was obtained (28 mg, white solid, yield 74%). ¹H NMR (400 MHz, MeOD) δ 7.88 - 7.79 (m, 1H), 7.63 - 7.51 (m, 5H), 7.47 - 7.38 (m, 3H), 6.94 (s, 1H), 6.88 (d, *J* = 8.4 Hz, 1H), 5.07 (dd, *J* = 13.6, 5.0 Hz, 1H), 4.42 - 4.28 (m, 4H), 3.82 (s, 2H), 3.05 (d, *J* = 12.8 Hz, 2H), 2.95 (d, *J* = 12.5 Hz, 2H), 2.86 (dd, *J* = 13.2, 5.1 Hz, 1H), 2.80 - 2.69 (m, 1H), 2.43 (dd, *J* = 13.1, 5.0 Hz, 1H), 2.22 - 2.06 (m, 3H), 1.57 - 1.44 (m, 2H). LCMS (ESI) calcd. for C₃₂H₃₃ClN₅O₃⁺ [M+H]⁺: 570.23, found, 570.3.

### Example 199: Preparation of compound GT-10233

With reference to the method of Scheme 1, the target compound GT-10233 was obtained (27 mg, white solid, yield 72%). ¹H NMR (400 MHz, MeOD) δ 7.90 (s, 1H), 7.78 (s, 1H), 7.72 - 7.64 (m, 2H), 7.61 - 7.56 (m, 3H), 7.48 (dd, *J* = 8.5, 1.6 Hz, 2H), 7.02 (s, 1H), 6.95 (d, *J* = 8.1 Hz, 1H), 5.08 (dd, *J* = 13.1, 3.7 Hz, 1H), 4.44 - 4.26 (m, 4H), 4.04 (s, 2H), 3.16 (d, *J* = 11.6 Hz, 2H), 3.06 (d, *J* = 11.9 Hz, 2H), 2.97 - 2.81 (m, 1H), 2.76 (d, *J* = 17.7 Hz, 1H), 2.56 - 2.33 (m, 5H), 2.19 - 2.05 (m, 1H). LCMS (ESI) calcd. for C₃₂H₃₃ClN₅O₃⁺ [M+H]⁺: 570.23, found, 570.3.

### Example 200: Preparation of compound GT-10234

With reference to the method of Scheme 1, the target compound GT-10234 was obtained (12 mg, white solid, yield 32%). ¹H NMR (400 MHz, MeOD) δ 7.55 (d, *J* = 8.4 Hz, 1H), 7.28 - 7.20 (m, 2H), 7.15 (t, *J* = 8.6 Hz, 2H), 6.96 (s, 1H), 6.90 (d, *J* = 8.4 Hz, 1H), 5.07 (dd, *J* = 13.5, 5.2 Hz, 1H), 4.43 - 4.26 (m, 2H), 3.89 (s, 2H), 3.64 (s, 2H), 3.11 - 2.98 (m, 4H), 2.91 - 2.81 (m, 1H), 2.77 - 2.67 (m, 1H), 2.45 - 2.35 (m, 3H), 2.31 (s, 2H), 2.23 - 2.07 (m, 3H), 1.82 (s, 4H), 1.54 - 1.40 (m, 2H). LCMS (ESI) calcd. for C₃₂H₃₇FN₅O₃⁺ [M+H]⁺: 558.29, found, 558.3.

### Example 201: Preparation of compound GT-10235

With reference to the method of Scheme 1, the target compound GT-10235 was obtained (13 mg, white solid, yield 34%). ¹H NMR (400 MHz, MeOD) δ 7.58 (d, *J* = 8.3 Hz, 1H), 7.52 (d, *J* = 8.3 Hz, 2H), 7.34 (d, *J* = 8.3 Hz, 2H), 7.00 (s, 1H), 6.94 (d, *J* = 8.1 Hz, 1H), 5.10 (dd, *J* = 13.3, 5.1 Hz, 1H), 4.48 - 4.27 (m, 5H), 3.97 - 3.92 (m, 3H), 3.71 (s, 2H), 3.15 - 3.05 (m, 4H), 2.97 - 2.85 (m, 1H), 2.76 (d, *J* = 15.7 Hz, 1H), 2.53 (s, 2H), 2.45 (dd, *J* = 13.2, 4.5 Hz, 1H), 2.21 - 2.06 (m, 3H), 1.46 - 1.44 (m, 2H). LCMS (ESI) calcd. for C₃₁H₃₅ClN₅O₄⁺ [M+H]⁺: 576.24, found, 576.3.

### Example 202: Preparation of compound GT-10236

With reference to the method of Scheme 1, the target compound GT-10236 was obtained (12 mg, white solid, yield 32%).¹H NMR (400 MHz, MeOD) δ 7.54 (d, *J* = 8.3 Hz, 1H), 7.35 - 7.28 (m, 2H), 7.22 - 7.17 (m, 2H), 6.95 (s, 1H), 6.89 (d, *J* = 8.4 Hz, 1H), 5.06 (dd, *J* = 13.3, 5.1 Hz, 1H), 4.38 (s, 2H), 4.33 (d, *J* = 7.7 Hz, 2H), 3.97 - 3.89 (m, 3H), 3.72 (s, 2H), 3.19 - 3.09 (m, 1H), 3.07 (s, 3H), 2.91 - 2.81 (m, 1H), 2.75 (d, *J* = 17.7 Hz, 1H), 2.51 (s, 2H), 2.46 - 2.37 (m, 1H), 2.33 - 2.30 (m, 1H), 2.18 - 2.05 (m, 3H), 1.45 - 1.42 (m, 2H). LCMS (ESI) calcd. for C₃₁H₃₅FN₅O₄⁺ [M+H]⁺: 560.27, found, 560.3.

### Example 203: Preparation of compound GT-10237

With reference to the method of Scheme 1, the target compound GT-10237 was obtained (12 mg, white solid, yield 30%). ¹H NMR (400 MHz, MeOD) δ 7.55 (d, *J* = 8.4 Hz, 1H), 7.47 (d, *J* = 8.3 Hz, 2H), 7.27 (d, *J* = 8.4 Hz, 2H), 6.95 (s, 1H), 6.89 (d, *J* = 8.4 Hz, 1H), 5.07 (dd, *J* = 13.3, 5.1 Hz, 1H), 4.40 (s, 2H), 4.34 (d, *J* = 7.2 Hz, 2H), 3.89 (s, 2H), 3.73 (s, 2H), 3.14 - 2.97 (m, 4H), 2.92 - 2.81 (m, 1H), 2.75 (d, *J* = 15.4 Hz, 1H), 2.43 (dd, *J* = 13.2, 4.7 Hz, 1H), 2.39 (s, 2H), 2.16 - 2.11 (m, 3H), 1.51 - 1.40 (m, 2H), 1.31 (s, 6H). LCMS (ESI) calcd. for C₃₃H₃₉ClN₅O₄⁺ [M+H]⁺: 604.27, found, 604.3.

### Example 204: Preparation of compound GT-10238

With reference to the method of Scheme 1, the target compound GT-10238 was obtained (12 mg, white solid, yield 30%). ¹H NMR (400 MHz, MeOD) δ 7.55 (d, *J* = 9.0 Hz, 1H), 7.47 (d, *J* = 7.3 Hz, 2H), 7.26 (d, *J* = 7.6 Hz, 2H), 6.96 (s, 1H), 6.90 (d, *J* = 8.9 Hz, 1H), 5.07 (dd, *J* = 12.4, 4.2 Hz, 1H), 4.34 (q, *J* = 17.3 Hz, 2H), 3.91 (s, 2H), 3.69 (s, 2H), 3.15 - 3.02 (m, 3H), 2.94 - 2.85 (m, 3H), 2.81 - 2.60 (m, 4H), 2.52 - 2.36 (m, 1H), 2.33 - 2.06 (m, 5H), 1.49 - 1.38 (m, 2H). LCMS (ESI) calcd. for C₃₂H₃₅ClF₂N₅O₃⁺ [M+H]⁺: 610.24, found, 610.3.

### Example 205: Preparation of compound GT-10239

With reference to the method of Scheme 1, the target compound GT-10239 was obtained (8 mg, white solid, yield 20%). ¹H NMR (400 MHz, MeOD) δ 7.56 (d, *J* = 8.5 Hz, 1H), 7.44 (d, *J* = 7.9 Hz, 2H), 7.22 (d, *J* = 8.1 Hz, 2H), 6.96 (s, 1H), 6.90 (d, *J* = 8.4 Hz, 1H), 5.07 (dd, *J* = 13.0, 4.7 Hz, 1H), 4.35 (q, *J* = 17.0 Hz, 2H), 3.91 (s, 2H), 3.64 (s, 2H), 3.33 (s, 3H), 3.12 - 3.01 (m, 4H), 2.86 (dd, *J* = 14.1, 5.9 Hz, 1H), 2.76 (d, *J* = 17.3 Hz, 1H), 2.61 - 2.48 (m, 1H), 2.47 - 2.29 (m, 4H), 2.21 - 2.01 (m, 4H), 1.82 - 1.65 (m, 1H), 1.52 - 1.40 (m, 2H), 1.35 (s, 3H). LCMS (ESI) calcd. for C₃₄H₄₁ClN₅O₄⁺ [M+H]⁺: 618.28, found, 618.3.

### Example 206: Preparation of compound GT-10240

With reference to the method of Scheme 1, the target compound GT-10240 was obtained (9 mg, white solid, yield 20%). ¹H NMR (400 MHz, MeOD) δ 7.55 (d, *J* = 8.3 Hz, 1H), 7.25 - 7.12 (m, 4H), 6.95 (s, 1H), 6.90 (d, *J* = 7.9 Hz, 1H), 5.07 (dd, *J* = 13.2, 5.1 Hz, 1H), 4.41 - 4.25 (m, 2H), 3.84 (s, 2H), 3.66 (s, 2H), 3.12 - 3.03 (m, 3H), 2.91 - 2.84 (m, 1H), 2.80 - 2.71 (m, 1H), 2.47 - 2.40 (m, 1H), 2.39 - 2.34 (m, 2H), 2.32 - 2.26 (m, 1H), 2.23 - 2.06 (m, 5H), 1.61 (t, *J* = 6.3 Hz, 2H), 1.52 - 1.38 (m, 2H), 1.03 (s, 6H). LCMS (ESI) calcd. for C₃₄H₃₄₁FN₅O₃⁺ [M+H]⁺: 586.32, found, 586.4.

### Example 207: Preparation of compound GT-10241

With reference to the method of Scheme 1, the target compound GT-10241 was obtained (22 mg, white solid, yield 59%). ¹H NMR (400 MHz, MeOD) δ 7.61 - 7.57 (m, 2H), 7.56 - 7.49 (m, 4H), 7.43 - 7.37 (m, 3H), 7.12 (s, 1H), 7.01 (d, *J* = 8.4 Hz, 1H), 5.08 (dd, *J* = 13.2, 5.0 Hz, 1H), 4.49 (d, *J* = 8.3 Hz, 2H), 4.36 (t, *J* = 12.7 Hz, 2H), 4.02 (d, *J* = 10.5 Hz, 2H), 3.74 (d, *J* = 10.7 Hz, 2H), 3.04 - 2.69 (m, 6H), 2.43 - 2.40 (m, 1H), 2.18 - 2.00 (m, 3H), 1.82 (s, 2H). LCMS (ESI) calcd. for C₃₃H₃₅ClN₅O₃⁺ [M+H]⁺: 584.24, found, 584.3.

### Example 208: Preparation of compound GT-10242

With reference to the method of Scheme 1, the target compound GT-10242 was obtained (23 mg, white solid, yield 62%). ¹H NMR (400 MHz, MeOD) δ 7.81 (s, 1H), 7.74 (d, *J* = 7.5 Hz, 1H), 7.67 (d, *J* = 8.5 Hz, 2H), 7.64 - 7.58 (m, 1H), 7.54 (dd, *J* = 14.5, 7.7 Hz, 2H), 7.47 (d, *J* = 8.4 Hz, 2H), 7.15 (s, 1H), 7.04 (d, *J* = 7.7 Hz, 1H), 5.08 (dd, *J* = 13.1, 4.9 Hz, 1H), 4.50 (s, 2H), 4.39 (q, *J* = 17.3 Hz, 2H), 4.12 (d, *J* = 10.6 Hz, 2H), 4.04 (d, *J* = 10.0 Hz, 2H), 3.13 - 2.92 (m, 3H), 2.91 - 2.84 (m, 2H), 2.75 (d, *J* = 15.5 Hz, 1H), 2.44 - 2.40 (m, 1H), 2.123 - 2.11 (m, 5H). LCMS (ESI) calcd. for C₃₃H₃₅ClN₅O₃⁺ [M+H]⁺: 584.24, found, 584.3.

### Example 209: Preparation of compound GT-10243

With reference to the method of Scheme 1, the target compound GT-10243 was obtained (15 mg, white solid, yield 41%). ¹H NMR (400 MHz, MeOD) δ 7.63 (d, *J* = 8.4 Hz, 1H), 7.28 - 7.20 (m, 2H), 7.19 - 7.11 (m, 3H), 7.07 (d, *J* = 7.2 Hz, 1H), 5.09 (dd, *J* = 13.3, 5.1 Hz, 1H), 4.40 (q, *J* = 16.9 Hz, 2H), 4.10 (d, *J* = 11.1 Hz, 2H), 3.78 (s, 2H), 3.68 (d, *J* = 11.2 Hz, 2H), 3.06 (brs, 2H), 2.98 - 2.82 (m, 3H), 2.76 (d, *J* = 17.4 Hz, 1H), 2.46 (dd, *J* = 13.3, 4.6 Hz, 1H), 2.36 (s, 2H), 2.16 (brs, 5H), 1.78 (brs, 6H). LCMS (ESI) calcd. for C₃₃H₃₉FN₅O₃⁺ [M+H]⁺: 572.30, found, 572.3.

### Example 210: Preparation of compound GT-10244

With reference to the method of Scheme 1, the target compound GT-10244 was obtained (20 mg, white solid, yield 53%). ¹H NMR (400 MHz, MeOD) δ 7.62 (d, *J* = 8.4 Hz, 1H), 7.47 (d, *J* = 8.3 Hz, 2H), 7.28 (d, *J* = 8.3 Hz, 2H), 7.14 (s, 1H), 7.03 (d, *J* = 8.1 Hz, 1H), 5.09 (dd, *J* = 13.2, 5.1 Hz, 1H), 4.39 (q, *J* = 17.0 Hz, 2H), 4.23 (s, 2H), 4.10 (d, *J* = 10.8 Hz, 2H), 3.92 - 3.86 (m, 4H), 3.66 (d, *J* = 11.1 Hz, 2H), 3.11 - 2.94 (m, 2H), 2.92 - 2.84 (m, 2H), 2.76 (d, *J* = 15.8 Hz, 2H), 2.54 - 2.42 (m, 3H), 2.18 - 2.04 (m, 3H), 1.78 (brs, 2H). LCMS (ESI) calcd. for C₃₂H₃₇ClN₅O₄⁺ [M+H]⁺: 590.25, found, 590.3.

### Example 211: Preparation of compound GT-10245

With reference to the method of Scheme 1, the target compound GT-10245 was obtained (17 mg, white solid, yield 46%). ¹H NMR (400 MHz, MeOD) δ 7.65 (d, *J* = 8.4 Hz, 1H), 7.34 - 7.27 (m, 2H), 7.24 - 7.16 (m, 3H), 7.08 (dd, *J* = 8.3, 1.8 Hz, 1H), 5.09 (dd, *J* = 13.3, 5.1 Hz, 1H), 4.41 (q, *J* = 17.1 Hz, 2H), 4.24 (s, 2H), 4.11 (d, *J* = 11.2 Hz, 2H), 3.90 (t, *J* = 5.5 Hz, 2H), 3.87 (s, 2H), 3.67 (d, *J* = 11.1 Hz, 2H), 3.19 - 3.12 (m, 2H), 2.98 - 2.92 (m, 2H), 2.87 (dd, *J* = 13.4, 5.3 Hz, 1H), 2.82 - 2.68 (m, 1H), 2.53 - 2.40 (m, 3H), 2.23 - 2.08 (m, 3H), 1.82 (s, 2H). LCMS (ESI) calcd. for C₃₂H₃₇FN₅O₄⁺ [M+H]⁺: 574.28, found, 574.3.

### Example 212: Preparation of compound GT-10246

With reference to the method of Scheme 1, the target compound GT-10246 was obtained (21 mg, white solid, yield 51%). ¹H NMR (400 MHz, MeOD) δ 7.62 (d, *J* = 8.4 Hz, 1H), 7.47 (d, *J* = 8.5 Hz, 2H), 7.26 (d, *J* = 8.5 Hz, 2H), 7.16 (s, 1H), 7.04 (d, *J* = 8.4 Hz, 1H), 5.09 (dd, *J* = 13.2, 5.1 Hz, 1H), 4.39 (q, *J* = 17.0 Hz, 2H), 4.26 (s, 2H), 4.10 (d, *J* = 10.7 Hz, 2H), 3.88 (s, 2H), 3.67 (d, *J* = 10.9 Hz, 2H), 3.02 (s, 2H), 2.96 - 2.81 (m, 3H), 2.76 (d, *J* = 15.5 Hz, 1H), 2.50 - 2.41 (m, 1H), 2.34 (s, 2H), 2.19 - 2.08 (m, 3H), 1.78 (s, 2H), 1.30 (s, 6H). LCMS (ESI) calcd. for C₃₄H₄₁ClN₅O₄⁺ [M+H]⁺: 618.28, found, 618.3.

### Example 213: Preparation of compound GT-10247

With reference to the method of Scheme 1, the target compound GT-10247 was obtained (16 mg, white solid, yield 40%). ¹H NMR (400 MHz, MeOD) δ 7.60 (d, *J* = 8.3 Hz, 1H), 7.47 (d, *J* = 7.5 Hz, 2H), 7.25 (d, *J* = 7.6 Hz, 2H), 7.12 (s, 1H), 7.01 (d, *J* = 8.0 Hz, 1H), 5.09 (dd, *J* = 13.2, 4.8 Hz, 1H), 4.38 (q, *J* = 17.4 Hz, 2H), 4.11 (d, *J* = 10.7 Hz, 2H), 3.83 (s, 2H), 3.68 (d, *J* = 11.2 Hz, 2H), 3.03 - 2.82 (m, 3H), 2.82 - 2.69 (m, 4H), 2.65 (s, 3H), 2.53 - 2.37 (m, 1H), 2.24 - 2.01 (m, 5H), 1.75 (s, 2H). LCMS (ESI) calcd. for C₃₃H₃₇ClF₂N₅O₃⁺ [M+H]⁺: 624.25, found, 624.3.

### Example 214: Preparation of compound GT-10248

With reference to the method of Scheme 1, the target compound GT-10248 was obtained (14 mg, white solid, yield 35%). ¹H NMR (400 MHz, MeOD) δ 7.61 (d, *J* = 8.3 Hz, 1H), 7.44 (d, *J* = 8.4 Hz, 2H), 7.21 (d, *J* = 8.4 Hz, 2H), 7.14 (s, 1H), 7.03 (d, *J* = 6.9 Hz, 1H), 5.09 (dd, *J* = 13.2, 4.9 Hz, 1H), 4.39 (q, *J* = 16.7 Hz, 2H), 4.08 (d, *J* = 10.8 Hz, 2H), 3.77 (s, 2H), 3.67 (t, *J* = 12.1 Hz, 2H), 3.27 (s, 3H), 3.07 - 2.94 (m, 2H), 2.95 - 2.69 (m, 4H), 2.53 - 2.43 (m, 2H), 2.32 (d, *J* = 18.3 Hz, 1H), 2.25 (s, 2H), 2.17 - 2.09 (m, 3H), 2.02 - 1.96 (m, 1H), 1.73 (s, 3H), 1.30 (s, 3H). LCMS (ESI) calcd. for C₃₅H₄₃ClN₅O₄⁺ [M+H]⁺: 632.30, found, 632.4.

### Example 215: Preparation of compound GT-10249

With reference to the method of Scheme 1, the target compound GT-10249 was obtained (11 mg, white solid, yield 29%). ¹H NMR (400 MHz, MeOD) δ 7.60 (d, *J* = 8.4 Hz, 1H), 7.22 - 7.14 (m, 4H), 7.11 (s, 1H), 7.01 (d, *J* = 8.3 Hz, 1H), 5.08 (dd, *J* = 13.2, 5.1 Hz, 1H), 4.38 (q, *J* = 17.2 Hz, 2H), 4.08 (d, *J* = 11.0 Hz, 2H), 3.80 (s, 2H), 3.65 (d, *J* = 11.1 Hz, 2H), 3.08 - 2.61 (m, 6H), 2.49 - 2.40 (m, 1H), 2.20 (s, 2H), 2.14 - 2.05 (m, 5H), 1.72 (s, 2H), 1.55 (t, *J* = 6.1 Hz, 2H), 1.02 (s, 6H). LCMS (ESI) calcd. for C₃₅H₄₃FN₅O₃⁺ [M+H]⁺: 600.33, found, 600.4.

### Example 216: Preparation of compound GT-10251

With reference to the method of Scheme 1, the target compound GT-10251 was obtained (22 mg, white solid, yield 58%). ¹H NMR (400 MHz, MeOD) δ 7.79 - 7.77 (m, 1H), 7.57 - 7.54 (m, 5H), 7.43 - 7.31 (m, 3H), 6.91 (s, 1H), 6.83 (d, *J* = 8.1 Hz, 1H), 5.07 (dd, *J* = 12.5, 3.7 Hz, 1H), 4.47 (s, 2H), 4.34 (q, *J* = 17.0 Hz, 2H), 3.43 - 3.40 (m, 2H), 3.21 - 3.14 (m, 2H), 3.11 - 3.01 (m, 1H), 3.01 - 2.87 (m, 3H), 2.88 - 2.73 (m, 2H), 2.49 - 2.40 (m, 1H), 2.20 - 2.04 (m, 1H), 1.87 (s, 2H). LCMS (ESI) calcd. for C₃₁H₃₃ClN₅O₃⁺ [M+H]⁺: 558.23, found, 558.3.

### Example 217: Preparation of compound GT-10252

With reference to the method of Scheme 1, the target compound GT-10252 was obtained (25 mg, white solid, yield 66%). ¹H NMR (400 MHz, MeOD) δ 7.87 (s, 1H), 7.79 - 7.76 (m, 1H), 7.71 - 7.67 (m, 2H), 7.63 - 7.52 (m, 3H), 7.51 - 7.43 (m, 2H), 7.02 (s, 1H), 6.94 (d, *J* = 6.7 Hz, 1H), 5.07 (dd, *J* = 16.1, 3.9 Hz, 1H), 4.52 (s, 2H), 4.42 - 4.25 (m, 2H), 3.67 - 3.55 (m, 2H), 3.55 - 3.43 (m, 2H), 3.37 - 3.35 (m, 1H), 3.25 - 3.21 (m, 2H), 3.16 - 2.98 (m, 1H), 2.87 (d, *J* = 13.8 Hz, 1H), 2.76 (d, *J* = 16.5 Hz, 1H), 2.49 - 2.35 (m, 1H), 2.25 - 2.16 (m, 1H), 2.16 - 2.02 (m, 2H). LCMS (ESI) calcd. for C₃₁H₃₃ClN₅O₃⁺ [M+H]⁺: 558.23, found, 558.3.

### Example 218: Preparation of compound GT-10253

With reference to the method of Scheme 1, the target compound GT-10253 was obtained (13 mg, white solid, yield 35%). ¹H NMR (400 MHz, MeOD) δ 7.53 (d, *J* = 8.2 Hz, 1H), 7.25 - 7.09 (m, 4H), 6.93 (s, 1H), 6.85 (d, *J* = 8.3 Hz, 1H), 5.07 (dd, *J* = 12.8, 2.4 Hz, 1H), 4.42 - 4.26 (m, 2H), 3.71 (s, 2H), 3.57 - 3.46 (m, 2H), 3.21 - 3.09 (m, 3H), 3.01 - 2.84 (m, 3H), 2.85 - 2.73 (m, 2H), 2.52 - 2.41 (m, 1H), 2.36 (s, 2H), 2.23 (s, 2H), 2.16 - 2.09 (m, 1H), 1.99 - 1.90 (m, 1H), 1.81 (brs, 5H). LCMS (ESI) calcd. for C₃₁H₃₇FN₅O₃⁺ [M+H]⁺: 546.29, found, 546.3.

### Example 219: Preparation of compound GT-10254

With reference to the method of Scheme 1, the target compound GT-10254 was obtained (22 mg, white solid, yield 57%). ¹H NMR (400 MHz, MeOD) δ 7.54 (d, *J* = 8.4 Hz, 1H), 7.47 (d, *J* = 8.3 Hz, 2H), 7.24 (d, *J* = 8.4 Hz, 2H), 6.93 (s, 1H), 6.84 (d, *J* = 8.7 Hz, 1H), 5.07 (dd, *J* = 13.5, 4.3 Hz, 1H), 4.39 - 4.31 (m, 4H), 3.92 (t, *J* = 5.4 Hz, 2H), 3.80 (s, 2H), 3.57 - 3.45 (m, 2H), 3.21 - 3.08 (m, 3H), 2.97 - 2.86 (m, 3H), 2.76 (d, *J* = 17.9 Hz, 1H), 2.56 - 2.34 (m, 4H), 2.16 - 2.07 (m, 1H), 2.01 - 1.77 (m, 2H). LCMS (ESI) calcd. for C₃₀H₃₅ClN₅O₄⁺ [M+H]⁺: 564.24, found, 564.3.

### Example 220: Preparation of compound GT-10255

With reference to the method of Scheme 1, the target compound GT-10255 was obtained (20 mg, white solid, yield 54%). ¹H NMR (400 MHz, MeOD) δ 7.53 (d, *J* = 8.4 Hz, 1H), 7.27 (dd, *J* = 8.6, 5.4 Hz, 2H), 7.19 (t, *J* = 8.7 Hz, 2H), 6.94 (s, 1H), 6.84 (d, *J* = 8.4 Hz, 1H), 5.07 (dd, *J* = 13.3, 5.1 Hz, 1H), 4.40 - 4.27 (m, 4H), 3.92 (t, *J* = 5.5 Hz, 2H), 3.80 (s, 2H), 3.60 - 3.43 (m, 2H), 3.21 - 3.10 (m, 3H), 3.02 - 2.83 (m, 4H), 2.80 - 2.71 (m, 1H), 2.53 - 2.39 (m, 3H), 2.15 - 2.11 (m, 1H), 1.93 (brs, 1H), 1.85 (brs, 1H). LCMS (ESI) calcd. for C₃₀H₃₅FN₅O₄⁺ [M+H]⁺: 548.27, found, 548.3.

### Example 221: Preparation of compound GT-10256

With reference to the method of Scheme 1, the target compound GT-10256 was obtained (23 mg, white solid, yield 57%). ¹H NMR (400 MHz, MeOD) δ 7.54 (d, *J* = 8.5 Hz, 1H), 7.46 (d, *J* = 7.7 Hz, 2H), 7.22 (d, *J* = 7.7 Hz, 2H), 6.94 (s, 1H), 6.85 (d, *J* = 7.6 Hz, 1H), 5.08 (dd, *J* = 12.6, 3.9 Hz, 1H), 4.45 - 4.25 (m, 4H), 3.81 (s, 2H), 3.61 - 3.43 (m, 2H), 3.24 - 3.06 (m, 3H), 3.06 - 2.84 (m, 4H), 2.76 (d, *J* = 17.2 Hz, 1H), 2.52 - 2.37 (m, 1H), 2.35 (s, 2H), 2.22 - 2.05 (m, 1H), 1.94 (brs, 1H), 1.87 (brs, 1H), 1.30 (s, 6H). LCMS (ESI) calcd. for C₃₂H₃₉ClN₅O₄⁺ [M+H]⁺: 592.27, found, 592.3.

### Example 222: Preparation of compound GT-10257

With reference to the method of Scheme 1, the target compound GT-10257 was obtained (18 mg, white solid, yield 44%). ¹H NMR (400 MHz, MeOD) δ 7.54 (d, *J* = 8.4 Hz, 1H), 7.47 (d, *J* = 8.3 Hz, 2H), 7.20 (d, *J* = 8.3 Hz, 2H), 6.93 (s, 1H), 6.84 (d, *J* = 8.1 Hz, 1H), 5.08 (dd, *J* = 13.1, 4.6 Hz, 1H), 4.42 - 4.25 (m, 2H), 3.76 (s, 2H), 3.61 - 3.43 (m, 2H), 3.25 - 3.08 (m, 3H), 3.06 - 2.74 (m, 7H), 2.67 (s, 2H), 2.46 - 2.40 (m, 1H), 2.31 - 2.17 (m, 2H), 2.14 - 2.10 (m, 1H), 1.95 (brs, 1H), 1.86 (brs, 1H). LCMS (ESI) calcd. for C₃₁H₃₅ClF₂N₅O₃⁺ [M+H]⁺: 598.24, found, 598.3.

### Example 223: Preparation of compound GT-10258

With reference to the method of Scheme 1, the target compound GT-10258 was obtained (11 mg, white solid, yield 27%). ¹H NMR (400 MHz, MeOD) δ 7.53 (d, *J* = 8.1 Hz, 1H), 7.43 (d, *J* = 7.9 Hz, 2H), 7.17 (d, *J* = 8.0 Hz, 2H), 6.94 (s, 1H), 6.85 (d, *J* = 7.1 Hz, 1H), 5.07 (dd, *J* = 13.1, 4.6 Hz, 1H), 4.34 (q, *J* = 17.1 Hz, 3H), 3.70 (s, 2H), 3.61 - 3.48 (m, 2H), 3,21 (s, 3H), 3.18 - 3.11 (m, 2H), 3.01 - 2.85 (m, 4H), 2.76 (d, *J* = 15.7 Hz, 1H), 2.53 - 2.45 (m, 2H), 2.35 - 2.29 (m, 4H), 2.18 - 2.11 (m, 1H), 2.05 - 1.98 (m, 2H), 1.86 (brs, 1H), 1.79 - 1.71 (m, 1H), 1.32 (s, 3H). LCMS (ESI) calcd. for C₃₃H₄₁ClN₅O₄⁺ [M+H]⁺: 606.28, found, 606.3.

### Example 224: Preparation of compound GT-10259

With reference to the method of Scheme 1, the target compound GT-10259 was obtained (16 mg, white solid, yield 41%). ¹H NMR (400 MHz, MeOD) δ 7.53 (d, *J* = 8.0 Hz, 1H), 7.23 - 7.11 (m, 4H), 6.93 (s, 1H), 6.84 (d, *J* = 7.9 Hz, 1H), 5.07 (dd, *J* = 14.8, 6.9 Hz, 1H), 4.34 (q, *J* = 16.9 Hz, 2H), 3.73 (s, 2H), 3.61 - 3.44 (m, 2H), 3.26 - 3.07 (m, 3H), 3.04 - 2.84 (m, 4H), 2.76 (d, *J* = 15.5 Hz, 1H), 2.52 - 2.37 (m, 1H), 2.27 (s, 2H), 2.14 (s, 3H), 1.92 (s, 1H), 1.85 (s, 1H), 1.58 (t, *J* = 6.0 Hz, 2H), 1.02 (s, 6H). LCMS (ESI) calcd. for C₃₃H₄₁FN₅O₃⁺ [M+H]⁺: 574.32, found, 574.4.

### Example 225: Preparation of compound GT-10287

With reference to the method of Scheme 1, the target compound GT-10287 was obtained (25 mg, white solid, yield 66%). ¹H NMR (400 MHz, MeOD) δ 7.81 (s, 1H), 7.62 - 7.50 (m, 5H), 7.46 - 7.33 (m, 3H), 6.99 (s, 1H), 6.90 (d, *J* = 8.0 Hz, 1H), 5.08 (dd, *J* = 14.8, 6.9 Hz,, 1H), 4.53 - 4.26 (m, 4H), 3.95 (s, 1H), 3.58 (s, 1H), 3.45 (d, *J* = 11.8 Hz, 1H), 3.05 (d, *J* = 11.6 Hz, 1H), 2.99 (brs, 1H), 2.92 - 2.81 (m, 1H), 2.79 - 2.72 (m, 1H), 2.48 - 2.41 (m, 1H), 2.34 (d, *J* = 11.7 Hz, 1H), 2.20 - 1.97 (m, 2H), 1.38 (d, *J* = 11.9 Hz, 1H). LCMS (ESI) calcd. for C₃₁H₃₁ClN₅O₃⁺ [M+H]⁺: 556.21, found, 556.3.

### Example 226: Preparation of compound GT-10288

With reference to the method of Scheme 1, the target compound GT-10288 was obtained (26 mg, white solid, yield 69%). ¹H NMR (400 MHz, MeOD) δ 7.90 (s, 1H), 7.79 - 7.75 (m, 1H), 7.69 (d, *J* = 8.3 Hz, 2H), 7.60 (t, *J* = 8.7 Hz, 2H), 7.52 (d, *J* = 8.3 Hz, 1H), 7.47 (d, *J* = 8.3 Hz, 2H), 7.03 (s, 1H), 6.94 (d, *J* = 8.2 Hz, 1H), 5.14 - 5.00 (m, 1H), 4.67 - 4.64 (m, 1H), 4.57 (d, *J* = 13.0 Hz, 1H), 4.47 - 4.24 (m, 4H), 3.79 - 3.73 (m, 1H), 3.65 - 3.54 (m, 1H), 3.51 - 3.45 (m, 1H), 3.12 (brs, 1H), 2.96 - 2.82 (m, 1H), 2.77 - 3.73 (m, 1H), 2.55 - 2.30 (m, 3H), 2.20 - 2.04 (m, 1H). LCMS (ESI) calcd. for C₃₁H₃₁ClN₅O₃⁺ [M+H]⁺: 556.21, found, 556.3.

### Example 227: Preparation of compound GT-10289

With reference to the method of Scheme 1, the target compound GT-10289 was obtained (14 mg, white solid, yield 38%). ¹H NMR (400 MHz, MeOD) δ 7.53 (d, *J* = 8.3 Hz, 1H), 7.31 - 7.10 (m, 4H), 7.02 (s, 1H), 6.93 (d, *J* = 8.1 Hz, 1H), 5.20 - 5.00 (m, 1H), 4.34 (q, *J* = 17.1 Hz, 2H), 4.20 (d, *J* = 20.3 Hz, 1H), 3.94 - 3.67 (m, 2H), 3.59 (s, 2H), 3.56 - 3.43 (m, 1H), 3.22 - 2.96 (m, 2H), 2.96 - 2.82 (m, 1H), 2.82 - 2.65 (m, 1H), 2.57 - 2.25 (m, 5H), 2.22 - 2.00 (m, 2H), 1.81 (brs, 4H), 1.28 (d, *J* = 10.5 Hz, 1H). LCMS (ESI) calcd. for C₃₁H₃₅FN₅O₃⁺ [M+H]⁺: 544.27, found, 544.3.

### Example 228: Preparation of compound GT-10290

With reference to the method of Scheme 1, the target compound GT-10290 was obtained (25 mg, white solid, yield 65%). ¹H NMR (400 MHz, MeOD) δ 7.59 - 7.43 (m, 3H), 7.35 - 7.22 (m, 2H), 7.01 (s, 1H), 6.92 (d, *J* = 7.8 Hz, 1H), 5.17 - 5.02 (m, 1H), 4.36 (s, 3H), 4.34 - 4.15 (m, 2H), 3.93 (s, 2H), 3.83 (d, *J* = 13.8 Hz, 2H), 3.76 - 3.45 (m, 3H), 3.18 - 2.98 (m, 2H), 2.96 - 2.71 (m, 3H), 2.55 - 2.44 (m, 3H), 2.41 - 2.27 (m, 1H), 2.21 - 2.02 (m, 2H), 1.30 (d, *J* = 11.2 Hz, 1H). LCMS (ESI) calcd. for C₃₀H₃₃ClN₅O₄⁺ [M+H]⁺: 562.22, found, 562.3.

### Example 229: Preparation of compound GT-10291

With reference to the method of Scheme 1, the target compound GT-10291 was obtained (18 mg, white solid, yield 48%). ¹H NMR (400 MHz, MeOD) δ 7.53 (d, *J* = 8.2 Hz, 1H), 7.43 - 7.12 (m, 4H), 7.01 (s, 1H), 6.92 (d, *J* = 7.1 Hz, 1H), 5.16 - 5.02 (m, 1H), 4.36 (s, 4H), 4.25 - 4.20 (m, 1H), 3.93 (s, 2H), 3.84 (d, *J* = 12.8 Hz, 1H), 3.75 - 3.45 (m, 3H), 3.21 - 2.98 (m, 2H), 2.98 - 2.72 (m, 2H), 2.62 - 2.25 (m, 4H), 2.23 - 2.00 (m, 2H), 1.28 (d, *J* = 10.7 Hz, 1H). LCMS (ESI) calcd. for C₃₀H₃₃FN₅O₄⁺ [M+H]⁺: 546.25, found, 546.3.

### Example 230: Preparation of compound GT-10292

With reference to the method of Scheme 1, the target compound GT-10292 was obtained (22 mg, white solid, yield 55%). ¹H NMR (400 MHz, MeOD) δ 7.61 - 7.42 (m, 3H), 7.36 - 7.20 (m, 2H), 7.01 (s, 1H), 6.92 (d, *J* = 7.2 Hz, 1H), 5.17 - 5.03 (m, 1H), 4.37 (s, 4H), 4.22 - 6.16 (m, 1H), 4.01 - 3.76 (m, 2H), 3.75 - 3.44 (m, 3H), 3.14 - 2.98 (m, 2H), 2.96 - 2.85 (m, 1H), 2.82 - 2.69 (m, 1H), 2.52 - 2.33 (m, 4H), 2.22 - 1.99 (m, 2H), 1.31 (s, 6H). LCMS (ESI) calcd. for C₃₂H₃₇ClN₅O₄⁺ [M+H]⁺: 590.25, found, 590.3.

### Example 231: Preparation of compound GT-10293

With reference to the method of Scheme 1, the target compound GT-10293 was obtained (20 mg, white solid, yield 49%). ¹H NMR (400 MHz, MeOD) δ 7.64 - 7.42 (m, 3H), 7.29 - 7.21 (m, 2H), 7.01 (s, 1H), 6.93 (d, *J* = 8.1 Hz, 1H), 5.18 - 5.02 (m, 1H), 4.49 - 4.32 (m, 2H), 4.32 - 4.15 (m, 1H), 3.99 - 3.73 (m, 2H), 3.73 - 3.44 (m, 3H), 3.20 - 2.95 (m, 2H), 2.96 - 2.56 (m, 7H), 2.56 - 2.35 (m, 1H), 2.35 - 2.18 (m, 2H), 2.18 - 2.01 (m, 2H). LCMS (ESI) calcd. for C₃₁H₃₃ClF₂N₅O₃⁺ [M+H]⁺: 596.22, found, 596.3.

### Example 232: Preparation of compound GT-10294

With reference to the method of Scheme 1, the target compound GT-10294 was obtained (20 mg, white solid, yield 49%). ¹H NMR (400 MHz, MeOD) δ 7.54 - 7.43 (m, 3H), 7.25 - 7.18 (m, 2H), 7.03 (s, 1H), 6.93 (d, *J* = 7.2 Hz, 1H), 5.16 - 5.02 (m, 1H), 4.49 - 4.31 (m, 2H), 4.24 - 4.14 (m, 1H), 3.95 - 3.69 (m, 2H), 3.61 - 3.51 (m, 3H), 3.28 (s, 3H), 3.19 - 2.95 (m, 2H), 2.93 - 2.74 (m, 3H), 2.52 - 2.30 (m, 5H), 2.23 - 1.96 (m, 3H), 1.86 - 1.68 (m, 1H), 1.33 (s, 3H). LCMS (ESI) calcd. for C₃₃H₃₉ClN₅O₄⁺ [M+H]⁺: 604.27, found, 604.3.

### Example 233: Preparation of compound GT-10295

With reference to the method of Scheme 1, the target compound GT-10295 was obtained (16 mg, white solid, yield 41%). ¹H NMR (400 MHz, MeOD) δ 7.53 (d, *J* = 8.2 Hz, 1H), 7.30 - 7.09 (m, 4H), 7.01 (s, 1H), 6.93 (d, *J* = 8.0 Hz, 1H), 5.16 - 5.01 (m, 1H), 4.45 - 4.33 (m, 2H), 4.16 (d, *J* = 27.3 Hz, 1H), 3.76 (d, *J* = 13.8 Hz, 1H), 3.63 (d, *J* = 7.0 Hz, 1H), 3.61 - 3.41 (m, 2H), 3.13 - 3.01 (m, 2H), 2.95 - 2.62 (m, 3H), 2.56 - 2.34 (m, 2H), 2.30 (s, 2H), 2.25 - 2.00 (m, 4H), 1.60 (s, 2H), 1.04 (s, 6H). LCMS (ESI) calcd. for C₃₃H₃₉FN₅O₃⁺ [M+H]⁺: 572.30, found, 572.3.

### Example 234: Preparation of compound GT-10330

With reference to the method of Scheme 2, the target compound GT-10330 was obtained (8 mg, white solid, yield 22%). LCMS (ESI) calcd. for C₃₂H₃₄N₅O₃⁺ [M+H]⁺: 536.27, found, 536.3.

### Example 235: Preparation of compound GT-10331

With reference to the method of Scheme 2, the target compound GT-10331 was obtained (10 mg, white solid, yield 28%). ¹H NMR (400 MHz, MeOD) δ 7.74 (d, *J* = 6.6 Hz, 3H), 7.63 - 7.40 (m, 7H), 7.28 (s, 1H), 7.19 (s, 1H), 7.08 (d, *J* = 7.9 Hz, 1H), 5.64 (s, 1H), 5.08 (dd, *J* = 13.3, 4.8 Hz, 1H), 4.47 - 4.31 (m, 2H), 3.60 (d, *J* = 11.5 Hz, 2H), 3.51 (s, 2H), 3.00 - 2.82 (m, 2H), 2.77 (d, *J* = 15.7 Hz, 1H), 2.54 - 2.41 (m, 1H), 2.33 (brs, 2H), 2.25 (brs, 1H), 2.19 - 2.00 (m, 3H). LCMS (ESI) calcd. for C₃₂H₃₄N₅O₃⁺ [M+H]⁺: 536.27, found, 536.3.

### Example 236: Preparation of compound GT-10332

With reference to the method of Scheme 2, the target compound GT-10332 was obtained (14 mg, white solid, yield 29%).¹H NMR (400 MHz, MeOD) δ 7.69 (s, 4H), 7.62 - 7.38 (m, 8H), 5.50 (s, 1H), 5.05 (dd, *J* = 12.7, 5.4 Hz, 1H), 3.54 - 3.38 (m, 2H), 3.18 (brs, 4H), 2.93 - 2.77 (m, 2H), 2.74 - 2.63 (m, 3H), 2.13 - 2.02 (m, 1H). LCMS (ESI) calcd. for C₃₀H₂₉FN₅O₄⁺ [M+H]⁺: 542.22, found, 542.3.

### Example 237: Preparation of compound GT-10333

With reference to the method of Scheme 2, the target compound GT-10333 was obtained (12 mg, white solid, yield 25%). ¹H NMR (400 MHz, MeOD) δ 7.68 (s, 4H), 7.58 - 7.40 (m, 6H), 7.09 (s, 1H), 6.83 (s, 1H), 5.48 (s, 1H), 5.09 - 5.00 (m, 1H), 3.43 (dd, *J* = 20.6, 15.5 Hz, 3H), 3.27 - 3.14 (m, 2H), 3.10 - 2.98 (m, 2H), 2.86 - 2.69 (m, 4H), 2.13 - 1.96 (m, 1H). LCMS (ESI) calcd. for C₃₀H₂₉FN₅O₄⁺ [M+H]⁺: 542.22, found, 542.3.

### Example 238: Preparation of compound GT-10334

With reference to the method of Scheme 1, the target compound GT-10334 was obtained (25 mg, white solid, yield 66%). ¹H NMR (400 MHz, MeOD) δ 7.84 (s, 1H), 7.65 - 7.47 (m, 5H), 7.45 - 7.35 (m, 3H), 6.98 (s, 1H), 6.90 (d, *J* = 7.5 Hz, 1H), 5.07 (dd, *J* = 13.3, 4.7 Hz, 1H), 4.48 (s, 2H), 4.34 (q, *J* = 16.8 Hz, 2H), 3.41 (s, 2H), 3.21 (d, *J* = 9.9 Hz, 2H), 3.15 - 3.04 (m, 2H), 2.94 - 2.83 (m, 1H), 2.76 (d, *J* = 17.2 Hz, 1H), 2.44 (dd, *J* = 13.1, 4.3 Hz, 1H), 2.28 (brs, 2H), 2.16 - 2.10 (m, 1H), 2.02 - 1.97 (m, 2H). LCMS (ESI) calcd. for C₃₂H₃₃ClN₅O₃⁺ [M+H]⁺: 570.23, found, 570.2.

### Example 239: Preparation of compound GT-10335

With reference to the method of Scheme 3, the target compound GT-10335 was obtained (35 mg, white solid, yield 70%). ¹H NMR (400 MHz, MeOD) δ 7.77 (s, 1H), 7.61 - 7.52 (m, 4H), 7.48 (t, *J* = 7.8 Hz, 2H), 7.41 - 7.36 (m, 3H), 5.04 (dd, *J* = 12.7, 5.4 Hz, 1H), 4.44 (s, 2H), 3.48 - 3.37 (m, 2H), 3.20 - 2.95 (m, 6H), 2.82 (dd, *J* = 17.8, 4.3 Hz, 1H), 2.77 - 2.63 (m, 2H), 2.17 - 2.03 (m, 1H). LCMS (ESI) calcd. for C₃₀H₂₈ClFN₅O₄⁺ [M+H]⁺: 576.18, found, 576.2.

### Example 240: Preparation of compound GT-10336

With reference to the method of Scheme 1, the target compound GT-10336 was obtained (30 mg, white solid, yield 60%). ¹H NMR (400 MHz, MeOD) δ 7.77 (s, 1H), 7.64 - 7.50 (m, 4H), 7.40 - 7.35 (m, 3H), 7.03 (s, 1H), 6.79 (s, 1H), 5.03 (dd, *J* = 12.6, 5.2 Hz, 1H), 4.44 (s, 2H), 3.41 - 3.35 (m, 2H), 3.08 (s, 4H), 2.98 - 2.87 (m, 2H), 2.82 (dd, *J* = 18.3, 4.8 Hz, 1H), 2.77 - 2.62 (m, 2H), 2.16 - 1.98 (m, 1H). LCMS (ESI) calcd. for C₃₀H₂₈ClFN₅O₄⁺ [M+H]⁺: 576.18, found, 576.2.

### Example 241: Preparation of compound GT-10337

With reference to the method of Scheme 2, the target compound GT-10337 was obtained (8 mg, white solid, yield 21%). ¹H NMR (400 MHz, MeOD) δ 7.94 (s, 2H), 7.66 - 7.49 (m, 3H), 7.43 - 7.24 (m, 4H), 7.02 (s, 1H), 6.95 (d, *J* = 5.1 Hz, 1H), 5.97 (s, 1H), 5.07 (dd, *J* = 13.5, 4.9 Hz, 1H), 4.36 (q, *J* = 16.6 Hz, 2H), 4.09 (brs, 1H), 3.21 - 3.18 (m, 4H), 3.17 - 3.09 (m, 1H), 2.94 - 2.83 (m, 2H), 2.76 (d, *J* = 15.0 Hz, 1H), 2.61 - 2.39 (m, 4H), 2.19 - 2.09 (m, 1H). LCMS (ESI) calcd. for C₃₂H₃₂F₂N₅O₃⁺ [M+H]⁺: 572.25, found, 572.3.

### Example 242: Preparation of compound GT-10339

With reference to the method of Scheme 2, the target compound GT-10339 was obtained (8 mg, white solid, yield 21%). ¹H NMR (400 MHz, MeOD) δ 7.77 - 7.43 (m, 7H), 7.24 (brs, 2H), 7.02 (s, 1H), 6.96 (d, *J* = 8.5 Hz, 1H), 5.51 (s, 1H), 5.07 (dd, *J* = 13.6, 5.2 Hz, 1H), 4.46 - 4.29 (m, 2H), 3.97 (brs, 1H), 3.29 - 3.17 (m, 4H), 3.14 (brs, 1H), 2.92 - 2.79 (m, 2H), 2.76 (d, *J* = 18.7 Hz, 1H), 2.64 - 2.35 (m, 4H), 2.20 - 2.04 (m, 1H). LCMS (ESI) calcd. for C₃₂H₃₂F₂N₅O₃⁺ [M+H]⁺: 572.25, found, 572.3.

### Example 243: Preparation of compound GT-10340

With reference to the method of Scheme 2, the target compound GT-10340 was obtained (10 mg, white solid, yield 25%). ¹H NMR (400 MHz, DMSO-d6) δ 7.72 (s, 3H), 7.65 - 7.37 (m, 6H), 7.01 (s, 1H), 6.95 (d, *J* = 7.2 Hz, 1H), 5.47 (s, 1H), 5.07 (dd, *J* = 13.5, 4.8 Hz, 1H), 4.36 (q, *J* = 16.6 Hz, 2H), 3.95 (s, 1H), 3.23 - 3.05 (m, 3H), 2.95 - 2.69 (m, 3H), 2.57 - 2.29 (m, 4H), 2.19 - 1.89 (m, 2H), 1.30 - 1.24 (m, 1H). LCMS (ESI) calcd. for C₃₂H₃₂Cl₂N₅O₃⁺ [M+H]⁺: 604.19, found, 604.2.

### Example 244: Preparation of compound GT-10430

With reference to the method of Scheme 2, the target compound GT-10430 was obtained (25 mg, white solid, yield 70%). ¹H NMR (400 MHz, DMSO-d6) δ 8.79 (s, 1H), 7.88 - 7.81 (m, 1H), 7.72 (s, 2H), 7.56 (d, *J* = 8.2 Hz, 1H), 7.52 - 7.45 (m, 4H), 7.18 (s, 1H), 7.03 (s, 1H), 6.97 (d, *J* = 7.9 Hz, 1H), 5.68 (s, 1H), 5.07 (dd, *J* = 13.3, 5.0 Hz, 1H), 4.36 (q, *J* = 17.2 Hz, 2H), 3.99 (brs, 1H), 3.67 (brs, 1H), 3.27 - 3.02 (m, 3H), 2.94 - 2.85 (m, 2H), 2.46 (brs, 5H), 2.35 - 2.27 (m, 1H), 2.21 - 2.03 (m, 1H). LCMS (ESI) calcd. for C₃₁H₃₃N₆O₃⁺ [M+H]⁺: 537.26, found, 537.3.

### Example 245: Preparation of compound GT-10431

With reference to the method of Scheme 2, the target compound GT-10431 was obtained (12 mg, white solid, yield 34%). ¹H NMR (400 MHz, DMSO-d6) δ 9.58 (s, 1H), 9.32 (d, *J* = 7.6 Hz, 1H), 8.91 (d, *J* = 5.3 Hz, 1H), 8.24 - 8.13 (m, 1H), 8.06 (s, 2H), 7.57 - 7.49 (m, 4H), 7.01 (s, 1H), 6.95 (d, *J* = 7.8 Hz, 1H), 5.83 (s, 1H), 5.07 (dd, *J* = 13.0, 4.9 Hz, 1H), 4.46 - 4.27 (m, 2H), 4.06 (brs, 1H), 3.94 (brs, 1H), 3.72 (brs, 1H), 3.60 (brs, 1H), 3.14 - 3.00 (m, 2H), 2.97 - 2.82 (m, 1H), 2.76 (d, *J* = 17.7 Hz, 1H), 2.68 - 2.65 (m, 1H), 2.51 - 2.42 (m, 4H), 2.22 - 2.05 (m, 1H). LCMS (ESI) calcd. for C₃₁H₃₃N₆O₃⁺ [M+H]⁺: 537.26, found, 537.3.

### Example 246: Preparation of compound GT-10459

With reference to the method of Scheme 2, the target compound GT-10459 was obtained (8 mg, white solid, yield 22%). ¹H NMR (400 MHz, DMSO-d6) δ 7.91 - 7.83 (m, 1H), 7.79 - 7.70 (m, 2H), 7.70 - 7.62 (m, 2H), 7.60 - 7.41 (m, 8H), 5.82 (s, 1H), 5.15 - 5.02 (m, 1H), 4.46 - 4.29 (m, 2H), 4.21 - 4.13 (m, 1H), 3.85 - 2.79 (m, 1H), 3.73 - 3.49 (m, 3H), 3.23 - 3.15 (m, 1H), 3.08 - 2.98 (m, 1H), 2.92 - 2.85 (m, 1H), 2.82 - 2.71 (m, 1H), 2.54 - 2.31 (m, 2H), 2.21 - 2.06 (m, 1H). LCMS (ESI) calcd. for C₃₁H₃₂N₅O₃⁺ [M+H]⁺: 522.25, found, 522.3.

### Example 247: Preparation of compound GT-10460

With reference to the method of Scheme 2, the target compound GT-10460 was obtained (8 mg, white solid, yield 21%). LCMS (ESI) calcd. for C₃₁H₃₀F₂N₅O₃⁺ [M+H]⁺: 558.23, found, 558.3.

### Example 248: Preparation of compound GT-10461

With reference to the method of Scheme 2, the target compound GT-10461 was obtained (14 mg, white solid, yield 36%). ¹H NMR (400 MHz, DMSO-d6) δ 7.70 - 7.38 (m, 7H), 7.30 - 7.15 (m, 2H), 7.05 (s, 1H), 6.97 (s, 1H), 5.61 (s, 1H), 5.17 - 5.02 (m, 1H), 4.50 - 4.31 (m, 2H), 4.28 - 4.14 (m, 1H), 3.92 - 3.80 (m, 2H), 3.69 - 3.52 (m, 1H), 3.21 - 3.07 (m, 1H), 2.91 - 2.85 (m, 2H), 2.65 - 2.41 (m, 3H), 2.22 - 2.05 (m, 1H), 1.37 - 1.24 (m, 1H). LCMS (ESI) calcd. for C₃₁H₃₀F₂N₅O₃⁺ [M+H]⁺: 558.23, found, 558.3.

### Example 249: Preparation of compound GT-10462

With reference to the method of Scheme 2, the target compound GT-10462 was obtained (8 mg, white solid, yield 21%). ¹H NMR (400 MHz, DMSO-d6) δ 7.87 - 7.68 (m, 3H), 7.59 - 7.52 (m, 1H), 7.41 - 7.31 (m, 1H), 7.31 - 7.17 (m, 4H), 7.10 - 6.95 (m, 2H), 5.16 - 5.03 (m, 1H), 4.47 - 4.37 (m, 2H), 4.17 (d, *J* = 23.0 Hz, 1H), 3.84 - 3.77 (m, 2H), 3.63 - 3.54 (m, 1H), 3.21 - 3.14 (m, 2H), 2.98 - 2.82 (m, 1H), 2.76 (d, *J* = 17.0 Hz, 1H), 2.45 - 2.36 (m, 2H), 2.21 - 2.06 (m, 1H), 1.32 - 1.29 (m, 1H), 1.27 - 1.23 (m, 1H). LCMS (ESI) calcd. for C₃₁H₃₀F₂N₅O₃⁺ [M+H]⁺: 558.23, found, 558.3.

### Example 250: Preparation of compound GT-10463

With reference to the method of Scheme 2, the target compound GT-10463 was obtained (12 mg, white solid, yield 30%). ¹H NMR (400 MHz, DMSO-d6) δ 7.88 - 7.62 (m, 4H), 7.60 - 7.42 (m, 5H), 7.37 - 7.28 (m, 1H), 7.02 - 6.89 (m, 1H), 5.16 - 5.00 (m, 1H), 4.52 - 4.29 (m, 2H), 4.27 - 4.11 (m, 1H), 3.92 - 3.69 (m, 2H), 3.64 - 3.59 (m, 1H), 3.25 - 3.06 (m, 1H), 2.98 - 2.70 (m, 3H), 2.67 - 2.51 (m, 1H), 2.44 - 2.33 (m, 2H), 2.23 - 2.05 (m, 1H), 1.38 - 1.20 (m, 1H). LCMS (ESI) calcd. for C₃₁H₃₀Cl₂N₅O₃⁺ [M+H]⁺: 590.17, found, 590.2.

### Example 251: Preparation of compound GT-10464

With reference to the method of Scheme 2, the target compound GT-10464 was obtained (20 mg, white solid, yield 56%). ¹H NMR (400 MHz, DMSO-d6) δ 8.77 (d, *J* = 16.8 Hz, 1H), 8.00 - 7.86 (m, 1H), 7.68 (dd, *J* = 20.1, 4.4 Hz, 2H), 7.62 - 7.52 (m, 2H), 7.54 - 7.37 (m, 4H), 7.21 - 7.12 (m, 1H), 7.02 (t, *J* = 9.1 Hz, 1H), 5.83 (s, 1H), 5.07 (d, *J* = 11.9 Hz, 1H), 4.49 - 4.28 (m, 2H), 4.02 - 3.81 (m, 3H), 3.43 - 4.39 (m, 1H), 3.18 (d, *J* = 12.8 Hz, 1H), 3.12 (brs, 1H), 2.98 - 2.82 (m, 1H), 2.76 (d, *J* = 16.2 Hz, 1H), 2.55 - 2.39 (m, 3H), 2.14 (brs, 1H). LCMS (ESI) calcd. for C₃₀H₃₁N₆O₃⁺ [M+H]⁺: 523.25, found, 523.3.

### Example 252: Preparation of compound GT-10465

With reference to the method of Scheme 2, the target compound GT-10465 was obtained (30 mg, white solid, yield 65%). ¹H NMR (400 MHz, DMSO-d6) δ 9.92 (s, 1H), 9.32 - 9.08 (m, 2H), 9.00 (s, 2H), 8.69 - 8.53 (m, 1H), 8.32 - 8.25 (m, 1H), 8.17 - 8.11 (m, 1H), 7.99 - 7.95 (m, 1H), 7.84 (s, 1H), 7.65 - 7.53 (m, 4H), 7.50 - 7.42 (m, 5H), 7.27 - 6.93 (m, 2H), 6.21 (s, 1H), 5.16 - 5.04 (m, 1H), 4.50 - 4.30 (m, 2H), 4.31 - 4.01 (m, 2H), 4.03 - 3.69 (m, 2H), 3.42 - 3.38 (m, 2H), 3.22 - 3.12 (m, 1H), 2.97 - 2.83 (m, 1H), 2.78 - 2.74 (m, 1H), 2.58 (s, 1H), 2.48 - 2.36 (m, 2H), 2.18 - 2.06 (m, 1H). LCMS (ESI) calcd. for C₄₂H₄₀N₇O₃⁺ [M+H]⁺: 690.32, found, 690.3.

### Example 253: Preparation of compound GT-10475

With reference to the method of Scheme 2, the target compound GT-10475 was obtained (6 mg, white solid, yield 17%). ¹H NMR (400 MHz, MeOD) δ 9.50 (s, 1H), 9.26 (d, *J* = 8.2 Hz, 1H), 8.93 (d, *J* = 5.6 Hz, 1H), 8.27 - 8.14 (m, 1H), 7.94 (d, *J* = 7.3 Hz, 2H), 7.63 (d, *J* = 8.2 Hz, 1H), 7.58 - 7.45 (m, 3H), 7.33 (s, 1H), 7.12 (s, 1H), 6.25 (s, 1H), 5.10 (dd, *J* = 13.3, 4.9 Hz, 2H), 4.60 - 4.35 (m, 2H), 3.66 (s, 4H), 3.39 (s, 4H), 3.02 - 2.84 (m, 2H), 2.77 (d, *J* = 15.8 Hz, 1H), 2.61 - 2.39 (m, 1H), 2.25 - 2.00 (m, 1H). LCMS (ESI) calcd. for C₃₁H₃₃N₆O₃⁺ [M+H]⁺: 537.26, found, 537.3.

### Example 254: Preparation of compound GT-10476

With reference to the method of Scheme 2, the target compound GT-10476 was obtained (12 mg, white solid, yield 26%). ¹H NMR (400 MHz, MeOD) δ 9.88 (s, 1H), 9.24 (d, *J* = 28.3 Hz, 1H), 9.16 - 8.90 (m, 3H), 8.83 - 8.70 (m, 1H), 8.27 (d, *J* = 21.9 Hz, 2H), 8.11 - 8.02 (m, 1H), 7.84 (s, 2H), 7.64 (s, 1H), 7.56 - 7.40 (m, 8H), 7.13 (s, 1H), 6.39 (s, 1H), 5.11 (d, *J* = 10.6 Hz, 1H), 4.58 - 4.36 (m, 2H), 3.87 - 3.58 (m, 2H), 3.53 - 3.38 (m, 4H), 3.30 - 3.08 (m, 5H), 2.95 - 2.84 (m, 1H), 2.77 (d, *J* = 16.0 Hz, 1H), 2.53 - 2.34 (m, 1H), 2.16 (brs, 1H). LCMS (ESI) calcd. for C₄₃H₄₂N₇O₃⁺ [M+H]⁺: 704.33, found, 704.4.

### Example 255: Preparation of compound GT-10477

With reference to the method of Scheme 2, the target compound GT-10477 was obtained (5 mg, white solid, yield 14%). LCMS (ESI) calcd. for C₃₁H₃₃N₆O₃⁺ [M+H]⁺: 537.26, found, 537.3.

### Example 256: Preparation of compound GT-10478

With reference to the method of Scheme 2, the target compound GT-10478 was obtained (16 mg, white solid, yield 45%). ¹H NMR (400 MHz, MeOD) δ 8.90 - 8.62 (m, 1H), 7.94 - 7.76 (m, 1H), 7.55 (brs, 3H), 7.45 (s, 4H), 7.35 (s, 1H), 7.00 (s, 1H), 6.91 (s, 1H), 5.90 (d, *J* = 6.7 Hz, 1H), 5.12 - 5.06 (m, 1H), 4.60 (s, 1H), 4.34 (brs, 2H), 3.70 (s, 1H), 3.55 - 3.48 (m, 1H), 3.21 - 2.70 (m, 5H), 2.47 (brs, 2H), 2.14 (brs, 2H). LCMS (ESI) calcd. for C₃₀H₃₁N₆O₃⁺ [M+H]⁺: 523.25, found, 523.3.

### Example 257: Preparation of compound GT-10479

With reference to the method of Scheme 2, the target compound GT-10479 was obtained (4 mg, white solid, yield 11%). LCMS (ESI) calcd. for C₃₀H₃₁N₆O₃⁺ [M+H]⁺: 523.25, found, 523.3.

### Example 258: Preparation of compound GT-10480

With reference to the method of Scheme 2, the target compound GT-10480 was obtained (10 mg, white solid, yield 22%). ¹H NMR (400 MHz, DMSO-d6) δ 9.19 - 9.02 (m, 1H), 9.00 - 8.85 (m, 2H), 8.77 (dd, *J* = 27.0, 4.9 Hz, 1H), 8.45 - 8.30 (m, 1H), 8.29 - 8.16 (m, 1H), 7.99 - 7.95 (m, 1H), 7.91 - 7.79 (m, 1H), 7.77 - 7.63 (m, 2H), 7.60 - 7.38 (m, 10H), 6.96 - 6.81 (m, 1H), 6.07 (s, 1H), 5.15 - 5.10 (m, 1H), 4.66 (brs, 1H), 4.42 - 4.19 (m, 2H), 3.62 (s, 1H), 3.44 - 3.33 (m, 3H), 3.11 - 2.84 (m, 3H), 2.81 - 2.74 (m, 1H), 2.50 - 2.32 (m, 2H), 2.25 - 1.98 (m, 2H). LCMS (ESI) calcd. for C₄₂H₄₀N₇O₃⁺ [M+H]⁺: 690.32, found, 690.4.

### Example 259: Preparation of compound GT-10518

With reference to the method of Scheme 2, the target compound GT-10518 was obtained (14 mg, white solid, yield 39%). ¹H NMR (400 MHz, MeOD) δ 8.73 (d, *J* = 4.4 Hz, 2H), 7.94 (t, *J* = 7.1 Hz, 2H), 7.74 (d, *J* = 7.8 Hz, 2H), 7.57 (d, *J* = 8.3 Hz, 1H), 7.49 (dd, *J* = 7.0, 5.2 Hz, 2H), 7.04 (s, 1H), 6.97 (d, *J* = 8.0 Hz, 1H), 6.08 (s, 1H), 5.07 (dd, *J* = 13.3, 4.9 Hz, 2H), 4.37 (q, *J* = 16.8 Hz, 2H), 3.89 (s, 2H), 3.43 - 3.35 (m, 2H), 3.13 (d, *J* = 11.0 Hz, 2H), 2.96 - 2.82 (m, 1H), 2.76 (d, *J* = 17.5 Hz, 1H), 2.51 - 2.43 (m, 4H), 2.22 - 2.04 (m, 1H). LCMS (ESI) calcd. for C₃₀H₃₂N₇O₃⁺ [M+H]⁺: 538.26, found, 538.3.

### Example 260: Preparation of compound GT-10519

With reference to the method of Scheme 2, the target compound GT-10519 was obtained (10 mg, white solid, yield 22%). ¹H NMR (400 MHz, MeOD) δ 8.67 (s, 2H), 7.88 (s, 2H), 7.63 - 7.49 (m, 3H), 7.45 (s, 2H), 7.00 (br, s), 6.92 (s, 1H), 6.06 (s, 1H), 5.11 - 5.05 (m, 2H), 4.60 (s, 1H), 4.41 - 4.26 (m, 2H), 3.74 (s, 1H), 3.05 (s, 2H), 2.88 (s, 2H), 2.79 - 2.75 (m, 1H), 2.52 - 2.46 (m, 2H), 2.24 (brs, 1H), 2.14 (brs, 1H). LCMS (ESI) calcd. for C₄₂H₄₀N₇O₃⁺ [M+H]⁺: 690.32, found, 690.4.

### Example 261: Preparation of compound GT-10520

With reference to the method of Scheme 2, the target compound GT-10520 was obtained (10 mg, white solid, yield 22%). ¹H NMR (400 MHz, MeOD) δ 8.74 (s, 2H), 8.08 (t, *J* = 7.0 Hz, 2H), 7.87 (dd, *J* = 23.9, 7.8 Hz, 2H), 7.65 - 7.55 (m, 3H), 7.22 (s, 1H), 7.09 (d, *J* = 7.6 Hz, 1H), 6.13 (s, 1H), 5.23 - 5.05 (m, 4H), 4.45 - 4.34 (m, 2H), 4.05 (d, *J* = 12.7 Hz, 2H), 3.61 (s, 1H), 2.99 - 2.83 (m, 1H), 2.76 (d, *J* = 16.8 Hz, 1H), 2.52 - 2.42(m, 3H), 2.15 (brs, 1H). LCMS (ESI) calcd. for C₄₂H₄₀N₇O₃⁺ [M+H]⁺: 690.32, found, 690.4.

### Example 262: Preparation of compound GT-10521

With reference to the method of Scheme 2, the target compound GT-10521 was obtained (10 mg, white solid, yield 22%). ¹H NMR (400 MHz, MeOD) δ 9.18 (s, 1H), 8.85 - 8.70 (m, 2H), 7.93 (s, 1H), 7.84 - 7.71 (m, 2H), 7.59 - 7.46 (m, 4H), 7.11 (s, 1H), 7.01 (s, 1H), 5.11 - 5.03 (m, 1H), 4.45 - 4.31 (m, 2H), 3.98 - 3.83 (m, 2H), 3.25 - 3.21 (m, 4H), 2.95 - 2.83 (m, 1H), 2.80 - 2.74 (m, 1H), 2.57 - 2.34 (m, 3H), 2.19 - 2.11 (m, 1H). LCMS (ESI) calcd. for C₄₂H₄₀N₇O₃⁺ [M+H]⁺: 690.32, found, 690.4.

### Example 263: Preparation of compound GT-10545

With reference to the method of Scheme 2, the target compound GT-10545 was obtained (10 mg, white solid, yield 22%). ¹H NMR (400 MHz, MeOD) δ 7.97 - 7.83 (m, 1H), 7.55 (d, *J* = 8.2 Hz, 1H), 7.52 - 7.45 (m, 2H), 7.40 - 7.33 (m, 2H), 7.29 - 7.24 (m, 2H), 7.07 (s, 1H), 6.94 (d, *J* = 8.4 Hz, 1H), 5.07 (dd, *J* = 13.2, 4.9 Hz, 1H), 4.51 - 4.31 (m, 2H), 3.76 - 3.70 (m, 1H), 3.59 (brs, 3H), 3.40 - 3.35(m, 2H), 3.11 (s, 2H), 2.95 - 2.82 (m, 1H), 2.76 (d, *J* = 15.4 Hz, 1H), 2.55 - 2.41 (m, 1H), 2.22 (s, 2H), 2.18 - 2.07 (m, 1H). LCMS (ESI) calcd. for C₄₂H₄₀N₇O₃⁺ [M+H]⁺: 690.32, found, 690.4.

### Example 264: Preparation of compound GT-10546

With reference to the method of Scheme 2, the target compound GT-10546 was obtained (10 mg, white solid, yield 22%). ¹H NMR (400 MHz, MeOD) δ 7.63 - 7.48 (m, 6H), 7.27 - 7.13 (m, 3H), 7.02 (s, 1H), 6.93 (dd, *J* = 9.9, 4.7 Hz, 1H), 5.75 (s, 1H), 5.13 - 5.02 (m, 1H), 4.51 - 4.39 (m, 1H), 4.35 (d, *J* = 6.9 Hz, 1H), 3.62 - 3.44 (m, 3H), 3.45 - 3.35 (m, 3H), 3.18 - 2.98 (m, 2H), 2.97 - 2.82 (m, 1H), 2.80 - 2.74 (m, 1H), 2.54 - 2.38 (m, 1H), 2.28 - 2.06 (m, 3H). LCMS (ESI) calcd. for C₄₂H₄₀N₇O₃⁺ [M+H]⁺: 690.32, found, 690.4.

### Example 265: Preparation of compound GT-10547

With reference to the method of Scheme 2, the target compound GT-10547 was obtained (10 mg, white solid, yield 22%). ¹H NMR (400 MHz, MeOD) δ 7.98 - 7.84 (m, 1H), 7.82 - 7.66 (m, 2H), 7.54 (d, *J* = 9.6 Hz, 1H), 7.39 - 7.33 (m, 1H), 7.31 - 7.16 (m, 3H), 7.12 - 6.98 (m, 2H), 6.97 - 6.88 (m, 1H), 5.72 (s, 1H), 4.43 - 4.27 (m, 1H), 3.70 - 3.55 (m, 2H), 3.55 - 3.46 (m, 4H), 3.26 - 3.15 (m, 1H), 3.15 - 2.97 (m, 1H), 2.98 - 2.83 (m, 1H), 2.83 - 2.71 (m, 1H), 2.52 - 2.36 (m, 1H), 2.30 - 2.05 (m, 2H), 1.37 - 1.25 (m, 3H). LCMS (ESI) calcd. for C₄₂H₄₀N₇O₃⁺ [M+H]⁺: 690.32, found, 690.4.

### Example 266: Preparation of compound GT-10548

With reference to the method of Scheme 2, the target compound GT-10548 was obtained (10 mg, white solid, yield 22%). ¹H NMR (400 MHz, D MeOD) δ 7.69 (d, *J* = 4.6 Hz, 4H), 7.52 (brs, 5H), 7.01 (s, 1H), 6.98 - 6.88 (m, 1H), 5.72 (s, 1H), 5.08 (dd, *J* = 13.6, 7.9 Hz, 1H), 4.47 - 4.29 (m, 2H), 3.66 - 3.60 (m, 1H), 3.58 - 3.40 (m, 4H), 3.15 - 3.03 (m, 2H), 2.99 - 2.70 (m, 3H), 2.55 - 2.37 (m, 1H), 2.25 - 2.15 (m, 3H). LCMS (ESI) calcd. for C₄₂H₄₀N₇O₃⁺ [M+H]⁺: 690.32, found, 690.4.

### Example 267: Preparation of compound GT-10563

With reference to the method of Scheme 2, the target compound GT-10563 was obtained (10 mg, white solid, yield 22%). ¹H NMR (400 MHz, MeOD) δ 7.75 - 7.58 (m, 2H), 7.54 - 7.44 (m, 3H), 7.38 - 7.27 (m, 4H), 7.07 - 6.99 (m, 1H), 6.94 - 6.76 (m, 1H), 5.21 - 5.00 (m, 1H), 4.49 - 4.22 (m, 2H), 3.77 - 3.71 (m, 1H), 3.26 - 3.17 (m, 3H), 3.09 - 2.97 (m, 2H), 2.96 - 2.84 (m, 2H), 2.79 - 2.74 (m, 1H), 2.48 - 2.41 (m, 2H), 2.25 - 2.01 (m, 2H). LCMS (ESI) calcd. for C₄₂H₄₀N₇O₃⁺ [M+H]⁺: 690.32, found, 690.4.

### Example 268: Preparation of compound GT-10564

With reference to the method of Scheme 2, the target compound GT-10564 was obtained (10 mg, white solid, yield 22%). ¹H NMR (400 MHz, MeOD) δ 7.67 - 7.47 (m, 3H), 7.44 - 7.28 (m, 3H), 7.26 - 7.14 (m, 2H), 7.12 - 6.74 (m, 3H), 5.19 - 5.02 (m, 1H), 4.53 - 4.16 (m, 3H), 3.82 - 3.50 (m, 2H), 3.41 - 3.38 (m, 2H), 3.15 - 3.12 (m, 1H), 2.98 - 2.64 (m, 3H), 2.54 - 3.44 (m, 2H), 2.27 - 1.89 (m, 2H). LCMS (ESI) calcd. for C₄₂H₄₀N₇O₃⁺ [M+H]⁺: 690.32, found, 690.4.

### Example 269: Preparation of compound GT-10565

With reference to the method of Scheme 2, the target compound GT-10565 was obtained (10 mg, white solid, yield 22%). ¹H NMR (400 MHz, MeOD) δ 7.70 - 7.62 (m, 1H), 7.56 - 7.48 (m, 2H), 7.39 - 7.30 (m, 2H), 7.28 - 7.24 (m, 2H), 7.21 - 7.10 (m, 2H), 7.07 - 7.01 (m, 2H), 5.08 (dd, *J* = 13.7, 5.6 Hz, 1H), 4.58 - 4.53 (m, 1H), 4.37 (d, *J* = 6.9 Hz, 1H), 4.34 - 4.23 (m, 2H), 3.65 - 3.52 (m, 3H), 2.97 - 2.81 (m, 2H), 2.80 - 2.72 (m, 2H), 2.52 - 2.30 (m, 2H), 2.16 - 2.09 (m, 2H). LCMS (ESI) calcd. for C₄₂H₄₀N₇O₃⁺ [M+H]⁺: 690.32, found, 690.4.

### Example 270: Preparation of compound GT-10566

With reference to the method of Scheme 2, the target compound GT-10566 was obtained (10 mg, white solid, yield 22%). ¹H NMR (400 MHz, MeOD) δ 7.67 - 7.61 (m, 2H), 7.56 - 7.44 (m, 8H), 6.99 - 6.94 (m, 1H), 5.18 - 5.04 (m, 1H), 4.46 - 4.15 (m, 4H), 3.74 - 3.64 (m, 3H), 2.88 - 2.81 (m, 2H), 2.82 - 2.71 (m, 2H), 2.55 - 2.30 (m, 2H), 2.22 - 2.04 (m, 2H). LCMS (ESI) calcd. for C₄₂H₄₀N₇O₃⁺ [M+H]⁺: 690.32, found, 690.4.

### Example 271: Preparation of compound GT-10568

With reference to the method of Scheme 2, the target compound GT-10568 was obtained (10 mg, white solid, yield 22%). ¹H NMR (400 MHz, MeOD) δ 7.67 (d, *J* = 6.3 Hz, 4H), 7.61 - 7.31 (m, 7H), 7.01 (s, 1H), 6.93 (d, *J* = 5.4 Hz, 1H), 5.67 (s, 1H), 5.07 (dd, *J* = 13.0, 5.1 Hz, 1H), 4.46 - 4.26 (m, 2H), 3.60 - 3.42 (m, 4H), 3.25 - 3.22 (m, 2H), 3.12 - 3.03 (m, 2H), 2.92 - 2.85 (m, 1H), 2.76 (d, *J* = 15.6 Hz, 1H), 2.54 - 2.43 (m, 1H), 2.33 - 2.17 (m, 1H), 2.17 - 2.01 (m, 2H). LCMS (ESI) calcd. for C₄₂H₄₀N₇O₃⁺ [M+H]⁺: 690.32, found, 690.4.

### Example 272: Preparation of compound GT-10569

With reference to the method of Scheme 2, the target compound GT-10569 was obtained (10 mg, white solid, yield 22%). ¹H NMR (400 MHz, MeOD) δ 8.77 (d, *J* = 3.2 Hz, 1H), 7.90 (td, *J* = 7.8, 1.6 Hz, 1H), 7.70 - 7.61 (m, 2H), 7.57 (d, *J* = 8.4 Hz, 1H), 7.51 (d, *J* = 8.1 Hz, 1H), 7.49 - 7.42 (m, 4H), 7.11 (s, 1H), 6.99 (d, *J* = 8.4 Hz, 1H), 5.90 (s, 1H), 5.07 (dd, *J* = 13.2, 5.1 Hz, 1H), 4.37 (q, *J* = 17.2 Hz, 2H), 3.55 - 3.50 (m, 1H), 3.47 - 3.33 (m, 5H), 3.17 (s, 2H), 2.86 (dd, *J* = 13.3, 5.0 Hz, 1H), 2.76 (d, *J* = 15.5 Hz, 1H), 2.45 (dd, *J* = 13.1, 4.5 Hz, 1H), 2.18 - 2.11 (m, 3H). LCMS (ESI) calcd. for C₄₂H₄₀N₇O₃⁺ [M+H]⁺: 690.32, found, 690.4.

### Example 273: Preparation of compound GT-10645

With reference to the method of Scheme 2, the target compound GT-10645 was obtained (10 mg, white solid, yield 22%). ¹H NMR (400 MHz, MeOD) δ 9.48 (s, 1H), 9.22 (d, *J* = 8.2 Hz, 1H), 8.90 (d, *J* = 5.5 Hz, 1H), 8.18 (dd, *J* = 8.1, 5.9 Hz, 1H), 7.91 (d, *J* = 7.5 Hz, 2H), 7.54 (dd, *J* = 14.4, 8.0 Hz, 3H), 7.48 (d, *J* = 7.2 Hz, 1H), 7.09 (s, 1H), 6.97 (d, *J* = 8.3 Hz, 1H), 6.14 (s, 1H), 5.08 (dd, *J* = 13.3, 5.1 Hz, 1H), 4.37 (q, *J* = 16.9 Hz, 2H), 3.54 (s, 4H), 3.47 - 3.35 (m, 2H), 3.23 - 3.08 (m, 2H), 2.91 - 2.82 (m, 1H), 2.77 (d, *J* = 15.7 Hz, 1H), 2.45 (qd, *J* = 13.4, 4.8 Hz, 1H), 2.27 (brs, 2H), 2.19 - 2.06 (m, 1H). LCMS (ESI) calcd. for C₄₂H₄₀N₇O₃⁺ [M+H]⁺: 690.32, found, 690.4.

### Example 274: Preparation of compound GT-10646

With reference to the method of Scheme 2, the target compound GT-10646 was obtained (10 mg, white solid, yield 22%). ¹H NMR (400 MHz, MeOD) δ 10.00 (d, *J* = 14.2 Hz, 1H), 9.31 - 9.10 (m, 2H), 9.09 - 8.94 (m, 2H), 8.81 - 8.63 (m, 1H), 8.35 - 8.18 (m, 2H), 7.98 (d, *J* = 8.8 Hz, 1H), 7.83 (t, *J* = 5.3 Hz, 2H), 7.56 (dd, *J* = 7.7, 3.3 Hz, 3H), 7.54 - 7.43 (m, 6H), 7.12 (s, 1H), 6.97 (d, *J* = 7.8 Hz, 1H), 6.29 (d, *J* = 8.9 Hz, 1H), 5.08 (dd, *J* = 13.3, 5.1 Hz, 1H), 4.50 - 4.26 (m, 2H), 3.49 (s, 4H), 3.36 (s, 2H), 3.24 - 3.03 (m, 2H), 2.87 (dd, *J* = 13.2, 5.1 Hz, 1H), 2.82 - 2.71 (m, 1H), 2.51 - 2.40 (m, 1H), 2.24 (brs, 2H), 2.18 - 2.07 (m, 1H). LCMS (ESI) calcd. for C₄₂H₄₀N₇O₃⁺ [M+H]⁺: 690.32, found, 690.4.

### Example 275: Preparation of compound GT-10647

With reference to the method of Scheme 2, the target compound GT-10647 was obtained (10 mg, white solid, yield 22%). ¹H NMR (400 MHz, MeOD) δ 8.71 (d, *J* = 4.7 Hz, 2H), 7.92 (td, *J* = 7.8, 1.7 Hz, 2H), 7.64 (d, *J* = 7.8 Hz, 2H), 7.58 (d, *J* = 8.4 Hz, 1H), 7.47 (dd, *J* = 7.2, 5.3 Hz, 2H), 7.09 (s, 1H), 6.98 (dd, *J* = 8.4, 1.6 Hz, 1H), 6.08 (s, 1H), 5.07 (dd, *J* = 13.3, 5.1 Hz, 1H), 4.84 - 4.77 (m, 2H), 4.38 (q, *J* = 16.8 Hz, 2H), 3.60 - 3.46 (m, 4H), 3.14 (s, 2H), 2.91 - 2.84 (m, 1H), 2.83 - 2.71 (m, 1H), 2.46 (qd, *J* = 13.1, 4.7 Hz, 1H), 2.22 - 2.09 (m, 3H). LCMS (ESI) calcd. for C₄₂H₄₀N₇O₃⁺ [M+H]⁺: 690.32, found, 690.4.

### Example 276: Preparation of compound GT-10648

With reference to the method of Scheme 2, the target compound GT-10648 was obtained (10 mg, white solid, yield 22%). ¹H NMR (400 MHz, MeOD) δ 7.87 - 7.85 (m, 1H), 7.64 - 7.60 (m, 3H), 7.54 - 7.41 (m, 8H), 6.98 - 6.96 (m, 1H), 5.09 (dd, *J* = 13.3, 5.0 Hz, 1H), 4.78 - 4.70 (m, 2H), 4.48 - 4.25 (m, 4H), 3.76 (d, *J* = 12.3 Hz, 1H), 3.62 - 3.51 (m, 1H), 3.28 - 3.23 (m, 2H), 2.92 - 2.82 (m, 1H), 2.82 - 2.74 (m, 2H), 2.50 - 2.38 (m, 1H), 2.19 - 2.07 (m, 1H). LCMS (ESI) calcd. for C₄₂H₄₀N₇O₃⁺ [M+H]⁺: 690.32, found, 690.4.

### Example 277: Preparation of compound GT-10649

With reference to the method of Scheme 2, the target compound GT-10649 was obtained (10 mg, white solid, yield 22%). ¹H NMR (400 MHz, MeOD) δ 7.90 - 7.82 (m, 1H), 7.69 - 7.64 (m, 4H), 7.55 - 7.48 (m, 5H), 7.43 (d, *J* = 8.4 Hz, 1H), 7.05 (s, 1H), 7.03 (d, *J* = 8.4 Hz, 1H), 5.10 (dd, *J* = 13.3, 5.0 Hz, 1H), 4.80 - 4.72 (m, 2H), 4.47 - 4.33 (m, 2H), 3.68 - 3.64 (m, 2H), 3.11 - 3.00 (m, 5H), 2.89 (dd, *J* = 13.2, 5.2 Hz, 1H), 2.78 (dd, *J* = 19.0, 3.1 Hz, 1H), 2.67 - 2.63 (m, 2H), 2.50 - 2.42 (m, 1H), 2.21 - 2.10 (m, 1H). LCMS (ESI) calcd. for C₄₂H₄₀N₇O₃⁺ [M+H]⁺: 690.32, found, 690.4.

### Example 278: Preparation of compound GT-10650

With reference to the method of Scheme 2, the target compound GT-10650 was obtained (10 mg, white solid, yield 22%). ¹H NMR (400 MHz, MeOD) δ 7.74 (t, *J* = 7.4 Hz, 2H), 7.58 (d, *J* = 8.4 Hz, 1H), 7.55 - 7.51 (m, 2H), 7.36 (t, *J* = 7.6 Hz, 2H), 7.34 - 7.27 (m, 2H), 7.08 (s, 1H), 7.01 (d, *J* = 7.7 Hz, 1H), 5.10 (dd, *J* = 13.3, 5.2 Hz, 1H), 4.45 - 4.33 (m, 2H), 3.91 - 3.76 (m, 2H), 3.28 - 3.17 (m, 3H), 3.16 - 3.05 (m, 4H), 2.93 - 2.84 (m, 1H), 2.81 - 2.75 (m, 1H), 2.70 - 2.64 (m, 2H), 2.46 (dd, *J* = 13.1, 4.7 Hz, 1H), 2.22 - 2.10 (m, 1H). LCMS (ESI) calcd. for C₄₂H₄₀N₇O₃⁺ [M+H]⁺: 690.32, found, 690.4.

### Example 279: Preparation of compound GT-10651

With reference to the method of Scheme 2, the target compound GT-10651 was obtained (10 mg, white solid, yield 22%).¹H NMR (400 MHz, MeOD) δ 7.61 - 7.52 (m, 5H), 7.50 (d, *J* = 1.9 Hz, 1H), 7.47 (d, *J* = 1.8 Hz, 1H), 7.26 - 7.19 (m, 2H), 7.06 (s, 1H), 7.03 (d, *J* = 8.8 Hz, 1H), 5.82 (s, 1H), 5.10 (dd, *J* = 13.2, 5.2 Hz, 1H), 4.48 - 4.33 (m, 2H), 3.72 (s, 2H), 3.20 - 3.02 (m, 6H), 2.93 - 2.85 (m, 1H), 2.77 (dd, *J* = 17.9, 4.8 Hz, 1H), 2.72 - 2.56 (m, 2H), 2.46 (dd, *J* = 13.0, 4.6 Hz, 1H), 2.20 - 2.14 (m, 1H). LCMS (ESI) calcd. for C₄₂H₄₀N₇O₃⁺ [M+H]⁺: 690.32, found, 690.4.

### Example 280: Preparation of compound GT-10652

With reference to the method of Scheme 2, the target compound GT-10652 was obtained (10 mg, white solid, yield 22%). ¹H NMR (400 MHz, MeOD) δ 7.90 (dd, *J* = 8.7, 5.1 Hz, 1H), 7.79 - 7.70 (m, 4H), 7.28 - 7.24 (m, 4H), 7.08 (s, 1H), 7.06 - 7.00 (m, 2H), 5.80 (s, 1H), 5.10 (dd, *J* = 13.3, 5.1 Hz, 1H), 4.46 - 4.33 (m, 2H), 3.65 (s, 2H), 3.11 - 3.03 (m, 6H), 2.89 (dd, *J* = 13.4, 5.4 Hz, 1H), 2.83 - 2.73 (m, 1H), 2.73 - 2.56 (m, 2H), 2.50 - 2.41 (m, 1H), 2.23 - 2.09 (m, 1H). LCMS (ESI) calcd. for C₄₂H₄₀N₇O₃⁺ [M+H]⁺: 690.32, found, 690.4.

### Example 281: Preparation of compound GT-10653

With reference to the method of Scheme 2, the target compound GT-10653 was obtained (10 mg, white solid, yield 22%). ¹H NMR (400 MHz, MeOD) δ 8.79 (d, *J* = 4.4 Hz, 1H), 7.95 (t, *J* = 7.5 Hz, 1H), 7.68 - 7.65 (m, 3H), 7.59 - 7.50 (m, 2H), 7.48 - 7.43 (m, 3H), 7.34 (s, 1H), 7.22 - 7.18 (m, 1H), 5.85 (s, 1H), 5.11 (dd, *J* = 13.3, 5.1 Hz, 1H), 4.45 (q, *J* = 17.2 Hz, 2H), 3.69 (s, 1H), 3.58 - 3.46 (m, 2H), 3.41 (s, 1H), 3.30 - 3.02 (m, 6H), 2.92 - 2.84 (m, 1H), 2.81 - 2.72 (m, 1H), 2.53 - 2.42 (m, 1H), 2.19 - 2.13 (m, 1H). LCMS (ESI) calcd. for C₄₂H₄₀N₇O₃⁺ [M+H]⁺: 690.32, found, 690.4.

### Example 282: Preparation of compound GT-10654

With reference to the method of Scheme 2, the target compound GT-10654 was obtained (10 mg, white solid, yield 22%). LCMS (ESI) calcd. for C₄₂H₄₀N₇O₃⁺ [M+H]⁺: 690.32, found, 690.4.

### Example 283: Preparation of compound GT-10655

With reference to the method of Scheme 2, the target compound GT-10655 was obtained (10 mg, white solid, yield 22%). ¹H NMR (400 MHz, MeOD) δ 8.77 - 8.70 (m, 2H), 8.03 (t, *J* = 7.6 Hz, 2H), 7.80 (d, *J* = 8.0 Hz, 2H), 7.66 (d, *J* = 8.4 Hz, 1H), 7.60 - 7.51 (m, 2H), 7.33 (s, 1H), 7.19 (d, *J* = 8.5 Hz, 1H), 6.00 (s, 1H), 5.11 (dd, *J* = 13.3, 5.1 Hz, 1H), 4.43 (q, *J* = 16.9 Hz, 2H), 3.61 - 3.46 (m, 2H), 3.40 (d, *J* = 7.4 Hz, 2H), 3.30 - 3.25 (m, 2H), 3.15 (s, 4H), 2.93 - 2.84 (m, 1H), 2.83 - 2.69 (m, 1H), 2.53 - 2.42 (m, 1H), 2.22 - 2.07 (m, 1H). LCMS (ESI) calcd. for C₄₂H₄₀N₇O₃⁺ [M+H]⁺: 690.32, found, 690.4.

### Example 284: Preparation of compound GT-09960

With reference to the method of Scheme 2, the target compound GT-09960 was obtained (white solid, 28 mg, yield 42 %). ¹H NMR (400 MHz, DMSO-d6) δ 12.16 (s, 1H), 10.95 (s, 1H), 8.08 - 7.41 (m, 6H), 7.41 - 7.07 (m, 5H), 5.74 (s, 1H), 5.04 (dd, *J* = 13.2, 5.0 Hz, 1H), 4.24 (dd, *J* = 49.9, 16.9 Hz, 2H), 3.38 - 3.18 (m, 5H), 3.14 - 2.72 (m, 4H), 2.59 (d, *J* = 16.8 Hz, 1H), 2.39 - 2.28 (m, 1H), 2.04 - 1.86 (m, 1H). LCMS (ESI) calcd. for C₃₀H₂₉F₃N₅O₃ ⁺ [M+H]⁺: 564.22, found, 564.3.

### Example 285: Preparation of compound GT-09961

With reference to the method of Scheme 2, the target compound GT-09961 was obtained (white solid, 15 mg, yield 23 %). ¹H NMR (400 MHz, DMSO-d6) δ 12.34 (s, 1H), 11.00 (s, 1H), 8.05 - 7.44 (m, 5H), 7.44 - 6.93 (m, 7H), 5.74 (s, 1H), 5.09 (dd, *J* = 13.2, 5.1 Hz, 1H), 4.28 (dd, *J* = 46.5, 17.5 Hz, 2H), 3.27 (s, 5H), 3.08 (s, 2H), 2.99 - 2.71 (m, 2H), 2.63 (d, *J* = 17.2 Hz, 1H), 2.41 - 2.30 (m, 1H), 2.09 - 1.98 (m, 1H). LCMS (ESI) calcd. for C₃₀H₃₀F₂N₅O₃ ⁺ [M+H]⁺: 546.23, found, 546.3.

### Example 286: Preparation of compound GT-09962

With reference to the method of Scheme 2, the target compound GT-09962 was obtained (white solid, 9 mg, yield 13 %). ¹H NMR (400 MHz, DMSO-d6) δ 12.02 (s, 1H), 11.00 (s, 1H), 7.94 (d, *J* = 7.3 Hz, 3H), 7.46 (t, *J* = 7.4 Hz, 4H), 7.41 - 7.33 (m, 2H), 7.32 - 7.22 (m, 2H), 7.11 (d, 1H), 7.05 (d, *J* = 7.3 Hz, 1H), 5.63 (d, *J* = 9.1 Hz, 1H), 5.09 (dd, *J* = 13.2, 5.0 Hz, 1H), 4.35 (d, *J* = 17.4 Hz, 1H), 4.23 (d, *J* = 17.5 Hz, 1H), 3.34 - 3.16 (m, 6H), 3.14 - 3.00 (m, 2H), 2.98 - 2.88 (m, 1H), 2.63 (d, *J* = 16.7 Hz, 1H), 2.42 - 2.29 (m, 1H), 2.08 - 1.97 (m, 1H). LCMS (ESI) calcd. for C₃₀H₃₂N₅O₃ ⁺ [M+H]⁺: 510.25, found, 510.3.

### Example 287: Preparation of compound GT-09963

With reference to the method of Scheme 2, the target compound GT-09963 was obtained (white solid, 5 mg, yield 8 %). ¹H NMR (400 MHz, DMSO-d6) δ 11.87 (s, 1H), 11.08 (s, 1H), 8.58 (s, 1H), 7.94 (s, 3H), 7.69 - 7.43 (m, 3H), 7.33 (s, 3H), 7.16 (s, 1H), 5.73 (s, 1H), 5.07 (dd, *J* = 12.7, 5.2 Hz, 1H), 3.33 - 3.15 (m, 4H), 3.07 (s, 2H), 2.93 - 2.77 (m, 2H), 2.70 - 2.53 (m, 2H), 2.46 (s, 1H), 2.06 - 1.96 (m, 1H). LCMS (ESI) calcd. for C₃₀H₂₇F₃N₅O₄ ⁺ [M+H]⁺: 600.18, found, 600.3.

### Example 288: Preparation of compound GT-09964

With reference to the method of Scheme 2, the target compound GT-09964 was obtained (white solid, 3 mg, yield 5 %). ¹H NMR (400 MHz, DMSO-d6) δ 11.57 (s, 1H), 10.89 (s, 1H), 8.01 - 7.54 (m, 4H), 7.38 - 7.09 (m, 6H), 5.60 (s, 1H), 4.96 (dd, *J* = 13.3, 5.1 Hz, 1H), 4.38 (d, *J* = 16.8 Hz, 1H), 4.20 (d, *J* = 16.7 Hz, 1H), 3.19 - 2.90 (m, 6H), 2.89 - 2.75 (m, 2H), 2.63 - 2.48 (m, 2H), 2.37 - 2.26 (m, 1H), 1.97 - 1.81 (m, 1H). LCMS (ESI) calcd. for C₃₀H₂₉F₃N₅O₃ ⁺ [M+H]⁺: 564.22, found, 564.3.

### Example 289: Preparation of compound GT-09965

With reference to the method of Scheme 2, the target compound GT-09965 was obtained (white solid, 11 mg, yield 17 %). ¹H NMR (400 MHz, DMSO-d6) δ 11.70 (s, 1H), 10.88 (s, 1H), 8.31 - 7.35 (m, 5H), 7.31 - 6.85 (m, 6H), 5.63 (s, 1H), 4.97 (dd, *J* = 13.3, 5.1 Hz, 1H), 4.24 (d, *J* = 16.9 Hz, 1H), 4.11 (d, *J* = 16.8 Hz, 1H), 3.26 - 3.04 (m, 5H), 2.98 (s, 2H), 2.86 - 2.67 (m, 2H), 2.52 (d, *J* = 16.4 Hz, 1H), 2.32 - 2.24 (m, 1H), 1.95 - 1.86 (m, 1H). LCMS (ESI) calcd. for C₃₀H₂₉F₃N₅O₃ ⁺ [M+H]⁺: 564.22, found, 564.3.

### Example 290: Preparation of compound GT-10076

With reference to the method of Scheme 2, the target compound GT-10076 was obtained (white solid, 11 mg, yield 17 %). ¹H NMR (400 MHz, DMSO-d6) δ 12.10 (s, 1H), 10.94 (s, 1H), 8.24 - 7.47 (m, 5H), 7.36 - 7.13 (m, 4H), 6.62 (d, *J* = 43.5 Hz, 2H), 5.67 (s, 1H), 4.98 (dd, *J* = 13.3, 5.1 Hz, 1H), 4.30 (d, *J* = 17.2 Hz, 1H), 4.16 (d, *J* = 17.2 Hz, 1H), 3.34 - 3.10 (m, 6H), 3.04 (s, 2H), 2.94 - 2.85 (m, 1H), 2.58 (d, *J* = 16.6 Hz, 1H), 2.35 - 2.26 (m, 1H), 1.99 - 1.87 (m, 1H). LCMS (ESI) calcd. for C₃₀H₂₉F₃N₅O₃ ⁺ [M+H]⁺: 564.22, found, 564.3.

### Example 291: Preparation of compound GT-10077

With reference to the method of Scheme 2, the target compound GT-10077 was obtained (white solid, 7 mg, yield 11 %). ¹H NMR (400 MHz, DMSO-d6) δ 12.01 (s, 1H), 11.00 (s, 1H), 7.88 (s, 3H), 7.58 - 6.91 (m, 9H), 5.70 (s, 1H), 5.09 (dd, *J* = 13.2, 5.0 Hz, 1H), 4.28 (dd, *J* = 46.0, 17.4 Hz, 2H), 3.28 - 3.16 (m, 4H), 3.09 (s, 2H), 2.97 - 2.87 (m, 1H), 2.79 (s, 1H), 2.63 (d, *J* = 17.3 Hz, 1H), 2.47 - 2.19 (m, 2H), 2.10 - 1.96 (m, 1H). LCMS (ESI) calcd. for C₃₀H₃₀F₂N₅O₃ ⁺ [M+H]⁺: 546.23, found, 546.2.

### Example 292: Preparation of compound GT-10078

With reference to the method of Scheme 2, the target compound GT-10078 was obtained (white solid, 9 mg, yield 13 %). ¹H NMR (400 MHz, DMSO-d6) δ 11.66 (s, 1H), 10.95 (s, 1H), 8.00 - 7.72 (m, 4H), 7.55 - 7.15 (m, 9H), 5.74 - 5.46 (m, 1H), 5.04 (dd, *J* = 13.2, 5.0 Hz, 1H), 4.24 (dd, *J* = 50.0, 16.9 Hz, 2H), 3.33 - 3.25 (m, 3H), 3.25 - 3.15 (m, 2H), 3.07 (s, 2H), 2.96 - 2.77 (m, 2H), 2.59 (d, *J* = 16.9 Hz, 1H), 2.40 - 2.30 (m, 1H), 2.02 - 1.90 (m, 1H). LCMS (ESI) calcd. for C₃₀H₃₁FN₅O₃ ⁺ [M+H]⁺: 528.24, found, 528.2.

### Example 293: Preparation of compound GT-10079

With reference to the method of Scheme 2, the target compound GT-10079 was obtained (yellow solid, 18 mg, yield 28 %). ¹H NMR (400 MHz, DMSO-d6) δ 12.14 (s, 1H), 11.09 (s, 1H), 8.74 - 7.70 (m, 4H), 7.69 - 7.27 (m, 7H), 5.73 (s, 1H), 5.07 (dd, *J* = 12.8, 5.4 Hz, 1H), 3.35 - 3.15 (m, 4H), 3.05 (s, 2H), 2.93 - 2.77 (m, 2H), 2.63 - 2.52 (m, 2H), 2.50 - 2.43 (m, 1H), 2.06 - 1.95 (m, 1H). LCMS (ESI) calcd. for C₃₀H₂₇Cl₂FN₅O₄ ⁺ [M+H]⁺: 610.14, found, 610.2.

### Example 294: Preparation of compound GT-10080

With reference to the method of Scheme 2, the target compound GT-10080 was obtained (white solid, 8 mg, yield 13 %). ¹H NMR (400 MHz, DMSO-d6) δ 11.86 (s, 1H), 10.95 (s, 1H), 8.06 - 7.73 (m, 3H), 7.71 - 7.10 (m, 8H), 5.71 (s, 1H), 5.03 (dd, *J* = 13.3, 5.0 Hz, 1H), 4.45 (d, *J* = 16.8 Hz, 1H), 4.27 (d, *J* = 16.7 Hz, 1H), 3.33 - 3.14 (m, 4H), 3.14 - 2.96 (m, 2H), 2.94 - 2.81 (m, 2H), 2.68 - 2.52 (m, 2H), 2.42 - 2.32 (m, 1H), 1.99 - 1.88 (m, 1H). LCMS (ESI) calcd. for C₃₀H₂₉Cl₂FN₅O₃ ⁺ [M+H]⁺: 596.16, found, 596.2.

### Example 295: Preparation of compound GT-10081

With reference to the method of Scheme 2, the target compound GT-10081 was obtained (white solid, 17 mg, yield 27 %). ¹H NMR (400 MHz, DMSO-d6) δ 12.04 (s, 1H), 10.95 (s, 1H), 8.07 - 7.65 (m, 4H), 7.64 - 7.38 (m, 5H), 7.34 (d, *J* = 10.6 Hz, 1H), 7.23 (d, *J* = 6.9 Hz, 1H), 5.72 (s, 1H), 5.12 - 4.97 (m, 1H), 4.24 (dd, *J* = 50.6, 16.9 Hz, 2H), 3.35 - 3.13 (m, 5H), 3.04 (s, 2H), 2.96 - 2.82 (m, 2H), 2.59 (d, *J* = 17.0 Hz, 1H), 2.40 - 2.29 (m, 1H), 2.02 - 1.90 (m, 1H). LCMS (ESI) calcd. for C₃₀H₂₉Cl₂FN₅O₃ ⁺ [M+H]⁺: 596.16, found, 596.2.

### Example 296: Preparation of compound GT-10082

With reference to the method of Scheme 2, the target compound GT-10082 was obtained (white solid, 14 mg, yield 23 %). ¹H NMR (400 MHz, DMSO-d6) δ 12.26 (s, 1H), 10.94 (s, 1H), 8.28 - 7.70 (m, 4H), 7.71 - 7.26 (m, 5H), 6.62 (d, *J* = 43.8 Hz, 2H), 5.68 (s, 1H), 4.98 (dd, *J* = 13.3, 5.1 Hz, 1H), 4.30 (d, *J* = 17.3 Hz, 1H), 4.16 (d, *J* = 17.3 Hz, 1H), 3.34 - 3.11 (m, 5H), 3.04 (s, 2H), 2.95 - 2.70 (m, 2H), 2.58 (d, *J* = 16.7 Hz, 1H), 2.38 - 2.27 (m, 1H), 2.00 - 1.86 (m, 1H). LCMS (ESI) calcd. for C₃₀H₂₉Cl₂FN₅O₃ ⁺ [M+H]⁺: 596.16, found, 596.2.

### Example 297: Preparation of compound GT-10083

With reference to the method of Scheme 2, the target compound GT-10083 was obtained (white solid, 13 mg, yield 22 %). ¹H NMR (400 MHz, DMSO-d6) δ 12.13 (s, 1H), 11.00 (s, 1H), 7.95 (s, 3H), 7.75 - 7.39 (m, 5H), 7.29 (t, *J* = 7.6 Hz, 2H), 7.15 - 6.99 (m, 2H), 5.74 (s, 1H), 5.09 (dd, *J* = 13.2, 5.1 Hz, 1H), 4.34 (d, *J* = 17.4 Hz, 1H), 4.23 (d, *J* = 17.5 Hz, 1H), 3.33 - 3.15 (m, 5H), 3.15 - 3.02 (m, 2H), 2.99 - 2.88 (m, 2H), 2.63 (d, *J* = 16.8 Hz, 1H), 2.40 - 2.30 (m, 1H), 2.10 - 1.93 (m, 1H). LCMS (ESI) calcd. for C₃₀H₃₀Cl₂N₅O₃ ⁺ [M+H]⁺: 578.17, found, 578.2.

### Example 298: Preparation of compound GT-10084

With reference to the method of Scheme 2, the target compound GT-10084 was obtained (yellow solid, 25 mg, yield 35 %). ¹H NMR (400 MHz, DMSO-d6) δ 11.07 (s, 1H), 8.77 (d, *J* = 4.1 Hz, 1H), 8.70 - 8.40 (m, 1H), 7.98 - 7.88 (m, 1H), 7.78 - 7.69 (m, 3H), 7.63 (d, *J* = 8.3 Hz, 1H), 7.50 - 7.38 (m, 4H), 7.29 - 7.23 (m, 1H), 7.11 - 7.01 (m, 1H), 5.89 (s, 1H), 5.05 (dd, *J* = 12.8, 5.4 Hz, 1H), 3.38 (s, 4H), 3.18 - 3.08 (m, 4H), 2.93 - 2.81 (m, 1H), 2.64 - 2.53 (m, 2H), 2.07 - 1.91 (m, 1H). LCMS (ESI) calcd. for C₂₉H₂₉N₆O₄ ⁺ [M+H]⁺: 525.22, found, 525.3.

### Example 299: Preparation of compound GT-10085

With reference to the method of Scheme 2, the target compound GT-10085 was obtained (yellow solid, 25 mg, yield 34 %). ¹H NMR (400 MHz, DMSO-d6) δ 11.09 (s, 1H), 8.76 (d, *J* = 8.6 Hz, 1H), 8.47 (s, 1H), 7.98 - 7.85 (m, 1H), 7.73 (d, *J* = 7.0 Hz, 2H), 7.71 - 7.62 (m, 2H), 7.54 (d, *J* = 7.0 Hz, 1H), 7.51 - 7.40 (m, 4H), 5.88 (s, 1H), 5.07 (dd, *J* = 12.8, 5.4 Hz, 1H), 3.39 (s, 3H), 3.26 - 2.96 (m, 5H), 2.95 - 2.82 (m, 1H), 2.62 - 2.52 (m, 2H), 2.08 - 1.94 (m, 1H). LCMS (ESI) calcd. for C₂₉H₂₈FN₆O₄ ⁺ [M+H]⁺: 543.22, found, 543.3.

### Example 300: Preparation of compound GT-10086

With reference to the method of Scheme 2, the target compound GT-10086 was obtained (red solid, 20 mg, yield 29 %). ¹H NMR (400 MHz, DMSO-d6) δ 10.95 (d, *J* = 11.1 Hz, 1H), 8.76 (d, *J* = 4.1 Hz, 1H), 7.98 - 7.87 (m, 1H), 7.76 - 7.69 (m, 3H), 7.49 - 7.38 (m, 5H), 7.01 - 6.82 (m, 2H), 5.91 (s, 1H), 5.10 - 4.98 (m, 1H), 4.30 (d, *J* = 16.9 Hz, 1H), 4.16 (d, *J* = 17.0 Hz, 1H), 3.50 - 3.30 (m, 4H), 3.21 (s, 2H), 3.09 (s, 4H), 2.94 - 2.83 (m, 1H), 2.59 (d, *J* = 16.6 Hz, 1H), 2.39 - 2.25 (m, 1H), 2.01 - 1.86 (m, 1H). LCMS (ESI) calcd. for C₂₉H₃₁N₆O₃ ⁺ [M+H]⁺: 511.25, found, 511.3.

### Example 301: Preparation of compound GT-10087

With reference to the method of Scheme 2, the target compound GT-10087 was obtained (red solid, 17 mg, yield 24 %). ¹H NMR (400 MHz, DMSO-d6) δ 10.96 (s, 1H), 8.75 (dd, *J* = 17.3, 4.0 Hz, 1H), 7.99 - 7.87 (m, 1H), 7.71 (dd, *J* = 21.7, 7.4 Hz, 4H), 7.49 - 7.39 (m, 5H), 7.28 (dd, *J* = 14.2, 6.4 Hz, 1H), 5.90 (s, 1H), 5.15 - 4.93 (m, 1H), 4.44 (d, *J* = 15.3 Hz, 1H), 4.26 (d, 1H), 3.37 - 3.29 (m, 2H), 3.30 - 3.04 (m, 6H), 2.96 - 2.84 (m, 1H), 2.62 - 2.54 (m, 1H), 2.45 - 2.32 (m, 1H), 2.00 - 1.92 (m, 1H). LCMS (ESI) calcd. for C₂₉H₃₀FN₆O₃ ⁺ [M+H]⁺: 529.24, found, 529.3.

### Example 302: Preparation of compound GT-10088

With reference to the method of Scheme 2, the target compound GT-10088 was obtained (red solid, 22 mg, yield 31 %). ¹H NMR (400 MHz, DMSO-d6) δ 10.96 (s, 1H), 8.75 (d, 1H), 7.97 - 7.90 (m, 1H), 7.79 - 7.64 (m, 4H), 7.50 - 7.40 (m, 4H), 7.35 (d, *J* = 11.3, 5.8 Hz, 1H), 7.26 (d, *J* = 7.1 Hz, 1H), 5.90 (s, 1H), 5.10 - 5.00 (m, 1H), 4.31 (d, *J* = 16.9 Hz, 1H), 4.16 (d, 1H), 3.37 - 3.29 (m, 2H), 3.27 - 3.04 (m, 6H), 2.95 - 2.82 (m, 1H), 2.59 (d, *J* = 17.2 Hz, 1H), 2.40 - 2.30 (m, 1H), 2.01 - 1.90 (m, 1H). LCMS (ESI) calcd. for C₂₉H₃₀FN₆O₃ ⁺ [M+H]⁺: 529.24, found, 529.3.

### Example 303: Preparation of compound GT-10089

With reference to the method of Scheme 2, the target compound GT-10089 was obtained (red solid, 17 mg, yield 24 %). ¹H NMR (400 MHz, DMSO-d6) δ 10.95 (s, 1H), 8.75 (d, *J* = 7.9 Hz, 1H), 7.93 (t, *J* = 7.7, 1.7 Hz, 1H), 7.76 - 7.70 (m, 3H), 7.49 - 7.40 (m, 4H), 6.81 - 6.51 (m, 2H), 5.88 (s, 1H), 4.98 (dd, *J* = 13.3, 5.1 Hz, 1H), 4.31 (d, *J* = 17.2 Hz, 1H), 4.17 (d, *J* = 17.2 Hz, 1H), 3.46 - 3.16 (m, 5H), 3.15 - 3.02 (m, 4H), 2.93 - 2.84 (m, 1H), 2.58 (d, *J* = 17.6 Hz, 1H), 2.36 - 2.25 (m, 1H), 1.99 - 1.88 (m, 1H). LCMS (ESI) calcd. for C₂₉H₃₀FN₆O₃ ⁺ [M+H]⁺: 529.24, found, 529.3.

### Example 304: Preparation of compound GT-10090

With reference to the method of Scheme 2, the target compound GT-10090 was obtained (red solid, 20 mg, yield 29 %). ¹H NMR (400 MHz, DMSO-d6) δ 11.02 (s, 1H), 8.75 (d, *J* = 4.1 Hz, 1H), 7.96 - 7.90 (m, 1H), 7.83 - 7.75 (m, 3H), 7.50 - 7.41 (m, 4H), 7.30 (t, *J* = 13.7, 6.0 Hz, 1H), 7.13 - 7.03 (m, 2H), 6.01 (s, 1H), 5.11 (dd, *J* = 13.2, 5.0 Hz, 1H), 4.39 (d, 1H), 4.27 (d, *J* = 17.6, 2.9 Hz, 1H), 3.38 - 3.06 (m, 8H), 2.99 - 2.88 (m, 1H), 2.63 (d, *J* = 16.8 Hz, 1H), 2.47 - 2.33 (m, 1H), 2.07 - 1.96 (m, 1H). LCMS (ESI) calcd. for C₂₉H₃₁N₆O₃ ⁺ [M+H]⁺: 511.25, found, 511.3.

### Example 305: Preparation of compound GT-10091

With reference to the method of Scheme 2, the target compound GT-10091 was obtained (yellow solid, 7 mg, yield 10 %). ¹H NMR (400 MHz, DMSO-d6) δ 12.54 (s, 1H), 11.08 (s, 1H), 9.08 (s, 1H), 8.78 - 8.33 (m, 3H), 7.94 (s, 2H), 7.71 - 7.38 (m, 6H), 5.79 (s, 1H), 5.07 (dd, *J* = 12.9, 5.3 Hz, 1H), 3.44 - 3.41 (m, 2H), 3.29 - 3.15 (m, 3H), 3.11 - 3.01 (m, 2H), 2.91 - 2.82 (m, 1H), 2.64 - 2.53 (m, 3H), 2.07 - 1.95 (m, 1H). LCMS (ESI) calcd. for C₂₉H₂₈FN₆O₄ ⁺ [M+H]⁺: 543.22, found, 543.3.

### Example 306: Preparation of compound GT-10092

With reference to the method of Scheme 2, the target compound GT-10092 was obtained (white solid, 5 mg, yield 6 %). ¹H NMR (400 MHz, DMSO-d6) δ 10.95 (s, 1H), 9.07 (s, 1H), 8.91 - 8.62 (m, 3H), 7.87 - 7.69 (m, 2H), 7.51 - 7.37 (m, 6H), 6.08 - 5.64 (m, 1H), 5.03 (dd, *J* = 13.3, 5.0 Hz, 1H), 4.45 (d, *J* = 16.8 Hz, 1H), 4.27 (d, *J* = 16.7 Hz, 1H), 3.19 - 3.04 (m, 5H), 2.95 - 2.84 (m, 2H), 2.60 - 2.54 (m, 2H), 2.40 - 2.32 (m, 1H), 1.99 - 1.93 (m, 1H). LCMS (ESI) calcd. for C₂₉H₃₀FN₆O₃ ⁺ [M+H]⁺: 529.24, found, 529.3.

### Example 307: Preparation of compound GT-10093

With reference to the method of Scheme 2, the target compound GT-10093 was obtained (white solid, 7 mg, yield 8 %). ¹H NMR (400 MHz, DMSO-d6) δ 12.55 (s, 1H), 10.95 (s, 1H), 9.12 (s, 1H), 8.72 (d, *J* = 4.9 Hz, 2H), 8.05 - 7.64 (m, 4H), 7.47 - 7.24 (m, 5H), 5.91 (d, *J* = 79.9 Hz, 1H), 5.04 (dd, *J* = 13.3, 5.1 Hz, 1H), 4.31 (d, *J* = 16.9 Hz, 1H), 4.18 (d, *J* = 16.8 Hz, 1H), 3.34 - 3.17 (m, 5H), 3.06 (s, 3H), 2.94 - 2.86 (m, 1H), 2.59 (d, *J* = 16.6 Hz, 1H), 2.38 - 2.29 (m, 1H), 2.00 - 1.91 (m, 1H). LCMS (ESI) calcd. for C₂₉H₃₀FN₆O₃ ⁺ [M+H]⁺: 529.24, found, 529.3.

### Example 308: Preparation of compound GT-10496

With reference to the method of Scheme 2, the target compound GT-10496 was obtained (white solid, 12 mg, yield 13 %). ¹H NMR (400 MHz, DMSO-d6) δ 12.81 (s, 1H), 10.94 (s, 1H), 8.84 - 8.72 (m, 2H), 8.12 - 7.96 (m, 2H), 7.77 (s, 1H), 7.52 - 7.38 (m, 4H), 6.84 - 6.50 (m, 2H), 5.84 (s, 1H), 4.98 (dd, *J* = 13.3, 5.0 Hz, 1H), 4.30 (d, *J* = 17.2 Hz, 1H), 4.16 (d, *J* = 17.2 Hz, 1H), 3.49 - 3.12 (m, 7H), 3.05 (s, 2H), 2.94 - 2.84 (m, 1H), 2.57 (d, *J* = 17.1 Hz, 1H), 2.40 - 2.22 (m, 1H), 2.04 - 1.86 (m, 1H). LCMS (ESI) calcd. for C₂₉H₃₀FN₆O₃ ⁺ [M+H]⁺: 529.24, found, 529.3.

### Example 309: Preparation of compound GT-10094

With reference to the method of Scheme 2, the target compound GT-10094 was obtained (white solid, 14 mg, yield 15 %). ¹H NMR (400 MHz, DMSO-d6) δ 12.81 (s, 1H), 10.99 (s, 1H), 8.93 - 8.73 (m, 3H), 8.03 - 7.93 (m, 2H), 7.84 - 7.74 (m, 1H), 7.43 - 7.34 (m, 3H), 7.31 - 7.25 (m, 2H), 7.12 (t, *J* = 7.7 Hz, 1H), 7.04 (d, *J* = 5.8 Hz, 1H), 6.02 (s, 1H), 5.10 (dd, *J* = 13.2, 5.1 Hz, 1H), 4.36 (d, *J* = 18.6 Hz, 1H), 4.24 (d, *J* = 17.6 Hz, 1H), 3.38 - 3.20 (m, 6H), 3.08 (s, 2H), 2.97 - 2.89 (m, 1H), 2.63 (d, *J* = 16.9 Hz, 1H), 2.44 - 2.36 (m, 1H), 2.07 - 1.99 (m, 1H). LCMS (ESI) calcd. for C₂₉H₃₁N₆O₃ ⁺ [M+H]⁺: 511.25, found, 511.3.

### Example 310: Preparation of compound GT-10166

With reference to the method of Scheme 1, the target compound GT-10166 was obtained (white solid, 28 mg, yield 39 %). ¹H NMR (400 MHz, DMSO-d6) δ 11.06 (s, 1H), 10.87 (s, 1H), 8.42 (s, 1H), 8.13 (d, *J* = 5.5 Hz, 1H), 7.62 - 7.51 (m, 5H), 7.43 - 7.37 (m, 2H), 7.36 - 7.28 (m, 1H), 7.18 (s, 1H), 7.01 (d, *J* = 7.1 Hz, 1H), 5.10 - 4.95 (m, 1H), 4.29 (s, 2H), 3.24 (d, *J* = 7.6 Hz, 2H), 3.18 - 2.91 (m, 6H), 2.91 - 2.84 (m, 1H), 2.60 - 2.52 (m, 2H), 2.05 - 1.92 (m, 1H). LCMS (ESI) calcd. for C₃₀H₂₉ClN₅O₄ ⁺ [M+H]⁺: 558.19, found, 558.2.

### Example 311: Preparation of compound GT-10167

With reference to the method of Scheme 1, the target compound GT-10167 was obtained (white solid, 30 mg, yield 41 %). ¹H NMR (400 MHz, DMSO-d6) δ 11.08 (s, 1H), 10.58 (s, 1H), 8.42 (s, 1H), 8.11 (t, *J* = 14.7 Hz, 1H), 7.65 - 7.46 (m, 6H), 7.40 (d, *J* = 8.3 Hz, 2H), 7.36 - 7.30 (m, 1H), 5.06 (dd, *J* = 12.8, 5.4 Hz, 1H), 4.31 (d, *J* = 33.0 Hz, 2H), 3.25 (d, *J* = 9.4 Hz, 2H), 3.07 (d, *J* = 47.1 Hz, 4H), 3.00 - 2.82 (m, 3H), 2.55 (dd, *J* = 18.5, 11.9 Hz, 2H), 2.06 - 1.92 (m, 1H). LCMS (ESI) calcd. for C₃₀H₂₈ClFN₅O₄ ⁺ [M+H]⁺: 576.18, found, 576.2.

### Example 312: Preparation of compound GT-10168

With reference to the method of Scheme 1, the target compound GT-10168 was obtained (white solid, 20 mg, yield 28 %). ¹H NMR (400 MHz, DMSO-d6) δ 10.95 (s, 1H), 10.64 (s, 1H), 8.07 (d, *J* = 5.5 Hz, 1H), 7.73 (s, 1H), 7.61 - 7.49 (m, 4H), 7.45 - 7.30 (m, 4H), 7.24 (t, *J* = 7.7 Hz, 1H), 5.03 (dd, *J* = 13.3, 5.0 Hz, 1H), 4.49 - 4.20 (m, 4H), 3.24 (d, *J* = 11.0 Hz, 2H), 3.15 - 3.04 (m, 2H), 3.03 - 2.84 (m, 5H), 2.58 (d, *J* = 17.8 Hz, 1H), 2.44 - 2.31 (m, 1H), 2.01 - 1.89 (m, 1H). LCMS (ESI) calcd. for C₃₀H₃₀ClFN₅O₃ ⁺ [M+H]⁺: 562.20, found, 562.3.

### Example 313: Preparation of compound GT-10169

With reference to the method of Scheme 1, the target compound GT-10169 was obtained (white solid, 22 mg, yield 30 %). ¹H NMR (400 MHz, DMSO-d6) δ 10.95 (s, 1H), 10.70 (s, 1H), 8.09 (d, *J* = 5.3 Hz, 1H), 7.72 (s, 1H), 7.63 - 7.49 (m, 4H), 7.40 (d, *J* = 8.4 Hz, 2H), 7.37 - 7.28 (m, 2H), 7.21 (d, *J* = 7.2 Hz, 1H), 5.03 (dd, *J* = 13.3, 5.1 Hz, 1H), 4.46 - 4.09 (m, 4H), 3.32 - 3.07 (m, 4H), 3.06 - 2.83 (m, 5H), 2.56 (s, 1H), 2.44 - 2.25 (m, 1H), 2.01 - 1.87 (m, 1H). LCMS (ESI) calcd. for C₃₀H₃₀ClFN₅O₃ ⁺ [M+H]⁺: 562.20, found, 562.3.

### Example 314: Preparation of compound GT-10170

With reference to the method of Scheme 1, the target compound GT-10170 was obtained (white solid, 23 mg, yield 32 %). ¹H NMR (400 MHz, DMSO-d6) δ 10.94 (s, 1H), 10.81 (s, 1H), 8.11 (s, 1H), 8.00 (s, 1H), 7.61 - 7.52 (m, 4H), 7.43 - 7.37 (m, 2H), 7.36 - 7.29 (m, 1H), 6.64 (s, 1H), 6.57 (d, *J* = 12.1 Hz, 1H), 4.97 (dd, *J* = 13.3, 5.1 Hz, 1H), 4.36 - 4.10 (m, 4H), 3.23 (d, *J* = 7.5 Hz, 2H), 3.09 - 2.83 (m, 7H), 2.61 - 2.53 (m, 1H), 2.37 - 2.25 (m, 1H), 1.98 - 1.88 (m, 1H). LCMS (ESI) calcd. for C₃₀H₃₀ClFN₅O₃ ⁺ [M+H]⁺: 562.20, found, 562.2.

### Example 315: Preparation of compound GT-10171

With reference to the method of Scheme 1, the target compound GT-10171 was obtained (white solid, 26 mg, yield 37 %). ¹H NMR (400 MHz, DMSO-d6) δ 10.97 (d, *J* = 21.5 Hz, 2H), 8.11 (s, 1H), 7.58 - 7.50 (m, 4H), 7.41 (d, *J* = 8.4 Hz, 2H), 7.36 - 7.25 (m, 2H), 7.19 (s, 1H), 7.05 (t, *J* = 7.5 Hz, 2H), 5.12 - 5.00 (m, 1H), 4.36 - 4.08 (m, 4H), 3.27 - 3.18 (m, 2H), 3.11 - 2.82 (m, 8H), 2.63 (d, *J* = 16.8 Hz, 2H), 2.34 - 2.23 (m, 1H), 2.06 - 1.93 (m, 1H). LCMS (ESI) calcd. for C₃₀H₃₁ClN₅O₃ ⁺ [M+H]⁺: 544.21, found, 544.3.

### Example 316: Preparation of compound GT-10172

With reference to the method of Scheme 1, the target compound GT-10172 was obtained (white solid, 5 mg, yield 8 %). ¹H NMR (400 MHz, DMSO-d6) δ 10.95 (s, 1H), 7.95 - 7.87 (m, 1H), 7.58 - 7.51 (m, 4H), 7.44 - 7.37 (m, 4H), 7.32 - 7.26 (m, 1H), 7.11 (d, 2H), 5.05 (dd, *J* = 13.3, 5.1 Hz, 1H), 4.39 - 4.18 (m, 2H), 3.60 - 3.57 (m, 2H), 3.50 - 3.46 (m, 4H), 3.23 - 3.13 (m, 4H), 2.96 - 2.84 (m, 1H), 2.59 (d, *J* = 16.9 Hz, 1H), 2.44 - 2.30 (m, 1H), 2.04 - 1.91 (m, 1H). LCMS (ESI) calcd. for C₃₀H₂₉ClN₅O₃ ⁺ [M+H]⁺: 542.20, found, 542.3.

### Example 317: Preparation of compound GT-10173

With reference to the method of Scheme 1, the target compound GT-10173 was obtained (white solid, 17 mg, yield 23 %). ¹H NMR (400 MHz, DMSO-d6) δ 11.09 (s, 1H), 11.01 (s, 1H), 8.50 (s, 1H), 8.03 (s, 1H), 7.78 (d, *J* = 8.5 Hz, 3H), 7.64 (d, *J* = 10.2 Hz, 1H), 7.61 - 7.53 (m, 5H), 5.08 (dd, *J* = 12.8, 5.4 Hz, 1H), 4.42 (d, *J* = 3.9 Hz, 2H), 3.42 - 3.37 (m, 4H), 3.24 - 3.09 (m, 4H), 2.92 - 2.83 (m, 1H), 2.64 - 2.52 (m, 2H), 2.06 - 1.97 (m, 1H). LCMS (ESI) calcd. for C₃₀H₂₈ClFN₅O₄ ⁺ [M+H]⁺: 576.18, found, 576.2.

### Example 318: Preparation of compound GT-10174

With reference to the method of Scheme 1, the target compound GT-10174 was obtained (white solid, 16 mg, yield 23 %). ¹H NMR (400 MHz, DMSO-d6) δ 10.96 (s, 2H), 8.01 (s, 1H), 7.78 (d, *J* = 8.5 Hz, 4H), 7.65 - 7.54 (m, 4H), 7.42 (d, *J* = 8.2 Hz, 1H), 7.30 (t, *J* = 7.7 Hz, 1H), 5.04 (dd, *J* = 13.3, 5.1 Hz, 1H), 4.44 (t, *J* = 13.1 Hz, 3H), 4.28 (d, *J* = 16.7 Hz, 1H), 3.39 (s, 2H), 3.30 (s, 2H), 3.09 (s, 4H), 2.95 - 2.85 (m, 1H), 2.59 (d, *J* = 17.4 Hz, 1H), 2.46 - 2.31 (m, 1H), 2.02 - 1.90 (m, 1H). LCMS (ESI) calcd. for C₃₀H₃₀ClFN₅O₃ ⁺ [M+H]⁺: 562.20, found, 562.2.

### Example 319: Preparation of compound GT-10175

With reference to the method of Scheme 1, the target compound GT-10175 was obtained (white solid, 15 mg, yield 21 %). ¹H NMR (400 MHz, DMSO-d6) δ 11.05 (s, 1H), 10.96 (s, 1H), 8.03 (s, 1H), 7.78 (d, *J* = 8.3 Hz, 4H), 7.65 - 7.54 (m, 4H), 7.35 (d, *J* = 10.6 Hz, 1H), 7.26 (d, *J* = 7.2 Hz, 1H), 5.05 (dd, *J* = 13.3, 5.1 Hz, 1H), 4.41 (s, 2H), 4.25 (dd, *J* = 49.7, 16.9 Hz, 2H), 3.43 - 3.39 (m, 2H), 3.31 - 3.24 (m, 2H), 3.17 - 3.02 (m, 4H), 2.96 - 2.85 (m, 1H), 2.59 (d, *J* = 17.2 Hz, 1H), 2.41 - 2.30 (m, 1H), 2.01 - 1.91 (m, 1H). LCMS (ESI) calcd. for C₃₀H₃₀ClFN₅O₃ ⁺ [M+H]⁺: 562.20, found, 562.2.

### Example 320: Preparation of compound GT-10176

With reference to the method of Scheme 1, the target compound GT-10176 was obtained (white solid, 14 mg, yield 20 %). ¹H NMR (400 MHz, DMSO-d6) δ 11.09 (s, 1H), 10.95 (s, 1H), 8.04 (d, *J* = 13.4 Hz, 2H), 7.79 (d, *J* = 8.5 Hz, 3H), 7.63 - 7.54 (m, 4H), 6.69 (s, 1H), 6.60 (d, *J* = 11.9 Hz, 1H), 4.99 (dd, *J* = 13.3, 5.1 Hz, 1H), 4.40 (s, 2H), 4.24 (dd, *J* = 55.1, 17.3 Hz, 2H), 3.43 - 3.38 (m, 2H), 3.31 - 3.23 (m, 2H), 3.12 - 2.96 (m, 4H), 2.94 - 2.84 (m, 1H), 2.58 (d, *J* = 17.3 Hz, 1H), 2.37 - 2.26 (m, 1H), 1.98 - 1.90 (m, 1H). LCMS (ESI) calcd. for C₃₀H₃₀ClFN₅O₃ ⁺ [M+H]⁺: 562.20, found, 562.2.

### Example 321: Preparation of compound GT-10177

With reference to the method of Scheme 1, the target compound GT-10177 was obtained (white solid, 13 mg, yield 19 %). ¹H NMR (400 MHz, DMSO-d6) δ 11.18 (s, 1H), 11.00 (s, 1H), 7.99 (s, 1H), 7.82 - 7.74 (m, 3H), 7.66 - 7.53 (m, 4H), 7.34 - 7.23 (m, 2H), 7.08 (dd, *J* = 13.1, 7.6 Hz, 2H), 5.09 (dd, *J* = 13.3, 5.0 Hz, 1H), 4.46 - 4.17 (m, 4H), 3.33 - 3.17 (m, 4H), 3.16 - 2.98 (m, 4H), 2.97 - 2.87 (m, 1H), 2.61 (d, *J* = 17.1 Hz, 1H), 2.42 - 2.28 (m, 1H), 2.07 - 1.94 (m, 1H). LCMS (ESI) calcd. for C₃₀H₃₁ClN₅O₃ ⁺ [M+H]⁺: 544.21, found, 544.3.

### Example 322: Preparation of compound GT-10178

With reference to the method of Scheme 1, the target compound GT-10178 was obtained (white solid, 14 mg, yield 19 %). ¹H NMR (400 MHz, DMSO-d6) δ 11.09 (s, 1H), 10.90 (s, 1H), 8.49 (s, 1H), 7.85 - 7.69 (m, 6H), 7.65 (d, *J* = 10.2 Hz, 1H), 7.60 - 7.51 (m, 3H), 5.14 - 5.00 (m, 1H), 4.40 (s, 2H), 3.51 - 3.39 (m, 3H), 3.12 (s, 4H), 2.93 - 2.83 (m, 1H), 2.64 - 2.54 (m, 2H), 2.50 - 2.44 (m, 1H), 2.08 - 1.96 (m, 1H). LCMS (ESI) calcd. for C₃₀H₂₈ClFN₅O₄ ⁺ [M+H]⁺: 576.18, found, 576.2.

### Example 323: Preparation of compound GT-10179

With reference to the method of Scheme 1, the target compound GT-10179 was obtained (white solid, 19 mg, yield 27 %). ¹H NMR (400 MHz, DMSO-d6) δ 10.96 (s, 1H), 10.87 (s, 1H), 7.86 - 7.68 (m, 7H), 7.58 - 7.51 (m, 2H), 7.42 (d, *J* = 8.2 Hz, 1H), 7.30 (t, *J* = 7.7 Hz, 1H), 5.04 (dd, *J* = 13.3, 5.1 Hz, 1H), 4.52 - 4.24 (m, 4H), 3.44 - 3.39 (m, 2H), 3.31 - 3.23 (m, 2H), 3.09 (s, 4H), 2.98 - 2.84 (m, 1H), 2.59 (d, *J* = 17.0 Hz, 1H), 2.44 - 2.31 (m, 1H), 2.02 - 1.91 (m, 1H). LCMS (ESI) calcd. for C₃₀H₃₀ClFN₅O₃ ⁺ [M+H]⁺: 562.20, found, 562.3.

### Example 324: Preparation of compound GT-10180

With reference to the method of Scheme 1, the target compound GT-10180 was obtained (white solid, 26 mg, yield 36 %). ¹H NMR (400 MHz, DMSO-d6) δ 10.96 (s, 1H), 10.91 (s, 1H), 7.83 - 7.70 (m, 7H), 7.55 (d, *J* = 8.6 Hz, 2H), 7.36 (d, *J* = 10.6 Hz, 1H), 7.26 (d, *J* = 7.2 Hz, 1H), 5.05 (dd, *J* = 13.3, 5.1 Hz, 1H), 4.39 (s, 2H), 4.34 - 4.14 (m, 2H), 3.53 - 3.43 (m, 2H), 3.27 (s, 2H), 3.10 (s, 4H), 2.97 - 2.86 (m, 1H), 2.59 (d, *J* = 16.6 Hz, 1H), 2.44 - 2.29 (m, 1H), 2.04 - 1.91 (m, 1H). LCMS (ESI) calcd. for C₃₀H₃₀ClFN₅O₃ ⁺ [M+H]⁺: 562.20, found, 562.3.

### Example 325: Preparation of compound GT-10181

With reference to the method of Scheme 1, the target compound GT-10181 was obtained (white solid, 32 mg, yield 44 %). ¹H NMR (400 MHz, DMSO-d6) δ 11.13 (s, 1H), 10.95 (s, 1H), 8.07 (s, 1H), 7.82 - 7.71 (m, 6H), 7.55 (d, *J* = 8.6 Hz, 2H), 6.69 (s, 1H), 6.61 (d, *J* = 12.0 Hz, 1H), 4.99 (dd, *J* = 13.3, 5.1 Hz, 1H), 4.36 (s, 2H), 4.33 - 4.12 (m, 2H), 3.43 - 3.38 (m, 2H), 3.32 - 3.20 (m, 2H), 3.16 - 2.95 (m, 4H), 2.94 - 2.84 (m, 1H), 2.58 (d, *J* = 17.0 Hz, 1H), 2.39 - 2.23 (m, 1H), 1.99 - 1.88 (m, 1H). LCMS (ESI) calcd. for C₃₀H₃₀ClFN₅O₃ ⁺ [M+H]⁺: 562.20, found, 562.3.

### Example 326: Preparation of compound GT-10182

With reference to the method of Scheme 1, the target compound GT-10182 was obtained (white solid, 27 mg, yield 39 %). ¹H NMR (400 MHz, DMSO-d6) δ 11.18 (s, 1H), 11.01 (s, 1H), 7.82 - 7.73 (m, 6H), 7.58 - 7.51 (m, 2H), 7.34 - 7.23 (m, 2H), 7.09 (dd, *J* = 15.2, 7.5 Hz, 2H), 5.10 (dd, *J* = 13.3, 5.1 Hz, 1H), 4.44 - 4.18 (m, 4H), 3.41 - 3.38 (m, 2H), 3.29 - 3.19 (m, 2H), 3.16 - 2.98 (m, 4H), 2.98 - 2.86 (m, 1H), 2.62 (d, *J* = 17.1 Hz, 1H), 2.44 - 2.32 (m, 1H), 2.09 - 1.94 (m, 1H). LCMS (ESI) calcd. for C₃₀H₃₁ClN₅O₃ ⁺ [M+H]⁺: 544.21, found, 544.3.

### Example 327: Preparation of compound GT-10183

With reference to the method of Scheme 2, the target compound GT-10183 was obtained (white solid, 23 mg, yield 33 %). ¹H NMR (400 MHz, DMSO-d6) δ 11.25 (s, 1H), 11.01 (s, 1H), 9.36 (d, *J* = 1.4 Hz, 1H), 9.02 (d, *J* = 1.4 Hz, 1H), 8.21 (d, *J* = 7.9, 1.7 Hz, 2H), 7.65 - 7.52 (m, 3H), 7.30 (t, *J* = 7.7 Hz, 1H), 7.08 (dd, *J* = 10.6, 7.7 Hz, 2H), 5.10 (dd, *J* = 13.3, 5.1 Hz, 1H), 4.65 (s, 2H), 4.37 (d, *J* = 17.5 Hz, 1H), 4.24 (d, *J* = 17.5 Hz, 1H), 3.58 - 3.50 (m, 4H), 3.14 (d, *J* = 11.4 Hz, 2H), 3.01 (s, 2H), 2.97 - 2.87 (m, 1H), 2.62 (d, *J* = 17.2 Hz, 1H), 2.45 - 2.31 (m, 1H), 2.09 - 1.97 (m, 1H). LCMS (ESI) calcd. for C₂₈H₃₀N₇O₃ ⁺ [M+H]⁺: 512.24, found, 512.3.

### Example 328: Preparation of compound GT-10207

With reference to the method of Scheme 2, the target compound GT-10207 was obtained (white solid, 30 mg, yield 42 %). ¹H NMR (400 MHz, DMSO-d6) δ 12.11 (s, 1H), 11.07 (s, 1H), 9.29 (s, 1H), 9.15 - 9.03 (m, 2H), 8.86 - 8.37 (m, 4H), 7.63 (d, *J* = 8.3 Hz, 1H), 7.23 (d, *J* = 3.9 Hz, 1H), 7.11 - 6.93 (m, 1H), 5.05 (dd, *J* = 12.8, 5.4 Hz, 1H), 4.55 (s, 2H), 4.21 - 4.11 (m, 2H), 3.39 (s, 3H), 3.27 - 3.07 (m, 4H), 3.03 - 2.82 (m, 2H), 2.58 (d, *J* = 17.4 Hz, 1H), 2.06 - 1.95 (m, 1H). LCMS (ESI) calcd. for C₂₈H₂₈N₇O₄ ⁺ [M+H]⁺: 526.22, found, 526.2.

### Example 329: Preparation of compound GT-10208

With reference to the method of Scheme 2, the target compound GT-10208 was obtained (white solid, 29 mg, yield 40 %). ¹H NMR (400 MHz, DMSO-d6) δ 11.75 (s, 1H), 11.09 (s, 1H), 9.38 - 8.85 (m, 3H), 8.81 - 8.22 (m, 4H), 7.64 (d, *J* = 10.2 Hz, 1H), 7.56 (t, *J* = 6.8 Hz, 1H), 5.07 (dd, *J* = 12.8, 5.4 Hz, 1H), 4.53 (s, 1H), 3.43 - 3.33 (m, 5H), 3.29 - 3.19 (m, 3H), 3.13 - 3.09 (m, 1H), 3.03 (s, 1H), 2.94 - 2.85 (m, 1H), 2.59 (d, *J* = 19.2 Hz, 1H), 2.05 - 1.97 (m, 1H). LCMS (ESI) calcd. for C₂₈H₂₇FN₇O₄ ⁺ [M+H]⁺: 544.21, found, 544.2.

### Example 330: Preparation of compound GT-10209

With reference to the method of Scheme 2, the target compound GT-10209 was obtained (white solid, 17 mg, yield 25 %). ¹H NMR (400 MHz, DMSO-d6) δ 11.98 (s, 1H), 10.94 (s, 1H), 9.16 - 9.01 (m, 3H), 8.71 (t, *J* = 7.7 Hz, 2H), 8.57 - 8.44 (m, 2H), 7.46 (d, *J* = 8.3 Hz, 1H), 6.94 (s, 1H), 6.88 (d, *J* = 8.4 Hz, 1H), 5.03 (dd, *J* = 13.3, 5.1 Hz, 1H), 4.54 (s, 2H), 4.30 (d, *J* = 16.9 Hz, 1H), 4.17 (d, *J* = 16.9 Hz, 1H), 3.41 - 3.27 (m, 5H), 3.10 (s, 4H), 2.95 - 2.86 (m, 1H), 2.59 (d, *J* = 16.8 Hz, 1H), 2.39 - 2.31 (m, 1H), 1.99 - 1.91 (m, 1H). LCMS (ESI) calcd. for C₂₈H₃₀N₇O₃ ⁺ [M+H]⁺: 512.24, found, 512.3.

### Example 331: Preparation of compound GT-10210

With reference to the method of Scheme 2, the target compound GT-10210 was obtained (white solid, 24 mg, yield 34 %). ¹H NMR (400 MHz, DMSO-d6) δ 11.85 (s, 1H), 10.96 (s, 1H), 9.10 - 9.03 (m, 2H), 8.69 (d, *J* = 6.6 Hz, 2H), 8.52 (d, *J* = 8.2 Hz, 1H), 8.50 - 8.43 (m, 1H), 7.82 (s, 1H), 7.44 - 7.38 (m, 1H), 7.34 - 7.26 (m, 1H), 5.04 (dd, *J* = 13.3, 5.1 Hz, 1H), 4.53 (s, 2H), 4.45 (d, *J* = 16.8 Hz, 1H), 4.27 (d, *J* = 16.8 Hz, 1H), 3.42 - 3.32 (m, 3H), 3.23 - 3.15 (m, 2H), 3.13 - 3.03 (m, 2H), 3.03 - 2.95 (m, 1H), 2.94 - 2.85 (m, 1H), 2.62 - 2.54 (m, 1H), 2.46 - 2.36 (m, 1H), 2.01 - 1.90 (m, 1H). LCMS (ESI) calcd. for C₂₈H₂₉FN₇O₃ ⁺ [M+H]⁺: 530.23, found, 530.3.

### Example 332: Preparation of compound GT-10211

With reference to the method of Scheme 2, the target compound GT-10211 was obtained (white solid, 18 mg, yield 26 %). ¹H NMR (400 MHz, DMSO-d6) δ 11.83 (s, 1H), 10.96 (s, 1H), 9.10 - 9.04 (m, 2H), 8.78 - 8.62 (m, 2H), 8.53 - 8.47 (m, 1H), 7.82 (s, 1H), 7.33 (dd, *J* = 12.1, 9.1 Hz, 1H), 7.28 (d, *J* = 7.0 Hz, 1H), 5.05 (dd, *J* = 13.3, 5.1 Hz, 1H), 4.54 (s, 2H), 4.32 (d, *J* = 16.9 Hz, 1H), 4.20 (d, *J* = 16.9 Hz, 1H), 3.44 - 3.29 (m, 4H), 3.22 - 3.14 (m, 2H), 3.13 - 3.02 (m, 2H), 2.96 - 2.86 (m, 1H), 2.59 (d, *J* = 17.2 Hz, 1H), 2.40 - 2.30 (m, 1H), 2.00 - 1.92 (m, 1H). LCMS (ESI) calcd. for C₂₈H₂₉FN₇O₃ ⁺ [M+H]⁺: 530.23, found, 530.3.

### Example 333: Preparation of compound GT-10212

With reference to the method of Scheme 2, the target compound GT-10212 was obtained (white solid, 21 mg, yield 30 %). ¹H NMR (400 MHz, DMSO-d6) δ 12.06 (s, 1H), 10.95 (s, 1H), 9.24 (s, 1H), 9.11 - 9.04 (m, 2H), 8.71 - 8.68 (m, 1H), 8.52 (d, *J* = 8.2 Hz, 1H), 8.49 - 8.39 (m, 1H), 8.34 - 7.86 (m, 1H), 6.84 - 6.47 (m, 2H), 4.99 (dd, *J* = 13.3, 5.1 Hz, 1H), 4.49 (s, 2H), 4.29 (d, 1H), 4.17 (d, *J* = 17.3 Hz, 1H), 3.42 - 3.29 (m, 3H), 3.21 (s, 1H), 3.09 (s, 3H), 3.00 - 2.83 (m, 2H), 2.58 (d, *J* = 17.5 Hz, 1H), 2.38 - 2.24 (m, 1H), 2.00 - 1.89 (m, 1H). LCMS (ESI) calcd. for C₂₈H₂₉FN₇O₃ ⁺ [M+H]⁺: 530.23, found, 530.3.

### Example 334: Preparation of compound GT-10296

With reference to the method of Scheme 2, the target compound GT-10296 was obtained (white solid, 25 mg, yield 36 %). ¹H NMR (400 MHz, DMSO-d6) δ 12.15 (s, 1H), 11.00 (s, 1H), 9.37 (s, 1H), 9.14 (s, 1H), 9.08 (d, *J* = 6.6 Hz, 2H), 8.76 (d, *J* = 6.6 Hz, 2H), 8.57 - 8.51 (m, 2H), 7.32 - 7.28 (m, 1H), 7.08 (dd, *J* = 19.0, 7.8 Hz, 3H), 5.12 - 5.09 (m, 1H), 4.56 (s, 2H), 4.37 - 4.34 (m, 1H), 4.27 - 4.25 (m, 1H), 3.36 (s, 3H), 3.12 (s, 4H), 2.99 - 2.93 (m, 2H), 2.62 (d, *J* = 16.7 Hz, 1H), 2.45 - 2.38 (m, 1H), 2.05 - 1.99 (m, 1H). LCMS (ESI) calcd. for C₂₈H₃₀N₇O₃ ⁺ [M+H]⁺: 512.24, found, 512.3.

### Example 335: Preparation of compound GT-10297

With reference to the method of Scheme 1, the target compound GT-10297 was obtained (white solid, 27 mg, yield 33 %). ¹H NMR (400 MHz, DMSO-d6) δ 11.30 (s, 1H), 11.07 (s, 1H), 8.47 (s, 1H), 7.75 (s, 1H), 7.63 (d, *J* = 8.3 Hz, 1H), 7.37 (s, 2H), 7.22 (s, 1H), 7.09 - 6.98 (m, 1H), 6.84 (d, *J* = 2.6 Hz, 1H), 6.70 - 6.53 (m, 1H), 5.05 (dd, *J* = 12.9, 5.4 Hz, 1H), 4.59 (s, 2H), 3.46 - 3.38 (m, 2H), 3.32 - 3.21 (m, 2H), 3.14 (d, *J* = 11.4 Hz, 2H), 3.04 (s, 2H), 2.93 - 2.84 (m, 1H), 2.62 - 2.52 (m, 2H), 2.04 - 1.91 (m, 1H). LCMS (ESI) calcd. for C₂₆H₂₅N₅NaO₅S ⁺ [M+H]⁺: 542.15, found, 542.2.

### Example 336: Preparation of compound GT-10298

With reference to the method of Scheme 1, the target compound GT-10298 was obtained (white solid, 33 mg, yield 39 %). ¹H NMR (400 MHz, DMSO-d6) δ 11.17 (s, 1H), 11.09 (s, 1H), 8.49 (s, 1H), 7.75 (s, 1H), 7.64 (d, *J* = 10.2 Hz, 1H), 7.54 (d, *J* = 6.9 Hz, 1H), 7.38 (d, *J* = 6.2 Hz, 2H), 6.84 (d, *J* = 2.8 Hz, 1H), 6.62 (s, 1H), 5.16 - 4.99 (m, 1H), 4.58 (s, 2H), 3.45 - 3.37 (m, 4H), 3.13 (s, 4H), 2.94 - 2.84 (m, 1H), 2.62 - 2.53 (m, 2H), 2.06 - 1.95 (m, 1H). LCMS (ESI) calcd. for C₂₆H₂₄FN₅NaO₅S ⁺ [M+H]⁺: 560.14, found, 560.2.

### Example 337: Preparation of compound GT-10299

With reference to the method of Scheme 1, the target compound GT-10299 was obtained (white solid, 24 mg, yield 30 %). ¹H NMR (400 MHz, DMSO-d6) δ 11.30 (s, 1H), 10.94 (s, 1H), 7.85 - 7.62 (m, 2H), 7.46 (d, *J* = 8.3 Hz, 1H), 7.43 - 7.33 (m, 2H), 6.93 (s, 1H), 6.88 - 6.81 (m, 2H), 6.65 - 6.60 (m, 1H), 5.10 - 4.97 (m, 1H), 4.58 (s, 2H), 4.28 (d, *J* = 12.5 Hz, 1H), 4.16 (d, *J* = 16.9 Hz, 1H), 3.47 - 3.42 (m, 2H), 3.29 - 3.17 (m, 2H), 3.12 (d, *J* = 11.0 Hz, 2H), 3.04 - 2.94 (m, 2H), 2.93 - 2.83 (m, 1H), 2.61 - 2.54 (m, 1H), 2.41 - 2.28 (m, 1H), 2.00 - 1.90 (m, 1H). LCMS (ESI) calcd. for C₂₆H₂₈N₅O₄S ⁺ [M+H]⁺: 506.19, found, 506.2.

### Example 338: Preparation of compound GT-10300

With reference to the method of Scheme 1, the target compound GT-10300 was obtained (white solid, 24 mg, yield 29 %). ¹H NMR (400 MHz, DMSO-d6) δ 11.10 (s, 1H), 10.96 (s, 1H), 7.75 (s, 2H), 7.47 - 7.34 (m, 3H), 7.29 (t, *J* = 7.7 Hz, 1H), 6.85 (d, *J* = 3.1 Hz, 1H), 6.67 - 6.53 (m, 1H), 5.04 (dd, *J* = 13.3, 5.0 Hz, 1H), 4.58 (s, 2H), 4.45 (d, *J* = 16.8 Hz, 1H), 4.28 (d, *J* = 16.8 Hz, 1H), 3.46 - 3.40 (m, 2H), 3.24 (s, 2H), 3.10 (s, 4H), 2.95 - 2.85 (m, 1H), 2.59 (d, *J* = 17.0 Hz, 1H), 2.47 - 2.31 (m, 1H), 2.01 - 1.88 (m, 1H). LCMS (ESI) calcd. for C₂₆H₂₇FN₅O₄S ⁺ [M+H]⁺: 524.18, found, 524.3.

### Example 339: Preparation of compound GT-10301

With reference to the method of Scheme 1, the target compound GT-10301 was obtained (white solid, 21 mg, yield 25.15 %). ¹H NMR (400 MHz, DMSO-d6) δ 11.11 (s, 1H), 10.96 (s, 1H), 7.86 - 7.59 (m, 2H), 7.48 - 7.30 (m, 3H), 7.25 (d, *J* = 7.1 Hz, 1H), 6.85 (d, *J* = 2.9 Hz, 1H), 6.62 (s, 1H), 5.05 (dd, *J* = 13.3, 5.0 Hz, 1H), 4.59 (s, 2H), 4.31 (d, *J* = 16.9 Hz, 1H), 4.19 (d, *J* = 16.9 Hz, 1H), 3.46 - 3.40 (m, 2H), 3.23 (s, 2H), 3.11 (s, 4H), 2.95 - 2.85 (m, 1H), 2.62 - 2.54 (m, 1H), 2.41 - 2.27 (m, 1H), 2.05 - 1.90 (m, 1H). LCMS (ESI) calcd. for C₂₆H₂₇FN₅O₄S ⁺ [M+H]⁺: 524.18, found, 524.3.

### Example 340: Preparation of compound GT-10302

With reference to the method of Scheme 1, the target compound GT-10302 was obtained (white solid, 21 mg, yield 26 %). ¹H NMR (400 MHz, DMSO-d6) δ 11.24 (s, 1H), 10.95 (s, 1H), 8.06 (s, 1H), 7.75 (s, 1H), 7.37 (s, 2H), 6.84 (d, *J* = 3.2 Hz, 1H), 6.75 - 6.55 (m, 3H), 4.99 (dd, *J* = 13.3, 5.1 Hz, 1H), 4.58 (s, 2H), 4.31 (d, *J* = 17.3 Hz, 1H), 4.17 (d, *J* = 17.2 Hz, 1H), 3.44 - 3.39 (m, 2H), 3.26 (s, 2H), 3.11 (d, *J* = 11.1 Hz, 2H), 2.97 (d, *J* = 18.6 Hz, 2H), 2.93 - 2.83 (m, 1H), 2.58 (d, *J* = 17.4 Hz, 1H), 2.39 - 2.25 (m, 1H), 2.00 - 1.87 (m, 1H). LCMS (ESI) calcd. for C₂₆H₂₇FN₅O₄S ⁺ [M+H]⁺: 524.18, found, 524.2.

### Example 341: Preparation of compound GT-10303

With reference to the method of Scheme 1, the target compound GT-10303 was obtained (white solid, 36 mg, yield 45 %). ¹H NMR (400 MHz, DMSO-d6) δ 11.38 (s, 1H), 11.00 (s, 1H), 7.75 (s, 1H), 7.43 - 7.34 (m, 2H), 7.34 - 7.19 (m, 2H), 7.14 - 7.00 (m, 2H), 6.84 (d, *J* = 3.3 Hz, 1H), 6.69 - 6.52 (m, 1H), 5.09 (dd, *J* = 13.2, 5.0 Hz, 1H), 4.58 (s, 2H), 4.35 (d, *J* = 17.4 Hz, 1H), 4.22 (d, *J* = 17.4 Hz, 1H), 3.37 - 3.18 (m, 4H), 3.13 (d, 2H), 3.09 - 2.99 (m, 2H), 2.98 - 2.86 (m, 1H), 2.66 - 2.55 (m, 1H), 2.43 - 2.29 (m, 1H), 2.10 - 1.94 (m, 1H). LCMS (ESI) calcd. for C₂₆H₂₈N₅O₄S ⁺ [M+H]⁺: 506.19, found, 506.2.

### Example 342: Preparation of compound GT-10304

With reference to the method of Scheme 3, the target compound GT-10304 was obtained (white solid, 26 mg, yield 41 %). ¹H NMR (400 MHz, DMSO-d6) δ 10.98 (s, 1H), 7.56 - 7.51 (m, 3H), 7.49 - 7.41 (m, 4H), 7.41 - 7.31 (m, 2H), 7.25 (t, *J* = 7.7 Hz, 1H), 7.02 (dd, *J* = 7.5, 5.0 Hz, 2H), 5.07 (dd, *J* = 13.3, 5.0 Hz, 1H), 4.26 (dd, *J* = 40.1, 17.4 Hz, 2H), 3.16 (s, 1H), 3.05 - 2.81 (m, 3H), 2.78 - 2.64 (m, 2H), 2.64 - 2.51 (m, 4H), 2.42 - 2.31 (m, 1H), 2.04 - 1.94 (m, 1H). LCMS (ESI) calcd. for C₃₀H₂₉ClN₅O₄ ⁺ [M+H]⁺: 558.19, found, 558.3.

### Example 343: Preparation of compound GT-10305

With reference to the method of Scheme 1, the target compound GT-10305 was obtained (white solid, 20 mg, yield 28 %). ¹H NMR (400 MHz, DMSO-d6) δ 11.00 (s, 1H), 10.37 (s, 1H), 7.26 (dd, *J* = 15.8, 8.1 Hz, 2H), 7.23 - 7.18 (m, 4H), 7.10 - 7.01 (m, 2H), 5.08 (dd, *J* = 13.3, 5.0 Hz, 1H), 4.29 (d, *J* = 17.5 Hz, 1H), 4.16 (d, *J* = 17.4 Hz, 1H), 3.54 (s, 2H), 3.36 - 3.31 (m, 2H), 3.17 - 3.04 (m, 2H), 3.01 - 2.72 (m, 5H), 2.62 (d, *J* = 17.0 Hz, 1H), 2.39 - 2.22 (m, 5H), 2.08 - 1.97 (m, 1H), 1.70 (s, 4H). LCMS (ESI) calcd. for C₃₀H₃₅FN₅O₃ ⁺ [M+H]⁺: 532.27, found, 532.3.

### Example 344: Preparation of compound GT-10306

With reference to the method of Scheme 1, the target compound GT-10306 was obtained (white solid, 15 mg, yield 21 %). ¹H NMR (400 MHz, DMSO-d6) δ 10.95 (s, 1H), 10.36 (s, 1H), 7.74 (s, 1H), 7.50 (d, *J* = 8.2 Hz, 2H), 7.33 (d, *J* = 10.6 Hz, 1H), 7.27 (d, *J* = 8.3 Hz, 2H), 7.22 (d, *J* = 7.2 Hz, 1H), 5.03 (dd, *J* = 13.3, 5.0 Hz, 1H), 4.38 (s, 2H), 4.28 (d, *J* = 16.9 Hz, 1H), 4.15 (d, *J* = 16.9 Hz, 1H), 3.84 (t, *J* = 5.3 Hz, 2H), 3.66 (s, 2H), 3.38 (s, 2H), 3.16 (s, 2H), 2.97 - 2.79 (m, 5H), 2.58 (d, *J* = 17.7 Hz, 1H), 2.40 - 2.26 (m, 3H), 2.02 - 1.82 (m, 1H). LCMS (ESI) calcd. for C₂₉H₃₂ClFN₅O₄ ⁺ [M+H]⁺: 568.21, found, 568.3.

### Example 345: Preparation of compound GT-10307

With reference to the method of Scheme 1, the target compound GT-10307 was obtained (white solid, 25 mg, yield 36 %). ¹H NMR (400 MHz, DMSO-d6) δ 11.00 (s, 1H), 10.78 (s, 1H), 7.55 - 7.44 (m, 2H), 7.32 - 7.25 (m, 3H), 7.06 (dd, *J* = 14.8, 7.6 Hz, 2H), 5.08 (dd, *J* = 13.2, 5.0 Hz, 1H), 4.41 (q, *J* = 16.7 Hz, 2H), 4.29 (d, *J* = 17.5 Hz, 1H), 4.16 (d, *J* = 17.4 Hz, 1H), 3.83 (t, *J* = 5.4 Hz, 2H), 3.63 (s, 2H), 3.37 - 3.33 (m, 2H), 3.20 - 3.05 (m, 2H), 3.04 - 2.71 (m, 5H), 2.63 (d, *J* = 16.7 Hz, 1H), 2.45 - 2.25 (m, 3H), 2.07 - 1.95 (m, 1H). LCMS (ESI) calcd. for C₂₉H₃₃ClN₅O₄ ⁺ [M+H]⁺: 550.22, found, 550.3.

### Example 346: Preparation of compound GT-10308

With reference to the method of Scheme 2, the target compound GT-10308 was obtained (white solid, 18 mg, yield 27 %). ¹H NMR (400 MHz, DMSO-d6) δ 12.31 (s, 1H), 11.06 (s, 1H), 8.80 - 7.83 (m, 2H), 7.61 (d, *J* = 8.3 Hz, 1H), 7.57 - 6.82 (m, 7H), 6.20 (s, 1H), 5.13 - 4.93 (m, 1H), 3.60 (s, 1H), 3.42 - 3.10 (m, 5H), 3.05 (s, 1H), 2.96 - 2.68 (m, 2H), 2.66 - 2.53 (m, 2H), 2.49 - 2.19 (m, 1H), 2.06 - 1.93 (m, 1H). LCMS (ESI) calcd. for C₃₀H₂₈F₂N₅O₄ ⁺ [M+H]⁺: 560.21, found, 560.3.

### Example 347: Preparation of compound GT-10309

With reference to the method of Scheme 2, the target compound GT-10309 was obtained (white solid, 3 mg, yield 5 %). ¹H NMR (400 MHz, DMSO-d6) δ 11.08 (s, 1H), 8.23 (s, 2H), 7.78 - 6.92 (m, 9H), 5.06 (dd, *J* = 12.8, 5.3 Hz, 1H), 3.42 (s, 2H), 3.32 - 3.11 (m, 3H), 3.09 - 2.77 (m, 5H), 2.68 - 2.55 (m, 2H), 2.07 - 1.97 (m, 1H). LCMS (ESI) calcd. for C₃₀H₂₇F₃N₅O₄ ⁺ [M+H]⁺: 578.20, found, 578.3.

### Example 348: Preparation of compound GT-10310

With reference to the method of Scheme 2, the target compound GT-10310 was obtained (white solid, 22 mg, yield 35 %). ¹H NMR (400 MHz, DMSO-d6) δ 12.35 (s, 1H), 10.97 (s, 1H), 8.38 (s, 1H), 8.01 - 7.41 (m, 4H), 7.33 (s, 4H), 7.11 - 6.70 (m, 2H), 6.17 (s, 1H), 5.02 (dd, *J* = 13.3, 5.0 Hz, 1H), 4.29 (d, *J* = 16.9 Hz, 1H), 4.16 (d, *J* = 16.9 Hz, 1H), 3.66 - 3.51 (m, 2H), 3.39 - 3.21 (m, 3H), 3.18 - 2.80 (m, 4H), 2.58 (d, *J* = 17.6 Hz, 1H), 2.42 - 2.26 (m, 1H), 2.07 - 1.88 (m, 1H), 1.33 - 1.24 (m, 1H). LCMS (ESI) calcd. for C₃₀H₃₀F₂N₅O₃ ⁺ [M+H]⁺: 546.23, found, 546.3.

### Example 349: Preparation of compound GT-10311

With reference to the method of Scheme 2, the target compound GT-10311 was obtained (white solid, 12 mg, yield 18 %). ¹H NMR (400 MHz, DMSO-d6) δ 12.10 (s, 1H), 10.95 (s, 1H), 8.32 (s, 1H), 7.85 - 7.13 (m, 9H), 6.18 (s, 1H), 5.04 (dd, *J* = 13.2, 5.0 Hz, 1H), 4.45 (d, *J* = 16.8 Hz, 1H), 4.27 (d, *J* = 16.7 Hz, 1H), 3.72 - 3.46 (m, 2H), 3.37 - 3.20 (m, 2H), 3.20 - 2.66 (m, 5H), 2.66 - 2.53 (m, 2H), 2.43 - 2.33 (m, 1H), 2.01 - 1.87 (m, 1H). LCMS (ESI) calcd. for C₃₀H₂₉F₃N₅O₃ ⁺ [M+H]⁺: 564.22, found, 564.3.

### Example 350: Preparation of compound GT-10312

With reference to the method of Scheme 2, the target compound GT-10312 was obtained (white solid, 15 mg, yield 23 %). ¹H NMR (400 MHz, DMSO-d6) δ 12.08 (s, 1H), 10.95 (s, 1H), 8.31 (s, 1H), 8.01 - 7.12 (m, 9H), 6.21 (s, 1H), 5.11 - 4.98 (m, 1H), 4.31 (d, *J* = 16.8 Hz, 1H), 4.18 (d, *J* = 16.8 Hz, 1H), 3.73 - 3.44 (m, 2H), 3.37 - 3.23 (m, 2H), 3.22 - 2.67 (m, 5H), 2.66 - 2.53 (m, 2H), 2.42 - 2.27 (m, 1H), 2.03 - 1.88 (m, 1H). LCMS (ESI) calcd. for C₃₀H₂₉F₃N₅O₃ ⁺ [M+H]⁺: 564.22, found, 564.3.

### Example 351: Preparation of compound GT-10313

With reference to the method of Scheme 2, the target compound GT-10313 was obtained (white solid, 10 mg, yield 15 %). ¹H NMR (400 MHz, DMSO-d6) δ 12.35 (s, 1H), 10.94 (s, 1H), 8.67 - 7.72 (m, 2H), 7.69 - 7.11 (m, 6H), 6.85 - 6.41 (m, 2H), 6.15 (s, 1H), 4.98 (dd, *J* = 13.2, 5.0 Hz, 1H), 4.30 (d, *J* = 17.2 Hz, 1H), 4.16 (d, *J* = 17.2 Hz, 1H), 3.73 - 3.45 (m, 2H), 3.36 - 3.12 (m, 3H), 3.10 - 2.67 (m, 4H), 2.65 - 2.52 (m, 2H), 2.36 - 2.26 (m, 1H), 1.99 - 1.87 (m, 1H). LCMS (ESI) calcd. for C₃₀H₂₉F₃N₅O₃ ⁺ [M+H]⁺: 564.22, found, 564.3.

### Example 352: Preparation of compound GT-10314

With reference to the method of Scheme 2, the target compound GT-10314 was obtained (white solid, 5 mg, yield 8 %). ¹H NMR (400 MHz, DMSO-d6) δ 12.42 (s, 1H), 11.00 (s, 1H), 8.42 (s, 1H), 7.53 - 7.02 (m, 9H), 6.17 (s, 1H), 5.07 (dd, *J* = 13.2, 5.1 Hz, 1H), 4.34 (d, *J* = 17.5 Hz, 1H), 4.24 (d, *J* = 17.4 Hz, 1H), 3.58 - 3.51 (m, 2H), 3.41 - 3.21 (m, 4H), 3.05 (s, 2H), 2.99 - 2.81 (m, 2H), 2.62 (d, *J* = 17.0 Hz, 1H), 2.40 - 2.31 (m, 1H), 2.07 - 1.95 (m, 1H). LCMS (ESI) calcd. for C₃₀H₃₀F₂N₅O₃ ⁺ [M+H]⁺: 546.23, found, 546.3.

### Example 353: Preparation of compound GT-10315

With reference to the method of Scheme 1, the target compound GT-10315 was obtained (white solid, 16 mg, yield 21 %). ¹H NMR (400 MHz, DMSO-d6) δ 11.08 (s, 1H), 10.15 (s, 1H), 8.43 (s, 1H), 7.66 - 7.59 (m, 1H), 7.50 (d, *J* = 6.7 Hz, 1H), 7.28 (d, *J* = 7.2 Hz, 4H), 5.06 (dd, *J* = 12.7, 5.4 Hz, 1H), 4.31 (s, 2H), 3.84 (t, *J* = 5.0 Hz, 2H), 3.68 (s, 2H), 3.44 - 3.37 (m, 2H), 3.25 - 3.10 (m, 2H), 3.07 - 2.80 (m, 5H), 2.68 - 2.53 (m, 2H), 2.37 (s, 2H), 2.06 - 1.91 (m, 1H). LCMS (ESI) calcd. for C₂₉H₃₀F₂N₅O₅ ⁺ [M+H]⁺: 566.22, found, 566.3.

### Example 354: Preparation of compound GT-10316

With reference to the method of Scheme 1, the target compound GT-10316 was obtained (white solid, 17 mg, yield 23 %). ¹H NMR (400 MHz, DMSO-d6) δ 10.96 (s, 1H), 10.43 (s, 1H), 7.66 (s, 1H), 7.37 (d, *J* = 8.2 Hz, 1H), 7.34 - 7.18 (m, 5H), 5.04 (dd, *J* = 13.3, 5.0 Hz, 1H), 4.57 - 4.35 (m, 3H), 4.26 (d, *J* = 16.7 Hz, 1H), 3.84 (t, *J* = 5.3 Hz, 2H), 3.65 (s, 2H), 3.42 - 3.40 (m, 1H), 3.15 (t, *J* = 11.3 Hz, 2H), 3.00 - 2.78 (m, 5H), 2.69 - 2.53 (m, 2H), 2.45 - 2.29 (m, 3H), 2.04 - 1.88 (m, 1H). LCMS (ESI) calcd. for C₂₉H₃₂F₂N₅O₄ ⁺ [M+H]⁺: 552.24, found, 552.3.

### Example 355: Preparation of compound GT-10317

With reference to the method of Scheme 1, the target compound GT-10317 was obtained (white solid, 14 mg, yield 19 %). ¹H NMR (400 MHz, DMSO-d6) δ 10.95 (s, 1H), 10.41 (s, 1H), 7.73 (s, 1H), 7.35 - 7.19 (m, 6H), 5.03 (dd, *J* = 13.3, 5.0 Hz, 1H), 4.38 (s, 2H), 4.21 (dd, *J* = 50.5, 16.9 Hz, 2H), 3.84 (t, *J* = 5.2 Hz, 2H), 3.65 (s, 2H), 3.42 - 3.40 (m, 1H), 3.15 (s, 2H), 3.02 - 2.77 (m, 5H), 2.68 - 2.52 (m, 2H), 2.41 - 2.26 (m, 3H), 2.01 - 1.89 (m, 1H). LCMS (ESI) calcd. for C₂₉H₃₂F₂N₅O₄ ⁺ [M+H]⁺: 552.24, found, 552.3.

### Example 356: Preparation of compound GT-10318

With reference to the method of Scheme 1, the target compound GT-10318 was obtained (white solid, 17 mg, yield 23 %). ¹H NMR (400 MHz, DMSO-d6) δ 10.94 (s, 1H), 10.63 (s, 1H), 8.03 (s, 1H), 7.32 - 7.23 (m, 4H), 6.65 (s, 1H), 6.58 (d, *J* = 12.0 Hz, 1H), 4.98 (dd, *J* = 13.3, 5.0 Hz, 1H), 4.40 (s, 2H), 4.28 (d, *J* = 17.1 Hz, 1H), 4.14 (d, *J* = 17.2 Hz, 1H), 3.83 (t, *J* = 5.3 Hz, 2H), 3.63 (s, 2H), 3.38 (s, 2H), 3.16 - 3.03 (m, 2H), 3.01 - 2.76 (m, 5H), 2.61 - 2.53 (m, 1H), 2.40 - 2.27 (m, 3H), 2.01 - 1.84 (m, 1H). LCMS (ESI) calcd. for C₂₉H₃₂F₂N₅O₄ ⁺ [M+H]⁺: 552.24, found, 552.3.

### Example 357: Preparation of compound GT-10319

With reference to the method of Scheme 1, the target compound GT-10319 was obtained (white solid, 21 mg, yield 29 %). ¹H NMR (400 MHz, DMSO-d6) δ 11.00 (s, 1H), 10.79 (s, 1H), 7.32 - 7.18 (m, 6H), 7.06 (t, *J* = 8.4 Hz, 2H), 5.08 (dd, *J* = 13.3, 5.0 Hz, 1H), 4.48 - 4.34 (m, 2H), 4.28 (d, 1H), 4.15 (d, *J* = 17.5 Hz, 1H), 3.83 (t, *J* = 5.4 Hz, 2H), 3.62 (s, 2H), 3.38 (d, *J* = 12.4 Hz, 3H), 3.18 - 3.07 (m, 2H), 3.02 - 2.91 (m, 3H), 2.91 - 2.77 (m, 2H), 2.62 (d, *J* = 17.0 Hz, 1H), 2.37 (s, 2H), 2.34 - 2.25 (m, 1H), 2.05 - 1.95 (m, 1H). LCMS (ESI) calcd. for C₂₉H₃₃FN₅O₄ ⁺ [M+H]⁺: 534.25, found, 534.3.

### Example 358: Preparation of compound GT-10320

With reference to the method of Scheme 1, the target compound GT-10320 was obtained (white solid, 33 mg, yield 41 %). ¹H NMR (400 MHz, DMSO-d6) δ 11.08 (s, 1H), 10.25 (s, 1H), 8.44 (s, 1H), 7.62 (d, *J* = 10.2 Hz, 1H), 7.50 (d, *J* = 7.7 Hz, 3H), 7.25 (d, *J* = 8.0 Hz, 2H), 5.06 (dd, *J* = 12.8, 5.4 Hz, 1H), 4.38 (s, 2H), 3.69 (s, 2H), 3.44 - 3.36 (m, 2H), 3.26 - 3.12 (m, 2H), 3.11 - 2.81 (m, 5H), 2.61 - 2.52 (m, 2H), 2.23 (s, 2H), 2.08 - 1.92 (m, 1H), 1.23 (s, 6H). LCMS (ESI) calcd. for C₃₁H₃₄ClFN₅O₅ ⁺ [M+H]⁺: 610.22, found, 610.3.

### Example 359: Preparation of compound GT-10321

With reference to the method of Scheme 1, the target compound GT-10321 was obtained (white solid, 26 mg, yield 33 %). ¹H NMR (400 MHz, DMSO-d6) δ 10.96 (s, 1H), 10.44 (s, 1H), 7.75 (s, 1H), 7.49 (d, *J* = 10.0 Hz, 2H), 7.37 (d, *J* = 8.2 Hz, 1H), 7.25 (d, *J* = 7.8 Hz, 3H), 5.04 (dd, *J* = 13.3, 5.0 Hz, 1H), 4.58 - 4.31 (m, 3H), 4.26 (d, *J* = 16.8 Hz, 1H), 3.64 (s, 2H), 3.36 (s, 2H), 3.25 - 3.07 (m, 2H), 3.05 - 2.80 (m, 5H), 2.61 - 2.54 (m, 1H), 2.44 - 2.30 (m, 1H), 2.23 (s, 2H), 2.02 - 1.90 (m, 1H), 1.23 (s, 6H). LCMS (ESI) calcd. for C₃₁H₃₆ClFN₅O₄ ⁺ [M+H]⁺: 596.24, found, 596.3.

### Example 360: Preparation of compound GT-10408

With reference to the method of Scheme 1, the target compound GT-10408 was obtained (white solid, 13 mg, yield 17 %). ¹H NMR (400 MHz, DMSO-d6) δ 10.95 (s, 1H), 10.20 (s, 1H), 7.73 (s, 1H), 7.50 (d, *J* = 8.4 Hz, 2H), 7.34 (d, *J* = 10.7 Hz, 1H), 7.30 - 7.20 (m, 3H), 5.03 (dd, *J* = 13.3, 5.0 Hz, 1H), 4.37 (s, 2H), 4.22 (dd, *J* = 50.8, 16.9 Hz, 2H), 3.67 (s, 2H), 3.13 (s, 2H), 3.01 - 2.82 (m, 5H), 2.58 (d, *J* = 17.6 Hz, 1H), 2.39 - 2.30 (m, 1H), 2.24 (s, 2H), 2.02 - 1.87 (m, 1H), 1.23 (s, 6H). LCMS (ESI) calcd. for C₃₁H₃₆ClFN₅O₄ ⁺ [M+H]⁺: 596.24, found, 596.3.

### Example 361: Preparation of compound GT-10409

With reference to the method of Scheme 1, the target compound GT-10409 was obtained (white solid, 25 mg, yield 32 %). ¹H NMR (400 MHz, DMSO-d6) δ 10.94 (s, 1H), 10.52 (s, 1H), 8.02 (s, 1H), 7.49 (d, *J* = 8.8 Hz, 2H), 7.25 (d, *J* = 8.3 Hz, 2H), 6.74 - 6.50 (m, 2H), 4.98 (dd, *J* = 13.3, 5.0 Hz, 1H), 4.40 (s, 2H), 4.29 (d, *J* = 17.2 Hz, 1H), 4.15 (d, *J* = 17.3 Hz, 1H), 3.65 (s, 2H), 3.10 (s, 2H), 2.99 - 2.80 (m, 5H), 2.57 (d, *J* = 17.3 Hz, 1H), 2.36 - 2.27 (m, 1H), 2.23 (s, 2H), 2.00 - 1.86 (m, 1H), 1.23 (s, 6H). LCMS (ESI) calcd. for C₃₁H₃₆ClFN₅O₄ ⁺ [M+H]⁺: 596.24, found, 596.3.

### Example 362: Preparation of compound GT-10410

With reference to the method of Scheme 1, the target compound GT-10410 was obtained (white solid, 23 mg, yield 30 %). ¹H NMR (400 MHz, DMSO-d6) δ 11.00 (s, 1H), 10.67 (s, 1H), 7.49 (d, *J* = 8.4 Hz, 2H), 7.30 - 7.13 (m, 4H), 7.06 (dd, *J* = 12.3, 7.7 Hz, 2H), 5.08 (dd, *J* = 13.3, 5.0 Hz, 1H), 4.51 - 4.34 (m, 2H), 4.29 (d, *J* = 17.5 Hz, 1H), 4.16 (d, *J* = 17.4 Hz, 1H), 3.64 (s, 2H), 3.32 (s, 2H), 3.17 - 3.05 (m, 2H), 3.01 - 2.76 (m, 5H), 2.63 (d, *J* = 17.2 Hz, 1H), 2.35 - 2.26 (m, 1H), 2.21 (s, 2H), 2.07 - 1.97 (m, 1H), 1.23 (s, 6H). LCMS (ESI) calcd. for C₃₁H₃₇ClN₅O₄ ⁺ [M+H]⁺: 578.25, found, 578.3.

### Example 363: Preparation of compound GT-10411

With reference to the method of Scheme 1, the target compound GT-10411 was obtained (white solid, 18 mg, yield 23 %). ¹H NMR (400 MHz, DMSO-d6) δ 11.07 (s, 1H), 10.43 (s, 1H), 8.44 (s, 1H), 7.61 (d, *J* = 8.3 Hz, 1H), 7.50 (d, *J* = 8.3 Hz, 2H), 7.26 - 7.16 (m, 3H), 7.02 (d, *J* = 7.7 Hz, 1H), 5.04 (dd, *J* = 12.8, 5.4 Hz, 1H), 3.65 (s, 2H), 3.49 - 3.38 (m, 3H), 3.26 - 2.81 (m, 10H), 2.62 - 2.54 (m, 2H), 2.27 - 2.15 (m, 2H), 2.03 - 1.93 (m, 1H). LCMS (ESI) calcd. for C₃₀H₃₁ClF₂N₅O₄ ⁺ [M+H]⁺: 598.20, found, 598.3.

### Example 364: Preparation of compound GT-10412

With reference to the method of Scheme 1, the target compound GT-10412 was obtained (white solid, 17 mg, yield 23 %). ¹H NMR (400 MHz, DMSO-d6) δ 10.93 (s, 1H), 10.43 (s, 1H), 7.68 (s, 1H), 7.50 (d, *J* = 8.4 Hz, 2H), 7.43 (d, *J* = 8.4 Hz, 1H), 7.21 (d, *J* = 8.1 Hz, 2H), 6.89 (s, 1H), 6.83 (d, *J* = 8.4 Hz, 1H), 5.08 - 4.92 (m, 1H), 4.27 (d, *J* = 16.9 Hz, 1H), 4.16 (d, *J* = 14.7 Hz, 1H), 3.60 (s, 2H), 3.47 - 3.39 (m, 3H), 3.16 - 2.99 (m, 4H), 2.98 - 2.81 (m, 5H), 2.61 - 2.54 (m, 2H), 2.37 - 2.26 (m, 1H), 2.25 - 2.13 (m, 2H), 2.00 - 1.88 (m, 1H). LCMS (ESI) calcd. for C₃₀H₃₃ClF₂N₅O₃ ⁺ [M+H]⁺: 584.22, found, 584.3.

### Example 365: Preparation of compound GT-10413

With reference to the method of Scheme 1, the target compound GT-10413 was obtained (white solid, 11 mg, yield 14 %). ¹H NMR (400 MHz, DMSO-d6) δ 10.96 (s, 1H), 10.10 (s, 1H), 7.74 (s, 1H), 7.51 (d, *J* = 8.1 Hz, 2H), 7.37 (d, *J* = 8.2 Hz, 1H), 7.29 - 7.18 (m, 3H), 5.04 (dd, *J* = 13.3, 5.1 Hz, 1H), 4.44 (d, *J* = 16.8 Hz, 1H), 4.26 (d, *J* = 16.8 Hz, 1H), 3.64 (s, 2H), 3.48 - 3.38 (m, 3H), 3.21 - 3.09 (m, 2H), 3.08 - 2.84 (m, 7H), 2.62 - 2.54 (m, 2H), 2.39 (dt, *J* = 13.3, 8.7 Hz, 1H), 2.28 - 2.13 (m, 2H), 2.01 - 1.91 (m, 1H). LCMS (ESI) calcd. for C₃₀H₃₂ClF₃N₅O₃ ⁺ [M+H]⁺: 602.21, found, 602.3.

### Example 366: Preparation of compound GT-10414

With reference to the method of Scheme 1, the target compound GT-10414 was obtained (white solid, 15 mg, yield 20 %). ¹H NMR (400 MHz, DMSO-d6) δ 10.95 (s, 1H), 10.27 (s, 1H), 7.74 (s, 1H), 7.49 (d, *J* = 8.8 Hz, 2H), 7.34 (d, *J* = 10.6 Hz, 1H), 7.23 (d, *J* = 8.1 Hz, 3H), 5.03 (dd, *J* = 13.3, 5.1 Hz, 1H), 4.28 (d, *J* = 16.9 Hz, 1H), 4.15 (d, *J* = 16.9 Hz, 1H), 3.64 (s, 2H), 3.52 - 3.39 (m, 3H), 3.18 (s, 2H), 3.06 - 2.85 (m, 6H), 2.65 - 2.52 (m, 3H), 2.39 - 2.28 (m, 1H), 2.27 - 2.13 (m, 2H), 2.01 - 1.88 (m, 1H). LCMS (ESI) calcd. for C₃₀H₃₂ClF₃N₅O₃ ⁺ [M+H]⁺: 602.21, found, 602.3.

### Example 367: Preparation of compound GT-10415

With reference to the method of Scheme 1, the target compound GT-10415 was obtained (white solid, 16 mg, yield 21 %). ¹H NMR (400 MHz, DMSO-d6) δ 10.94 (s, 1H), 10.34 (s, 1H), 8.02 (s, 1H), 7.49 (d, *J* = 8.8 Hz, 2H), 7.22 (d, *J* = 8.1 Hz, 2H), 6.70 - 6.49 (m, 2H), 4.98 (dd, *J* = 13.3, 5.0 Hz, 1H), 4.29 (d, *J* = 17.2 Hz, 1H), 4.15 (d, *J* = 17.2 Hz, 1H), 3.60 (s, 2H), 3.46 - 3.38 (m, 3H), 3.12 - 2.80 (m, 9H), 2.61 - 2.54 (m, 2H), 2.36 - 2.18 (m, 3H), 1.97 - 1.88 (m, 1H). LCMS (ESI) calcd. for C₃₀H₃₂ClF₃N₅O₃ ⁺ [M+H]⁺: 602.21, found, 602.3.

### Example 368: Preparation of compound GT-10416

With reference to the method of Scheme 1, the target compound GT-10416 was obtained (white solid, 22 mg, yield 29 %). ¹H NMR (400 MHz, DMSO-d6) δ 11.00 (s, 1H), 10.60 (s, 1H), 7.48 (d, *J* = 6.9 Hz, 2H), 7.32 - 7.21 (m, 3H), 7.07 (dd, *J* = 15.8, 7.6 Hz, 2H), 5.08 (dd, *J* = 13.3, 5.0 Hz, 1H), 4.29 (d, *J* = 17.5 Hz, 1H), 4.16 (d, *J* = 17.4 Hz, 1H), 3.68 - 3.55 (m, 2H), 3.39 (s, 3H), 3.22 - 2.78 (m, 9H), 2.63 (d, *J* = 17.1 Hz, 1H), 2.58 - 2.53 (m, 1H), 2.39 - 2.28 (m, 1H), 2.27 - 2.13 (m, 2H), 2.07 - 1.97 (m, 1H). LCMS (ESI) calcd. for C₃₀H₃₃ClF₂N₅O₃ ⁺ [M+H]⁺: 584.22, found, 584.3.

### Example 369: Preparation of compound GT-10417

With reference to the method of Scheme 1, the target compound GT-10417 was obtained (white solid, 23 mg, yield 30 %). ¹H NMR (400 MHz, DMSO-d6) δ 11.07 (s, 1H), 10.16 (s, 1H), 8.45 (s, 1H), 7.61 (d, *J* = 8.3 Hz, 1H), 7.47 (d, *J* = 8.3 Hz, 2H), 7.18 (d, *J* = 8.2 Hz, 3H), 7.03 (d, *J* = 7.9 Hz, 1H), 5.04 (dd, *J* = 12.8, 5.4 Hz, 1H), 3.60 (d, *J* = 3.9 Hz, 2H), 3.36 (s, 3H), 3.19 (s, 3H), 3.14 (s, 2H), 3.03 - 2.83 (m, 5H), 2.58 (d, *J* = 18.0 Hz, 1H), 2.48 - 2.33 (m, 3H), 2.20 (d, *J* = 18.1 Hz, 1H), 2.03 - 1.95 (m, 1H), 1.87 - 1.78 (m, 1H), 1.70 - 1.61 (m, 1H), 1.23 (s, 3H). LCMS (ESI) calcd. for C₃₂H₃₇ClN₅O₅ ⁺ [M+H]⁺: 606.25, found, 606.3.

### Example 370: Preparation of compound GT-10482

With reference to the method of Scheme 1, the target compound GT-10482 was obtained (white solid, 10 mg, yield 14 %). ¹H NMR (400 MHz, DMSO-d6) δ 10.94 (s, 1H), 10.15 (s, 1H), 7.82 - 7.54 (m, 1H), 7.48 - 7.42 (m, 2H), 7.17 (d, *J* = 9.0 Hz, 2H), 6.94 - 6.79 (m, 2H), 5.02 (dd, *J* = 13.2, 5.0 Hz, 1H), 4.29 (d, 1H), 4.16 (d, *J* = 14.6 Hz, 1H), 3.58 (d, *J* = 4.4 Hz, 2H), 3.36 - 3.31 (m, 3H), 3.19 (s, 3H), 3.07 (s, 2H), 2.99 - 2.84 (m, 4H), 2.58 (d, *J* = 19.9 Hz, 1H), 2.48 - 2.30 (m, 4H), 2.21 (d, *J* = 18.1 Hz, 1H), 1.98 - 1.78 (m, 2H), 1.72 - 1.60 (m, 1H), 1.22 (s, 3H). LCMS (ESI) calcd. for C₃₂H₃₉ClN₅O₄ ⁺ [M+H]⁺: 592.27, found, 592.4.

### Example 371: Preparation of compound GT-10483

With reference to the method of Scheme 1, the target compound GT-10483 was obtained (white solid, 10 mg, yield 13 %). ¹H NMR (400 MHz, DMSO-d6) δ 10.96 (s, 1H), 10.01 (s, 1H), 7.74 (s, 1H), 7.48 (d, *J* = 8.4 Hz, 2H), 7.37 (d, *J* = 8.2 Hz, 1H), 7.26 (t, *J* = 7.7 Hz, 1H), 7.19 (d, *J* = 8.3 Hz, 2H), 5.04 (dd, *J* = 13.3, 5.1 Hz, 1H), 4.45 (d, *J* = 16.7 Hz, 1H), 4.26 (d, *J* = 16.7 Hz, 1H), 3.58 (d, *J* = 4.3 Hz, 2H), 3.19 (s, 3H), 3.17 - 3.06 (m, 2H), 2.98 - 2.78 (m, 5H), 2.58 (d, *J* = 17.2 Hz, 1H), 2.46 - 2.30 (m, 4H), 2.25 - 2.16 (m, 1H), 2.01 - 1.89 (m, 1H), 1.90 - 1.77 (m, 1H), 1.73 - 1.61 (m, 1H), 1.22 (s, 3H). LCMS (ESI) calcd. for C₃₂H₃₈ClFN₅O₄ ⁺ [M+H]⁺: 610.26, found, 610.3.

### Example 372: Preparation of compound GT-10484

With reference to the method of Scheme 1, the target compound GT-10484 was obtained (white solid, 7 mg, yield 10 %). ¹H NMR (400 MHz, DMSO-d6) δ 10.95 (s, 1H), 9.76 (s, 1H), 7.72 (s, 1H), 7.47 (d, *J* = 8.3 Hz, 2H), 7.34 (d, *J* = 10.7 Hz, 1H), 7.23 (d, *J* = 7.1 Hz, 1H), 7.18 (d, *J* = 8.2 Hz, 2H), 5.03 (dd, *J* = 13.3, 5.1 Hz, 1H), 4.28 (d, *J* = 16.9 Hz, 1H), 4.15 (d, *J* = 16.9 Hz, 1H), 3.60 (s, 2H), 3.38 (s, 2H), 3.18 (s, 3H), 3.11 (s, 2H), 3.01 - 2.79 (m, 5H), 2.58 (d, *J* = 19.2 Hz, 1H), 2.45 - 2.29 (m, 4H), 2.25 - 2.17 (m, 1H), 2.00 - 1.90 (m, 1H), 1.88 - 1.77 (m, 1H), 1.71 - 1.62 (m, 1H), 1.22 (s, 3H). LCMS (ESI) calcd. for C₃₂H₃₈ClFN₅O₄ ⁺ [M+H]⁺: 610.26, found, 610.4.

### Example 373: Preparation of compound GT-10485

With reference to the method of Scheme 1, the target compound GT-10485 was obtained (white solid, 11 mg, yield 15 %). ¹H NMR (400 MHz, DMSO-d6) δ 10.94 (s, 1H), 10.26 (s, 1H), 8.05 (s, 1H), 7.46 (d, *J* = 9.8 Hz, 2H), 7.16 (d, *J* = 20.3, 11.1 Hz, 2H), 6.68 - 6.53 (m, 2H), 4.98 (dd, *J* = 13.3, 5.1 Hz, 1H), 4.29 (d, *J* = 17.2 Hz, 1H), 4.15 (d, *J* = 17.3 Hz, 1H), 3.57 (s, 2H), 3.39 (s, 2H), 3.19 (s, 3H), 3.10 (s, 2H), 2.99 - 2.82 (m, 5H), 2.57 (d, *J* = 18.4 Hz, 1H), 2.47 - 2.19 (m, 5H), 1.97 - 1.89 (m, 1H), 1.87 - 1.78 (m, 1H), 1.70 - 1.60 (m, 1H), 1.22 (s, 3H). LCMS (ESI) calcd. for C₃₂H₃₈ClFN₅O₄ ⁺ [M+H]⁺: 610.26, found, 610.3.

### Example 374: Preparation of compound GT-10486

With reference to the method of Scheme 1, the target compound GT-10486 was obtained (white solid, 12 mg, yield 16 %). ¹H NMR (400 MHz, DMSO-d6) δ 11.00 (s, 1H), 10.18 (s, 1H), 7.46 (d, *J* = 8.3 Hz, 2H), 7.28 (t, *J* = 7.7 Hz, 1H), 7.19 (d, *J* = 8.3 Hz, 3H), 7.07 (dd, *J* = 14.2, 7.7 Hz, 2H), 5.08 (dd, *J* = 13.3, 5.0 Hz, 1H), 4.30 (d, *J* = 17.4 Hz, 1H), 4.17 (d, *J* = 17.4 Hz, 1H), 3.62 - 3.48 (m, 3H), 3.18 (s, 3H), 3.13 - 2.77 (m, 8H), 2.63 (d, *J* = 16.9 Hz, 1H), 2.46 - 2.29 (m, 4H), 2.23 - 2.16 (m, 1H), 2.04 - 1.96 (m, 1H), 1.87 - 1.79 (m, 1H), 1.71 - 1.63 (m, 1H), 1.22 (s, 3H). LCMS (ESI) calcd. for C₃₂H₃₉ClN₅O₄ ⁺ [M+H]⁺: 592.27, found, 592.3.

### Example 375: Preparation of compound GT-10487

With reference to the method of Scheme 1, the target compound GT-10487 was obtained (white solid, 20 mg, yield 26 %). ¹H NMR (400 MHz, DMSO-d6) δ 11.00 (s, 1H), 10.29 (s, 1H), 7.46 (d, *J* = 8.2 Hz, 2H), 7.28 (t, *J* = 7.7 Hz, 1H), 7.23 - 7.12 (m, 3H), 7.07 (dd, *J* = 14.1, 7.7 Hz, 2H), 5.08 (dd, *J* = 13.3, 5.0 Hz, 1H), 4.30 (d, *J* = 17.5 Hz, 1H), 4.17 (d, *J* = 17.4 Hz, 1H), 3.58 (s, 2H), 3.32 - 3.26 (m, 2H), 3.17 - 3.03 (m, 2H), 2.98 - 2.81 (m, 4H), 2.63 (d, *J* = 17.5 Hz, 1H), 2.55 - 2.52 (m, 1H), 2.41 - 2.26 (m, 3H), 2.02 (s, 3H), 1.48 (t, *J* = 6.2 Hz, 2H), 0.97 (s, 6H). LCMS (ESI) calcd. for C₃₂H₃₉ClN₅O₃ ⁺ [M+H]⁺: 576.27, found, 576.3.

### Example 376: Preparation of compound GT-10488

With reference to the method of Scheme 1, the target compound GT-10488 was obtained (white solid, 14 mg, yield 18 %). ¹H NMR (400 MHz, DMSO-d6) δ 11.00 (s, 1H), 10.28 (s, 1H), 7.31 - 7.15 (m, 6H), 7.06 (t, *J* = 7.8 Hz, 2H), 5.08 (dd, *J* = 13.3, 5.0 Hz, 1H), 4.29 (d, *J* = 17.4 Hz, 1H), 4.16 (d, *J* = 17.4 Hz, 1H), 3.57 (s, 2H), 3.33 - 3.29 (m, 2H), 3.16 - 3.02 (m, 2H), 3.00 - 2.75 (m, 5H), 2.62 (d, *J* = 16.8 Hz, 1H), 2.39 - 2.24 (m, 3H), 2.09 - 1.96 (m, 3H), 1.48 (t, *J* = 6.2 Hz, 2H), 0.97 (s, 6H). LCMS (ESI) calcd. for C₃₂H₃₉FN₅O₃ ⁺ [M+H]⁺: 560.30, found, 560.4.

### Example 377: Preparation of compound GT-10489

With reference to the method of Scheme 3, the target compound GT-10489 was obtained (white solid, 22 mg, yield 31 %). ¹H NMR (400 MHz, DMSO-d6) δ 10.97 (s, 1H), 7.40 (d, *J* = 8.5 Hz, 2H), 7.29 - 7.22 (m, 3H), 7.01 (dd, *J* = 10.3, 7.7 Hz, 2H), 6.92 - 6.41 (m, 1H), 5.06 (dd, *J* = 13.3, 5.1 Hz, 1H), 4.24 (dd, *J* = 39.2, 17.5 Hz, 2H), 3.41 - 3.30 (m, 2H), 2.96 - 2.87 (m, 1H), 2.69 - 2.53 (m, 3H), 2.44 - 2.27 (m, 4H), 2.23 - 1.82 (m, 4H), 1.56 - 1.36 (m, 2H), 0.98 (d, *J* = 9.3 Hz, 6H). LCMS (ESI) calcd. for C₃₂H₃₇ClN₅O₄ ⁺ [M+H]⁺: 590.25, found, 590.3.

### Example 378: Preparation of compound GT-10490

With reference to the method of Scheme 3, the target compound GT-10490 was obtained (white solid, 25 mg, yield 34 %). ¹H NMR (400 MHz, DMSO-d6) δ 10.97 (s, 1H), 7.30 - 7.21 (m, 3H), 7.16 (t, *J* = 8.8 Hz, 2H), 7.00 (dd, *J* = 14.6, 7.7 Hz, 2H), 6.79 (s, 1H), 5.06 (dd, *J* = 13.3, 5.0 Hz, 1H), 4.24 (dd, *J* = 39.9, 17.5 Hz, 2H), 3.43 - 3.00 (m, 5H), 2.95 - 2.85 (m, 1H), 2.60 (d, *J* = 17.4 Hz, 2H), 2.35 (d, *J* = 16.9 Hz, 4H), 2.19 - 1.82 (m, 4H), 1.54 - 1.37 (m, 2H), 0.99 (d, *J* = 11.8 Hz, 6H). LCMS (ESI) calcd. for C₃₂H₃₇FN₅O₄ ⁺ [M+H]⁺: 574.28, found, 574.3.

### Example 379: Preparation of compound GT-10491

With reference to the method of Scheme 1, the target compound GT-10491 was obtained (white solid, 9 mg, yield 13 %). ¹H NMR (400 MHz, DMSO-d6) δ 10.95 (s, 1H), 7.58 - 7.50 (m, 1H), 7.47 - 7.36 (m, 3H), 7.24 (d, *J* = 9.2 Hz, 2H), 7.12 - 7.03 (m, 2H), 5.04 (dd, *J* = 13.3, 5.0 Hz, 1H), 4.33 (d, 1H), 4.21 (d, *J* = 16.8, 6.8 Hz, 1H), 3.43 (s, 5H), 3.03 (s, 4H), 2.95 - 2.85 (m, 1H), 2.62 - 2.54 (m, 1H), 2.49 - 2.28 (m, 5H), 2.18 (s, 2H), 1.99 - 1.90 (m, 1H), 1.48 - 1.41 (m, 2H), 1.00 - 0.92 (m, 6H). LCMS (ESI) calcd. for C₃₂H₃₉ClN₅O₃ ⁺ [M+H]⁺: 574.27, found, 574.3.

### Example 380: Preparation of compound GT-10492

With reference to the method of Scheme 1, the target compound GT-10492 was obtained (white solid, 30 mg, yield 38 %). ¹H NMR (400 MHz, DMSO-d6) δ 11.08 (s, 1H), 10.34 (s, 1H), 8.46 (s, 1H), 7.62 (d, *J* = 10.3 Hz, 1H), 7.50 (d, *J* = 8.2 Hz, 3H), 7.22 (d, *J* = 8.1 Hz, 2H), 5.06 (dd, *J* = 12.8, 5.4 Hz, 1H), 3.74 (d, *J* = 65.7 Hz, 2H), 3.54 - 3.37 (m, 3H), 3.30 - 3.17 (m, 2H), 3.09 - 2.94 (m, 5H), 2.93 - 2.73 (m, 2H), 2.61 - 2.52 (m, 2H), 2.50 (s, 1H), 2.27 - 2.15 (m, 2H), 2.06 - 1.93 (m, 1H). LCMS (ESI) calcd. for C₃₀H₃₀ClF₃N₅O₄ ⁺ [M+H]⁺: 616.19, found, 616.2.

### Example 381: Preparation of compound GT-10493

With reference to the method of Scheme 1, the target compound GT-10493 was obtained (white solid, 21 mg, yield 27 %). ¹H NMR (400 MHz, DMSO-d6) δ 11.08 (s, 1H), 9.82 (s, 1H), 8.43 (s, 1H), 7.63 (d, *J* = 10.2 Hz, 1H), 7.56 - 7.40 (m, 3H), 7.18 (d, *J* = 7.0 Hz, 2H), 5.06 (dd, *J* = 12.6, 5.2 Hz, 1H), 3.62 (s, 2H), 3.37 (s, 2H), 3.25 - 3.13 (m, 5H), 3.10 - 2.83 (m, 5H), 2.67 - 2.54 (m, 2H), 2.45 - 2.29 (m, 3H), 2.26 - 2.14 (m, 1H), 2.07 - 1.95 (m, 1H), 1.91 - 1.78 (m, 1H), 1.73 - 1.62 (m, 1H), 1.23 (s, 3H). LCMS (ESI) calcd. for C₃₂H₃₆ClFN₅O₅ ⁺ [M+H]⁺: 624.24, found, 624.3.

### Example 382: Preparation of compound GT-10494

With reference to the method of Scheme 2, the target compound GT-10494 was obtained (white solid, 6 mg, yield 8 %). ¹H NMR (400 MHz, DMSO-d6) δ 12.80 (s, 1H), 11.06 (s, 1H), 8.79 (d, *J* = 5.7 Hz, 1H), 8.63 - 7.75 (m, 4H), 7.60 (d, *J* = 8.0 Hz, 1H), 7.57 - 7.28 (m, 4H), 7.21 (s, 1H), 7.03 (s, 1H), 5.04 (dd, *J* = 12.8, 5.4 Hz, 1H), 3.65 (s, 2H), 3.35 - 3.22 (m, 3H), 3.05 (s, 3H), 2.95 - 2.81 (m, 2H), 2.64 - 2.55 (m, 2H), 2.06 - 1.89 (m, 1H). LCMS (ESI) calcd. for C₂₉H₂₉N₆O₄ ⁺ [M+H]⁺: 525.22, found, 525.3.

### Example 383: Preparation of compound GT-10495

With reference to the method of Scheme 2, the target compound GT-10495 was obtained (white solid, 7 mg, yield 9 %). ¹H NMR (400 MHz, DMSO-d6) δ 13.50 - 11.94 (m, 1H), 11.08 (s, 1H), 8.81 (d, *J* = 5.9 Hz, 2H), 8.20 (s, 2H), 8.00 - 7.65 (m, 2H), 7.63 (d, *J* = 10.2 Hz, 1H), 7.59 - 7.20 (m, 5H), 5.06 (dd, *J* = 12.7, 5.4 Hz, 1H), 3.70 (s, 2H), 3.32 - 3.22 (m, 2H), 3.03 (s, 4H), 2.94 - 2.81 (m, 2H), 2.58 (d, *J* = 19.5 Hz, 2H), 2.05 - 1.96 (m, 1H). LCMS (ESI) calcd. for C₂₉H₂₈FN₆O₄ ⁺ [M+H]⁺: 543.22, found, 543.3.

### Example 384: Preparation of compound GT-10509

With reference to the method of Scheme 2, the target compound GT-10509 was obtained (white solid, 23 mg, yield 32 %). ¹H NMR (400 MHz, DMSO-d6) δ 11.07 (s, 1H), 8.74 - 8.67 (m, 2H), 8.04 - 7.90 (m, 2H), 7.78 (d, *J* = 7.9 Hz, 2H), 7.62 (d, *J* = 8.3 Hz, 1H), 7.55 - 7.40 (m, 2H), 7.25 (s, 1H), 7.08 (d, *J* = 8.1 Hz, 1H), 6.08 (s, 1H), 5.09 - 5.02 (m, 1H), 4.39 (s, 2H), 3.36 (s, 3H), 3.14 (s, 4H), 2.93 - 2.81 (m, 1H), 2.58 (d, *J* = 17.8 Hz, 1H), 2.09 - 1.94 (m, 1H). LCMS (ESI) calcd. for C₂₈H₂₈N₇O₄ ⁺ [M+H]⁺: 526.22, found, 526.3.

### Example 385: Preparation of compound GT-10510

With reference to the method of Scheme 2, the target compound GT-10510 was obtained (white solid, 36 mg, yield 49 %). ¹H NMR (400 MHz, DMSO-d6) δ 11.09 (s, 1H), 8.71 (d, *J* = 4.1 Hz, 2H), 8.49 (s, 1H), 8.00 - 7.88 (m, 2H), 7.77 (d, *J* = 7.9 Hz, 2H), 7.65 (d, *J* = 10.3 Hz, 1H), 7.55 (d, *J* = 7.0 Hz, 1H), 7.51 - 7.42 (m, 2H), 6.05 (s, 1H), 5.07 (dd, *J* = 12.8, 5.4 Hz, 1H), 3.26 (d, *J* = 56.4 Hz, 9H), 2.93 - 2.83 (m, 1H), 2.55 (d, *J* = 9.1 Hz, 1H), 2.07 - 1.96 (m, 1H). LCMS (ESI) calcd. for C₂₈H₂₇FN₇O₄ ⁺ [M+H]⁺: 544.21, found, 544.3.

### Example 386: Preparation of compound GT-10511

With reference to the method of Scheme 2, the target compound GT-10511 was obtained (white solid, 26 mg, yield 37 %). ¹H NMR (400 MHz, DMSO-d6) δ 10.95 (s, 1H), 8.69 (d, *J* = 8.9 Hz, 2H), 7.99 - 7.91 (m, 2H), 7.76 (d, *J* = 7.9 Hz, 2H), 7.49 - 7.39 (m, 3H), 6.95 (s, 1H), 6.89 (d, *J* = 8.3 Hz, 1H), 6.07 (s, 1H), 5.02 (dd, *J* = 13.1, 4.9 Hz, 1H), 4.29 (d, *J* = 17.0 Hz, 1H), 4.16 (d, *J* = 17.0 Hz, 1H), 3.33 (s, 4H), 3.10 (s, 4H), 2.94 - 2.84 (m, 1H), 2.58 (d, *J* = 16.8 Hz, 1H), 2.41 - 2.28 (m, 1H), 1.99 - 1.90 (m, 1H). LCMS (ESI) calcd. for C₂₈H₃₀N₇O₃ ⁺ [M+H]⁺: 512.24, found, 512.3.

### Example 387: Preparation of compound GT-10512

With reference to the method of Scheme 2, the target compound GT-10512 was obtained (white solid, 10 mg, yield 14 %). ¹H NMR (400 MHz, DMSO-d6) δ 10.96 (s, 1H), 8.76 - 8.65 (m, 2H), 7.99 - 7.92 (m, 2H), 7.76 (d, *J* = 7.8 Hz, 2H), 7.52 - 7.46 (m, 2H), 7.41 (d, *J* = 8.2 Hz, 1H), 7.29 (t, *J* = 7.7 Hz, 1H), 6.06 (s, 1H), 5.04 (dd, *J* = 13.3, 5.0 Hz, 1H), 4.44 (d, 1H), 4.28 (d, 1H), 3.32 (s, 4H), 3.14 (s, 4H), 2.96 - 2.85 (m, 1H), 2.56 (d, 1H), 2.47 - 2.32 (m, 1H), 2.01 - 1.90 (m, 1H). LCMS (ESI) calcd. for C₂₈H₂₉FN₇O₃ ⁺ [M+H]⁺: 530.23, found, 530.3.

### Example 388: Preparation of compound GT-10513

With reference to the method of Scheme 2, the target compound GT-10513 was obtained (white solid, 16 mg, yield 23 %). ¹H NMR (400 MHz, DMSO-d6) δ 10.95 (s, 1H), 8.71 (d, *J* = 4.8 Hz, 2H), 7.94 (t, *J* = 8.0 Hz, 2H), 7.76 (d, *J* = 9.1 Hz, 3H), 7.52 - 7.43 (m, 2H), 7.37 (d, *J* = 8.7 Hz, 1H), 7.26 (d, *J* = 7.1 Hz, 1H), 6.04 (s, 1H), 5.04 (dd, *J* = 13.3, 5.0 Hz, 1H), 4.31 (d, *J* = 17.0 Hz, 1H), 4.18 (d, *J* = 16.9 Hz, 1H), 3.31 (s, 4H), 3.14 (s, 4H), 2.97 - 2.87 (m, 1H), 2.59 (d, *J* = 17.2 Hz, 1H), 2.41 - 2.30 (m, 1H), 2.00 - 1.90 (m, 1H). LCMS (ESI) calcd. for C₂₈H₂₉FN₇O₃ ⁺ [M+H]⁺: 530.23, found, 530.3.

### Example 389: Preparation of compound GT-10514

With reference to the method of Scheme 2, the target compound GT-10514 was obtained (white solid, 20 mg, yield 28 %). ¹H NMR (400 MHz, DMSO-d6) δ 10.95 (s, 1H), 8.72 - 8.67 (m, 2H), 7.98 - 7.93 (m, 2H), 7.79 (d, *J* = 7.8 Hz, 2H), 7.48 (dd, *J* = 7.6, 4.9 Hz, 2H), 6.75 - 6.60 (m, 2H), 6.08 (s, 1H), 4.98 (dd, *J* = 13.3, 5.0 Hz, 1H), 4.31 (d, 1H), 4.18 (d, *J* = 6.0 Hz, 1H), 3.34 (s, 4H), 3.11 (s, 4H), 2.96 - 2.84 (m, 1H), 2.58 (d, *J* = 17.3 Hz, 1H), 2.35 - 2.23 (m, 1H), 2.00 - 1.89 (m, 1H). LCMS (ESI) calcd. for C₂₈H₂₉FN₇O₃ ⁺ [M+H]⁺: 530.23, found, 530.3.

### Example 390: Preparation of compound GT-10515

With reference to the method of Scheme 2, the target compound GT-10515 was obtained (white solid, 15 mg, yield 22 %). ¹H NMR (400 MHz, DMSO-d6) δ 11.01 (s, 1H), 8.70 (d, *J* = 4.1 Hz, 2H), 8.03 - 7.91 (m, 2H), 7.77 (d, *J* = 7.8 Hz, 2H), 7.47 (dd, *J* = 6.8, 4.9 Hz, 2H), 7.28 (t, *J* = 7.7 Hz, 1H), 7.07 (dd, *J* = 13.1, 7.6 Hz, 2H), 6.13 (s, 1H), 5.10 (dd, *J* = 13.3, 5.0 Hz, 1H), 4.36 (d, 1H), 4.27 (d, 1H), 3.32 (s, 4H), 3.11 (s, 4H), 2.99 - 2.87 (m, 1H), 2.64 (d, *J* = 17.5 Hz, 2H), 2.43 - 2.34 (m, 1H), 2.10 - 1.99 (m, 1H). LCMS (ESI) calcd. for C₂₈H₃₀N₇O₃ ⁺ [M+H]⁺: 512.24, found, 512.3.

### Example 391: Preparation of compound GT-10615

With reference to the method of Scheme 2, the target compound GT-10615 was obtained (white solid, 7 mg, yield 10 %). ¹H NMR (400 MHz, DMSO-d6) δ 12.49 (s, 1H), 10.93 (s, 1H), 7.75 (d, *J* = 7.3 Hz, 4H), 7.47 - 7.37 (m, 6H), 6.99 - 6.74 (m, 2H), 5.89 (d, *J* = 10.4 Hz, 1H), 5.02 (dd, *J* = 13.2, 5.1 Hz, 1H), 4.29 (d, 1H), 4.15 (d, *J* = 16.8 Hz, 1H), 3.82 (d, *J* = 22.0 Hz, 4H), 3.40 - 3.23 (m, 4H), 2.93 - 2.85 (m, 1H), 2.71 - 2.54 (m, 3H), 2.37 - 2.31 (m, 1H), 2.14 - 1.75 (m, 4H). LCMS (ESI) calcd. for C₃₃H₃₆N₅O₃ ⁺ [M+H]⁺: 550.28, found, 550.3.

### Example 392: Preparation of compound GT-10616

With reference to the method of Scheme 2, the target compound GT-10616 was obtained (yellow solid, 7 mg, yield 10 %). ¹H NMR (400 MHz, DMSO-d6) δ 12.79 (s, 1H), 10.93 (s, 2H), 7.99 - 7.94 (m, 2H), 7.50 - 7.45 (m, 3H), 7.33 - 7.29 (m, 4H), 6.93 - 6.86 (m, 1H), 6.48 - 6.40 (m, 1H), 5.02 (dd, *J* = 13.2, 5.1 Hz, 2H), 4.28 (d, *J* = 9.4 Hz, 1H), 4.18 (d, 1H), 3.94 (s, 5H), 3.34 - 3.26 (m, 2H), 2.92 - 2.86 (m, 2H), 2.58 (d, *J* = 16.1 Hz, 2H), 2.35 - 2.30 (m, 2H), 1.95 (s, 3H). LCMS (ESI) calcd. for C₃₃H₃₄F₂N₅O₃ ⁺ [M+H]⁺: 586.26, found, 586.3.

### Example 393: Preparation of compound GT-10617

With reference to the method of Scheme 2, the target compound GT-10617 was obtained (yellow solid, 10 mg, yield 14 %). ¹H NMR (400 MHz, DMSO-d6) δ 12.87 (s, 1H), 10.93 (s, 2H), 7.75 (d, *J* = 9.9 Hz, 2H), 7.62 (d, *J* = 8.0 Hz, 2H), 7.54 - 7.48 (m, 3H), 7.25 (t, *J* = 7.5 Hz, 3H), 6.86 (s, 1H), 6.13 - 5.92 (m, 1H), 5.09 - 4.91 (m, 2H), 4.30 (d, *J* = 8.1 Hz, 1H), 4.19 (d, *J* = 8.4 Hz, 1H), 3.91 - 3.78 (m, 4H), 3.38 - 3.30 (m, 3H), 2.94 - 2.87 (m, 2H), 2.56 (s, 2H), 2.37 - 2.30 (m, 2H), 1.97 - 1.90 (m, 3H). LCMS (ESI) calcd. for C₃₃H₃₄F₂N₅O₃ ⁺ [M+H]⁺: 586.26, found, 586.3.

### Example 394: Preparation of compound GT-10618

With reference to the method of Scheme 2, the target compound GT-10618 was obtained (white solid, 5 mg, yield 7 %). ¹H NMR (400 MHz, DMSO-d6) δ 12.70 (s, 1H), 10.93 (s, 1H), 7.87 - 7.76 (m, 4H), 7.43 (d, *J* = 8.4 Hz, 1H), 7.29 (t, *J* = 8.7 Hz, 4H), 6.97 - 6.81 (m, 2H), 5.95 (d, *J* = 10.4 Hz, 1H), 5.02 (dd, *J* = 13.1, 4.9 Hz, 1H), 4.29 (d, *J* = 16.8 Hz, 1H), 4.15 (d, *J* = 16.8 Hz, 1H), 3.81 (s, 4H), 3.72 - 3.61 (m, 2H), 3.39 - 3.28 (m, 2H), 2.93 - 2.85 (m, 1H), 2.75 - 2.56 (m, 3H), 2.39 - 2.27 (m, 2H), 2.00 - 1.86 (m, 3H). LCMS (ESI) calcd. for C₃₃H₃₄F₂N₅O₃ ⁺ [M+H]⁺: 586.26, found, 586.3.

### Example 395: Preparation of compound GT-10619

With reference to the method of Scheme 2, the target compound GT-10619 was obtained (yellow solid, 14 mg, yield 18 %). ¹H NMR (400 MHz, DMSO-d6) δ 12.85 (s, 1H), 10.93 (s, 1H), 7.79 (d, *J* = 7.9 Hz, 4H), 7.52 (d, *J* = 7.4 Hz, 5H), 7.44 (d, *J* = 8.1 Hz, 1H), 6.98 - 6.86 (m, 2H), 5.99 (d, *J* = 10.0 Hz, 1H), 5.06 - 5.01 (m, 1H), 4.30 (d, 1H), 4.16 (d, *J* = 16.8 Hz, 1H), 3.84 (s, 5H), 3.46 - 3.33 (m, 2H), 2.94 - 2.86 (m, 2H), 2.56 (d, 2H), 2.38 - 2.29 (m, 2H), 1.94 (s, 4H). LCMS (ESI) calcd. for C₃₃H₃₄Cl₂N₅O₃ ⁺ [M+H]⁺: 618.20, found, 618.3.

### Example 396: Preparation of compound GT-10620

With reference to the method of Scheme 2, the target compound GT-10620 was obtained (red solid, 26 mg, yield 36 %). ¹H NMR (400 MHz, DMSO-d6) δ 11.85 (s, 1H), 10.95 (s, 1H), 8.69 (d, *J* = 8.3 Hz, 1H), 7.89 (t, *J* = 7.7, 1.7 Hz, 1H), 7.64 (d, *J* = 6.4 Hz, 2H), 7.57 - 7.50 (m, 2H), 7.47 - 7.42 (m, 4H), 7.28 - 7.15 (m, 2H), 6.16 (s, 1H), 5.05 (dd, *J* = 13.3, 5.0 Hz, 1H), 4.05 - 3.81 (m, 6H), 3.29 - 3.01 (m, 3H), 3.00 - 2.84 (m, 2H), 2.59 (d, *J* = 17.5 Hz, 1H), 2.39 - 2.33 (m, 1H), 2.27 - 2.05 (m, 3H), 2.03 - 1.82 (m, 2H). LCMS (ESI) calcd. for C₃₂H₃₅N₆O₃ ⁺ [M+H]⁺: 551.28, found, 551.3.

### Example 397: Preparation of compound GT-10621

With reference to the method of Scheme 2, the target compound GT-10621 was obtained (red solid, 8 mg, yield 12 %). ¹H NMR (400 MHz, DMSO-d6) δ 10.94 (s, 2H), 8.67 - 8.63 (m, 2H), 7.96 - 7.90 (m, 2H), 7.70 (d, 2H), 7.52 - 7.42 (m, 4H), 7.12 - 6.99 (m, 2H), 5.95 (s, 1H), 5.04 (dd, *J* = 13.3, 5.0 Hz, 2H), 4.33 (d, 2H), 4.19 (d, 2H), 3.10 (s, 1H), 2.94 - 2.86 (m, 3H), 2.59 (d, *J* = 16.3 Hz, 2H), 2.40 - 2.33 (m, 2H), 1.99 - 1.83 (m, 5H). LCMS (ESI) calcd. for C₃₂H₃₅N₆O₃ ⁺ [M+H]⁺: 551.28, found, 551.3.

### Example 398: Preparation of compound GT-10622

With reference to the method of Scheme 2, the target compound GT-10622 was obtained (yellow solid, 10 mg, yield 14 %). ¹H NMR (400 MHz, DMSO-d6) δ 13.18 (s, 1H), 10.94 (s, 1H), 9.16 (s, 1H), 8.77 (d, *J* = 4.1 Hz, 1H), 8.66 (d, *J* = 8.0 Hz, 1H), 7.97 - 7.80 (m, 3H), 7.51 - 7.40 (m, 4H), 7.09 - 6.77 (m, 2H), 6.20 (s, 1H), 5.03 (dd, *J* = 13.1, 5.1 Hz, 1H), 4.31 (d, 1H), 4.17 (d, *J* = 15.6 Hz, 1H), 3.66 - 3.50 (m, 2H), 3.37 (d, 1H), 3.19 - 3.04 (m, 1H), 2.91 - 2.88 (m, 2H), 2.85 - 2.67 (m, 3H), 2.58 (d, *J* = 17.8 Hz, 2H), 2.37 - 2.31 (m, 1H), 2.24 - 1.89 (m, 4H). LCMS (ESI) calcd. for C₃₁H₃₄N₇O₃ ⁺ [M+H]⁺: 552.27, found, 552.3.

### Example 399: Preparation of compound GT-09104

With reference to the method of Scheme 2, the target compound GT-09104 was obtained (white solid, 17 mg, yield 21 %). ¹H NMR (400 MHz, DMSO) δ 10.94 (s, 2H), 8.77 (s, 2H), 7.65 (s, 1H), 7.46 (d, *J* = 8.3 Hz, 1H), 6.94 (s, 1H), 6.88 (d, *J* = 8.4 Hz, 1H), 5.03 (dd, *J* = 13.3, 5.1 Hz, 1H), 4.24 (dd, *J* = 53.7, 16.9 Hz, 2H), 3.49 (d, *J* = 11.7 Hz, 2H), 3.25 - 3.14 (m, 2H), 3.05 (s, 6H), 2.95 - 2.87 (m, 1H), 2.82 (s, 2H), 2.59 (d, *J* = 17.4 Hz, 1H), 2.41 - 2.28 (m, 1H), 2.11 (s, 3H), 1.97 - 1.92 (m, 1H), 1.88 (s, 6H), 1.78 - 1.54 (m, 10H), 1.33 (s, 6H). LCMS (ESI) calcd. for C₃₄H₅₁N₆O₃⁺ [M+H]⁺: 591.40, found, 591.4.

### Example 400: Preparation of compound GT-09105

With reference to the method of Scheme 2, the target compound GT-09105 was obtained (white solid, 7 mg, yield 9 %). ¹H NMR (400 MHz, DMSO) δ 10.96 (s, 1H), 10.67 (s, 1H), 8.76 (s, 2H), 7.78 (s, 1H), 7.42 (d, *J* = 8.2 Hz, 1H), 7.31 (t, *J* = 7.7 Hz, 1H), 5.04 (dd, *J* = 13.3, 5.1 Hz, 1H), 4.46 (d, *J* = 16.8 Hz, 1H), 4.28 (d, *J* = 16.7 Hz, 1H), 3.51 (d, *J* = 10.2 Hz, 2H), 3.28 - 3.14 (m, 3H), 3.13 - 3.04 (m, 5H), 2.93 - 2.77 (m, 3H), 2.59 (d, *J* = 17.0 Hz, 1H), 2.45 - 2.34 (m, 1H), 2.11 (s, 3H), 1.99 - 1.93 (m, 1H), 1.88 (s, 6H), 1.71 (d, *J* = 11.1 Hz, 2H), 1.70 - 1.57 (m, 8H), 1.33 (s, 6H). LCMS (ESI) calcd. for C₃₄H₅₀FN₆O₃⁺ [M+H]⁺: 609.39, found, 609.5.

### Example 401: Preparation of compound GT-09106

With reference to the method of Scheme 2, the target compound GT-09106 was obtained (white solid, 20 mg, yield 25 %). ¹H NMR (400 MHz, DMSO) δ 10.96 (s, 1H), 10.69 (s, 1H), 8.80 (s, 2H), 7.78 (s, 1H), 7.36 (d, *J* = 10.6 Hz, 1H), 7.28 (d, *J* = 7.1 Hz, 1H), 5.05 (dd, *J* = 13.2, 5.0 Hz, 1H), 4.32 (d, *J* = 17.0 Hz, 1H), 4.19 (d, *J* = 16.8 Hz, 1H), 3.50 (d, *J* = 9.2 Hz, 2H), 3.28 - 3.14 (m, 3H), 3.13 - 3.01 (m, 5H), 2.95 - 2.86 (m, 1H), 2.86 - 2.76 (m, 2H), 2.59 (d, *J* = 17.5 Hz, 1H), 2.41 - 2.29 (m, 1H), 2.11 (s, 3H), 1.99 - 1.93 (m, 1H), 1.88 (s, 6H), 1.72 (s, 2H), 1.69 - 1.55 (m, 8H), 1.33 (s, 6H). LCMS (ESI) calcd. for C₃₄H₅₀FN₆O₃⁺ [M+H]⁺: 609.39, found, 609.5.

### Example 402: Preparation of compound GT-09107

With reference to the method of Scheme 2, the target compound GT-09107 was obtained (white solid, 19 mg, yield 23 %). ¹H NMR (400 MHz, DMSO) δ 10.95 (s, 2H), 8.77 (s, 2H), 8.13 (s, 1H), 6.70 (s, 1H), 6.62 (d, *J* = 12.4 Hz, 1H), 4.99 (dd, *J* = 13.3, 5.1 Hz, 1H), 4.31 (d, *J* = 17.3 Hz, 1H), 4.17 (d, *J* = 17.3 Hz, 1H), 3.47 (d, 2H), 3.21 (s, 3H), 3.07 (s, 7H), 3.00 - 2.74 (m, 4H), 2.58 (d, *J* = 16.8 Hz, 1H), 2.37 - 2.27 (m, 1H), 2.11 (s, 3H), 1.97 - 1.89 (m, 2H), 1.73 (s, 2H), 1.71 - 1.54 (m, 10H), 1.33 (s, 6H). LCMS (ESI) calcd. for C₃₄H₅₀FN₆O₃⁺ [M+H]⁺: 609.39, found, 609.4.

### Example 403: Preparation of compound GT-09108

With reference to the method of Scheme 2, the target compound GT-09108 was obtained (white solid, 23 mg, yield 29 %). ¹H NMR (400 MHz, DMSO) δ 11.00 (s, 1H), 10.97 (s, 1H), 8.76 (s, 2H), 7.29 (d, *J* = 7.7 Hz, 1H), 7.12 - 7.00 (m, 2H), 5.10 (dd, *J* = 13.3, 5.0 Hz, 1H), 4.36 (d, *J* = 17.5 Hz, 1H), 4.23 (d, *J* = 17.5 Hz, 1H), 3.50 (s, 2H), 3.24 - 3.10 (m, 3H), 3.10 - 3.02 (m, 5H), 2.97 - 2.88 (m, 1H), 2.81 (s, 2H), 2.62 (d, *J* = 17.1 Hz, 1H), 2.44 - 2.31 (m, 1H), 2.09 (d, *J* = 19.4 Hz, 3H), 2.06 - 1.97 (m, 1H), 1.88 (s, 6H), 1.74 (s, 2H), 1.69 - 1.57 (m, 8H), 1.33 (s, 6H). LCMS (ESI) calcd. for C₃₄H₅₁N₆O₃⁺ [M+H]⁺: 591.40, found, 591.4.

### Example 404: Preparation of compound GT-09109

With reference to the method of Scheme 2, the target compound GT-09109 was obtained (white solid, 20 mg, yield 24 %). ¹H NMR (400 MHz, DMSO) δ 10.94 (s, 1H), 10.87 (s, 1H), 8.79 (s, 2H), 7.44 (d, *J* = 8.4 Hz, 1H), 7.07 - 6.85 (m, 2H), 5.03 (dd, *J* = 13.2, 5.0 Hz, 1H), 4.31 (d, *J* = 16.8 Hz, 1H), 4.18 (d, *J* = 16.8 Hz, 1H), 3.77 (s, 2H), 3.36 (s, 1H), 3.20 (d, *J* = 4.9 Hz, 1H), 3.06 (s, 1H), 3.01 - 2.73 (m, 8H), 2.70 - 2.53 (m, 3H), 2.39 - 2.26 (m, 1H), 2.11 (s, 3H), 2.00 - 1.93 (m, 1H), 1.89 (s, 6H), 1.76 - 1.54 (m, 10H), 1.34 (s, 6H). LCMS (ESI) calcd. for C₃₆H₅₃N₆O₃⁺ [M+H]⁺: 617.42, found, 617.5.

### Example 405: Preparation of compound GT-09110

With reference to the method of Scheme 2, the target compound GT-09110 was obtained (white solid, 24 mg, yield 29 %). ¹H NMR (400 MHz, DMSO) δ 10.94 (s, 1H), 10.75 (s, 1H), 8.79 (s, 2H), 7.63 (s, 1H), 7.46 (d, *J* = 8.3 Hz, 1H), 6.97 (s, 1H), 6.90 (d, *J* = 8.8 Hz, 1H), 5.03 (dd, *J* = 13.2, 5.0 Hz, 1H), 4.31 (d, *J* = 16.9 Hz, 1H), 4.18 (d, *J* = 16.9 Hz, 1H), 4.04 (s, 1H), 3.26 (d, *J* = 11.4 Hz, 2H), 2.97 - 2.91 (m, 4H), 2.82 (s, 3H), 2.59 (d, *J* = 16.4 Hz, 1H), 2.35 - 2.28 (m, 1H), 2.18 (s, 4H), 2.11 (s, 4H), 1.99 - 1.94 (m, 1H), 1.89 (s, 6H), 1.77 (s, 2H), 1.67 - 1.58 (m, 8H), 1.34 (s, 6H). LCMS (ESI) calcd. for C₃₆H₅₃N₆O₃⁺ [M+H]⁺: 617.42, found, 617.5.

### Example 406: Preparation of compound GT-09111

With reference to the method of Scheme 2, the target compound GT-09111 was obtained (white solid, 13 mg, yield 16 %). ¹H NMR (400 MHz, DMSO) δ 11.29 (s, 1H), 10.94 (s, 1H), 8.81 (s, 2H), 7.47 (d, *J* = 8.3 Hz, 1H), 7.11 - 6.91 (m, 2H), 5.03 (dd, *J* = 13.2, 5.1 Hz, 1H), 4.32 (d, 1H), 4.18 (d, *J* = 17.0 Hz, 1H), 3.90 (s, 2H), 3.84 - 3.78 (m, 4H), 3.13 (d, *J* = 7.7 Hz, 2H), 2.93 - 2.84 (m, 2H), 2.81 (s, 3H), 2.59 (d, *J* = 17.0 Hz, 2H), 2.38 - 2.31 (m, 1H), 2.11 (s, 3H), 1.97 - 1.93 (m, 2H), 1.89 (s, 6H), 1.70 - 1.54 (m, 10H), 1.52 (s, 2H), 1.31 (s, 6H). LCMS (ESI) calcd. for C₃₇H₅₅N₆O₃⁺ [M+H]⁺: 631.43, found, 631.5.

### Example 407: Preparation of compound GT-09112

With reference to the method of Scheme 2, the target compound GT-09112 was obtained (white solid, 17 mg, yield 21 %). ¹H NMR (400 MHz, DMSO) δ 10.94 (s, 1H), 10.85 (s, 1H), 8.74 (s, 2H), 7.70 (s, 1H), 7.45 (d, *J* = 8.4 Hz, 1H), 6.96 (s, 1H), 6.88 (d, *J* = 8.2 Hz, 1H), 5.03 (dd, *J* = 13.2, 5.1 Hz, 1H), 4.30 (d, *J* = 16.7 Hz, 1H), 4.17 (d, *J* = 16.9 Hz, 1H), 3.54 (s, 2H), 3.28 (d, *J* = 13.5 Hz, 4H), 3.15 - 3.06 (m, 3H), 3.03 - 2.73 (m, 5H), 2.58 (d, *J* = 17.6 Hz, 1H), 2.38 - 2.28 (m, 1H), 2.11 (s, 3H), 2.04 (s, 1H), 1.98 - 1.92 (m, 1H), 1.88 (s, 6H), 1.73 (s, 2H), 1.68 - 1.55 (m, 8H), 1.33 (s, 6H). LCMS (ESI) calcd. for C₃₅H₅₃N₆O₃⁺ [M+H]⁺: 605.42, found, 605.5.

### Example 408: Preparation of compound GT-09113

With reference to the method of Scheme 2, the target compound GT-09113 was obtained (white solid, 15 mg, yield 19 %). ¹H NMR (400 MHz, DMSO) δ 10.95 (s, 1H), 9.57 (s, 1H), 9.00 - 8.58 (m, 3H), 7.49 - 7.35 (m, 1H), 7.11 - 6.78 (m, 2H), 5.04 (d, 1H), 4.38 - 4.17 (m, 3H), 2.99 - 2.89 (m, 2H), 2.87 - 2.75 (m, 4H), 2.58 (d, *J* = 16.6 Hz, 1H), 2.34 (d, *J* = 10.4 Hz, 1H), 2.10 (s, 3H), 1.98 - 1.93 (m, 1H), 1.88 (s, 8H), 1.69 - 1.56 (m, 12H), 1.34 (d, *J* = 6.8 Hz, 8H). LCMS (ESI) calcd. for C₃₅H₅₁N₆O₃⁺ [M+H]⁺: 603.40, found, 603.5.

### Example 409: Preparation of compound GT-09114

With reference to the method of Scheme 2, the target compound GT-09114 was obtained (white solid, 22 mg, yield 27 %). ¹H NMR (400 MHz, DMSO) δ 11.25 (s, 1H), 10.94 (s, 1H), 8.79 (s, 2H), 8.14 (s, 1H), 7.45 (dd, *J* = 8.3, 3.6 Hz, 1H), 7.04 - 6.81 (m, 2H), 5.03 (dd, *J* = 13.2, 5.0 Hz, 1H), 4.24 (dd, *J* = 53.9, 16.8 Hz, 3H), 3.53 - 3.42 (m, 2H), 3.23 - 2.99 (m, 3H), 2.96 - 2.72 (m, 4H), 2.59 (d, *J* = 16.6 Hz, 1H), 2.44 - 2.26 (m, 2H), 2.19 (s, 1H), 2.11 (s, 3H), 1.98 - 1.92 (m, 1H), 1.89 (s, 6H), 1.72 (s, 1H), 1.72 - 1.55 (m, 10H), 1.35 (s, 6H). LCMS (ESI) calcd. for C₃₅H₅₁N₆O₃⁺ [M+H]⁺: 603.40, found, 603.5.

### Example 410: Preparation of compound GT-09115

With reference to the method of Scheme 2, the target compound GT-09115 was obtained (white solid, 22 mg, yield 27 %). ¹H NMR (400 MHz, DMSO) δ 10.95 (d, *J* = 7.5 Hz, 1H), 10.15 (s, 1H), 8.75 (s, 2H), 7.45 (d, *J* = 8.4 Hz, 1H), 7.10 (s, 1H), 7.05 (d, *J* = 5.8 Hz, 1H), 5.03 (dd, *J* = 13.3, 5.0 Hz, 1H), 4.30 (d, *J* = 16.8 Hz, 1H), 4.17 (d, *J* = 16.9 Hz, 1H), 3.46 - 3.22 (m, 7H), 3.03 (s, 1H), 2.91 - 2.76 (m, 3H), 2.59 (d, *J* = 17.1 Hz, 1H), 2.40 - 2.33 (m, 1H), 2.17 (s, 3H), 2.11 (s, 4H), 1.98 - 1.92 (m, 1H), 1.88 (s, 6H), 1.75 (s, 2H), 1.69 - 1.57 (m, 8H), 1.31 (d, *J* = 17.3 Hz, 6H). LCMS (ESI) calcd. for C₃₆H₅₃N₆O₃⁺ [M+H]⁺: 617.42, found, 617.5.

### Example 411: Preparation of compound GT-09116

With reference to the method of Scheme 2, the target compound GT-09116 was obtained (yellow solid, 30 mg, yield 36.28 %). ¹H NMR (400 MHz, DMSO) δ 11.07 (s, 1H), 10.98 (s, 1H), 8.75 (s, 2H), 8.57 (d, *J* = 46.0 Hz, 1H), 7.64 (d, *J* = 8.3 Hz, 1H), 7.24 (s, 1H), 7.06 (s, 1H), 5.05 (dd, *J* = 12.8, 5.4 Hz, 1H), 3.51 (d, *J* = 10.3 Hz, 2H), 3.25 (s, 2H), 3.09 (s, 6H), 2.88 - 2.75 (m, 3H), 2.56 (d, 1H), 2.11 (s, 3H), 2.04 - 1.97 (m, 1H), 1.88 (s, 6H), 1.74 (s, 2H), 1.63 (dd, *J* = 29.4, 12.4 Hz, 9H), 1.33 (s, 6H). LCMS (ESI) calcd. for C₃₄H₄₉N₆O₄⁺ [M+H]⁺: 605.38, found, 605.4.

### Example 412: Preparation of compound GT-09117

With reference to the method of Scheme 2, the target compound GT-09117 was obtained (yellow solid, 23 mg, yield 28 %). ¹H NMR (400 MHz, DMSO) δ 11.09 (s, 1H), 10.81 (s, 1H), 8.81 (s, 2H), 8.50 (s, 1H), 7.64 (d, *J* = 10.2 Hz, 1H), 7.56 (d, *J* = 7.0 Hz, 1H), 5.07 (dd, *J* = 12.8, 5.4 Hz, 1H), 3.51 (d, *J* = 10.0 Hz, 2H), 3.28 - 3.15 (m, 4H), 3.09 (d, *J* = 10.1 Hz, 4H), 2.92 - 2.78 (m, 3H), 2.59 (d, *J* = 18.6 Hz, 1H), 2.11 (s, 3H), 2.06 - 1.98 (m, 1H), 1.88 (d, *J* = 7.8 Hz, 6H), 1.73 (s, 2H), 1.69 - 1.56 (m, 9H), 1.33 (s, 6H). LCMS (ESI) calcd. for C₃₄H₄₈FN₆O₄⁺ [M+H]⁺: 623.37, found, 623.4.

### Biological activity assay

### Reagents and Materials

| Reagents and materials | Suppliers or Source |
|---|---|
| Human multiple myeloma cell line: MM.1S | ATCC (American Type Culture Collection) |
| Human B lymphocyte leukemia cell line: RPMI-8226 | ATCC |
| Human diffuse large B-cell lymphoma cell line: TMD8 | Kyinno Biotechnology Co., Ltd |
| Human myelomonocytic leukemia cell line: MV-4-11 | ATCC |
| Human small cell lung cancer cell line: H196 | ATCC |
| human pancreatic cancer cell line: CFPAC-1 | Dalian Meilun Biotech Co., Ltd. |
| human malignant melanoma cell: A375 | Dalian Meilun Biotech Co., Ltd. |
| RPMI-1640 Medium | ATCC |
| IMDM Medium | Gibco company |
| DMEM Medium | Dalian Meilun Biotech Co., Ltd. |
| CellTiter-Meiluncell Luminescent Cell Viability Assay Kit | Dalian Meilun Biotech Co., Ltd. |
| Fetal bovine serum (FBS) | Sunrise Science Products company |
| Penicillin-Streptomycin | Beijing TransGen Biotech Co., Ltd. |
| Mycoplasma detection kit | Beijing TransGen Biotech Co., Ltd. |
| STR genotype detection | Shanghai Biowing Applied Biotechnology Co. Ltd. |

### Methods:

### Cell cultures

The tumor cells used in the present disclosure were cultured in the cell culture media listed in Table A at 37 °C in an incubator containing 5% CO₂. All cells were authenticated as the correct cell lines by STR profiling and confirmed to be mycoplasma-negative using a mycoplasma detection kit prior to the experiments.

**Table A. Tumor cells and cell culture medium used in the present disclosure**

| Cell line | Cell culture medium used |
|---|---|
| MM.1S | RPMI 1640 culture medium supplemented with 10% FBS, as well as penicillin at a final concentration of 100 U/mL and streptomycin at a final concentration of 100 µg/mL |
| RPMI-8226 | |
| H196 | |
| TMD8 | |
| MV-4-11 | IMDM culture medium supplemented with 10% FBS, as well as penicillin at a final concentration of 100 U/mL and streptomycin at a final concentration of 100 µg/mL |
| CFPAC-1 | |
| A375 | DMEM high-glucose medium supplemented with 10% FBS, as well as penicillin at a final concentration of 100 U/mL and streptomycin at a final concentration of 100 µg/mL |

### I. Determination of half inhibitory concentration (IC₅₀) of the compounds of the present disclosure against tumor cells:

The IC₅₀ values of the compounds of the present disclosure (including the compounds in Table 1, Table 2, and Examples) were determined using the CellTiter-Meiluncell Luminescent Cell Viability Assay Kit from Dalian Meilun Biotech Co., Ltd. The detailed procedures are as follows: Tumor cells were seeded in 96-well cell culture plates at the densities specified in Table B. On the following day, the test compounds of the present disclosure were subjected to serial dilution. The dilution can be performed according to one of the following two methods:
(1) The first dilution method: the test compounds of the present disclosure (including the compounds in Tables 1 and 2, and Examples) were subjected to 5-fold serial dilutions starting from a highest concentration of 2000 µM, resulting in a total of 9 concentrations from high to low (2000, 400, 80, 16, 3.2, 0.64, 0.128, 0.0256, 0.00512 µM). Subsequently, 0.5 µL of the test compound dilution was transferred into the 96-well plate containing the cells, with each well containing 100 µL of cell culture medium. This resulted in final test compound concentrations of 10, 2, 0.4, 0.08, 0.016, 0.0032, 0.00064, 0.000128, and 0.0000256 µM, respectively, or
(2) As an alternative, a second dilution method can be used: the test compounds of the present disclosure (including the compounds in Tables 1 and 2, and the example compounds) were subjected to a 50-fold gradient dilution starting from the highest concentration of 100 µM, with a total of 2 concentrations (100 µM and 2 µM) being set. In a 96-well plate, the diluted test compounds of the present disclosure and the corresponding detection reagents were added to each well to ensure that the final test concentrations of the test compound in each well reached 500 nM and 10 nM, respectively, so as to evaluate the inhibitory activity of the test compound on tumor cell proliferation at these two concentration levels. The experimental procedure is described below using the diluted test compound at 2 µM as an example (the operating steps for the 100 µM concentration and the gradient concentrations obtained by the first dilution method are similar thereto).

0.5 µL of the above diluted test compounds of the present disclosure (including the compounds in Tables 1 and 2, and the example compounds) were added to 100 µL of seeded cells, resulting in a final compound concentration of 10 nM. After the cells were treated with the compounds of the present disclosure for a certain period of time, a cell viability detection reagent was added to the culture medium according to the manufacturer's instructions of the kit to determine cell viability. The negative control was DMSO, and the reference compounds were the corresponding commercially available immunomodulatory agents (including lenalidomide and pomalidomide), all of which were used to treat the cells in the same manner as the compounds of the present disclosure. The growth inhibition of the cells by the compounds of the present disclosure was plotted using Prism GraphPad software, from which the IC₅₀ values of the compounds of the present disclosure were calculated.

**Table B. Seeding protocol and treatment time for tumor cells**

| Cells | Cells seeding protocol | The times for treating cells with the compounds of the present disclosure (hours) |
|---|---|---|
| MM.1S | cells were seeded at a density of 10,000 cells per well in 100 µL of serum-containing RPMI-1640 medium per well | 96 hours |
| RPMI-8226 | | |
| TMD8 | | |
| A375 | cells were seeded at a density of 4,000 cells per well in 100 µL of serum-containing DMEM high-glucose medium per well | 96 hours |
| H196 | cells were seeded at a density of 1,000 cells per well in 100 µL of serum-containing RPMI-1640 medium per well | 96 hours |
| MV-4-11 | cells were seeded at a density of 10,000 cells per well in 100 µL of serum-containing IMDM medium per well | 96 hours |
| CFPAC-1 | cells were seeded at a density of 3,000 cells per well in 100 µL of serum-containing IMDM medium per well | 96 hours |

The results of tumor cell proliferation inhibition are presented in Tables I-1 to I-8.

**Table I-1: inhibitory activity of the compounds of the present disclosure against the proliferation of human myelomonocytic leukemia cells MV-4-11 (inhibition rate %)**

| Comp. No. /names | Inhibition Rate (%) at 500 nM | Inhibition Rate (%) at 10 nM | Comp. No. /names | Inhibition Rate (%) at 500 nM | Inhibition Rate (%) at 10 nM | Comp. No. /names | Inhibition Rate (%) at 500 nM | Inhibition Rate (%) at 10 nM |
|---|---|---|---|---|---|---|---|---|
| pomalidomide | 1a2 | 1a1 | GT-09988 | 1a8 | 1a6 | GT-10414 | 1a7 | 1a4 |
| lenalidomide | 1a4 | 1a2 | GT-09989 | 1a7 | 1a4 | GT-10459 | 1a10 | 1a10 |
| GT-09107 | 1a7 | 1a5 | GT-10026 | 1a7 | 1a6 | GT-10461 | 1a9 | 1a7 |
| GT-09131 | 1a7 | 1a7 | GT-10028 | 1a9 | 1a7 | GT-10462 | 1a9 | 1a8 |
| GT-09133 | 1a7 | 1a7 | GT-10029 | 1a9 | 1a7 | GT-10465 | 1a8 | 1a7 |
| GT-09134 | 1a4 | 1a9 | GT-10056 | 1a11 | 1a8 | GT-10478 | 1a9 | 1a7 |
| GT-09199 | 1a8 | 1a4 | GT-10057 | 1a11 | 1a10 | GT-10479 | 1a9 | 1a5 |
| GT-09200 | 1a8 | 1a5 | GT-10058 | 1a11 | 1a6 | GT-10482 | 1a7 | 1a6 |
| GT-06467 | 1a8 | 1a5 | GT-10067 | 1a7 | 1a5 | GT-10484 | 1a8 | 1a8 |
| GT-06542 | 1a11 | 1a4 | GT-10068 | 1a7 | 1a6 | GT-10485 | 1a5 | 1a7 |
| GT-06544 | 1a11 | 1a5 | GT-10128 | 1a11 | 1a9 | GT-10487 | 1a9 | 1a8 |
| GT-06547 | 1a9 | 1a4 | GT-10129 | 1a8 | 1a6 | GT-10488 | 1a7 | 1a6 |
| GT-06441 | 1a11 | 1a5 | GT-10138 | 1a7 | 1a6 | GT-10489 | 1a8 | 1a6 |
| GT-06443 | 1a11 | 1a6 | GT-10142 | 1a8 | 1a6 | GT-10490 | 1a9 | 1a6 |
| GT-06444 | 1a11 | 1a8 | GT-10169 | 1a11 | 1a4 | GT-10476 | 1a8 | 1a8 |
| GT-06445 | 1a11 | 1a2 | GT-10211 | 1a9 | 1a8 | GT-10520 | 1a7 | 1a5 |
| GT-09960 | 1a9 | 1a7 | GT-10287 | 1a8 | 1a7 | GT-10521 | 1a7 | 1a6 |
| GT-09961 | 1a7 | 1a5 | GT-10289 | 1a7 | 1a7 | GT-10545 | 1a11 | 1a7 |
| GT-09963 | 1a7 | 1a6 | GT-10290 | 1a9 | 1a7 | GT-10546 | 1a8 | 1a6 |
| GT-09965 | 1a8 | 1a6 | GT-10291 | 1a9 | 1a6 | GT-10563 | 1a10 | 1a10 |
| GT-10078 | 1a7 | 1a4 | GT-10292 | 1a9 | 1a9 | GT-10566 | 1a9 | 1a8 |
| GT-10081 | 1a8 | 1a6 | GT-10293 | 1a7 | 1a6 | GT-10647 | 1a11 | 1a11 |
| GT-10086 | 1a7 | 1a4 | GT-10294 | 1a9 | 1a7 | GT-10650 | 1a11 | 1a11 |
| GT-09973 | 1a12 | 1a11 | GT-10331 | 1a7 | 1a6 | GT-10652 | 1a11 | 1a11 |
| GT-09975 | 1a7 | 1a5 | GT-10332 | 1a8 | 1a7 | GT-10655 | 1a11 | 1a11 |
| GT-09977 | 1a7 | 1a5 | GT-10305 | 1a7 | 1a1 | GT-10616 | 1a6 | 1a7 |
| GT-09978 | 1a11 | 1a11 | GT-10312 | 1a7 | 1a3 | GT-10618 | 1a7 | 1a5 |
| GT-09985 | 1a10 | 1a8 | GT-10314 | 1a8 | 1a8 | GT-10619 | 1a9 | 1a9 |
| GT-09986 | 1a8 | 1a7 | GT-10316 | 1a8 | 1a6 | GT-10621 | 1a8 | 1a7 |

| | | | | | | | | |
|---|---|---|---|---|---|---|---|---|
| Note: in table I-1, the symbols 1a1, 1a2, 1a3, 1a4, 1a5, 1a6, 1a7, 1a8, 1a9, 1a10, 1a11, and 1a12 are defined as follows: -10% < 1a1 < 0, 0 < 1a2 < 5%, 5% ≤ 1a3 < 10%, 10% ≤ 1a4 < 20%, 20% ≤ 1a5 < 30%, 30% ≤ 1a6 < 40%, 40% ≤ 1a7 < 50%, 50% ≤ 1a8 < 60%, 60% ≤ 1a9 < 70%, 70% ≤ 1a10 < 80%, 80% ≤ 1a11 ≤ 100%, 100% < 1a12 ≤ 105%. | | | | | | | | |

**Table I-2: inhibitory activity of the compounds of the present disclosure against the proliferation of human multiple myeloma cells MM.1S (inhibition rate %)**

| Comp. No. /names | Inhibition Rate (%) at 500 nM | Inhibition Rate (%) at 10 nM | Comp. No. /names | Inhibition Rate (%) at 500 nM | Inhibition Rate (%) at 10 nM | Comp. No. /names | Inhibition Rate (%) at 500 nM | Inhibition Rate (%) at 10 nM |
|---|---|---|---|---|---|---|---|---|
| lenalidomide | 1b4 | 1b2 | GT-10246 | 1b4 | 1b5 | GT-10462 | 1b6 | 1b5 |
| GT-06402 | 1b5 | 1b4 | GT-10248 | 1b5 | 1b5 | GT-10463 | 1b6 | 1b5 |
| GT-06902 | 1b5 | 1b4 | GT-10257 | 1b5 | 1b5 | GT-10483 | 1b6 | 1b5 |
| GT-06905 | 1b5 | 1b2 | GT-10169 | 1b6 | 1b2 | GT-10486 | 1b7 | 1b6 |
| GT-06441 | 1b9 | 1b3 | GT-10171 | 1b6 | 1b5 | GT-10487 | 1b7 | 1b5 |
| GT-06443 | 1b9 | 1b9 | GT-10174 | 1b5 | 1b5 | GT-10488 | 1b7 | 1b5 |
| GT-06444 | 1b9 | 1b9 | GT-10183 | 1b5 | 1b2 | GT-10489 | 1b5 | 1b3 |
| GT-06445 | 1b9 | 1b10 | GT-10332 | 1b7 | 1b6 | GT-10490 | 1b6 | 1b3 |
| GT-10083 | 1b6 | 1b5 | GT-10334 | 1b6 | 1b6 | GT-10545 | 1b7 | 1b2 |
| GT-10086 | 1b4 | 1b5 | GT-10296 | 1b5 | 1b3 | GT-10646 | 1b5 | 1b2 |
| GT-09973 | 1b9 | 1b9 | GT-10311 | 1b5 | 1b6 | GT-10647 | 1b6 | 1b5 |
| GT-09978 | 1b9 | 1b9 | GT-10314 | 1b8 | 1b7 | GT-10615 | 1b5 | 1b4 |
| GT-09987 | 1b5 | 1b3 | GT-10316 | 1b7 | 1b5 | GT-10616 | 1b8 | 1b7 |
| GT-10057 | 1b9 | 1b8 | GT-10319 | 1b5 | 1b2 | GT-10619 | 1b7 | 1b7 |
| GT-10058 | 1b9 | 1b5 | GT-10414 | 1b5 | 1b4 | GT-10621 | 1b6 | 1b5 |
| GT-10129 | 1b5 | 1b4 | GT-10415 | 1b6 | 1b6 | GT-08865 | 1b6 | 1b5 |
| GT-10234 | 1b5 | 1b4 | GT-10459 | 1b5 | 1b5 | | | |
| GT-10243 | 1b4 | 1b5 | GT-10461 | 1b6 | 1b5 | | | |

| | | | | | | | | |
|---|---|---|---|---|---|---|---|---|
| Note: in table I-2, the symbols 1b1, 1b2, 1b3, 1b4, 1b5, 1b6, 1b7, 1b8, 1b9, and 1b10 are defined as follows: 0 < 1b1 < 10%, 10% ≤ 1b2 < 20%, 20% ≤ 1b3 < 30%, 30% ≤ 1b4 < 40%, 40% ≤ 1b5 < 50%, 50% ≤ 1b6 < 60%, 60% ≤ 1b7 < 70%, 70% ≤ 1b8 < 80%, 80% ≤ 1b9 ≤ 100%, 100% < 1b10 ≤ 105%. | | | | | | | | |

**Table I-3: inhibitory activity of the compounds of the present disclosure against the proliferation of human small cell lung cancer cells H196 (inhibition rate %)**

| Comp. No. /names | Inhibition Rate (%) at 500 nM | Inhibition Rate (%) at 10 nM | Comp. No. /names | Inhibition Rate (%) at 500 nM | Inhibition Rate (%) at 10 nM | Comp. No. /names | Inhibition Rate (%) at 500 nM | Inhibition Rate (%) at 10 nM |
|---|---|---|---|---|---|---|---|---|
| pomalidomide | 1c2 | 1c2 | GT-10235 | 1c5 | 1c4 | GT-10300 | 1c5 | 1c5 |
| lenalidomide | 1c2 | 1c3 | GT-10236 | 1c4 | 1c5 | GT-10302 | 1c4 | 1c5 |
| GT-06441 | 1c8 | 1c1 | GT-10237 | 1c6 | 1c7 | GT-10305 | 1c4 | 1c5 |
| GT-06443 | 1c8 | 1c1 | GT-10238 | 1c5 | 1c6 | GT-10307 | 1c3 | 1c5 |
| GT-06444 | 1c9 | 1c1 | GT-10239 | 1c5 | 1c4 | GT-10308 | 1c6 | 1c6 |
| GT-06445 | 1c9 | 1c6 | GT-10240 | 1c6 | 1c6 | GT-10309 | 1c5 | 1c4 |
| GT-09962 | 1c5 | 1c4 | GT-10243 | 1c5 | 1c6 | GT-10310 | 1c5 | 1c3 |
| GT-10083 | 1c6 | 1c6 | GT-10246 | 1c7 | 1c6 | GT-10311 | 1c7 | 1c6 |
| GT-10086 | 1c6 | 1c5 | GT-10248 | 1c6 | 1c7 | GT-10314 | 1c7 | 1c7 |
| GT-10088 | 1c5 | 1c5 | GT-10251 | 1c6 | 1c6 | GT-10316 | 1c6 | 1c6 |
| GT-10091 | 1c4 | 1c5 | GT-10254 | 1c6 | 1c4 | GT-10319 | 1c6 | 1c6 |
| GT-10092 | 1c5 | 1c5 | GT-10257 | 1c5 | 1c4 | GT-10320 | 1c4 | 1c5 |
| GT-09973 | 1c9 | 1c9 | GT-10166 | 1c7 | 1c6 | GT-10321 | 1c6 | 1c5 |
| GT-09976 | 1c6 | 1c6 | GT-10168 | 1c4 | 1c5 | GT-10431 | 1c6 | 1c5 |
| GT-09978 | 1c9 | 1c7 | GT-10170 | 1c5 | 1c4 | GT-10409 | 1c6 | 1c6 |
| GT-09981 | 1c5 | 1c3 | GT-10171 | 1c6 | 1c4 | GT-10412 | 1c6 | 1c3 |
| GT-10029 | 1c6 | 1c6 | GT-10177 | 1c5 | 1c5 | GT-10413 | 1c7 | 1c4 |
| GT-10057 | 1c7 | 1c2 | GT-10180 | 1c7 | 1c7 | GT-10414 | 1c5 | 1c4 |
| GT-10058 | 1c7 | 1c5 | GT-10183 | 1c5 | 1c2 | GT-10415 | 1c6 | 1c6 |
| GT-10067 | 1c5 | 1c2 | GT-10207 | 1c5 | 1c2 | GT-10459 | 1c5 | 1c5 |
| GT-10068 | 1c5 | 1c3 | GT-10208 | 1c7 | 1c6 | GT-10460 | 1c5 | 1c3 |
| GT-10069 | 1c6 | 1c6 | GT-10211 | 1c6 | 1c6 | GT-10461 | 1c7 | 1c6 |
| GT-10132 | 1c3 | 1c5 | GT-10212 | 1c5 | 1c6 | GT-10462 | 1c5 | 1c4 |
| GT-10135 | 1c6 | 1c4 | GT-10289 | 1c6 | 1c5 | GT-10464 | 1c5 | 1c6 |
| GT-10136 | 1c6 | 1c5 | GT-10331 | 1c6 | 1c3 | GT-10478 | 1c6 | 1c6 |
| GT-10137 | 1c7 | 1c7 | GT-10332 | 1c8 | 1c7 | GT-10480 | 1c5 | 1c1 |
| GT-10138 | 1c5 | 1c4 | GT-10334 | 1c7 | 1c7 | GT-10487 | 1c6 | 1c6 |
| GT-10142 | 1c4 | 1c5 | GT-10336 | 1c5 | 1c3 | GT-10490 | 1c7 | 1c6 |
| GT-10145 | 1c5 | 1c5 | GT-10337 | 1c7 | 1c7 | GT-10491 | 1c7 | 1c6 |
| GT-10146 | 1c5 | 1c6 | GT-10339 | 1c3 | 1c5 | GT-10492 | 1c6 | 1c5 |
| GT-10149 | 1c5 | 1c2 | GT-10340 | 1c5 | 1c6 | GT-10493 | 1c5 | 1c5 |
| GT-10232 | 1c7 | 1c6 | GT-10296 | 1c5 | 1c4 | | | |
| GT-10234 | 1c6 | 1c6 | GT-10297 | 1c5 | 1c5 | | | |

| | | | | | | | | |
|---|---|---|---|---|---|---|---|---|
| Note: in table I-3, the symbols 1c1, 1c2, 1c3, 1c4, 1c5, 1c6, 1c7, 1c8, 1c9, and 1c10 are defined as follows: 0 < 1c1 < 10%, 10% ≤ 1c2 < 20%, 20% ≤ 1c3 < 30%, 30% ≤ 1c4 < 40%, 40% ≤ 1c5 < 50%, 50% ≤ 1c6 < 60%, 60% ≤ 1c7 < 70%, 70% ≤ 1c8 < 80%, 80% ≤ 1c9 ≤ 100%, 100% < 1c10 ≤ 105%. | | | | | | | | |

**Table I-4: inhibitory activity of the compounds of the present disclosure against the proliferation of human pancreatic cancer cells CFPAC-1 (inhibition rate %)**

| Comp. No. /names | Inhibition Rate (%) at 500 nM | Inhibition Rate (%) at 10 nM | Comp. No. /names | Inhibition Rate (%) at 500 nM | Inhibition Rate (%) at 10 nM | Comp. No. /names | Inhibition Rate (%) at 500 nM | Inhibition Rate (%) at 10 nM |
|---|---|---|---|---|---|---|---|---|
| pomalidomide | 1d1 | 1d1 | GT-10080 | 1d6 | 1d2 | GT-10168 | 1d8 | 1d9 |
| lenalidomide | 1d1 | 1d1 | GT-10081 | 1d6 | 1d3 | GT-10172 | 1d8 | 1d6 |
| GT-06398 | 1d6 | 1d2 | GT-10082 | 1d6 | 1d3 | GT-10330 | 1d6 | 1d5 |
| GT-06399 | 1d6 | 1d1 | GT-10083 | 1d5 | 1d1 | GT-10337 | 1d6 | 1d5 |
| GT-06400 | 1d5 | 1d2 | GT-10088 | 1d5 | 1d4 | GT-10339 | 1d7 | 1d7 |
| GT-06420 | 1d9 | 1d3 | GT-10089 | 1d5 | 1d4 | GT-10340 | 1d6 | 1d6 |
| GT-06421 | 1d5 | 1d1 | GT-10092 | 1d7 | 1d3 | GT-10297 | 1d7 | 1d4 |
| GT-06426 | 1d7 | 1d2 | GT-09973 | 1d10 | 1d9 | GT-10298 | 1d5 | 1d2 |
| GT-06427 | 1d7 | 1d2 | GT-09975 | 1d9 | 1d8 | GT-10299 | 1d5 | 1d4 |
| GT-06428 | 1d6 | 1d4 | GT-09976 | 1d8 | 1d3 | GT-10300 | 1d5 | 1d4 |
| GT-06962 | 1d9 | 1d4 | GT-09978 | 1d9 | 1d9 | GT-10301 | 1d6 | 1d5 |
| GT-06467 | 1d9 | 1d3 | GT-10057 | 1d8 | 1d3 | GT-10302 | 1d6 | 1d5 |
| GT-06543 | 1d6 | 1d2 | GT-10058 | 1d9 | 1d3 | GT-10303 | 1d5 | 1d2 |
| GT-06441 | 1d9 | 1d4 | GT-10135 | 1d5 | 1d3 | GT-10310 | 1d5 | 1d2 |
| GT-06443 | 1d9 | 1d9 | GT-10136 | 1d8 | 1d7 | GT-10312 | 1d5 | 1d4 |
| GT-06444 | 1d9 | 1d9 | GT-10137 | 1d7 | 1d4 | GT-10316 | 1d7 | 1d5 |
| GT-06445 | 1d9 | 1d9 | GT-10138 | 1d6 | 1d3 | GT-10413 | 1d9 | 1d7 |
| GT-09960 | 1d5 | 1d4 | GT-10139 | 1d6 | 1d2 | GT-10483 | 1d9 | 1d6 |
| GT-09964 | 1d8 | 1d4 | GT-10232 | 1d7 | 1d3 | GT-10548 | 1d5 | 1d1 |
| GT-09965 | 1d5 | 1d5 | GT-10235 | 1d7 | 1d5 | GT-10652 | 1d5 | 1d4 |
| GT-10076 | 1d5 | 1d3 | GT-10238 | 1d7 | 1d4 | GT-10654 | 1d6 | 1d6 |
| GT-10078 | 1d5 | 1d5 | GT-10239 | 1d5 | 1d3 | | | |

| | | | | | | | | |
|---|---|---|---|---|---|---|---|---|
| Note: in table I-4, the symbols 1d1, 1d2, 1d3, 1d4, 1d5, 1d6, 1d7, 1d8, 1d9, and 1d10 are defined as follows: 0 < 1d1 < 10%, 10% ≤ 1d2 < 20%, 20% ≤ 1d3 < 30%, 30% ≤ 1d4 < 40%, 40% ≤ 1d5 < 50%, 50% ≤ 1d6 < 60%, 60% ≤ 1d7 < 70%, 70% ≤ 1d8 < 80%, 80% ≤ 1d9 ≤ 100%, 100% < 1d10 ≤ 105%. | | | | | | | | |

**Table I-5: inhibitory activity of the compounds of the present disclosure against the proliferation of human malignant melanoma cells A375 (inhibition rate %)**

| Comp. No. /names | Inhibition Rate (%) at 500 nM | Inhibition Rate (%) at 10 nM | Comp. No. /names | Inhibition Rate (%) at 500 nM | Inhibition Rate (%) at 10 nM | Comp. No. /names | Inhibition Rate (%) at 500 nM | Inhibition Rate (%) at 10 nM |
|---|---|---|---|---|---|---|---|---|
| pomalidomide | 1e1 | 1e1 | GT-10093 | 1e5 | 1e3 | GT-10339 | 1e8 | 1e8 |
| lenalidomide | 1e1 | 1e2 | GT-09971 | 1e5 | 1e2 | GT-10340 | 1e7 | 1e8 |
| GT-06467 | 1e5 | 1e4 | GT-09973 | 1e9 | 1e9 | GT-10297 | 1e8 | 1e3 |
| GT-06441 | 1e8 | 1e2 | GT-09974 | 1e6 | 1e4 | GT-10298 | 1e5 | 1e2 |
| GT-06443 | 1e9 | 1e4 | GT-09975 | 1e7 | 1e5 | GT-10299 | 1e6 | 1e6 |
| GT-06444 | 1e9 | 1e4 | GT-09976 | 1e7 | 1e4 | GT-10300 | 1e6 | 1e3 |
| GT-06445 | 1e9 | 1e7 | GT-09978 | 1e9 | 1e8 | GT-10301 | 1e7 | 1e6 |
| GT-09960 | 1e6 | 1e4 | GT-09981 | 1e6 | 1e4 | GT-10302 | 1e7 | 1e6 |
| GT-09964 | 1e7 | 1e5 | GT-10057 | 1e7 | 1e3 | GT-10303 | 1e6 | 1e2 |
| GT-09965 | 1e6 | 1e5 | GT-10058 | 1e6 | 1e2 | GT-10310 | 1e5 | 1e2 |
| GT-10076 | 1e5 | 1e3 | GT-10136 | 1e6 | 1e5 | GT-10312 | 1e7 | 1e5 |
| GT-10078 | 1e6 | 1e6 | GT-10137 | 1e5 | 1e2 | GT-10316 | 1e7 | 1e4 |
| GT-10080 | 1e5 | 1e1 | GT-10232 | 1e5 | 1e3 | GT-10430 | 1e5 | 1e1 |
| GT-10081 | 1e6 | 1e2 | GT-10235 | 1e5 | 1e4 | GT-10431 | 1e5 | 1e3 |
| GT-10082 | 1e6 | 1e3 | GT-10238 | 1e5 | 1e3 | GT-10413 | 1e9 | 1e8 |
| GT-10083 | 1e5 | 1e1 | GT-10168 | 1e5 | 1e7 | GT-10459 | 1e5 | 1e4 |
| GT-10086 | 1e5 | 1e2 | GT-10172 | 1e5 | 1e4 | GT-10483 | 1e8 | 1e7 |
| GT-10088 | 1e5 | 1e4 | GT-10330 | 1e7 | 1e7 | GT-10496 | 1e6 | 1e3 |
| GT-10089 | 1e5 | 1e3 | GT-10335 | 1e5 | 1e3 | GT-10654 | 1e6 | 1e5 |
| GT-10092 | 1e6 | 1e4 | GT-10337 | 1e7 | 1e6 | | | |

| | | | | | | | | |
|---|---|---|---|---|---|---|---|---|
| Note: in table I-5, the symbols 1e1, 1e2, 1e3, 1e4, 1e5, 1e6, 1e7, 1e8, 1e9, and 1e10 are defined as follows: 0 < 1e1 < 10%, 10% ≤ 1e2 < 20%, 20% ≤ 1e3 < 30%, 30% ≤ 1e4 < 40%, 40% ≤ 1e5 < 50%, 50% ≤ 1e6 < 60%, 60% ≤ 1e7 < 70%, 70% ≤ 1e8 < 80%, 80% ≤ 1e9 ≤ 100%, 100% < 1e10 ≤ 105%. | | | | | | | | |

**Table I-6: inhibitory activity of the compounds of the present disclosure against the proliferation of human B lymphocyte leukemia cells RPMI-8226 (inhibition rate %)**

| Comp. No. /names | Inhibition Rate (%) at 500 nM | Inhibition Rate (%) at 10 nM | Comp. No. /names | Inhibition Rate (%) at 500 nM | Inhibition Rate (%) at 10 nM | Comp. No. /names | Inhibition Rate (%) at 500 nM | Inhibition Rate (%) at 10 nM |
|---|---|---|---|---|---|---|---|---|
| pomalido | 1f3 | 1f1 | GT-09973 | 1f9 | 1f9 | GT-10647 | 1f5 | 1f4 |
| mide | | | | | | | | |
| lenalidomide | 1f2 | 1f2 | GT-09978 | 1f9 | 1f5 | GT-10652 | 1f5 | 1f5 |
| GT-06441 | 1f9 | 1f3 | GT-10057 | 1f8 | 1f3 | GT-08710 | 1f7 | 1f2 |
| GT-06443 | 1f9 | 1f4 | GT-10058 | 1f5 | 1f2 | GT-08711 | 1f6 | 1f1 |
| GT-06444 | 1f9 | 1f4 | GT-10461 | 1f6 | 1f4 | | | |
| GT-06445 | 1f9 | 1f9 | GT-10492 | 1f5 | 1f4 | | | |

| | | | | | | | | |
|---|---|---|---|---|---|---|---|---|
| Note: in table I-6, the symbols 1f1, 1f2, 1f3, 1f4, 1f5, 1f6, 1f7, 1f8, 1f9, and 1f10 are defined as follows: 0 < 1f1 < 10%, 10% ≤ 1f2 < 20%, 20% ≤ 1f3 < 30%, 30% ≤ 1f4 < 40%, 40% ≤ 1f5 < 50%, 50% ≤ 1f6 < 60%, 60% ≤ 1f7 < 70%, 70% ≤ 1f8 < 80%, 80% ≤ 1f9 ≤ 100%, 100% < 1f10 ≤ 105%. | | | | | | | | |

**Table I-7: inhibitory activity of the compounds of the present disclosure against the proliferation of human diffuse large B-cell lymphoma cells TMD8 (inhibition rate %)**

| Comp. No. /names | Inhibition Rate (%) at 500 nM | Inhibition Rate (%) at 10 nM | Comp. No. /names | Inhibition Rate (%) at 500 nM | Inhibition Rate (%) at 10 nM | Comp. No. /names | Inhibition Rate (%) at 500 nM | Inhibition Rate (%) at 10 nM |
|---|---|---|---|---|---|---|---|---|
| pomalido mide | 1g2 | 1g2 | GT-09115 | 1g5 | 1g2 | GT-06445 | 1g9 | 1g4 |
| lenalidomi de | 1g2 | 1g2 | GT-09129 | 1g5 | 1g3 | GT-09973 | 1g8 | 1g5 |
| GT-09109 | 1g7 | 1g3 | GT-09131 | 1g7 | 1g4 | GT-09978 | 1g6 | 1g4 |
| GT-09110 | 1g5 | 1g4 | GT-09133 | 1g7 | 1g3 | GT-10459 | 1g5 | 1g3 |
| GT-09111 | 1g7 | 1g3 | GT-09134 | 1g7 | 1g6 | GT-10463 | 1g5 | 1g4 |
| GT-09112 | 1g6 | 1g2 | GT-06441 | 1g5 | 1g2 | GT-10476 | 1g6 | 1g3 |
| GT-09113 | 1g8 | 1g4 | GT-06443 | 1g6 | 1g2 | | | |
| GT-09114 | 1g6 | 1g2 | GT-06444 | 1g8 | 1g3 | | | |

| | | | | | | | | |
|---|---|---|---|---|---|---|---|---|
| Note: in table I-7, the symbols 1g1, 1g2, 1g3, 1g4, 1g5, 1g6, 1g7, 1g8, 1g9, and 1g10 are defined as follows: 0 < 1g1 < 10%, 10% ≤ 1g2 < 20%, 20% ≤ 1g3 < 30%, 30% ≤ 1g4 < 40%, 40% ≤ 1g5 < 50%, 50% ≤ 1g6 < 60%, 60% ≤ 1g7 < 70%, 70% ≤ 1g8 < 80%, 80% ≤ 1g9 ≤ 100%, 100% < 1g10 ≤ 105%. | | | | | | | | |

**Table I-8: Inhibitory activity (IC₅₀, nM) of the compounds of the present disclosure against tumor cell proliferation**

| Comp. No. /names | RPMI-8226 | MM.1S | TMD8 | MV-4-11 |
|---|---|---|---|---|
| lenalidomide | F | F | F | F |
| pomalidomide | D | F | F | F |
| GT-06441 | A | A+ | B | - |
| GT-06545 | - | - | C | - |
| GT-06444 | A+ | A++ | - | - |
| GT-06445 | A++ | A++ | A++ | - |
| GT-06443 | A+ | A++ | A+ | - |
| GT-06869 | - | - | - | B |
| GT-06902 | - | - | - | C |
| GT-06903 | - | - | - | C |
| GT-07990 | - | - | - | A++ |
| GT-07996 | - | - | - | A+ |
| GT-06907 | - | - | - | B |

| | | | | |
|---|---|---|---|---|
| Note: in table I-8, the symbols A+++, A++, A+, A, B, C, D, E, and F are defined as follows: 0 < A+++ ≤ 1 nM; 1 nM < A++ ≤ 10 nM; 10 nM < A+ ≤ 50 nM; 50 nM < A ≤ 100 nM; 100 nM < B ≤ 500 nM; 500 nM < C ≤ 1000 nM; 1000 nM < D ≤ 5000 nM; 5000 nM < E ≤ 10000 nM; F > 10000 nM. The symbol "-" indicates "not tested". | | | | |

The results demonstrated that the compounds of the present disclosure (including the compounds listed in Tables 1 and 2, as well as the example compounds) significantly inhibited the proliferation of MV-4-11 cells (human myelomonocytic leukemia cells), MM.1S cells (human multiple myeloma cells), H196 cells (human small cell lung cancer cells), CFPAC-1 cells (human pancreatic cancer cells), A375 cells (human malignant melanoma cells), RPMI-8226 cells (human B-lymphocyte leukemia cells), and TMD8 cells (human diffuse large B-cell lymphoma cells) (see Tables I-1 to I-8), and the inhibitory effects were superior to those of the corresponding positive control agents.

### II. Western Blot Assay

Conventional Western blot assay was performed to evaluate the effects of the compounds of the present disclosure on the expression of target proteins in cells.
(1) Cell Seeding and Protein Harvesting: Human peripheral blood mononuclear cells (hPBMCs) at a density of 1×10⁶ cells/mL were thawed according to the PBMC Recovery Protocol (Milecell Biotechnologies Inc, Shanghai). The cells were then treated with the compounds of the present disclosure (including the compounds listed in Table 1 and the Examples compounds) at concentrations of 50 nM and 500 nM. Controls included a negative control (DMSO) and a positive control (the commercial immunomodulatory drug lenalidomide), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay.
(2) To the collected supernatant, 5X SDS sample loading buffer was added, followed by denaturation at 95°C for 5 minutes. The denatured samples were then separated by SDS-PAGE electrophoresis on a polyacrylamide gel, transferred onto a nitrocellulose membrane (0.45 µm NC membrane), blocked with blocking buffer at room temperature for 1 hour, and subjected to antibody incubation and development procedures according to the antibody manufacturer's instructions (Cell Signaling Technology Company).

Half-Maximal Degradation Concentration value (the drug concentration required for degrading proteins by 50%, abbreviated as DC₅₀) reads method: comparing the grayscale values of Western blot bands from drug-treated samples with those from blank DMSO-treated controls, and identifying the drug concentration range at which the grayscale value is half that of the DMSO-treated control bands.

DC₅₀ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in cells after treatment with protein degrader compounds.

Calculation method for target protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

Target protein degradation rate (%) = 1 - target protein remaining (%).

The effects of the compounds of the present disclosure (including the compounds listed in Tables 1 and 2, and the Examples compounds) on target proteins expression in cells were shown in Table II-1 below.

**Table II-1: degradation of target proteins WEE1, IKZF1, IKZF2, IKZF3, CK1α, and GSPT1 by the compounds of the present disclosure - target protein remaining (%)**

| Comp. No. /names | WEE1 | IKZF1 | IKZF2 | IKZF3 | GSPT1 | CK1α |
|---|---|---|---|---|---|---|
| lenalidomide | B | A | B | A | B | B |
| GT-09741 | B | B | A | A | B | B |
| GT-09708 | B | B | B | B | B | A |
| GT-09112 | B | B | B | B | B | A |
| GT-09113 | B | B | B | B | B | A |
| GT-09114 | B | B | B | B | B | A |
| GT-09197 | A | B | B | B | B | B |
| GT-09199 | B | B | B | B | B | A |
| GT-09213 | B | B | A | A | A | B |
| GT-06867 | B | B | B | B | B | A |
| GT-08040 | B | B | B | B | A | B |
| GT-07996 | A | A | A | B | A | A |
| GT-06398 | B | B | B | B | A | B |
| GT-06399 | B | B | B | B | A | B |
| GT-06401 | B | B | B | B | A | B |
| GT-06402 | B | B | B | B | A | B |
| GT-06423 | A | B | B | B | B | B |
| GT-06427 | B | B | B | B | B | A |
| GT-06500 | A | B | B | B | B | B |
| GT-06502 | B | B | B | B | A | B |
| GT-06505 | B | B | B | B | A | B |
| GT-06507 | B | B | B | B | A | B |
| GT-06508 | A | B | B | B | B | B |
| GT-06510 | B | B | B | B | A | B |
| GT-06511 | B | B | B | B | A | B |
| GT-06865 | B | B | B | B | A | B |
| GT-06866 | A | B | B | B | B | A |
| GT-06460 | B | B | A | A | A | B |
| GT-06461 | A | B | B | A | A | B |
| GT-06463 | B | B | A | A | B | B |
| GT-06465 | B | B | B | A | B | B |
| GT-06466 | A | B | B | B | A | B |
| GT-06467 | A | B | A | A | A | B |
| GT-06468 | A | B | B | B | A | B |
| GT-06471 | B | B | B | A | B | B |
| GT-06536 | B | B | B | B | A | B |
| GT-06537 | B | B | B | B | A | B |
| GT-06538 | B | B | B | B | A | A |
| GT-06539 | A | B | B | B | A | A |
| GT-06540 | B | B | B | B | A | A |
| GT-06544 | B | B | A | A | A | B |
| GT-06545 | B | B | A | B | B | B |
| GT-06905 | B | B | B | A | A | B |
| GT-06436 | B | B | B | A | B | B |
| GT-06438 | B | B | A | B | B | B |
| GT-06439 | B | B | A | B | B | B |
| GT-06440 | A | B | B | A | B | B |
| GT-06441 | B | B | B | A | B | B |
| GT-06445 | B | B | B | A | B | B |
| GT-06446 | A | B | B | B | A | A |
| GT-06447 | B | B | B | B | B | A |
| GT-07045 | B | B | A | A | A | A |
| GT-06902 | A | A | B | A | A | A |
| GT-06903 | B | B | B | A | A | A |
| GT-07211 | B | B | B | B | A | B |
| GT-07209 | B | B | A | B | A | A |
| GT-07212 | A | A | B | A | A | A |
| GT-06904 | A | A | B | A | A | B |
| GT-07214 | B | B | B | B | A | B |
| GT-07066 | B | B | B | B | A | B |
| GT-07057 | B | B | A | A | A | B |
| GT-07059 | B | B | B | A | A | A |
| GT-09961 | B | A | B | B | A | B |
| GT-09962 | B | A | B | B | B | B |
| GT-09963 | A | B | A | B | B | B |
| GT-09964 | B | B | A | B | B | B |
| GT-09965 | A | B | A | B | B | B |
| GT-10077 | B | A | B | B | B | B |
| GT-10080 | B | B | B | A | B | B |
| GT-10082 | B | B | B | B | A | B |
| GT-10083 | B | B | B | B | A | A |
| GT-10084 | B | B | B | B | A | B |
| GT-10085 | B | B | B | B | A | B |
| GT-10086 | B | B | B | B | A | B |
| GT-10088 | A | A | B | B | B | A |
| GT-10090 | B | A | B | B | B | B |
| GT-10093 | A | B | B | B | B | A |
| GT-10094 | A | B | B | B | B | B |
| GT-09972 | B | B | B | B | A | B |
| GT-09977 | B | B | B | B | B | A |
| GT-09980 | B | B | A | B | B | B |
| GT-09983 | A | B | A | A | B | B |
| GT-09985 | B | B | A | A | B | A |
| GT-09986 | A | B | A | A | B | A |
| GT-09988 | A | B | A | A | B | A |
| GT-09990 | B | B | A | A | B | B |
| GT-09991 | B | B | A | A | B | B |
| GT-09994 | B | B | B | A | B | A |
| GT-09992 | A | B | B | B | B | B |
| GT-10028 | B | B | A | B | B | A |
| GT-10029 | B | B | A | A | B | A |
| GT-10056 | B | B | A | A | B | A |
| GT-10059 | B | A | B | B | B | B |
| GT-10065 | B | B | A | B | B | B |
| GT-10066 | A | A | A | B | B | B |
| GT-10069 | B | B | B | A | B | B |
| GT-10124 | A | B | B | A | B | A |
| GT-10127 | B | B | B | A | B | A |
| GT-10128 | B | B | B | B | B | A |
| GT-10129 | A | A | A | A | B | A |
| GT-10131 | A | B | B | B | B | B |
| GT-10134 | A | A | B | A | B | A |
| GT-10136 | A | B | B | B | B | B |
| GT-10138 | A | B | B | B | B | B |
| GT-10143 | B | B | B | B | B | A |
| GT-10145 | A | B | A | A | A | A |
| GT-10146 | B | B | B | B | A | A |
| GT-10147 | B | B | B | B | A | A |
| GT-10148 | A | B | B | B | B | B |
| GT-10149 | B | B | B | B | A | A |
| GT-10240 | B | B | A | B | B | B |
| GT-10241 | B | B | A | B | B | B |
| GT-10242 | B | A | B | B | A | B |
| GT-10243 | B | B | B | B | A | B |
| GT-10244 | B | B | B | B | A | B |
| GT-10245 | B | B | B | A | A | A |
| GT-10246 | A | B | A | A | A | A |
| GT-10247 | B | A | A | A | B | B |
| GT-10248 | B | A | A | A | B | A |
| GT-10249 | B | B | A | A | B | B |
| GT-10251 | B | B | B | B | B | A |
| GT-10252 | A | B | B | B | B | B |
| GT-10253 | B | B | B | B | B | A |
| GT-10255 | B | B | B | B | B | A |
| GT-10256 | B | B | B | B | B | A |
| GT-10257 | A | A | A | A | A | A |
| GT-10258 | A | A | A | A | A | A |
| GT-10259 | B | A | A | A | A | A |
| GT-10170 | B | B | B | B | A | B |
| GT-10177 | A | B | A | A | A | B |
| GT-10178 | A | A | A | A | A | B |
| GT-10179 | A | B | A | A | A | B |
| GT-10180 | B | B | B | A | A | B |
| GT-10182 | A | B | B | A | B | B |
| GT-10183 | B | B | B | B | A | B |
| GT-10207 | A | B | B | A | A | B |
| GT-10208 | A | B | B | B | A | B |
| GT-10209 | A | B | B | B | B | B |
| GT-10210 | A | B | B | A | B | B |
| GT-10211 | A | B | B | B | B | A |
| GT-10287 | A | A | A | A | A | A |
| GT-10288 | A | A | A | A | A | A |
| GT-10289 | B | B | B | A | A | A |
| GT-10290 | B | B | B | A | B | A |
| GT-10291 | B | B | B | B | B | A |
| GT-10292 | A | A | A | A | A | A |
| GT-10293 | A | A | A | A | A | A |
| GT-10294 | A | A | A | A | A | A |
| GT-10295 | B | A | B | A | A | A |
| GT-10331 | A | B | A | B | A | B |
| GT-10334 | B | B | A | B | B | B |
| GT-10335 | B | B | A | B | B | B |
| GT-10339 | B | B | B | B | A | B |
| GT-10340 | B | B | B | B | A | B |
| GT-10296 | B | A | B | A | B | A |
| GT-10303 | B | B | B | B | A | A |
| GT-10304 | B | B | B | B | A | B |
| GT-10305 | B | B | B | B | A | B |
| GT-10306 | A | B | B | B | B | B |
| GT-10307 | B | B | B | B | A | A |
| GT-10308 | B | B | B | B | A | A |
| GT-10310 | A | B | B | B | A | B |
| GT-10312 | A | B | B | B | A | A |
| GT-10313 | B | B | B | B | A | B |
| GT-10314 | B | B | B | B | A | B |
| GT-10317 | A | B | B | B | A | B |
| GT-10318 | B | B | B | B | A | B |
| GT-10320 | B | B | A | A | B | B |
| GT-10408 | A | B | B | B | A | A |
| GT-10414 | B | B | B | B | A | A |
| GT-10459 | A | A | B | A | A | A |
| GT-10460 | A | B | B | A | A | A |
| GT-10461 | A | A | A | A | A | A |
| GT-10462 | A | A | A | A | A | A |
| GT-10463 | A | A | A | A | A | A |
| GT-10464 | A | B | B | B | A | A |
| GT-10465 | A | B | A | B | B | B |
| GT-10478 | B | B | A | B | B | B |
| GT-10479 | B | B | A | B | B | B |
| GT-10480 | B | B | A | B | B | B |
| GT-10482 | B | B | A | B | B | B |
| GT-10484 | A | B | B | B | A | B |
| GT-10488 | B | B | B | B | B | A |
| GT-10489 | B | B | A | B | B | A |
| GT-10490 | B | B | A | B | B | B |
| GT-10491 | B | B | B | A | B | B |
| GT-10521 | A | B | B | B | B | A |
| GT-10510 | B | B | A | B | B | B |
| GT-10511 | B | B | A | B | B | B |
| GT-10513 | A | B | B | B | B | B |
| GT-10515 | A | B | B | B | B | B |
| GT-10545 | A | A | A | B | A | B |
| GT-10546 | A | B | A | B | A | B |
| GT-10547 | A | B | B | B | A | B |
| GT-10548 | B | B | B | B | B | A |
| GT-10563 | B | B | B | B | A | B |
| GT-10564 | A | B | B | A | A | A |
| GT-10565 | B | B | B | B | B | A |
| GT-10566 | B | B | B | A | B | A |
| GT-10568 | B | B | B | B | A | A |
| GT-10569 | A | B | B | B | A | A |
| GT-10651 | B | B | B | B | A | B |
| GT-10652 | B | B | A | B | B | B |
| GT-10653 | B | B | A | B | B | B |
| GT-10654 | B | B | B | B | A | B |
| GT-10655 | B | B | A | B | A | A |
| GT-10616 | B | B | A | B | B | B |
| GT-10617 | B | B | A | B | B | B |
| GT-10618 | B | B | A | B | B | B |
| GT-10622 | A | B | A | A | B | B |
| GT-08710 | B | B | B | B | A | A |
| GT-09057 | B | B | B | B | A | A |
| GT-06906 | B | B | A | B | B | B |
| GT-06907 | A | B | B | B | A | A |
| GT-06908 | A | A | B | A | B | B |
| GT-06909 | A | A | B | A | B | B |
| GT-06910 | B | B | B | B | B | A |
| GT-07058 | B | B | B | B | A | A |

| | | | | | | |
|---|---|---|---|---|---|---|
| **Note:** In Table II-1, "A" is defined as 0% ≤ target protein remaining % ≤ 80%, and "B" is defined as 80%< target protein remaining %≤ 100%. | | | | | | |

The degradation effects of the compounds of the present invention on the target substrate proteins were shown in Table II-1 and Figure 1. From the experimental results in Table II-1 and Figure 1, it can be concluded that the compounds of the present invention significantly degrade the target substrate proteins (e.g., proteins WEE1, IKZF1/2/3, CK1α, GSPT1 etc.). Compared to lenalidomide, the compounds of the present invention exhibited significantly enhanced efficiency in inducing target substrate proteins degradation and also showed selective induction of degradation, thereby potentially enabling their use in treating indications associated with these target substrate proteins.

### III. Determination of CRBN-binding affinity of compounds of the present disclosure:

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:
**1.** According to the instructions of the CEREBLON BINDING KITS, serial dilutions were performed for the test compounds of the present disclosure (including the compounds in Table 1, Table 2, and Examples) and for lenalidomide. Specifically, diluent #9 (1X) solution was used as the diluent, and multiple concentration gradients were set. The dilution operation can be performed according to one of the following two methods:
   (1) The first dilution method: The test compounds of the present disclosure (including the compounds in Table 1, Table 2, and Examples) were subjected to 5-fold serial dilutions starting from a highest concentration of 40 µM, resulting in a total of 7 concentrations from high to low (40, 8, 1.6, 0.32, 0.064, 0.0128, 0.00256 µM). Subsequently, the diluted test compounds and corresponding detection reagents were added to each well of a 96-well plate, resulting in final test compound concentrations of 10, 2, 0.4, 0.08, 0.016, 0.0032, and 0.00064 µM, respectively.
   (2) As an alternative, a second dilution method can be used: The test compounds of the present disclosure (including the compounds in Table 1, Table 2, and Examples) were subjected to 4-fold gradient dilutions starting from a highest concentration of 8 µM, resulting in 2 concentrations (8 µM, 2 µM). Into the 96-well plate, the diluted test compounds and corresponding detection reagents were added, resulting in final test compound concentrations of 2 µM and 0.5 µM, respectively. The experimental procedure is described below using the diluted 8 µM test compound and lenalidomide solutions as examples.
**2.** 2.5 µL of the above 8 µM of the tested compounds and lenalidomide solution, as well as the same volume of diluent #9 (1X) solution (solvent control group, Std0) were added to each well of a 96-well plate, respectively. Then, 2.5 µL of human Cereblon WT GST-tagged protein solution was added to each well. Finally, 5 µL of the thoroughly mixed Thalidomide-Re reagent and GST Eu antibody working solution were added to each of the aforementioned wells. The final concentration of the tested compounds and lenalidomide in each well was 2 µM.
**3.** The blank control wells were sequentially added with 2.5 µL of diluent #9 (1X) solution, 2.5 µL of PROTAC binding buffer, and 5 µL of thoroughly mixed Thalidomide-Re reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).
The corresponding CRBN binding inhibition rate was calculated using the following method: ${R}_{compound} = OD 665 {nm}_{compound} / OD 620 {nm}_{compound} - OD 665 {nm}_{control} / OD 620 {nm}_{control}$ ${R}_{Std 0} = OD 665 {nm}_{Std 0} / OD 620 {nm}_{Std 0} - OD 665 {nm}_{control} / OD 620 {nm}_{control}$ $inhibition rate \left(%\right) = \left(1-{R}_{compound}/{R}_{Std 0}\right) * 100$
Note: OD 665 nm _{compound} is the absorbance value of each well of the compounds to be tested of the present disclosure at an emission wavelength of 665 nm.
OD 620 nm _{compound} is the absorbance value of each well of the compounds to be tested of the present disclosure at an emission wavelength of 620 nm.
OD 665 nm _{control} is the absorbance value of the blank control well at an emission wavelength of 665 nm.
OD 620 nm _{control} is the absorbance value of the blank control well at an emission wavelength of 620 nm.
OD 665 nm _{Std0} is the absorbance value of the solvent control well at an emission wavelength of 665 nm.
OD 620 nm _{Std0} is the absorbance value of the solvent control well at an emission wavelength of 620 nm.

The test results are shown in Tables III-1 and III-2 below.

**Table III-1. HTRF inhibitory activity (IC₅₀, µM) of the compounds of the present disclosure (including the compounds in Tables 1, 2, and the Examples compounds)**

| Comp. No. | HTRF IC₅₀ (µM) | Comp. No. | HTRF IC₅₀ (µM) | Comp. No. | HTRF IC₅₀ (µM) | Comp. No. | HTRF IC₅₀ (µM) |
|---|---|---|---|---|---|---|---|
| lenalidomide | e | GT-06502 | a | GT-06472 | d | GT-06442 | c |
| GT-06871 | a | GT-06508 | b | GT-06536 | b | GT-06443 | a |
| GT-06867 | c | GT-06511 | a | GT-06476 | c | GT-06444 | a |
| GT-06961 | c | GT-06864 | a | GT-06538 | c | GT-06445 | a |
| GT-06863 | b | GT-06869 | b | GT-06539 | b | GT-06446 | c |
| GT-06398 | a | GT-06870 | b | GT-06540 | b | GT-06447 | a |
| GT-06399 | a | GT-06962 | c | GT-06543 | d | GT-07045 | c |
| GT-06400 | a | GT-06963 | a | GT-06546 | c | GT-06904 | c |
| GT-06401 | b | GT-06460 | d | GT-06911 | c | GT-07066 | a |
| GT-06421 | b | GT-06462 | a | GT-06903 | c | GT-07057 | b |
| GT-06422 | d | GT-06463 | a | GT-06436 | d | GT-07059 | a |
| GT-06426 | b | GT-06464 | a | GT-06438 | b | GT-06906 | a |
| GT-06427 | a | GT-06465 | d | GT-06439 | a | GT-06908 | b |
| GT-06428 | b | GT-06467 | a | GT-06440 | b | GT-06909 | b |
| GT-06501 | a | GT-06468 | a | GT-06441 | c | GT-07058 | b |

| | | | | | | | |
|---|---|---|---|---|---|---|---|
| Note: in Table III-1, the symbols a, b, c, d, e, and f are defined as follows: 0<a≤0.5µM, 0.5µM<b≤1µM, 1µM<c≤1.5µM, 1.5µM<d<1.9µM, 1.9µM ≤ e ≤10µM, 10µM < f. | | | | | | | |

**Table III-2: Screening of CRBN Binding Affinity of the Compounds of the Present Invention (including the compounds in Tables 1 and 2, and Examples Compounds) at Concentrations of 2 µM and 0.5 µM**

| Comp. No. | Inhibition Rate (%) at 2 µM | Inhibition Rate (%) at 0.5 µM | Comp. No. | Inhibition Rate (%) at 2 µM | Inhibition Rate (%) at 0.5 µM | Comp. No. | Inhibition Rate (%) at 2 µM | Inhibition Rate (%) at 0.5 µM |
|---|---|---|---|---|---|---|---|---|
| lenalidomide | a4 | a2 | GT-10129 | a8 | a5 | GT-10311 | a8 | a4 |
| GT-09709 | a7 | a4 | GT-10130 | a8 | a5 | GT-10312 | a7 | a2 |
| GT-09708 | a5 | a4 | GT-10131 | a7 | a2 | GT-10313 | a8 | a4 |
| GT-09104 | a7 | a4 | GT-10132 | a7 | a3 | GT-10430 | a8 | a6 |
| GT-09105 | a7 | a4 | GT-10134 | a8 | a4 | GT-10431 | a8 | a6 |
| GT-09106 | a6 | a2 | GT-10135 | a8 | a7 | GT-10408 | a6 | a2 |
| GT-09107 | a8 | a4 | GT-10136 | a8 | a5 | GT-10409 | a8 | a4 |
| GT-09108 | a7 | a3 | GT-10137 | a8 | a7 | GT-10412 | a7 | a2 |
| GT-09109 | a7 | a4 | GT-10138 | a8 | a7 | GT-10415 | a7 | a2 |
| GT-09110 | a7 | a3 | GT-10139 | a8 | a7 | GT-10459 | a8 | a5 |
| GT-09111 | a8 | a7 | GT-10141 | a8 | a5 | GT-10460 | a8 | a8 |
| GT-09112 | a8 | a6 | GT-10142 | a7 | a4 | GT-10461 | a8 | a6 |
| GT-09113 | a8 | a6 | GT-10143 | a8 | a5 | GT-10462 | a7 | a4 |
| GT-09114 | a8 | a6 | GT-10144 | a8 | a6 | GT-10463 | a7 | a4 |
| GT-09115 | a8 | a5 | GT-10145 | a8 | a5 | GT-10464 | a8 | a6 |
| GT-09116 | a5 | a2 | GT-10146 | a8 | a6 | GT-10465 | a8 | a6 |
| GT-09117 | a5 | a3 | GT-10147 | a8 | a5 | GT-10478 | a8 | a7 |
| GT-09129 | a6 | a2 | GT-10148 | a8 | a5 | GT-10479 | a8 | a7 |
| GT-09130 | a6 | a2 | GT-10149 | a7 | a4 | GT-10480 | a8 | a8 |
| GT-09132 | a5 | a2 | GT-10232 | a5 | a2 | GT-10482 | a8 | a5 |
| GT-09133 | a5 | a1 | GT-10233 | a6 | a2 | GT-10483 | a8 | a5 |
| GT-09134 | a7 | a4 | GT-10234 | a8 | a4 | GT-10484 | a5 | a2 |
| GT-09135 | a6 | a2 | GT-10235 | a8 | a4 | GT-10485 | a7 | a4 |
| GT-09197 | a8 | a5 | GT-10236 | a7 | a4 | GT-10486 | a6 | a3 |
| GT-09198 | a8 | a6 | GT-10237 | a7 | a3 | GT-10489 | a5 | a2 |
| GT-09199 | a8 | a5 | GT-10238 | a6 | a2 | GT-10491 | a6 | a4 |
| GT-09200 | a8 | a4 | GT-10239 | a7 | a4 | GT-10494 | a8 | a5 |
| GT-09201 | a7 | a4 | GT-10240 | a5 | a1 | GT-10495 | a6 | a3 |
| GT-09213 | a7 | a5 | GT-10241 | a7 | a3 | GT-10496 | a7 | a4 |
| GT-08034 | a7 | a4 | GT-10242 | a7 | a3 | GT-10475 | a8 | a6 |
| GT-08040 | a5 | a1 | GT-10243 | a8 | a5 | GT-10476 | a8 | a7 |
| GT-09960 | a5 | a1 | GT-10244 | a8 | a4 | GT-10477 | a8 | a4 |
| GT-10076 | a6 | a2 | GT-10245 | a8 | a4 | GT-10518 | a8 | a4 |
| GT-10078 | a7 | a2 | GT-10246 | a8 | a5 | GT-10519 | a8 | a6 |
| GT-10080 | a6 | a2 | GT-10247 | a8 | a4 | GT-10520 | a8 | a6 |
| GT-10082 | a5 | a1 | GT-10248 | a8 | a4 | GT-10521 | a8 | a4 |
| GT-10084 | a7 | a4 | GT-10249 | a8 | a5 | GT-10509 | a5 | a2 |
| GT-10086 | a7 | a4 | GT-10251 | a7 | a4 | GT-10511 | a8 | a5 |
| GT-10087 | a8 | a5 | GT-10252 | a7 | a3 | GT-10512 | a7 | a4 |
| GT-10088 | a7 | a3 | GT-10253 | a7 | a4 | GT-10513 | a6 | a3 |
| GT-10089 | a8 | a6 | GT-10254 | a8 | a4 | GT-10514 | a7 | a4 |
| GT-10090 | a6 | a3 | GT-10255 | a8 | a4 | GT-10545 | a8 | a5 |
| GT-10091 | a6 | a2 | GT-10256 | a8 | a5 | GT-10546 | a6 | a3 |
| GT-10093 | a7 | a4 | GT-10257 | a5 | a2 | GT-10547 | a6 | a3 |
| GT-09971 | a6 | a4 | GT-10258 | a7 | a3 | GT-10548 | a7 | a4 |
| GT-09972 | a8 | a7 | GT-10259 | a7 | a1 | GT-10563 | a8 | a6 |
| GT-09973 | a8 | a4 | GT-10172 | a6 | a3 | GT-10564 | a8 | a7 |
| GT-09974 | a8 | a7 | GT-10175 | a5 | a2 | GT-10565 | a8 | a4 |
| GT-09975 | a8 | a6 | GT-10177 | a5 | a1 | GT-10566 | a8 | a5 |
| GT-09976 | a8 | a5 | GT-10181 | a6 | a2 | GT-10568 | a8 | a5 |
| GT-09977 | a8 | a7 | GT-10183 | a7 | a4 | GT-10569 | a8 | a4 |
| GT-09978 | a8 | a4 | GT-10209 | a7 | a3 | GT-10645 | a8 | a6 |
| GT-09980 | a8 | a7 | GT-10210 | a7 | a4 | GT-10646 | a8 | a7 |
| GT-09981 | a8 | a3 | GT-10211 | a6 | a3 | GT-10647 | a8 | a5 |
| GT-09982 | a8 | a6 | GT-10212 | a7 | a4 | GT-10648 | a8 | a6 |
| GT-09983 | a8 | a4 | GT-10287 | a8 | a5 | GT-10649 | a8 | a4 |
| GT-09984 | a8 | a6 | GT-10288 | a8 | a3 | GT-10650 | a8 | a4 |
| GT-09985 | a8 | a6 | GT-10289 | a8 | a6 | GT-10651 | a8 | a4 |
| GT-09986 | a8 | a4 | GT-10290 | a8 | a5 | GT-10652 | a8 | a4 |
| GT-09987 | a8 | a7 | GT-10291 | a8 | a5 | GT-10653 | a8 | a6 |
| GT-09988 | a8 | a5 | GT-10292 | a8 | a5 | GT-10654 | a7 | a7 |
| GT-09989 | a8 | a6 | GT-10293 | a8 | a4 | GT-10655 | a8 | a4 |
| GT-09990 | a8 | a6 | GT-10294 | a8 | a5 | GT-10615 | a7 | a4 |
| GT-09991 | a8 | a6 | GT-10295 | a8 | a4 | GT-10616 | a8 | a8 |
| GT-09994 | a8 | a7 | GT-10330 | a8 | a4 | GT-10617 | a8 | a8 |
| GT-09992 | a8 | a5 | GT-10331 | a8 | a4 | GT-10618 | a8 | a6 |
| GT-10026 | a8 | a6 | GT-10332 | a7 | a2 | GT-10619 | a8 | a5 |
| GT-10028 | a8 | a6 | GT-10334 | a6 | a2 | GT-10620 | a8 | a7 |
| GT-10029 | a7 | a4 | GT-10337 | a7 | a2 | GT-10621 | a8 | a7 |
| GT-10056 | a8 | a4 | GT-10339 | a7 | a4 | GT-10622 | a8 | a6 |
| GT-10061 | a6 | a2 | GT-10340 | a6 | a3 | GT-08644 | a5 | a3 |
| GT-10065 | a7 | a4 | GT-10296 | a6 | a3 | GT-08710 | a7 | a3 |
| GT-10066 | a6 | a2 | GT-10299 | a7 | a4 | GT-08881 | a5 | a3 |
| GT-10067 | a8 | a4 | GT-10301 | a8 | a4 | GT-08866 | a6 | a3 |
| GT-10068 | a7 | a2 | GT-10302 | a8 | a6 | GT-08865 | a6 | a3 |
| GT-10069 | a8 | a4 | GT-10303 | a7 | a4 | GT-08880 | a7 | a4 |
| GT-10124 | a8 | a6 | GT-10304 | a6 | a2 | GT-09138 | a6 | a2 |
| GT-10125 | a7 | a4 | GT-10306 | a8 | a3 | GT-09057 | a5 | a3 |
| GT-10126 | a8 | a5 | GT-10307 | a6 | a2 | GT-09058 | a5 | a2 |
| GT-10127 | a8 | a6 | GT-10309 | a7 | a3 | | | |
| GT-10128 | a8 | a7 | GT-10310 | a8 | a3 | | | |

| | | | | | | | | |
|---|---|---|---|---|---|---|---|---|
| Note: in table III-2, the symbols a, a1, a2, a3, a4, a5, a6, a7 , and a8 are defined as follows: 0% < a ≤ 10%, 10% < a1 ≤ 20%, 20% < a2 ≤ 30%, 30% < a3 ≤ 40%, 40% < a4 ≤ 53%, 53% < a5 ≤ 60%, 60% < a6 ≤ 70%, 70% < a7 ≤80%, 80% < a8 ≤100%. | | | | | | | | |

The results in Table III-1 and Table III-2 demonstrate that the compounds of the present invention (including but not limited to the compounds in Tables 1 and 2, and Examples) exhibit lower or comparable IC₅₀ values, or higher inhibition rates compared to lenalidomide, indicating stronger binding affinity to CRBN.

### IV. TNF-α Activity Inhibition Assay

PBMCs cells were cultured at 37°C with 5% CO₂ and seeded in 96-well plates at 1×10⁷ cells/well. The compounds to be tested (including those listed in Tables 1 and 2, and Examples compounds) were dissolved in DMSO and diluted to appropriate concentrations, ensuring that the final DMSO concentration in cell culture did not exceed 0.5%. After 1-hour incubation in medium with or without the compounds, the cells were stimulated with Lipopolysaccharide (LPS, 1 ng/ml) and continued to be cultured for 18-20 hours. Culture supernatants were then collected, diluted with serum-free medium, and analyzed for TNF-α levels using an ELISA kit. IC₅₀ values were calculated using GraphPad Prism 7.0.

TNF-α downregulation is a key mechanism for the anti-tumor action of immunomodulators. The compounds of the present disclosure demonstrated dose-dependent inhibition of TNF-α levels.

### V. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds in Tables 1 and 2, and the Examples compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). The experimental animals used in this study were mice.

The experimental animals were divided into two groups: a control group (for collecting blank plasma) and an oral administration group (administered at a dose of 10 mg/kg or 30mg/kg). Blood samples were collected at predetermined time points after administration, such as 0.25 h, 0.5 h, 1 h, 2 h, 4 h, 8 h, and 24 h post-dose. Blank plasma was collected from the control group.

Prior to analysis, plasma samples were processed as follows: To 10 µL of plasma sample were added 10 µL of 50% acetonitrile and 200 µL of internal standard solution (containing 50 ng/mL dexamethasone in acetonitrile). For blank control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

### VI. Effects of the compounds of the present disclosure on the release of inflammatory cytokines, including TNF-α, IL-2, IL-12, IL-10, IL-1α, IL-1β, IL-6, IL-8, and IFN-γ, from PBMCs induced by phytohemagglutinin (PHA) or the eBioscience^{™} Cell Stimulation Cocktail (PMA + Ionomycin)

The present inventors examined the effects of the compound of the present invention on the release of a panel of inflammatory cytokines from peripheral blood mononuclear cells (PBMCs) triggered by phytohemagglutinin (PHA) or the eBioscience^{™} Cell Stimulation Cocktail (containing PMA and Ionomycin). These inflammatory cytokines include TNF-α, IL-2, IL-5, IL-12, IL-10, IL-1α, IL-6, IL-8, IFN-γ, IL-1β, and granulocyte-macrophage colony-stimulating factor (GM-CSF), among others. Previous studies have suggested that these inflammatory cytokines may play important roles in the onset and progression of cancer, autoimmune diseases, infectious diseases, and inflammatory diseases. In autoimmune diseases such as rheumatoid arthritis (RA), psoriatic arthritis (PsA), and ankylosing spondylitis (AS), the expression level of TNF-α is significantly elevated, and it exerts its effects by stimulating synovial cell proliferation and promoting inflammatory responses. IL-1α is associated with cancer, autoimmune diseases, infectious diseases, and other inflammatory conditions, and participates in the inflammatory processes of autoimmune diseases. In diseases such as rheumatoid arthritis and systemic lupus erythematosus, elevated IL-1β levels can reflect the extent and activity of inflammatory responses. IL-2, IL-5, IL-10, IFN-γ, and GM-CSF are also associated with various inflammatory diseases and autoimmune diseases.

The following materials were used in this study:

### VI.1 Reagents and Consumable materials

| Name | Supplier or Source |
|---|---|
| Human PBMC cell | Milestone Biotechnologies |
| eBioscience^{™} Cell Stimulation Cocktail | Invitrogen |
| Phytohemagglutinin (PHA) | MCE |
| RPMI 1640 Medium | Gibco |
| Human IFN-**γ** CBA Flex Set E7 100Tst | Becton, Dickinson and Company |
| Human IL-2 CBA Flex Set A4 100Tst | Becton, Dickinson and Company |
| Human TNF-α CBA Flex Set D9 100Tst | Becton, Dickinson and Company |
| Human IL-10 CBA Flex Set B7 100Tst | Becton, Dickinson and Company |
| Human IL-6 CBA Flex Set A7 100Tst | Becton, Dickinson and Company |
| Human IL-8 CBA Flex Set A9 100Tst | Becton, Dickinson and Company |
| Human IL-1β CBA Flex Set B4 100Tst | Becton, Dickinson and Company |
| Human IL-12/IL-23p40 CBA Flex Set E5 100Tst | Becton, Dickinson and Company |
| Human IL-17A CBA Flex Set B5 100Tst | Becton, Dickinson and Company |
| Human IL-1α CBA Flex Set D6 100Tst | Becton, Dickinson and Company |
| Human IL-5 CBA Flex Set A6 100Tst | Becton, Dickinson and Company |
| Human IL-13 CBA Flex Set E6 100Tst | Becton, Dickinson and Company |
| Human IL-4 CBA Flex Set A5 100Tst | Becton, Dickinson and Company |
| Human GM-CSF CBA Flex Set C9 100Tst | Becton, Dickinson and Company |
| Sheath Fluid for Flow Cytometric Analysis (20L) | Becton, Dickinson and Company |
| Human Solbl Ptein CBA Buf Kit 500Tst | Becton, Dickinson and Company |

### VI.2 Methods

### VI.2.1 Cell Thawing and Plating

A complete medium was prepared and pre-warmed to 37°C. Cryovials containing the cells were retrieved from liquid nitrogen, and the area immediately below the cap-notch interface was firmly gripped with forceps. The cryovials were immersed in a 37°C water bath, and shaken occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of 1×10⁵ cells/well (75 µL/well) and incubated in an incubator for 2 h at 37°C with 5% CO₂ under high humidity conditions.

### VI.2.2 Compound Preparation and Addition to Cell Plates

1) The test compounds (including the compounds in Tables 1 and 2, and the Examples compounds) were prepared as 10 mM stock solutions in DMSO.
2) The test compounds were serially diluted 10-fold in DMSO starting from 1 mM (highest concentration) to generate 2 concentration gradients, i.e., 1 mM and 0.1 mM.
3) The test compounds were further diluted in culture medium to five times their final working concentration intended for use.
4) The diluted compounds were added to designated wells according to the plate layout at 25 µL/well, achieving final concentrations starting from 1 µM with 10-fold serial dilution (2 concentration points in total), i.e., 1 µM and 0.1 µM.
5) After 1 h incubation, PHA or PMA+Ionomycin (25 µL/well) was added to the cell wells to reach final concentrations of: 1 µg/mL PHA (TNF-α, IL-10, IL-6, IL-1β, IFN-γ, IL-2, IL-12p40, IL-1α, IL-8), 81nM/mL PMA (IL-4, IL-5, IL-13, GM-CSF, and IL-17A), or 1.34µM/mL Ionomycin (IL-4, IL-5, IL-13, GM-CSF, and IL-17A).
6) The cell plates were incubated in an incubator for 24 h at 37°C with 5% CO₂ under high humidity. The negative control was DMSO, and the positive control drug was the corresponding commercial immunomodulatory agent (e.g., lenalidomide), all of which were used to treat the cells in the same manner as the compounds of the present disclosure.

### Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | H₂O | | | | | | | | | | | |
| B | DMSO | PHA/ PMA+Ionomycin | Cmpd 1-5# (1 µM and 0.1µM, 2 points) | | | | | | | | | |
| C | Cmpd 6-11# (1 µM and 0.1µM, 2 points) | | | | | | | | | | | |
| D | Cmpd 12-17# (1 µM and 0.1µM, 2 points) | | | | | | | | | | | |
| E | Cmpd 18-23# (1 µM and 0.1µM, 2 points) | | | | | | | | | | | |
| F | Cmpd 24-29# (1 µM and 0.1µM, 2 points) | | | | | | | | | | | |
| G | Cmpd 30-35# (1 µM and 0.1µM, 2 points) | | | | | | | | | | | |
| H | Cmpd 36-41# (1 µM and 0.1µM, 2 points) | | | | | | | | | | | |

### VI.2.3 Detection

(1) The cell plate was centrifuged and the culture supernatant was aspirated. TNF-α, IL-10, IL-6, IL-1β, IFN-γ, IL-2, IL-12p40, IL-1α, IL-8, IL-4, IL-5, IL-13, GM-CSF, and IL-17A cytokines in the culture supernatant were detected according to the instructions of the flow cytometry CBA (Cytometric Bead Array) assay.
(2) The flow cytometry CBA assay was performed as follows:
   Dilution of standards and samples: Standards and samples were diluted at appropriate ratios to enable accurate quantitative analysis in the assay.
   Bead mixing: Capture beads were mixed with samples or standards and incubated in the dark for 1 hour.
   Antibody mixing: Detection antibodies were mixed with samples or standards and incubated in the dark for 2 hours. These two steps are the core of the flow cytometry CBA technique, enabling quantitative detection of cytokines through the binding of beads to target molecules.
   Flow cytometric detection: The mixtures were analyzed using a flow cytometer. The flow cytometer excites fluorescent labels on the beads with a laser, collects and analyzes the fluorescence signals, thereby quantitatively determining the cytokine concentrations in the samples.
(3) Data collected by the flow cytometer were further analyzed, typically using software (FACP) for data
   processing, to generate detailed cytokine concentration reports (for TNF-α, IL-10, IL-6, IL-1β, IFN-γ, IL-2, IL-12p40, IL-1α, IL-8, IL-4, IL-5, IL-13, GM-CSF, and IL-17A). The inhibition rate (%) was calculated as follows:$Inhibition rate \left(%\right) = \left[\left(Ac-As\right)/\left(Ac-Ab\right)\right] \times 100 %$ where:
      As = OA of the sample (cells + PHA / PMA+Ionomycin + test compound)
      Ac = OA of the positive cell control (cells + PHA / PMA+Ionomycin + DMSO)
      Ab = OA of the blank control (cells + medium + DMSO)
(4) IC₅₀ curve fitting and IC₅₀ value calculation were performed using GraphPad Prism 6 software with the following formula/analysis: XY-analysis/Nonlinear regression (curve fit)/Dose response-Inhibition/log (inhibitor) vs. response-Variable slope (four parameters).

The results are presented in Table IV below.

**Table IV: Inhibition rates (%) of the compounds of the present disclosure (including but not limited to the compounds in Tables 1 and 2, and the Example compounds) on inflammatory cytokines at concentrations of 1 µM and 0.1 µM.**

| Comp. No. | GM-CSF | | IL-1α | | IL-1β | | IL-12 | | TNF-α | | IL-2 | | IL-4 | | IL-5 | |
|---|---|---|---|---|---|---|---|---|---|---|---|---|---|---|---|---|
| | 1µ M | 0.1 µM | 1µ M | 0.1 µM | 1µ M | 0.1 µM | 1µ M | 0.1 µM | 1µ M | 0.1 µM | 1µ M | 0.1 µM | 1µ M | 0.1 µM | 1µ M | 0.1 µM |
| lenalido mide | IV 4 | IV4 | IV 10 | IV1 0 | IV 12 | IV1 2 | IV 13 | IV1 2 | IV 4 | IV6 | IV 0 | IV1 | IV 4 | IV4 | IV 7 | IV6 |
| GT-06400 | IV 9 | IV1 0 | - | - | - | - | - | - | - | - | IV 7 | IV9 | - | - | - | - |
| GT-06401 | IV 9 | IV7 | - | - | IV 9 | IV7 | IV 9 | IV6 | - | - | - | - | - | - | IV 9 | IV7 |
| GT-06421 | IV 10 | IV1 0 | - | - | - | - | - | - | IV 8 | IV9 | IV 7 | IV1 1 | - | - | IV 8 | IV9 |
| GT-06422 | IV 10 | IV1 1 | IV 7 | IV9 | IV 6 | IV9 | - | - | IV 9 | IV8 | IV 10 | IV1 1 | IV 9 | IV9 | IV 9 | IV1 1 |
| GT-06427 | IV 10 | IV1 1 | IV 9 | IV8 | IV 9 | IV7 | IV 9 | IV6 | IV 9 | IV7 | IV 11 | IV8 | IV 7 | IV9 | IV 10 | IV1 1 |
| GT-06428 | IV 11 | IV1 0 | IV 10 | IV9 | IV 9 | IV8 | - | - | - | - | IV 10 | IV6 | - | - | IV 10 | IV1 0 |
| GT-06499 | IV 9 | IV5 | IV 11 | IV6 | IV 10 | IV5 | IV 9 | IV7 | - | - | IV 11 | IV1 1 | - | - | IV 9 | IV5 |
| GT-06501 | IV 6 | IV9 | - | - | - | - | - | - | - | - | IV 6 | IV1 0 | - | - | IV 5 | IV9 |
| GT-06502 | IV 11 | IV1 0 | IV 9 | IV1 0 | IV 7 | IV1 0 | - | - | IV 7 | IV9 | IV 7 | IV1 2 | IV 7 | IV9 | IV 10 | IV1 0 |
| GT-06506 | IV 11 | IV1 2 | IV 9 | IV9 | - | - | - | - | IV 8 | IV9 | IV 8 | IV1 1 | IV 10 | IV1 0 | IV 10 | IV1 2 |
| GT-06509 | IV 11 | IV1 1 | IV 10 | IV9 | IV 9 | IV9 | - | - | IV 9 | IV8 | IV 12 | IV1 1 | IV 10 | IV1 0 | IV 11 | IV1 1 |
| GT-06511 | IV 12 | IV1 0 | IV 11 | IV9 | IV 10 | IV9 | - | - | IV 9 | IV7 | IV 10 | IV9 | IV 9 | IV8 | IV 10 | IV1 0 |
| GT-06437 | IV 10 | IV6 | IV 9 | IV1 0 | - | - | - | - | - | - | IV 11 | IV1 1 | - | - | IV 9 | IV5 |
| GT-06462 | IV 11 | IV1 1 | IV 6 | IV1 0 | - | - | - | - | IV 6 | IV1 0 | - | - | - | - | IV 11 | IV1 0 |
| GT- | IV | IV7 | IV | IV1 | IV | IV1 | IV | IV1 | IV | IV1 | IV | IV1 | - | - | IV | IV6 |
| 06464 | 11 | | 8 | 0 | 8 | 0 | 6 | 0 | 8 | 0 | 10 | 3 | | | 11 | |
| GT-06469 | IV 11 | IV1 1 | - | - | - | - | - | - | - | - | IV 8 | IV1 0 | IV 11 | IV1 0 | IV 12 | IV1 1 |
| GT-06470 | IV 10 | IV1 1 | - | - | - | - | - | - | - | - | IV 10 | IV8 | IV 9 | IV8 | IV 11 | IV1 0 |
| GT-06472 | IV 11 | IV6 | - | - | IV 9 | IV6 | - | - | - | - | IV 8 | IV1 1 | - | - | IV 10 | IV5 |
| GT-06473 | IV 6 | IV9 | IV 11 | IV9 | IV 9 | IV7 | - | - | - | - | - | - | - | - | - | - |
| GT-06474 | IV 11 | IV1 1 | - | - | - | - | - | - | - | - | IV 8 | IV1 0 | - | - | IV 10 | IV1 0 |
| GT-06475 | IV 12 | IV1 1 | IV 8 | IV9 | - | - | - | - | - | - | IV 9 | IV1 0 | - | - | IV 11 | IV1 0 |
| GT-06476 | IV 11 | IV1 2 | IV 9 | IV6 | - | - | - | - | - | - | IV 9 | IV1 0 | IV 7 | IV9 | IV 9 | IV1 0 |
| GT-06541 | IV 12 | IV1 0 | IV 9 | IV 1 0 | IV 8 | IV9 | - | - | - | - | - | - | IV 10 | IV7 | IV 12 | IV9 |
| GT-06543 | IV 10 | IV6 | IV 9 | IV7 | - | - | - | - | - | - | IV 9 | IV1 0 | - | - | - | - |
| GT-06545 | IV 9 | IV1 0 | - | - | - | - | - | - | - | - | - | - | - | - | IV 8 | IV9 |
| GT-06863 | IV 10 | IV1 1 | - | - | - | - | - | - | - | - | - | - | - | - | IV 9 | IV9 |
| GT-06864 | IV 9 | IV1 0 | - | - | - | - | - | - | - | - | - | - | - | - | IV 9 | IV8 |
| GT-06868 | IV 10 | IV9 | - | - | - | - | - | - | - | - | IV 10 | IV6 | - | - | IV 9 | IV8 |
| GT-06869 | IV 9 | IV6 | - | - | - | - | - | - | - | - | - | - | - | - | IV 9 | IV6 |
| GT-06870 | - | - | - | - | - | - | - | - | - | - | - | - | - | - | IV 6 | IV9 |
| GT-06871 | - | - | IV 9 | IV8 | - | - | - | - | - | - | IV 10 | IV6 | - | - | IV 7 | IV9 |
| GT-06904 | IV 5 | IV1 0 | IV 11 | IV1 1 | IV 9 | IV9 | IV 13 | IV1 2 | - | - | - | - | - | - | IV 8 | IV1 0 |
| GT-06905 | - | - | IV 11 | IV 1 0 | IV 9 | IV7 | IV 11 | IV9 | - | - | - | - | - | - | - | - |
| GT-09129 | IV 10 | IV5 | IV 9 | IV8 | IV 9 | IV8 | IV 8 | IV9 | - | - | IV 11 | IV1 0 | - | - | IV 10 | IV6 |
| GT-09130 | IV 7 | IV9 | IV 8 | IV9 | IV 8 | IV9 | IV 9 | IV9 | IV 7 | IV9 | IV 9 | IV9 | - | - | - | - |
| GT-09131 | IV 6 | IV1 1 | - | - | IV 8 | IV9 | IV 8 | IV9 | - | - | IV 10 | IV 1 0 | - | - | IV 7 | IV 1 0 |
| GT-09132 | - | - | - | - | - | - | IV 9 | IV7 | - | - | IV 11 | IV8 | - | - | - | - |
| GT-09133 | - | - | IV 9 | IV7 | - | - | IV 8 | IV9 | - | - | IV 10 | IV 1 2 | - | - | - | - |
| GT-09134 | - | - | - | - | - | - | - | - | - | - | IV 9 | IV9 | - | - | - | - |
| GT-09135 | IV 11 | IV7 | IV 7 | IV9 | IV 8 | IV9 | - | - | - | - | IV 11 | IV8 | - | - | IV 11 | IV7 |
| GT-09104 | IV 9 | IV1 0 | IV 8 | IV9 | - | - | IV 9 | IV8 | IV 9 | IV8 | IV 9 | IV1 0 | - | - | IV 8 | IV1 0 |
| GT-09105 | IV 9 | IV1 1 | IV 7 | IV9 | IV 6 | IV9 | IV 9 | IV8 | IV 10 | IV8 | IV 12 | IV1 1 | - | - | IV 9 | IV1 0 |
| GT-09106 | IV 9 | IV9 | IV 8 | IV6 | - | - | - | - | - | - | IV 11 | IV8 | - | - | - | - |
| GT-09107 | - | - | IV 9 | IV5 | IV 9 | IV5 | IV 10 | IV6 | IV 9 | IV6 | IV 12 | IV1 0 | - | - | - | - |
| GT-09109 | IV 10 | IV8 | - | - | - | - | - | - | - | - | IV 10 | IV8 | - | - | IV 11 | IV8 |
| GT-09110 | IV 10 | IV1 0 | IV 8 | IV1 0 | IV 7 | IV9 | - | - | - | - | IV 7 | IV1 0 | - | - | IV 9 | IV1 0 |
| GT-09111 | IV 9 | IV1 1 | IV 10 | IV1 0 | IV 9 | IV9 | - | - | - | - | IV 10 | IV1 1 | - | - | IV 9 | IV1 1 |
| GT-09115 | IV 9 | IV9 | IV 9 | IV7 | - | - | - | - | - | - | IV 9 | IV7 | - | - | - | - |
| GT-09116 | - | - | - | - | - | - | IV 9 | IV7 | - | - | IV 11 | IV9 | - | - | - | - |
| GT-09117 | - | - | IV 8 | IV1 0 | - | - | - | - | - | - | IV 5 | IV1 1 | - | - | - | - |
| GT-09198 | IV 11 | IV5 | IV 9 | IV9 | - | - | - | - | - | - | IV 11 | IV9 | - | - | IV 10 | IV7 |
| GT-09200 | | | IV 9 | IV1 0 | IV 8 | IV9 | - | - | - | - | IV 10 | IV1 2 | - | - | - | - |
| GT-09201 | IV 7 | IV9 | IV 9 | IV1 0 | IV 7 | IV9 | - | - | - | - | IV 10 | IV8 | - | - | - | - |
| GT-09203 | - | - | IV 10 | IV9 | IV 9 | IV7 | IV 11 | IV8 | IV 9 | IV5 | IV 9 | IV1 0 | - | - | - | - |
| GT-09708 | - | - | IV 10 | IV8 | - | - | IV 9 | IV8 | - | - | IV 9 | IV9 | - | - | - | - |
| GT-09709 | IV 11 | IV5 | IV 9 | IV7 | - | - | IV 9 | IV7 | - | - | - | - | - | - | IV 11 | IV5 |
| GT-09974 | - | - | IV 9 | IV8 | - | - | IV 10 | IV1 0 | - | - | IV 9 | IV8 | - | - | - | - |
| GT-09975 | - | - | IV 8 | IV9 | - | - | IV 12 | IV1 0 | IV 9 | IV7 | - | - | - | - | - | - |
| GT-09982 | - | - | IV 9 | IV8 | - | - | - | - | - | - | IV 8 | IV9 | - | - | - | - |
| GT-09984 | - | - | IV 9 | IV1 1 | - | - | IV 8 | IV1 0 | - | - | IV 8 | IV1 1 | - | - | - | - |
| GT-09987 | - | - | IV 10 | IV9 | - | - | IV 10 | IV9 | - | - | IV 11 | IV1 1 | - | - | - | - |
| GT-09989 | - | - | IV 11 | IV8 | - | - | IV 10 | IV8 | - | - | IV 11 | IV1 0 | - | - | - | - |
| GT-09993 | - | - | IV 9 | IV1 0 | - | - | IV 9 | IV1 1 | IV 6 | IV9 | IV 8 | IV1 2 | - | - | - | - |
| GT-10026 | - | - | - | - | - | - | - | - | - | - | IV 10 | IV8 | - | - | - | - |
| GT-10079 | - | - | IV 9 | IV5 | IV 9 | IV5 | IV 13 | IV6 | IV 11 | IV8 | IV 5 | IV9 | - | - | - | - |
| GT-10087 | - | - | - | - | - | - | - | - | - | - | IV 8 | IV1 0 | - | - | - | - |
| GT-10126 | - | - | - | - | - | - | - | - | IV 10 | IV7 | - | - | - | - | - | - |
| GT-10132 | IV 9 | IV9 | - | - | - | - | - | - | IV 9 | IV8 | IV 10 | IV1 0 | - | - | - | - |
| GT-10133 | - | - | - | - | - | - | - | - | IV 6 | IV9 | IV 9 | IV1 0 | - | - | - | - |
| GT-10144 | - | - | - | - | - | - | - | - | - | - | IV 9 | IV8 | - | - | - | - |
| GT-10233 | - | - | - | - | - | - | - | - | IV 7 | IV9 | IV 8 | IV1 0 | - | - | - | - |
| GT- | IV | IV9 | IV | IV7 | - | - | - | - | - | - | IV | IV1 | - | - | IV | IV9 |
| 10236 | 9 | | 9 | | | | | | | | 7 | 0 | | | 9 | |
| GT-10239 | IV 9 | IV6 | - | - | - | - | - | - | IV 10 | IV9 | IV 9 | IV1 1 | - | - | - | - |
| GT-10167 | - | - | - | - | - | - | - | - | - | - | - | - | IV 9 | IV5 | - | - |
| GT-10173 | - | - | - | - | - | - | - | - | - | - | IV 8 | IV9 | - | - | - | - |
| GT-10174 | - | - | - | - | - | - | - | - | IV 9 | IV6 | - | - | - | - | - | - |
| GT-10175 | - | - | - | - | - | - | - | - | - | - | IV 8 | IV9 | - | - | - | - |
| GT-10176 | - | - | - | - | - | - | - | - | - | - | IV 7 | IV9 | - | - | - | - |
| GT-10181 | - | - | - | - | - | - | - | - | - | - | IV 8 | IV1 1 | - | - | - | - |
| GT-10333 | - | - | IV 12 | IV1 1 | IV 11 | IV9 | IV 13 | IV1 2 | IV 11 | IV9 | | | - | - | - | - |
| GT-10410 | - | - | IV 8 | IV1 0 | - | - | IV 8 | IV1 2 | IV 7 | IV1 0 | IV 5 | IV9 | - | - | - | - |
| GT-10411 | - | - | - | - | - | - | - | - | - | - | IV 6 | IV9 | - | - | - | - |
| GT-10417 | - | - | IV 8 | IV1 0 | - | - | IV 9 | IV9 | - | - | IV 8 | IV1 1 | - | - | - | - |
| GT-10485 | - | - | IV 9 | IV7 | - | - | - | - | - | - | IV 11 | IV1 2 | - | - | - | - |
| GT-10494 | - | - | IV 11 | IV8 | IV 9 | IV5 | - | - | - | - | IV 11 | IV9 | - | - | - | - |
| GT-10495 | - | - | IV 6 | IV1 0 | - | - | IV 8 | IV9 | IV 8 | IV1 0 | IV 8 | IV1 1 | - | - | - | - |
| GT-10475 | - | - | - | - | - | - | IV 10 | IV8 | IV 13 | IV7 | IV 10 | IV1 0 | - | - | - | - |
| GT-10477 | - | - | - | - | - | - | IV 9 | IV8 | - | - | IV 10 | IV9 | - | - | - | - |
| GT-10519 | - | - | - | - | - | - | - | - | IV 9 | IV5 | - | - | - | - | - | - |
| GT-10514 | - | - | IV 10 | IV9 | - | - | - | - | IV 9 | IV9 | IV 8 | IV9 | - | - | - | - |
| GT-10645 | - | - | IV 9 | IV9 | - | - | - | - | IV 10 | IV8 | IV 10 | IV8 | - | - | - | - |
| GT-10646 | - | - | IV 9 | IV8 | - | - | - | - | IV 9 | IV7 | IV 9 | IV7 | - | - | - | - |
| GT-10648 | - | - | IV 9 | IV9 | IV 8 | IV9 | - | - | - | - | - | - | - | - | IV 9 | IV7 |
| GT-10649 | - | - | - | - | - | - | - | - | - | - | IV 10 | IV5 | - | - | - | - |
| GT-10620 | - | - | IV 9 | IV1 0 | IV 8 | IV9 | - | - | IV 6 | IV9 | IV 6 | IV1 0 | - | - | - | - |

| | | | | | | | | | | | | | | | | |
|---|---|---|---|---|---|---|---|---|---|---|---|---|---|---|---|---|
| Note: in table IV, the symbols IV0, IV1, IV2, IV3, IV4, IV5, IV6, IV7, IV8, IV9, IV10, IV11, IV12, IV13, and IV14 are defined as follows: -320% ≤ IV0 < -200%, -200% ≤ IV1 < -100%, -100% ≤ IV2 < -50%, -50% ≤ IV3 < -20%, -20% ≤ IV4 < 0%, 0% < IV5 ≤ 10%, 10% < IV6 ≤ 20%, 20% < IV7 ≤ 30%, 30% < IV8 ≤ 40%, 40% < IV9 ≤ 50%, 50% < IV10 ≤ 60%, 60% < IV11 ≤ 70%, 70% < IV12 ≤ 80%, 80% < IV13 ≤ 90%, 90% < IV14 < 100%, 100% ≤ IV15 ≤ 110%. The symbol "-" indicates "not tested". | | | | | | | | | | | | | | | | |

The experimental results demonstrate that the compounds of the present disclosure (including the compounds in Tables 1 and 2, and the Example compounds) exhibit excellent modulatory capacity in regulating the production levels of cytokines associated with inflammatory diseases and autoimmune diseases, with inhibitory effects significantly more pronounced than those of the positive control drug lenalidomide. The positive control drug lenalidomide elevates IL-2 levels, thereby activating immune responses; in contrast, the compounds of the present disclosure suppress IL-2 or show no significant stimulatory effect, which confers greater advantages in the treatment of immune system disorders. The compounds of the present disclosure (including the compounds in Tables 1 and 2, and the Example compounds) hold broad promise as potential therapeutic agents for the treatment of various inflammatory diseases and autoimmune diseases, such as rheumatoid arthritis, ankylosing spondylitis, psoriasis and psoriatic arthritis, systemic lupus erythematosus, atopic dermatitis, and hidradenitis suppurativa, among others.

### VII. Efficacy experiment of the compounds of the present disclosure on Psoriasis-like skin lesions induced by Imiquimod in Mice

The compounds of the present disclosure (test compounds, including the compounds in Tables 1, 2, and Examples) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |

| | | | | | | |
|---|---|---|---|---|---|---|
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a vehicle control, a positive control drug, or the test compounds, according to the above table, once daily for 7 consecutive days.

All clinical symptoms of each animal were observed at the beginning and throughout the experiment. The animals' weights were measured and recorded every 2 days.

### Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by CO₂ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-α were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean ± SD. Statistical analysis between two groups was performed using SPSS, with p < 0.05 considered statistically significant.

The experimental results demonstrated that the compounds of the present disclosure exhibited ameliorative effects on psoriasis-like skin lesions on the back of mice induced by imiquimod and can be used for the treatment of psoriasis.

### VIII. Efficacy Experiment of the compounds of the present disclosure on the CIA Model in Rats Induced by Type II Collagen

The compounds of the present disclosure (test compounds, including the compounds in Tables 1, 2, and Examples) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

### Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |

| | | | | | | |
|---|---|---|---|---|---|---|
| Note: ig. = oral gavage; sc. = subcutaneous injection | | | | | | |

Administration commenced between 3 and 7 days after the second booster immunization, once the AI score of the affected paws reached 1-2. According to the table above, animals in each group received daily oral gavage (or subcutaneous injection) of either the vehicle control, positive control drug, or the test compounds for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

### Scoring

Arthritis Index (AI): The foot volume was measured twice a week, and the swelling of the four toes was scored simultaneously. Each foot was scored on a scale from 0 to 4, with a maximum score of 16 for each rat.

### Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-α, IL-1β, and IL-6 in serum were measured by ELISA.

After the experiment, the animals were euthanized by CO₂ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

### Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.
Scoring and Counting Method: 0, 1, 2, 3, and 4 levels, where "0" indicates no observation; "1" is mild; "2" is moderate; "3" is severe; and "4" is extremely severe.

Experimental data were presented as Mean ± SEM. Data between two groups were analyzed using Excel-T test, with p<0.05 considered statistically significant. Scoring data were analyzed using the non-parametric Mann-Whitney U test in SPSS, with p<0.05 considered statistically significant.

The experimental results demonstrated that the compounds of the present disclosure regulated the serum inflammatory cytokines levels in the bovine type II collagen-induced rat model of collagen-induced arthritis (CIA), significantly ameliorated limb swelling in the rats, and thus can be applicable for the treatment of arthritis.

The foregoing has shown and described the basic principles, main features, and advantages of the present invention. It should be understood by those skilled in the art that the present invention is not limited to the above-described embodiments, and that various changes and modifications may be made thereto without departing from the spirit and scope of the present invention, and such changes and modifications all fall within the scope of the invention as claimed. The scope of protection sought by the present invention is defined by the appended claims and their equivalents.

## Claims

1. A compound of Formula (I) 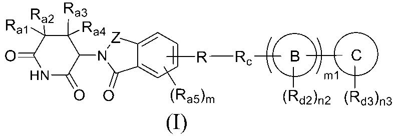 or salts, enantiomers, stereoisomers, isotopically enriched analogs, solvates, or polymorphs thereof as claimed in claim,
wherein Z represents C(O), CH₂, or CD₂;
Rₐ₁, Rₐ₂, Rₐ₃, and Rₐ₄ each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, or C₂₋₆ alkynyl;
m represents an integer of 0, 1, 2, or 3;
R represents: 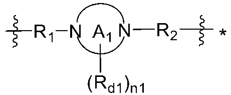
wherein ring A₁ represents heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
R₁ represents N(R_{w}), where R_{w} represents hydrogen or C₁₋₃ alkyl, and R₂ represents a bond, CH₂, or C(O); or
R₁ represents a bond, and R₂ represents N(R_{w}), where R_{w} represents hydrogen or C₁₋₃ alkyl, or R₂ represents N(R_{w})C(O) *, where R_{w} represents hydrogen or C₁₋₃ alkyl, or R₂ represents -N=CH-*; or
R represents: 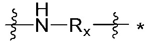 wherein R_{X} represents NH, -N=CH-*, or N(R_{w})C(O) *, wherein R_{w} represents hydrogen or C₁₋₃ alkyl; or
R represents: 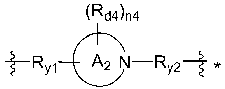
wherein ring A₂ represents heterocyclylene containing one nitrogen atom, and (R_{d4})ₙ₄ indicates that ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20; and
wherein R_{y1} represents 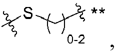 symbol ** indicates the point of attachment to ring A₂; and R_{y2} represents N(R_{w}) or N(R_{w})C(O) *, where R_{w} represents hydrogen or C₁₋₃ alkyl;
and the symbol * in each of the above formulae indicates the point of attachment to R_{c}; and
R_{c} represents CR_{c1}R_{c2}, where R_{c1} and R_{c2} each independently represent H, deuterium, halogen, optionally substituted linear or branched alkyl, optionally substituted cycloalkyl, optionally substituted heterocyclyl, optionally substituted aryl, or optionally substituted heteroaryl, or R_{c} represents a bond;
ring B represents cycloalkylene, heterocyclylene, arylene, or heteroarylene, m1 represents an integer of 0 or 1, and (R_{d2})ₙ₂ indicates that ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n2 represents an integer of 0-20; and
ring C represents heterocyclyl, cycloalkyl, aryl, or heteroaryl, and (R_{d3})ₙ₃ indicates that ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n3 represents an integer of 0-20;
with the proviso that the following compounds are excluded:
3-(5-((4-benzhydrylpiperazin-1-yl)amino)-1-oxoisoindolin-2-yl)piperidine-2,6-dione;
3-(5-((4-((4'-chloro-[1,1'-biphenyl]-2-yl)methyl)piperazin-1-yl)amino)-1-oxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione; and
3-(5-((4-(4'-chloro-5,5-dimethyl-3,4,5,6-tetrahydro-[1,1'-biphenyl]-2-carbonyl)piperazin-1-yl)amino)-1-oxoisoindolin-2-yl)piperidine-2,6-dione.

2. The compound of Formula (I) or salts, enantiomers, stereoisomers, solvates, or polymorphs thereof as claimed in claim 1, wherein
(i) Rₐ₁, Rₐ₂, Rₐ₃, and Rₐ₄ each independently represent H; and/or
(ii) ring A₁ represents 4- to 20-membered heterocyclylene containing at least two nitrogen atoms (including 4- to 20-membered heterocyclene and 4- to 15-membered heterocyclene), and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and/or
(iii) R₁ represents N(R_{w}), where R_{w} represents hydrogen or C₁₋₃ alkyl, and R₂ represents a bond; or
(iv) R₁ represents N(R_{w}), where R_{w} represents hydrogen or C₁₋₃ alkyl, and R₂ represents CH₂ or C(O); or
(v) R₁ represents a bond, and R₂ represents N(R_{w}), where R_{w} represents hydrogen or C₁₋₃ alkyl; or
(vi) R₁ represents a bond, and R₂ represents N(R_{w})C(O) *, where R_{w} represents hydrogen or C₁₋₃ alkyl; or
(vii) R₁ represents a bond, and R₂ represents -N=CH-*; and/or
(viii) ring A₂ represents 4- to 30-membered heterocyclylene containing one nitrogen atom (including 4- to 20-membered heterocyclylene and 4- to 15-membered heterocyclylene), and (R_{d4})ₙ₄ indicates that ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20; and/or
(iv) R_{c} represents CR_{c1}R_{c2}, where R_{c1} and R_{c2} each independently represent H, deuterium, halogen, optionally substituted linear or branched C₁₋₁₀ alkyl, optionally substituted C₃₋₃₀ cycloalkyl, optionally substituted C₅₋₃₀ aryl, optionally substituted 4- to 30-membered heterocyclyl, or optionally substituted 5- to 30-membered heteroaryl, or R_{c} represents a bond; and/or
(v) ring B represents 4- to 30-membered heterocyclylene, C₃₋₃₀ cycloalkylene, C₅₋₃₀ arylene, or 5- to 30-membered heteroarylene, m1 represents an integer of 0 or 1, and (R_{d2})ₙ₂ indicates that ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n2 represents an integer of 0-20; and/or
(vi) ring C represents C₃₋₃₀ cycloalkyl, 4- to 30-membered heterocyclyl, C₅₋₃₀ aryl, or 5- to 30-membered heteroaryl, and (R_{d3})ₙ₃ indicates that ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n3 represents an integer of 0-20.

3. The compound of Formula (I) or salts, enantiomers, stereoisomers, solvates, or polymorphs thereof as claimed in claim 1, wherein R represents one of the following formulae: 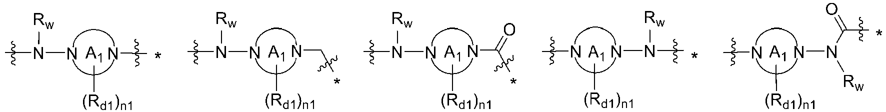 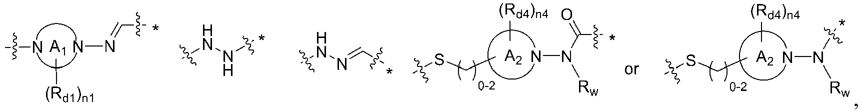
wherein each R_{w} independently represents hydrogen or C₁₋₃ alkyl;
each ring A₁ independently represents heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20;
ring A₂ represents heterocyclylene containing one nitrogen atom, and (R_{d4})ₙ₄ indicates that ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20; and
symbol * indicates the point of attachment to R_{c}.

4. The compound of Formula (I) or salts, enantiomers, stereoisomers, solvates, or polymorphs thereof as claimed in any one of claims 1-3, wherein
(i) each ring A₁ independently represents 4- to 20-membered heterocyclylene containing at least two nitrogen atoms (including 4- to 15-membered heterocyclylene), and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; or
(ii) ring A₂ represents 4- to 20-membered heterocyclylene containing one nitrogen atom (including 4- to 15-membered heterocyclylene), and (R_{d4})ₙ₄ indicates that ring A₂ is optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20; and/or
(iii) the R_{c1} and R_{c2} each independently represent H, deuterium, halogen, optionally substituted linear or branched C₁₋₆ alkyl, optionally substituted C₃₋₂₀ cycloalkyl (including optionally substituted C₃₋₁₅ cycloalkyl), optionally substituted C₅₋₂₀ aryl (including optionally substituted C₅₋₁₅ aryl), optionally substituted 4- to 20-membered heterocyclyl (including optionally substituted 4- to 15-membered heterocyclyl), or optionally substituted 5- to 20-membered heteroaryl (including optionally substituted 5- to 15-membered heteroaryl); and/or
(iv) ring B represents 4- to 20-membered heterocyclylene (including 4- to 15-membered heterocyclylene), C₃₋₂₀ cycloalkylene (including C₃₋₁₅ cycloalkylene), C₅₋₂₀ arylene (including C₅₋₁₅ arylene), or 5- to 20-membered heteroarylene (including 5- to 15-membered heteroarylene), m1 represents an integer of 0 or 1, and (R_{d2})ₙ₂ indicates that ring B is optionally substituted with n2 R_{d2} groups, wherein each R_{d2} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n2 represents an integer of 0-20; and/or
(v) ring C represents C₃₋₂₀ cycloalkyl (including C₃₋₁₅ cycloalkyl), 4- to 20-membered heterocyclyl (including 4- to 15-membered heterocyclyl), C₅₋₂₀ aryl (including C₅₋₁₅ aryl), or 5- to 20-membered heteroaryl (including 5- to 15-membered heteroaryl), and (R_{d3})ₙ₃ indicates that ring C is optionally substituted with n3 R_{d3} groups, wherein each R_{d3} independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and n3 represents an integer of 0-20.

5. The compound of Formula (I) or salts, enantiomers, stereoisomers, solvates, or polymorphs thereof as claimed in claim 1, wherein
(i) Z represents C(O) or CH₂; and/or
(ii) each ring A₁ independently represents 1,3-diazetidinylidene, imidazolidylene, pyrazolidylene, piperazinylene, diazacycloheptanylene, diazacyclooctylene, diazabicyclo[3.1.1]heptanylene, diazabicyclo[2.2.1]heptanylene, diazabicyclo[3.2.1]octanylene, diazabicyclo[2.2.2]octanylene, 2,6-diazaspiro[3.3]heptanylene, 2,7-diazaspiro[3.5]nonanylene, 2,8-diazaspiro[4.5]decanylene, 3,9-diazaspiro[5.5]undecanylene, or octahydropyrrolo[3,4-c]pyrrolylene, each optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20 (e.g., an integer of 0-10); or
(iii) ring A₂ represents azetidinylene, pyrrolidinylene, piperidinylene, dihydroxypiperidinylene, difluoropiperidinylene, azepanylene, azacyclooctylene, monoazabridged-cycloalkylene (e.g., 6- to 20-membered monoazabridged-cycloalkylene, such as 6-azabicyclo[3.1.1]heptanylene, 3-azabicyclo[3.2.1]octanylene, and quinuclidinylene), or monoazaspirocycloalkylene (e.g., 5- to 20-membered monoazaspirocycloalkylene, such as 3-azaspiro[5.5]undecanylene and 7-azaspiro[3.5]nonanylene), each optionally substituted with n4 R_{d4} groups, wherein each R_{d4} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n4 represents an integer of 0-20 (e.g., an integer of 0-10); and/or
(iv) R_{c1} and R_{c2} each independently represent:
H, deuterium, halogen, or optionally substituted linear or branched C₁₋₁₀ alkyl; or
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m*-menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl), each optionally substituted with one or more (e.g., 1-10) substituents independently selected from the group consisting of deuterium, halogen, hydroxyl, mercapto, nitro, amino, cyano, oxo, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl; or
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each optionally substituted with one or more (e.g., 1-10) substituents independently selected from the group consisting of deuterium, halogen, hydroxyl, mercapto, amino, cyano, oxo, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, and C₂₋₆ alkynyl; or
phenyl or naphthyl, each optionally substituted with one or more (e.g., 1-7) substituents independently selected from the group consisting of deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, halogenated C₁₋₆ alkoxy, C₂₋₆ alkenyl, and C₂₋₆ alkynyl; or
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each optionally substituted with one or more (e.g., 1-10) substituents independently selected from the group consisting of deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, and C₂₋₆ alkynyl; and/or
(v) ring B represents:
cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., C₅-C₂₀ spiro-cycloalkylene, such as spiro[3.3]heptylene, spiro[2.5]octylene, spiro[3.5]nonylene, spiro[3.5]nonenylene, spiro[4.4]nonylene, spiro[4.5]decylene, spiro[4.5]decenylene, and spiro[5.5]undecylene), *p*-menthanylene, *m*-menthanylene, or bridged cycloalkylene (e.g., C₆-C₂₀ bridged cycloalkylene, such as adamantanylene, noradamantanylene, bornylene, norbornylene, bicyclo[2.2.1]heptylene, 2-oxobicyclo[2.2.1]heptylene, or bicyclo[2.2.1]heptentylene), each optionally substituted with 0-20 (e.g., 0-10) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl; or
azetidinylene, oxetanylene, pyrrolidinylene, imidazolidylene, pyrazolidylene, oxetanylene, dihydropyranylene, tetrahydropyranylene, tetrahydrothienylene, tetrahydrothiopyranylene, oxazolidinylene, thiazolidinylene, piperidinylene, piperazinylene, tetrahydropyridinylene, dihydroxypiperidinylene, difluoropiperidinylene, morpholinylene, thiomorpholinylene, dioxacyclohexylene, azepanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6- to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene, each optionally substituted with 0-20 (e.g., 0-10) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl; or
phenylene or naphthylene, each optionally substituted with 0-20 (e.g., 0-6) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl; or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, indolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, or 6,7-dihydro-5H-cyclopenta[4,5]thieno[2,3-d]pyrimidinylene, each optionally substituted with 0-20 (e.g., 0-10) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl; and/or
(vi) ring C represents:
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m*-menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl), each optionally substituted with 0-20 (e.g., 1-10) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl; or
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5- diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each optionally substituted with 0-20 (e.g., 1-10) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, and C₂₋₆ alkenyl; or
phenyl or naphthyl, each optionally substituted with 0-20 (e.g., 0-6) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl; or
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each optionally substituted with 0-20 (e.g., 1-10) substituents independently selected from the group consisting of deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, C₂₋₆ alkynyl, and C₂₋₆ alkenyl.

6. The compound of Formula (I) or salts, enantiomers, stereoisomers, solvates, or polymorphs thereof as claimed in claim 1, wherein
(i) R represents one of the following structures: 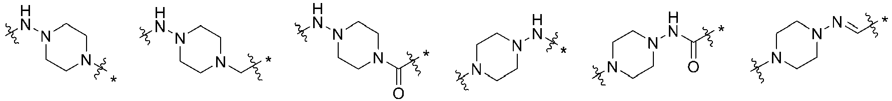 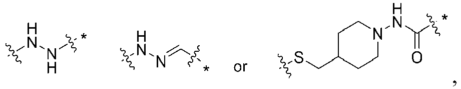 wherein symbol * indicates the point of attachment to R_{c} ; and/or
(ii) the 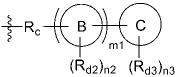 moiety of the compound of Formula (I) represents one of the following structures:

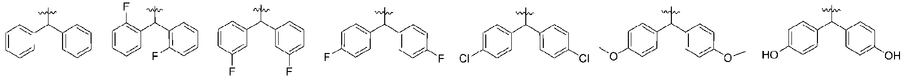

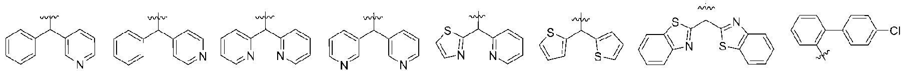

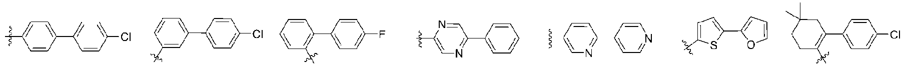

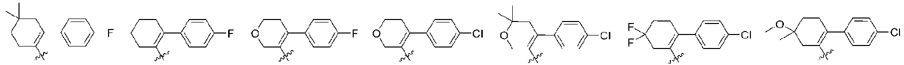

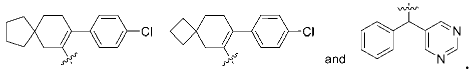

7. The compound of Formula (I) or salts, enantiomers, stereoisomers, solvates, or polymorphs thereof as claimed in claim 1, which is selected from the compounds in Table 1.

8. The compound of Formula (I) or salts, enantiomers, stereoisomers, solvates, or polymorphs thereof as claimed in any one of claims 1-7, which is hydrohalides (including hydrochlorides, hydrobromides), sulfates, citrates, maleates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, trifluoroacetate, glycolate, or 4-methylbenzenesulfonate of the compound of Formula (I) or (I').

9. A compound of Formula (I') 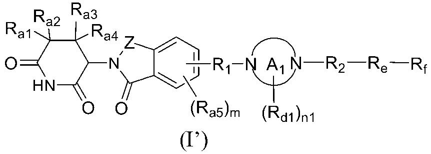 or salts, enantiomers, stereoisomers, isotopically enriched analogs, solvates, or polymorphs thereof,
wherein Z represents C(O), CH₂, or CD₂;
Rₐ₁, Rₐ₂, Rₐ₃, and Rₐ₄ each independently represent hydrogen, deuterium, halogen, C₁₋₆ alkyl, halogenated C₁₋₆ alkyl, deuterated C₁₋₆ alkyl, C₁₋₆ alkoxy, deuterated C₁₋₆ alkoxy, or halogenated C₁₋₆ alkoxy;
(Rₐ₅)ₘ indicates that the isoindoline ring to which it is attached is optionally substituted with m Rₐ₅, wherein each Rₐ₅ is identical or different and independently represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, C₂₋₆ alkynyl, or heterocyclyl, wherein said heterocyclyl is optionally substituted with one or more substituents independently selected from the group consisting of halogen, C₁₋₆ alkyl, and tert-butoxycarbonyl;
m represents an integer of 0, 1, 2, or 3;
ring A₁ represents heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and
R₁ represents N(R_{w}), where R_{w} represents hydrogen or C₁₋₃ alkyl optionally substituted with one or more substituents independently selected from the group consisting of optionally substituted aryl and optionally substituted heteroaryl, and R₂ represents a bond; or
R₁ represents a bond, and R₂ represents N(R_{w}), where R_{w} represents hydrogen or C₁₋₃ alkyl; and
Rₑ represents a bond or C(O)O, or Rₑ represents the structure of the following formula: 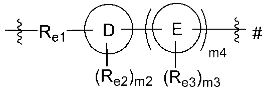
Rₑ₁ represents C(O), CH₂ or halogenated CH₂;
ring D represents arylene, and (Rₑ₂)ₘ₂ indicates that the ring D is optionally substituted with m2 Rₑ₂ groups, wherein each Rₑ₂ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m2 represents an integer of 0-20; and
ring E represents nitrogen-containing heterocyclylene, m4 represents an integer of 0 or 1, and (Rₑ₃)ₘ₃ indicates that the ring E is optionally substituted with m3 Rₑ₃ groups, wherein each Rₑ₃ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m3 represents an integer of 0-20;
symbol # indicates the point of attachment to R_{f} ;
R_{f} represents C₁₋₁₀ alkyl, wherein said C₁₋₁₀ alkyl is optionally substituted with one or more substituents independently selected from the group consisting of: halogen, optionally substituted aryl, optionally substituted heteroaryl, optionally substituted heterocyclyl, optionally substituted cycloalkyl, and NR₃R₄, where R₃ and R₄ each independently represent H, C₁₋₃ alkyl, or optionally substituted cycloalkyl;
with the proviso that the following compounds are excluded:
3-(5-((4-benzhydrylpiperazin-1-yl)amino)-1-oxoisoindolin-2-yl)piperidine-2,6-dione;
3-(5-((4-((4'-chloro-[1,1'-biphenyl]-2-yl)methyl)piperazin-1-yl)amino)-1-oxoisoindolin-2-yl)piperidine-2,6-dione; and
3-(5-((4-((4'-chloro-5,5-dimethyl-3,4,5,6-tetrahydro-[1,1'-biphenyl]-2-yl)methyl)piperazin-1-yl)amino)-1-oxoisoindolin-2-yl)piperidine-2,6-dione.

10. The compound of Formula (I') or salts, enantiomers, stereoisomers, solvates, or polymorphs thereof as claimed in claim 9, wherein
(i) Rₐ₁, Rₐ₂, Rₐ₃, and Rₐ₄ each independently represent H; and/or
(ii) ring A₁ represents 4- to 20-membered heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and/or
(iii) R₁ represents N(R_{w}), where R_{w} represents hydrogen or C₁₋₃ alkyl optionally substituted with one or more substituents independently selected from the group consisting of optionally substituted C₅₋₃₀ aryl and optionally substituted 5- to 30-membered heteroaryl, and R₂ represents a bond; or
(iv) R₁ represents a bond, and R₂ represents N(R_{w}), where R_{w} represents hydrogen or C₁₋₃ alkyl; and/or
(v) Rₑ represents a bond or C(O)O; or
(vi) ring D represents C₅₋₃₀ arylene, and (Rₑ₂)ₘ₂ indicates that the ring D is optionally substituted with m2 Rₑ₂ groups, wherein each Rₑ₂ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m2 represents an integer of 0-20; and/or
(vii) ring E represents 4- to 30-membered nitrogen-containing heterocyclylene, m4 represents an integer of 0 or 1, and (Rₑ₃)ₘ₃ indicates that the ring E is optionally substituted with m3 Rₑ₃ groups, wherein each Rₑ₃ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m3 represents an integer of 0-20; and/or
(viii) R_{f} represents C₁₋₁₀ alkyl, wherein said C₁₋₁₀ alkyl is optionally substituted with one or more substituents independently selected from the group consisting of: halogen, optionally substituted C₅₋₃₀ aryl, optionally substituted 5- to 30-membered heteroaryl, optionally substituted 4- to 30-membered heterocyclyl, optionally substituted C₃₋₃₀ cycloalkyl, and NR₃R₄, where R₃ and R₄ each independently represent H, C₁₋₃ alkyl, or optionally substituted C₃₋₃₀ cycloalkyl; and/or
(ix) Rₐ₅ represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, C₂₋₆ alkynyl, or 4- to 20-membered heterocyclyl, wherein said 4- to 20-membered heterocyclyl is optionally substituted with one or more substituents independently selected from the group consisting of halogen, C₁₋₆ alkyl, and tert-butoxycarbonyl;
wherein said C₅₋₃₀ aryl and 5- to 30-membered heteroaryl are each independently optionally substituted with one or more substituents selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl; and said 4- to 30-membered heterocyclyl and C₃₋₃₀ cycloalkyl are each independently optionally substituted with one or more substituents selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and optionally substituted C₅₋₁₅ aryl, wherein said C₅₋₁₅ aryl is optionally substituted with one or more substituents selected from the group consisting of halogen, C₁₋₆ alkyl, and C₁₋₆ alkoxy.

11. The compound of Formula (I') or salts, enantiomers, stereoisomers, solvates, or polymorphs thereof as claimed in claim 9 or 10, wherein
(i) ring A₁ represents 4- to 15-membered heterocyclylene containing at least two nitrogen atoms, and (R_{d1})ₙ₁ indicates that ring A₁ is optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20; and/or
(ii) R₁ represents N(R_{w}), where R_{w} represents hydrogen or C₁₋₃ alkyl optionally substituted with one or more substituents selected from the group consisting of optionally substituted C₅₋₂₀ aryl (including optionally substituted C₅₋₁₅ aryl) and optionally substituted 5- to 20-membered heteroaryl (including optionally substituted 5- to 15-membered heteroaryl), and R₂ represents a bond; or
(iii) ring D represents C₅₋₂₀ arylene (including C₅₋₁₅ arylene), and (Rₑ₂)ₘ₂ indicates that the ring D is optionally substituted with m2 Rₑ₂ groups, wherein each Rₑ₂ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m2 represents an integer of 0-20; and/or
(iv) ring E represents 4- to 20-membered nitrogen-containing heterocyclylene (including 4- to 15-membered nitrogen-containing heterocyclylene), m4 represents an integer of 0 or 1, and (Rₑ₃)ₘ₃ indicates that the ring E is optionally substituted with m3 Rₑ₃ groups, wherein each Rₑ₃ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m3 represents an integer of 0-20; and/or
(v) R_{f} represents C₁₋₁₀ alkyl, wherein said C₁₋₁₀ alkyl is optionally substituted with one or more substituents independently selected from the group consisting of: halogen, optionally substituted C₅₋₂₀ aryl (including optionally substituted C₅₋₁₅ aryl), optionally substituted 5- to 20-membered heteroaryl (including optionally substituted 5- to 15-membered heteroaryl), optionally substituted 4- to 20-membered heterocyclyl (including optionally substituted 4- to 15-membered heterocyclyl), optionally substituted C₃₋₂₀ cycloalkyl (including optionally substituted C₃₋₁₅ cycloalkyl), and NR₃R₄, where R₃ and R₄ each independently represent H, C₁₋₃ alkyl, or optionally substituted C₃₋₂₀ cycloalkyl (including optionally substituted C₃₋₁₅ cycloalkyl); and/or
(vi) Rₐ₅ represents deuterium, halogen, hydroxyl, mercapto, nitro, amino, cyano, C₁₋₆ alkyl, halogenated C₁₋₆ alkyl, deuterated C₁₋₆ alkyl, C₁₋₆ alkoxy, deuterated C₁₋₆ alkoxy, halogenated C₁₋₆ alkoxy, C₂₋₆ alkenyl, C₂₋₆ alkynyl, or 4- to 15-membered heterocyclyl, wherein said 4- to 15-membered heterocyclyl is optionally substituted with one or more substituents independently selected from the group consisting of halogen, C₁₋₆ alkyl, and tert-butoxycarbonyl;
wherein said C₅₋₂₀ aryl and 5- to 20-membered heteroaryl are each independently optionally substituted with one or more substituents selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl; and said 4- to 20-membered heterocyclyl and C₃₋₂₀ cycloalkyl are each independently optionally substituted with one or more substituents selected from the group consisting of: halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and C₅₋₁₅ aryl optionally substituted with one or more substituents selected from the group consisting of halogen, C₁₋₆ alkyl, and C₁₋₆ alkoxy.

12. The compound of Formula (I') or salts, enantiomers, stereoisomers, solvates, or polymorphs thereof as claimed in any one of claims 9-11, wherein
(i) ring A₁ represents 1,3-diazetidinylidene, imidazolidylene, pyrazolidylene, piperazinylene, diazacycloheptanylene, diazacyclooctylene, diazabicyclo[3.1.1]heptanylene, diazabicyclo[2.2.1]heptanylene, diazabicyclo[3.2.1]octanylene, diazabicyclo[2.2.2]octanylene, 2,6-diazaspiro[3.3]heptanylene, 2,7-diazaspiro[3.5]nonanylene, 2,8-diazaspiro[4.5]decanylene, 3,9-diazaspiro[5.5]undecanylene, or octahydropyrrolo[3,4-c]pyrrolylene, each optionally substituted with n1 R_{d1} groups, wherein each R_{d1} independently represents deuterium, halogen, hydroxyl, mercapto, nitro, amino, cyano, oxo, C₁₋₆ alkyl, deuterated C₁₋₆ alkyl, halogenated C₁₋₆ alkyl, halogenated C₁₋₆ alkoxy, or C₁₋₆ alkoxy, and n1 represents an integer of 0-20 (e.g., an integer of 0-10); and/or
(ii) R_{w} represents hydrogen or C₁₋₃ alkyl, wherein the C₁₋₃ alkyl is optionally substituted with one or more substituents selected from the group consisting of:
phenyl or naphthyl, each optionally substituted with one or more (e.g., 1-7) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl; and
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each optionally substituted with one or more (e.g., 1-10) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl; and/or
(iii) ring D represents phenylene or naphthylene, each optionally substituted with m2 Rₑ₂ groups, wherein each Rₑ₂ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m2 represents an integer of 0-20 (e.g., an integer of 0-10); and/or
(iv) ring E represents azetidinylene, pyrrolidinylene, imidazolidylene, pyrazolidylene, piperidinylene, piperazinylene, tetrahydropyridinylene, dihydroxypiperidinylene, difluoropiperidinylene, azepanylene, azacyclooctylene, diazacycloheptanylene (e.g., 1,4-diazacycloheptanylene, 4,5-diazacycloheptanylene, 1,3-diazacycloheptanylene), diazacyclooctylene, nitrogen-containing bridged heterocyclylene (e.g., 6- to 20-membered nitrogen-containing bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene, each optionally substituted with m3 Rₑ₃ groups, wherein each Rₑ₃ independently represents deuterium, C₁₋₆ alkyl, deuterated C₁₋₆ alkyl, halogenated C₁₋₆ alkyl, C₁₋₆ alkoxy, halogenated C₁₋₆ alkoxy, halogen, amino, hydroxyl, mercapto, cyano, oxo, C₂₋₆ alkynyl, or C₂₋₆ alkenyl, and m3 represents an integer of 0-20 (e.g., an integer of 0-10), and m4 represents an integer of 0 or 1; and/or
(v) R_{f} represents C₁₋₁₀ alkyl, wherein said C₁₋₁₀ alkyl is optionally substituted with one or more substituents independently selected from the group consisting of:
halogen;
phenyl or naphthyl, each optionally substituted with one or more (e.g., 1-7) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl;
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, indolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl, quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each optionally substituted with one or more (e.g., 1-10) substituents independently selected from the group consisting of halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, and C₂₋₆ alkenyl;
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each optionally substituted with one or more (e.g., 1-10) substituents independently selected from the group consisting of: halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and C₅₋₁₅ aryl optionally substituted with one or more (e.g., 1-5) substituents selected from the group consisting of halogen, C₁₋₆ alkyl, and C₁₋₆ alkoxy;
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m-*menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl), each optionally substituted with one or more (e.g., 1-10) substituents independently selected from the group consisting of: halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and C₅₋₁₅ aryl optionally substituted with one or more (e.g., 1-5) substituents selected from the group consisting of halogen, C₁₋₆ alkyl, and C₁₋₆ alkoxy; and
NR₃R₄, where R₃ and R₄ each independently represent:
H;
C₁₋₃ alkyl; or
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., C₅-C₂₀ spiro-cycloalkyl, such as spiro[3.3]heptyl, spiro[2.5]octyl, spiro[3.5]nonyl, spiro[3.5]nonenyl, spiro[4.4]nonyl, spiro[4.5]decyl, spiro[4.5]decenyl, and spiro[5.5]undecyl), *p*-menthanyl, *m*-menthanyl, or bridged cycloalkyl (e.g., C₆-C₂₀ bridged cycloalkyl, such as adamantanyl, noradamantanyl, bornyl, norbornyl, bicyclo[2.2.1]heptanyl, 2-oxobicyclo[2.2.1]heptyl, or bicyclo[2.2.1]heptenyl), each optionally substituted with one or more (e.g., 1-10) substituents independently selected from the group consisting of: halogen, amino, hydroxyl, mercapto, cyano, oxo, C₁₋₆ alkyl, deuterated C₁₋₆ alkyl, halogenated C₁₋₆ alkyl, C₁₋₆ alkoxy, halogenated C₁₋₆ alkoxy, C₂₋₆ alkynyl, C₂₋₆ alkenyl, tert-butoxycarbonyl, and C₅₋₁₅ aryl optionally substituted with one or more (e.g., 1-5) substituents selected from the group consisting of halogen, C₁₋₆ alkyl, and C₁₋₆ alkoxy.

13. The compound of Formula (I') or salts, enantiomers, stereoisomers, solvates, or polymorphs thereof as claimed in any one of claims 9-12, wherein
(i) R_{w} represents H or 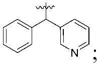
(ii) Rₑ represents a bond, C(O)O, 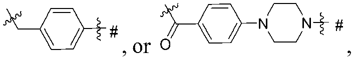 where symbol # indicates the point of attachment to R_{f} ;
(iii) R_{f} represents: methyl, isopropyl, tert-butyl, 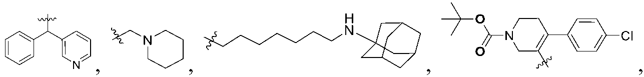 or

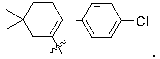

14. The compound of Formula (I') or salts, enantiomers, stereoisomers, solvates, or polymorphs thereof as claimed in claim 9, which is selected from the compounds in Table 2.

15. The compound of Formula (I') or salts, enantiomers, stereoisomers, solvates, or polymorphs thereof as claimed in any one of claims 9-14, which is hydrohalides (including hydrochlorides, hydrobromides), sulfates, citrates, maleates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, trifluoroacetate, glycolate, or 4-methylbenzenesulfonate of the compound of Formula (I) or Formula (I').

16. A pharmaceutical composition, comprising:
the compound of Formula (I) or a pharmaceutically acceptable salt thereof as claimed in any one of claims 1 to 8, and at least one pharmaceutically acceptable carrier or excipient; or
the compound of Formula (I') or a pharmaceutically acceptable salt thereof as claimed in any one of claims 9 to 15, and at least one pharmaceutically acceptable carrier or excipient;
and optionally comprising a second therapeutic agent, e.g., an anticancer agent.

17. The compound of Formula (I) or Formula (I') or a pharmaceutically acceptable salt thereof as claimed in any one of claims 1 to 15, for use in the prevention and/or treatment of a disease or disorder associated with cereblon protein.

18. The compound of Formula (I) or Formula (I') or a pharmaceutically acceptable salt thereof as claimed in claim 17, wherein the disease or disorder associated with cereblon protein is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

19. The compound of Formula (I) or Formula (I') or a pharmaceutically acceptable salt thereof as claimed in claim 17, wherein the disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type I diabetes mellitus, urticaria, inflammatory bowel disease (including Crohn's disease and ulcerative colitis); keratoconjunctivitis sicca; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

20. Use of the compound of Formula (I) or Formula (I') or a pharmaceutically acceptable salt thereof as claimed in any one of claims 1-15, or the pharmaceutical composition as claimed in claim 15, for the manufacture of a medicament for the prevention or treatment of a disease or disorder associated with cereblon protein.

21. A method for treating or preventing a disease or disorder associated with cereblon protein in a subject, comprising administering to the subject a therapeutically effective amount of the compound of Formula (I) or Formula (I') or a pharmaceutically acceptable salt thereof as claimed in any one of claims 1-15, or the pharmaceutical composition as claimed in claim 16.

22. The use as claimed in claim 20, or the method as claimed in claim 21, wherein the disease or disorder associated with cereblon protein is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

23. The use as claimed in claim 20, or the method as claimed in claim 21, wherein the disease or disorder associated with cereblon protein is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, B-cell acute lymphoblastic leukemia, T-cell lymphocytic leukemia, T-cell acute lymphoblastic leukemia, lymphoma cell leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, psoriatic arthritis, systemic lupus erythematosus, multiple sclerosis, recurrent oral ulcers, Kawasaki disease, polymyositis/dermatomyositis, Sjogren's syndrome, atopic dermatitis, hidradenitis suppurativa, gout, type I diabetes mellitus, urticaria, inflammatory bowel disease (including Crohn's disease and ulcerative colitis); keratoconjunctivitis sicca; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

24. A method for preparing the compound of Formula (I) as claimed in claim 1, comprising using a compound of Formula (M1) and a compound of Formula (M2) as starting materials to obtain the compound of Formula (I): 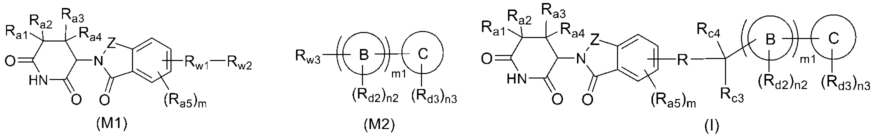
wherein Rₐ₁, Rₐ₂, Rₐ₃, Rₐ₄, (Rₐ₅)ₘ, Z, R, ring B, ring C, (R_{d2})ₙ₂, (R_{d3})ₙ₃, and m1 are as defined in claim 1; and
R_{w3} represents CHO, COOH, or 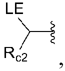
wherein R_{c2} is as defined in claim 1, and the group LE represents Cl, Br, I, OMs, OTs, or ONs;
wherein (1) -R_{w1}-R_{w2} represents 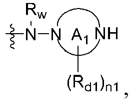 and R represents 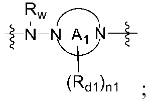 or
(2) -R_{w1}-R_{w2} represents 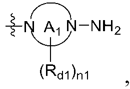 and R represents 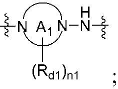 or
(3) -R_{w1}-R_{w2} represents -NH-NH₂, and R represents -NH-NH-; or
(4) -R_{w1}-R_{w2} represents 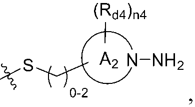 and R represents 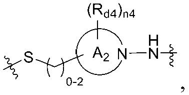
wherein R_{w}, ring A₁, (R_{d1})ₙ₁, ring A₂, and (R_{d4})ₙ₄ are as defined in claim 1;
wherein when R_{w3} represents CHO, R_{c3} and R_{c4} represent H; or
when R_{w3} represents COOH, R_{c3} and R_{c4} together form a carbonyl group; or
when R_{w3} represents 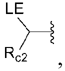 R_{c3} represents H, and R_{c4} represents R_{c2}.

25. The method as claimed in claim 24, wherein
(1) when R_{w3} represents CHO, the compound of Formula (M1) and the compound of Formula (M2) are subjected to a reductive amination reaction to give the compound of Formula (I), wherein R_{c3} and R_{c4} in the compound of Formula (I) represent H;
(2) when R_{w3} represents COOH, the compound of Formula (M1) and the compound of Formula (M2) are subjected to an amide condensation reaction to give the compound of Formula (I), wherein R_{c3} and R_{c4} in the compound of Formula (I) together form a carbonyl group; or
(3) when R_{w3} represents 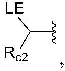 the compound of Formula (M1) and the compound of Formula (M2) are subjected to an aminoalkylation reaction to give the compound of Formula (I), wherein in the compound of Formula (I), R_{c3} represents H, and R_{c4} represents R_{c2}.

26. The method as claimed in claim 24, wherein
when -R_{w1}-R_{w2} in the compound of Formula (M1) represents 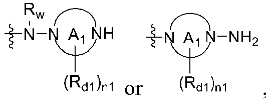 the compound of Formula (M1) is prepared by a reaction using the compound of Formula (M3) and the compound of Formula (M4) as starting materials, 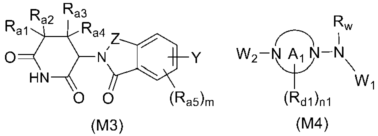
Y represents F or Br;
W₁ represents H, and W₂ represents an amino protecting group; or
W₁ represents an amino protecting group, and W₂ represents H.
